# EUROPEAN PATENT APPLICATION

(11) **EP 2 426 218 A1**
(43) Date of publication of application: **07.03.2012**
(21) Application number: 11006811.1
(22) Date of filing: 29.05.2008
(51) Int. Cl.: C12Q 1/68, C07H 21/02

(54) **Riboswitches and methods and compositions for use of and with riboswitches**

(30) Priority: 29.05.2007 US 932112 P
(62) Divisional of application: 08825955.1
(71) Applicant: Yale University, New Haven, CT 06511 (US)
(72) Inventor: Breaker, Ronald, Guilford, CT 06537 (US); Weinberg, Zasha, New Haven, CT 06511 (US); Sudarsan, Narasimhan, New Haven, CT 06511 (US); Wang, Joy Xin, Arlington, Massachusetts 02476 (US); Meyer, Michelle, Brighton, Massachusetts 02135 (US); Roth, Adam, New Haven, CT 06511 (US); Regulski, Elizabeth, New Haven, CT 06511 (US)
(74) Representative: Elbel, Michaela

(57) **Abstract**

Riboswitches are targets for antibiotics and other small molecule therapies. Riboswitches and portions thereof can be used to regulate the expression or function of RNA molecules and other elements and molecules. Riboswitches and portions thereof can be used in a variety of other methods to, for example, identify or detect compounds. Compounds can be used to stimulate, active, inhibit and/or inactivate the riboswitch. Riboswitches and portions thereof, both alone and in combination with other nucleic acids, can be used in a variety of constructs and RNA molecules and can be encoded by nucleic acids.

SEQUENCE LISTING

<110> Yale Univeristy

<120> Riboswitches and Methods and Compositions for Use of and with
Riboswitches

<130> P30027

<140> EP08825955.1
<141> 2008-05-29

<150> US60/932112
<151> 2007-05-29

<160> 102

<170> BiSSAP 1.0

<210> 1
<211> 39
<212> DNA
<213> Streptococcus pneumoniae

<220>
<221> source
<222> 1..39
<223> /mol_type="DNA"
      /organism="Streptococcus pneumoniae"

<220>
<221> 5'UTR
<222> 1..39

<400> 1
ggaattcaac aagtctaaca gaaaagtaga aaggcgggc                    39


<210> 2
<211> 45
<212> DNA
<213> Streptococcus pneumoniae

<220>
<221> source
<222> 1..45
<223> /mol_type="DNA"
      /organism="Streptococcus pneumoniae"

<220>
<221> 5'UTR
<222> 1..45

<400> 2
ttttgtcatt ttttctcctt taacgtctgg ataactctca aaagc             45


<210> 3
<211> 39
<212> RNA
<213> Unknown

<220>
<221> source
<222> 1..39

<223> /mol_type="RNA"
      /note="Consensus sequences"
      /organism="Unknown"

<220>
<221> unsure
<222> 1..39

<220>
<221> 5'UTR
<222> 1..39

<220>
<221> misc_feature
<222> 2..2
<223> /note="n is a, c, g, or u"

<220>
<221> misc_feature
<222> 9..11
<223> /note="n is a, c, g, or u"

<220>
<221> misc_feature
<222> 14..14
<223> /note="n is a, c, g, or u"

<220>
<221> misc_feature
<222> 18..20
<223> /note="n is a, c, g, or u"

<220>
<221> misc_feature
<222> 27..27
<223> /note="n is a, c, g, or u"

<400> 3
rnyaaacynn nygnrarnnn gggrcgnaaa gcyyrggyc                              39


<210> 4
<211> 26
<212> DNA
<213> Escherichia coli

<220>
<221> source
<222> 1..26
<223> /mol_type="DNA"
      /organism="Escherichia coli"

<220>
<221> unsure
<222> 1..26

<400> 4
gaattccctg gagtcagatt atccgc                                            26


<210> 5
<211> 28
<212> DNA
<213> Escherichia coli

<220>
<221> source
<222> 1..28
<223> /mol_type="DNA"
      /organism="Escherichia coli"

<220>
<221> unsure
<222> 1..28

<400> 5
ctggatccag ttgtgaagcc atgtacac                                          28


<210> 6
<211> 67
<212> DNA
<213> Escherichia coli

<220>
<221> source
<222> 1..67
<223> /mol_type="DNA"
      /organism="Escherichia coli"

<220>
<221> unsure
<222> 1..67

<400> 6
aggaagaaat gacttcgcct cccgtatctg gaaaggtgta catggctatt ccggggatcc      60

gtcgacc                                                                 67


<210> 7
<211> 70
<212> DNA
<213> Escherichia coli

<220>
<221> source
<222> 1..70
<223> /mol_type="DNA"
      /organism="Escherichia coli"

<220>
<221> unsure
<222> 1..70

<400> 7
acctgactca tctgatctct ccttttgacg ttttagccgc caatgtacga tgtaggctgg      60

agctgcttcg                                                              70


<210> 8
<211> 70
<212> DNA
<213> Escherichia coli

<220>
<221> source
<222> 1..70
<223> /mol_type="DNA"

/organism="Escherichia coli"

<220>
<221> unsure
<222> 1..70

<400> 8
tgtatcattc tgtttaacga gactgtttaa acggaaaaat cttgatgaat attccgggga     60

tccgtcgacc                                                            70


<210> 9
<211> 70
<212> DNA
<213> Escherichia coli

<220>
<221> source
<222> 1..70
<223> /mol_type="DNA"
      /organism="Escherichia coli"

<220>
<221> unsure
<222> 1..70

<400> 9
ttgagatccc cccgctcggg gggatttttt tattcgctaa cgtcgcgtct tgtaggctgg     60

agctgcttcg                                                            70


<210> 10
<211> 70
<212> DNA
<213> Escherichia coli

<220>
<221> source
<222> 1..70
<223> /mol_type="DNA"
      /organism="Escherichia coli"

<220>
<221> unsure
<222> 1..70

<400> 10
gacataatag gcaaattcga ttttgcctcc gcaggagtgt tttcatggaa attccgggga     60

tccgtcgacc                                                            70


<210> 11
<211> 70
<212> DNA
<213> Escherichia coli

<220>
<221> source
<222> 1..70
<223> /mol_type="DNA"
      /organism="Escherichia coli"

<220>
<221> unsure
<222> 1..70

<400> 11
cagcatctcc tgatcgctga gttccgccat tacaggcctc cgaacaacgc tgtaggctgg     60

agctgcttcg                                                            70


<210> 12
<211> 70
<212> DNA
<213> Escherichia coli

<220>
<221> source
<222> 1..70
<223> /mol_type="DNA"
      /organism="Escherichia coli"

<220>
<221> unsure
<222> 1..70

<400> 12
gaatggctgg ccagaatcag ccgtgaacgg gcggggcgct aatcatgctg attccgggga     60

tccgtcgacc                                                            70


<210> 13
<211> 69
<212> DNA
<213> Escherichia coli

<220>
<221> source
<222> 1..69
<223> /mol_type="DNA"
      /organism="Escherichia coli"

<220>
<221> unsure
<222> 1..69

<400> 13
ttgcccagtt catttatagc cacctgatta atcaggcggt tatcgacact gtaggctgga     60

gctgcttcg                                                             69


<210> 14
<211> 70
<212> DNA
<213> Escherichia coli

<220>
<221> source
<222> 1..70
<223> /mol_type="DNA"
      /organism="Escherichia coli"

<220>
<221> unsure

<222> 1..70

<400> 14
gacacgttag cagggtcaat cccacaataa aagaggcgat atcggtgaat attccgggga 60

tccgtcgacc 70


<210> 15
<211> 70
<212> DNA
<213> Escherichia coli

<220>
<221> source
<222> 1..70
<223> /mol_type="DNA"
/organism="Escherichia coli"

<220>
<221> unsure
<222> 1..70

<400> 15
actccacgcg gcaaaggcga gtaacggtat catcgttttt cctgccatcg tgtaggctgg 60

agctgcttcg 70


<210> 16
<211> 20
<212> DNA
<213> Escherichia coli

<220>
<221> source
<222> 1..20
<223> /mol_type="DNA"
/organism="Escherichia coli"

<220>
<221> unsure
<222> 1..20

<400> 16
cagtcatagc cgaatagcct 20


<210> 17
<211> 20
<212> DNA
<213> Escherichia coli

<220>
<221> source
<222> 1..20
<223> /mol_type="DNA"
/organism="Escherichia coli"

<220>
<221> unsure
<222> 1..20

<400> 17
cggtgccctg aatgaactgc 20

<210> 18
<211> 21
<212> DNA
<213> Escherichia coli

<220>
<221> source
<222> 1..21
<223> /mol_type="DNA"
      /organism="Escherichia coli"

<220>
<221> unsure
<222> 1..21

<400> 18
cgctagtatc ggcataacca c                                                21


<210> 19
<211> 21
<212> DNA
<213> Escherichia coli

<220>
<221> source
<222> 1..21
<223> /mol_type="DNA"
      /organism="Escherichia coli"

<220>
<221> unsure
<222> 1..21

<400> 19
gaatcgcgca gatagtgacc g                                                21


<210> 20
<211> 25
<212> DNA
<213> Escherichia coli

<220>
<221> source
<222> 1..25
<223> /mol_type="DNA"
      /organism="Escherichia coli"

<220>
<221> unsure
<222> 1..25

<400> 20
gctacctctt ctgaagcctg tctgt                                            25


<210> 21
<211> 25
<212> DNA
<213> Escherichia coli

<220>

<221> source
<222> 1..25
<223> /mol_type="DNA"
      /organism="Escherichia coli"

<220>
<221> unsure
<222> 1..25

<400> 21
atgggtgaag tactgaacga gcagt                                          25


<210> 22
<211> 25
<212> DNA
<213> Escherichia coli

<220>
<221> source
<222> 1..25
<223> /mol_type="DNA"
      /organism="Escherichia coli"

<220>
<221> unsure
<222> 1..25

<400> 22
gatggatatg gcatgtaaag gcagg                                          25


<210> 23
<211> 25
<212> DNA
<213> Escherichia coli

<220>
<221> source
<222> 1..25
<223> /mol_type="DNA"
      /organism="Escherichia coli"

<220>
<221> unsure
<222> 1..25

<400> 23
agcacgcgag aatctttcag cgcct                                          25


<210> 24
<211> 21
<212> DNA
<213> Escherichia coli

<220>
<221> source
<222> 1..21
<223> /mol_type="DNA"
      /organism="Escherichia coli"

<220>
<221> unsure
<222> 1..21

<400> 24
gagcggcgag actgtgcatt a                                                21

<210> 25
<211> 20
<212> DNA
<213> Escherichia coli

<220>
<221> source
<222> 1..20
<223> /mol_type="DNA"
      /organism="Escherichia coli"

<220>
<221> unsure
<222> 1..20

<400> 25
gcaacgccga tgacgcggta                                                  20

<210> 26
<211> 27
<212> DNA
<213> Escherichia coli

<220>
<221> source
<222> 1..27
<223> /mol_type="DNA"
      /organism="Escherichia coli"

<220>
<221> unsure
<222> 1..27

<400> 26
cttcattcag acgtttacat ttcatag                                          27

<210> 27
<211> 26
<212> DNA
<213> Escherichia coli

<220>
<221> source
<222> 1..26
<223> /mol_type="DNA"
      /organism="Escherichia coli"

<220>
<221> unsure
<222> 1..26

<400> 27
catccatatc atggtgcgta tgctta                                           26

<210> 28
<211> 83
<212> DNA

<213> Escherichia coli

<220>
<221> source
<222> 1..83
<223> /mol_type="DNA"
      /organism="Escherichia coli"

<220>
<221> unsure
<222> 1..83

<400> 28
gaattcctca ttaggcaccc caggctttac actttatgct tccggctcgt ataatgtgtg     60

gaaccactaa acactctagc ctc                                             83


<210> 29
<211> 28
<212> DNA
<213> Escherichia coli

<220>
<221> source
<222> 1..28
<223> /mol_type="DNA"
      /organism="Escherichia coli"

<220>
<221> unsure
<222> 1..28

<400> 29
ctggatccag ttgtgaagcc atgtacac                                        28


<210> 30
<211> 37
<212> DNA
<213> Escherichia coli

<220>
<221> source
<222> 1..37
<223> /mol_type="DNA"
      /organism="Escherichia coli"

<220>
<221> unsure
<222> 1..37

<400> 30
ctaggtacct ctggaaaggt gtacatggct tcacaac                              37


<210> 31
<211> 35
<212> DNA
<213> Escherichia coli

<220>
<221> source
<222> 1..35
<223> /mol_type="DNA"

/organism="Escherichia coli"

<220>
<221> unsure
<222> 1..35

<400> 31
tagaagctta actaccagcg ttttgccgcc tgctg                                35


<210> 32
<211> 39
<212> DNA
<213> Streptomyces coelicolor

<220>
<221> source
<222> 1..39
<223> /mol_type="DNA"
      /organism="Streptomyces coelicolor"

<220>
<221> unsure
<222> 1..39

<400> 32
gaagaggtga cgtcatggat ccgaacaaag gtgtaatgc                            39


<210> 33
<211> 34
<212> DNA
<213> Streptomyces coelicolor

<220>
<221> source
<222> 1..34
<223> /mol_type="DNA"
      /organism="Streptomyces coelicolor"

<220>
<221> unsure
<222> 1..34

<400> 33
ctaggatccg ccacgcgtag cggccctggt gtgt                                 34


<210> 34
<211> 33
<212> DNA
<213> Streptomyces coelicolor

<220>
<221> source
<222> 1..33
<223> /mol_type="DNA"
      /organism="Streptomyces coelicolor"

<220>
<221> unsure
<222> 1..33

<400> 34
gtaggatcca cagcggtggg tacggacatg gcg                                  33

<210> 35
<211> 46
<212> DNA
<213> Streptomyces coelicolor

<220>
<221> source
<222> 1..46
<223> /mol_type="DNA"
      /organism="Streptomyces coelicolor"

<220>
<221> unsure
<222> 1..46

<400> 35
taatacgact cactataggt ttttcgacag gtcatgagtg acagtc                    46


<210> 36
<211> 30
<212> DNA
<213> Streptomyces coelicolor

<220>
<221> source
<222> 1..30
<223> /mol_type="DNA"
      /organism="Streptomyces coelicolor"

<220>
<221> unsure
<222> 1..30

<400> 36
aggggtccgc gcttgccgtg gaccttgctg                                      30


<210> 37
<211> 16
<212> RNA
<213> Unknown

<220>
<221> source
<222> 1..16
<223> /mol_type="RNA"
      /note="Consensus sequences"
      /organism="Unknown"

<220>
<221> unsure
<222> 1..16

<220>
<221> 5'UTR
<222> 1..16

<400> 37
grycyrggyu gccrrr                                                     16


<210> 38

<211> 20
<212> RNA
<213> Unknown

<220>
<221> source
<222> 1..20
<223> /mol_type="RNA"
      /note="Consensus sequences"
      /organism="Unknown"

<220>
<221> stem_loop
<222> 1..20

<220>
<221> misc_feature
<222> 1..5
<223> /note="n is a, c, g, or u"

<220>
<221> misc_feature
<222> 16..20
<223> /note="n is a, c, g, or u"

<400> 38
nnnnnrggac gaccynnnnn                                                   20


<210> 39
<211> 14
<212> RNA
<213> Unknown

<220>
<221> source
<222> 1..14
<223> /mol_type="RNA"
      /note="Consensus sequences"
      /organism="Unknown"

<220>
<221> stem_loop
<222> 1..14

<220>
<221> misc_feature
<222> 1..2
<223> /note="n is a, c, g, or u"

<220>
<221> misc_feature
<222> 13..14
<223> /note="n is a, c, g, or u"

<400> 39
nngggacgrc ccnn                                                         14


<210> 40
<211> 32
<212> RNA
<213> Unknown

<220>

<221> source
<222> 1..32
<223> /mol_type="RNA"
      /note="Consensus sequences"
      /organism="Unknown"

<220>
<221> 5'UTR
<222> 1..32

<220>
<221> misc_feature
<222> 6..7
<223> /note="n is a, c, g, or u"

<220>
<221> misc_feature
<222> 10..11
<223> /note="n is a, c, g, or u"

<220>
<221> misc_feature
<222> 26..26
<223> /note="n is a, c, g, or u"

<220>
<221> misc_feature
<222> 28..29
<223> /note="n is a, c, g, or u"

<220>
<221> misc_feature
<222> 31..32
<223> /note="n is a, c, g, or u"

<400> 40
yygccnncyn nycgaggrgc gcugcnannr nn                                    32


<210> 41
<211> 23
<212> RNA
<213> Unknown

<220>
<221> source
<222> 1..23
<223> /mol_type="RNA"
      /note="Consensus sequences"
      /organism="Unknown"

<220>
<221> 5'UTR
<222> 1..23

<220>
<221> misc_feature
<222> 1..2
<223> /note="n is a, c, g, or u"

<220>
<221> misc_feature
<222> 4..6
<223> /note="n is a, c, g, or u"

<220>
<221> misc_feature
<222> 16..17
<223> /note="n is a, c, g, or u"

<220>
<221> misc_feature
<222> 20..20
<223> /note="n is a, c, g, or u"

<220>
<221> misc_feature
<222> 22..23
<223> /note="n is a, c, g, or u"

<400> 41
nnynnncagg cucgrnnggn rnn                                              23


<210> 42
<211> 15
<212> RNA
<213> Unknown

<220>
<221> source
<222> 1..15
<223> /mol_type="RNA"
      /note="Consensus sequences"
      /organism="Unknown"

<220>
<221> 5'UTR
<222> 1..15

<220>
<221> misc_feature
<222> 1..2
<223> /note="n is a, c, g, or u"

<220>
<221> misc_feature
<222> 4..4
<223> /note="n is a, c, g, or u"

<400> 42
nnyncaacgg cgcyc                                                       15


<210> 43
<211> 53
<212> RNA
<213> Unknown

<220>
<221> source
<222> 1..53
<223> /mol_type="RNA"
      /note="Consensus sequences"
      /organism="Unknown"

<220>
<221> stem_loop
<222> 1..53

<220>
<221> misc_feature
<222> 4..4
<223> /note="n is a, c, g, or u"

<220>
<221> misc_feature
<222> 10..11
<223> /note="n is a, c, g, or u"

<220>
<221> misc_feature
<222> 16..18
<223> /note="n is a, c, g, or u"

<220>
<221> misc_feature
<222> 22..22
<223> /note="n is a, c, g, or u"

<220>
<221> misc_feature
<222> 24..24
<223> /note="n is a, c, g, or u"

<220>
<221> misc_feature
<222> 36..36
<223> /note="n is a, c, g, or u"

<220>
<221> misc_feature
<222> 41..44
<223> /note="n is a, c, g, or u"

<220>
<221> misc_feature
<222> 50..50
<223> /note="n is a, c, g, or u"

<400> 43
guangruyyn nugugnnngu gnynuuuuar ruugcnrccu nnnnrrryyn ucu          53


<210> 44
<211> 18
<212> RNA
<213> Unknown

<220>
<221> source
<222> 1..18
<223> /mol_type="RNA"
      /note="Consensus sequences"
      /organism="Unknown"

<220>
<221> stem_loop
<222> 1..18

<220>
<221> misc_feature
<222> 1..1
<223> /note="n is a, c, g, or u"

<220>
<221> misc_feature
<222> 11..11
<223> /note="n is a, c, g, or u"

<220>
<221> misc_feature
<222> 18..18
<223> /note="n is a, c, g, or u"

<400> 44
nuggcuuuuu nrrgyyan                                                    18


<210> 45
<211> 18
<212> RNA
<213> Unknown

<220>
<221> source
<222> 1..18
<223> /mol_type="RNA"
      /note="Consensus sequences"
      /organism="Unknown"

<220>
<221> 5'UTR
<222> 1..18

<220>
<221> misc_feature
<222> 16..16
<223> /note="n is a, c, g, or u"

<400> 45
cggcyayuug ggyuynyr                                                    18


<210> 46
<211> 43
<212> RNA
<213> Unknown

<220>
<221> source
<222> 1..43
<223> /mol_type="RNA"
      /note="Consensus sequences"
      /organism="Unknown"

<220>
<221> stem_loop
<222> 1..43

<220>
<221> misc_feature
<222> 11..11
<223> /note="n is a, c, g, or u"

<220>
<221> misc_feature
<222> 36..36
<223> /note="n is a, c, g, or u"

<400> 46
gcaugggryc nrrgyrrgcg cyraagcgcy racyyngryc gac                43

<210> 47
<211> 40
<212> RNA
<213> Unknown

<220>
<221> source
<222> 1..40
<223> /mol_type="RNA"
      /note="Consensus sequences"
      /organism="Unknown"

<220>
<221> 5'UTR
<222> 1..40

<220>
<221> misc_feature
<222> 5..5
<223> /note="n is a, c, g, or u"

<220>
<221> misc_feature
<222> 15..15
<223> /note="n is a, c, g, or u"

<220>
<221> misc_feature
<222> 21..21
<223> /note="n is a, c, g, or u"

<400> 47
yygcnauccg cyaancgguy nrgccguguc gcggaagguu                40

<210> 48
<211> 18
<212> RNA
<213> Unknown

<220>
<221> source
<222> 1..18
<223> /mol_type="RNA"
      /note="Consensus sequences"
      /organism="Unknown"

<220>
<221> 5'UTR
<222> 1..18

<220>
<221> misc_feature
<222> 5..5
<223> /note="n is a, c, g, or u"

<220>
<221> misc_feature
<222> 10..12
<223> /note="n is a, c, g, or u"

<220>
<221> misc_feature
<222> 16..18
<223> /note="n is a, c, g, or u"

<400> 48
aaccnryurn nnyuynnn                                                    18


<210> 49
<211> 18
<212> RNA
<213> Unknown

<220>
<221> source
<222> 1..18
<223> /mol_type="RNA"
      /note="Consensus sequences"
      /organism="Unknown"

<220>
<221> 5'UTR
<222> 1..18

<220>
<221> misc_feature
<222> 1..3
<223> /note="n is a, c, g, or u"

<220>
<221> misc_feature
<222> 7..7
<223> /note="n is a, c, g, or u"

<220>
<221> misc_feature
<222> 9..10
<223> /note="n is a, c, g, or u"

<220>
<221> misc_feature
<222> 14..14
<223> /note="n is a, c, g, or u"

<400> 49
nnnrrrnrnn ggunagcg                                                    18


<210> 50
<211> 23
<212> RNA
<213> Unknown

<220>
<221> source
<222> 1..23
<223> /mol_type="RNA"
      /note="Consensus sequences"
      /organism="Unknown"

<220>
<221> 5'UTR
<222> 1..23

<220>
<221> misc_feature
<222> 12..12
<223> /note="n is a, c, g, or u"

<220>
<221> misc_feature
<222> 18..18
<223> /note="n is a, c, g, or u"

<400> 50
urugyuauac unrruaangu ugy                                              23


<210> 51
<211> 19
<212> RNA
<213> Unknown

<220>
<221> source
<222> 1..19
<223> /mol_type="RNA"
      /note="Consensus sequences"
      /organism="Unknown"

<220>
<221> 5'UTR
<222> 1..19

<220>
<221> misc_feature
<222> 1..1
<223> /note="n is a, c, g, or u"

<220>
<221> misc_feature
<222> 15..16
<223> /note="n is a, c, g, or u"

<400> 51
ncgyyaaagg gaaannaug                                                   19


<210> 52
<211> 47
<212> RNA
<213> Unknown

<220>
<221> source
<222> 1..47
<223> /mol_type="RNA"
      /note="Consensus sequences"
      /organism="Unknown"

<220>
<221> 5'UTR
<222> 1..47

<220>
<221> misc_feature
<222> 47..47
<223> /note="n is a, c, g, or u"

<400> 52
rcaacccuug rugcuuaruu cccuuucayc aagayauuau acrrcgn                47


<210> 53
<211> 72
<212> RNA
<213> Desulfotomaculum reducens

<220>
<221> source
<222> 1..72
<223> /mol_type="RNA"
      /organism="Desulfotomaculum reducens"

<220>
<221> stem_loop
<222> 1..72

<400> 53
ggcaaacuuu ccgaaaggaa aggacgcaaa gccacgggcc uaaggacauu gucuaugguc      60

cgggccgccu ua                                                          72


<210> 54
<211> 103
<212> RNA
<213> Geobacter sulfurreducens

<220>
<221> source
<222> 1..103
<223> /mol_type="RNA"
      /organism="Geobacter sulfurreducens"

<220>
<221> stem_loop
<222> 1..103

<400> 54
acuaaaccau ccgcgaggau ggggcggaaa gcccacaggg ucucacgaag acccggguug      60

ccgaacuauc acaccacgau agggcggcgg cccggcuuuu uuu                       103


<210> 55
<211> 47
<212> RNA
<213> Unknown

<220>
<221> source
<222> 1..47
<223> /mol_type="RNA"
      /note="Consensus sequences"
      /organism="Unknown"

<220>
<221> stem_loop
<222> 1..47

<220>
<221> misc_feature
<222> 2..3

<223> /note="n is a, c, g, or u"

<220>
<221> misc_feature
<222> 12..14
<223> /note="n is a, c, g, or u"

<220>
<221> misc_feature
<222> 21..23
<223> /note="n is a, c, g, or u"

<220>
<221> misc_feature
<222> 39..39
<223> /note="n is a, c, g, or u"

<220>
<221> misc_feature
<222> 46..47
<223> /note="n is a, c, g, or u"

<400> 55
annggcaaay ynnnygaaar nnnrrgrcgc aaagcyryng gycuann 47


<210> 56
<211> 19
<212> RNA
<213> Unknown

<220>
<221> source
<222> 1..19
<223> /mol_type="RNA"
/note="Consensus sequences"
/organism="Unknown"

<220>
<221> stem_loop
<222> 1..19

<220>
<221> misc_feature
<222> 3..3
<223> /note="n is a, c, g, or u"

<220>
<221> misc_feature
<222> 8..9
<223> /note="n is a, c, g, or u"

<220>
<221> misc_feature
<222> 18..19
<223> /note="n is a, c, g, or u"

<400> 56
yangrycnnr gyugccrnn 19


<210> 57
<211> 42
<212> RNA
<213> Unknown

<220>
<221> source
<222> 1..42
<223> /mol_type="RNA"
      /note="Concenses sequence"
      /organism="Unknown"

<220>
<221> stem_loop
<222> 1..42

<220>
<221> misc_feature
<222> 3..3
<223> /note="n is a, c, g, or u"

<220>
<221> misc_feature
<222> 5..5
<223> /note="n is a, c, g, or u"

<220>
<221> misc_feature
<222> 12..15
<223> /note="n is a, c, g, or u"

<220>
<221> misc_feature
<222> 20..23
<223> /note="n is a, c, g, or u"

<220>
<221> misc_feature
<222> 30..30
<223> /note="n is a, c, g, or u"

<220>
<221> misc_feature
<222> 37..37
<223> /note="n is a, c, g, or u"

<400> 57
aanrnyaaac cnnnngyran nnngggrcgn aaagccnygg gu                42


<210> 58
<211> 16
<212> RNA
<213> Unknown

<220>
<221> source
<222> 1..16
<223> /mol_type="RNA"
      /note="Consensus sequence"
      /organism="Unknown"

<220>
<221> stem_loop
<222> 1..16

<400> 58
gayccgggyu gccgaa                                             16

<210> 59
<211> 107
<212> RNA
<213> Vibrio cholerae

<220>
<221> source
<222> 1..107
<223> /mol_type="RNA"
      /organism="Vibrio cholerae"

<220>
<221> 5'UTR
<222> 1..107

<400> 59
   ggaaaaaugu cacgcacagg gcaaaccauu cgaaagagug ggacgcaaag ccccggccua      60
   aaccagaaga caugguaggu cgggguuacc gauggcaaaa ugcauac                   107


<210> 60
<211> 25
<212> RNA
<213> Unknown

<220>
<221> source
<222> 1..25
<223> /mol_type="RNA"
      /note="Consensus sequence"
      /organism="Unknown"

<220>
<221> stem_loop
<222> 1..25

<220>
<221> misc_feature
<222> 1..4
<223> /note="n is a, c, g, or u"

<220>
<221> misc_feature
<222> 23..25
<223> /note="n is a, c, g, or u"

<400> 60
nnnnycgagg rgcgyugcrr crnnn                                            25


<210> 61
<211> 19
<212> RNA
<213> Unknown

<220>
<221> source
<222> 1..19
<223> /mol_type="RNA"
      /note="Consensus sequence"
      /organism="Unknown"

<220>

<221> stem_loop
<222> 1..19

<220>
<221> misc_feature
<222> 1..3
<223> /note="n is a, c, g, or u"

<220>
<221> misc_feature
<222> 16..19
<223> /note="n is a, c, g, or u"

<400> 61
nnnygycagg cucgrnnnn                                                    19


<210> 62
<211> 21
<212> RNA
<213> Unknown

<220>
<221> source
<222> 1..21
<223> /mol_type="RNA"
      /note="Consensus sequence"
      /organism="Unknown"

<220>
<221> stem_loop
<222> 1..21

<220>
<221> misc_feature
<222> 1..7
<223> /note="n is a, c, g, or u"

<400> 62
nnnnnnncaa cggcgcycry y                                                 21


<210> 63
<211> 50
<212> RNA
<213> Dechloromonas aromatica

<220>
<221> source
<222> 1..50
<223> /mol_type="RNA"
      /organism="Dechloromonas aromatica"

<220>
<221> 5'UTR
<222> 1..50

<400> 63
ggucugccga ggagcgcugc gacccuuuaa uucgggggcc aggcucggca                  50


<210> 64
<211> 18
<212> RNA
<213> Dechloromonas aromatica

<220>
<221> source
<222> 1..18
<223> /mol_type="RNA"
      /organism="Dechloromonas aromatica"

<220>
<221> 5'UTR
<222> 1..18

<400> 64
augaucaacg gcgcucgc                                                    18


<210> 65
<211> 61
<212> RNA
<213> Pseudoalteromonas syringae

<220>
<221> source
<222> 1..61
<223> /mol_type="RNA"
      /organism="Pseudoalteromonas syringae"

<220>
<221> 5'UTR
<222> 1..61

<400> 65
ccguucccgc caccuguccg aggggcgcug cagcagguuu acccugucag gcucggaugg      60

g                                                                      61


<210> 66
<211> 86
<212> RNA
<213> Pseudoalteromonas syringae

<220>
<221> source
<222> 1..86
<223> /mol_type="RNA"
      /organism="Pseudoalteromonas syringae"

<220>
<221> 5'UTR
<222> 1..86

<400> 66
gcguuguuug ucgguugauc acagccggca agccuuaaac gcacaacggc gcccauucca      60

uuuuuaugga auggagacuc ucaaug                                           86


<210> 67
<211> 22
<212> RNA
<213> Unknown

<220>
<221> source
<222> 1..22

<223> /mol_type="RNA"
      /note="Consensus sequence"
      /organism="Unknown"

<220>
<221> 5'UTR
<222> 1..22

<220>
<221> misc_feature
<222> 2..3
<223> /note="n is a, c, g, or u"

<220>
<221> misc_feature
<222> 5..6
<223> /note="n is a, c, g, or u"

<220>
<221> misc_feature
<222> 8..8
<223> /note="n is a, c, g, or u"

<220>
<221> misc_feature
<222> 10..10
<223> /note="n is a, c, g, or u"

<220>
<221> misc_feature
<222> 14..14
<223> /note="n is a, c, g, or u"

<220>
<221> misc_feature
<222> 17..17
<223> /note="n is a, c, g, or u"

<220>
<221> misc_feature
<222> 19..19
<223> /note="n is a, c, g, or u"

<220>
<221> misc_feature
<222> 22..22
<223> /note="n is a, c, g, or u"

<400> 67
unngnnanan urrnaangnu gn                                              22


<210> 68
<211> 97
<212> RNA
<213> Streptococcus mutans

<220>
<221> source
<222> 1..97
<223> /mol_type="RNA"
      /organism="Streptococcus mutans"

<220>
<221> unsure

<222> 1..97

<220>
<221> misc_feature
<222> 23..25
<223> /note="n is a, c, g, or u"

<220>
<221> misc_feature
<222> 73..77
<223> /note="n is a, c, g, or u"

<400> 68
uaugcuauac uggauaaguu gunnnacaac ccuuggugcu uauuucccuu ucaccaagca     60

uauuacaaac ggnnnnnccg uuaaaggaga aacaaug                              97


<210> 69
<211> 97
<212> RNA
<213> Streptococcus pneumoniae R6

<220>
<221> source
<222> 1..97
<223> /mol_type="RNA"
      /organism="Streptococcus pneumoniae R6"

<220>
<221> unsure
<222> 1..97

<220>
<221> misc_feature
<222> 23..27
<223> /note="n is a, c, g, or u"

<220>
<221> misc_feature
<222> 74..77
<223> /note="n is a, c, g, or u"

<400> 69
ugugcuauac uaguaagguu gannnnnuca acccuuggug cuuagcuucu uucaccaagc     60

auauuacacg cggnnnnccg ccaaaggaga aaagaug                              97


<210> 70
<211> 97
<212> RNA
<213> Streptococcus pneumoniae SP18-BS74

<220>
<221> source
<222> 1..97
<223> /mol_type="RNA"
      /organism="Streptococcus pneumoniae SP18-BS74"

<220>
<221> unsure
<222> 1..97

<220>

<221> misc_feature
<222> 23..27
<223> /note="n is a, c, g, or u"

<220>
<221> misc_feature
<222> 74..77
<223> /note="n is a, c, g, or u"

<400> 70
ugugcuauac uaguaagguu gannnnnuca acccuuggug cuuagcuucu uucaccaagc     60

auauuacacg cggnnnnccg ccaaaggaga aaagaug                              97


<210> 71
<211> 97
<212> RNA
<213> Streptococcus pneumoniae SP19-BS75

<220>
<221> source
<222> 1..97
<223> /mol_type="RNA"
      /organism="Streptococcus pneumoniae SP19-BS75"

<220>
<221> unsure
<222> 1..97

<220>
<221> misc_feature
<222> 23..27
<223> /note="n is a, c, g, or u"

<220>
<221> misc_feature
<222> 74..77
<223> /note="n is a, c, g, or u"

<400> 71
ugugcuauac uaguaggguu gannnnngca acccuuggug cuuagcuucu uucaccaagc     60

auauuacacg cggnnnnccg ccaaaggaga aaagaug                              97


<210> 72
<211> 97
<212> RNA
<213> Streptococcus pneumoniae SP6-BS73

<220>
<221> source
<222> 1..97
<223> /mol_type="RNA"
      /organism="Streptococcus pneumoniae SP6-BS73"

<220>
<221> unsure
<222> 1..97

<220>
<221> misc_feature
<222> 23..27
<223> /note="n is a, c, g, or u"

<220>
<221> misc_feature
<222> 74..77
<223> /note="n is a, c, g, or u"

<400> 72
ugugcuauac uaguaggguu gannnnngca acccuuggug uuuagcuucu uucaccaagc      60

auauuacacg cggnnnnccg ccaaaggaga aaagaug                               97


<210> 73
<211> 116
<212> RNA
<213> Streptococcus pyogenes

<220>
<221> source
<222> 1..116
<223> /mol_type="RNA"
      /organism="Streptococcus pyogenes"

<220>
<221> unsure
<222> 1..116

<220>
<221> misc_feature
<222> 23..25
<223> /note="n is a, c, g, or u"

<220>
<221> misc_feature
<222> 78..89
<223> /note="n is a, c, g, or u"

<400> 73
uaugguacaa uguaaccguu gcnnngcaac ccuuggugcu uaguucccuu ucaccaagca      60

uauuagaaac guagauannn nnnnnnnnnu auccagacgu uaaaggagaa aaaaug         116


<210> 74
<211> 114
<212> RNA
<213> Streptococcus agalactiae 2630V/R

<220>
<221> source
<222> 1..114
<223> /mol_type="RNA"
      /organism="Streptococcus agalactiae 2630V/R"

<220>
<221> unsure
<222> 1..114

<220>
<221> misc_feature
<222> 23..27
<223> /note="n is a, c, g, or u"

<220>
<221> misc_feature

<222> 79..89
<223> /note="n is a, c, g, or u"

<400> 74
ugugauauac uuaguaaguu gunnnnnaca acccuugaug cuuaguuccc uuucaucaag      60

cauauuacaa acguuagann nnnnnnnnnu cuaaacguua aaggagaaau uaug           114


<210> 75
<211> 114
<212> RNA
<213> Streptococcus agalactiae

<220>
<221> source
<222> 1..114
<223> /mol_type="RNA"
      /organism="Streptococcus agalactiae"

<220>
<221> unsure
<222> 1..114

<220>
<221> misc_feature
<222> 23..27
<223> /note="n is a, c, g, or u"

<220>
<221> misc_feature
<222> 79..89
<223> /note="n is a, c, g, or u"

<400> 75
ugugauauac uuaguaaguu gunnnnnaca acccuugaug cuuaguuccc uuucaucaag      60

cauauuacaa acguuagann nnnnnnnnnu cuaaacguua aaggagaaau uaug           114


<210> 76
<211> 107
<212> RNA
<213> Streptococcus sanguinis

<220>
<221> source
<222> 1..107
<223> /mol_type="RNA"
      /organism="Streptococcus sanguinis"

<220>
<221> unsure
<222> 1..107

<220>
<221> misc_feature
<222> 25..34
<223> /note="n is a, c, g, or u"

<220>
<221> misc_feature
<222> 83..87
<223> /note="n is a, c, g, or u"

<400> 76
ugggggaauc aacuaaugau gcucnnnnnn nnnnaugcaa cccuuggcgc uuagcuucuu     60

ucgccaagca uauuacacgc ggnnnnnccg cuaaaggaga aaacaug                  107


<210> 77
<211> 103
<212> RNA
<213> Streptococcus suis

<220>
<221> source
<222> 1..103
<223> /mol_type="RNA"
      /organism="Streptococcus suis"

<220>
<221> unsure
<222> 1..103

<220>
<221> misc_feature
<222> 26..32
<223> /note="n is a, c, g, or u"

<220>
<221> misc_feature
<222> 79..84
<223> /note="n is a, c, g, or u"

<400> 77
uaugcuauac uaaucaacgu uguugnnnnn nncaaugacc cuuggggcuu gcucacuuua     60

cccaagcaua uuuuaagcnn nnnngcuaaa ggagaauuuu aug                      103


<210> 78
<211> 107
<212> RNA
<213> Lactococcus lactis subsp. lactis

<220>
<221> source
<222> 1..107
<223> /mol_type="RNA"
      /organism="Lactococcus lactis subsp. lactis"

<220>
<221> unsure
<222> 1..107

<220>
<221> misc_feature
<222> 25..31
<223> /note="n is a, c, g, or u"

<220>
<221> misc_feature
<222> 81..86
<223> /note="n is a, c, g, or u"

<400> 78
ucugcuaaaa uaaaaacguu gcucnnnnnn ngaguuaacc cuugagccuu caucccuuug     60

cucaagcaua uuauaggcga nnnnnnucgc caaaggagaa ccuuuug                107

<210> 79
<211> 108
<212> RNA
<213> Lactococcus lactis subsp. cremoris SK11

<220>
<221> source
<222> 1..108
<223> /mol_type="RNA"
      /organism="Lactococcus lactis subsp. cremoris SK11"

<220>
<221> unsure
<222> 1..108

<220>
<221> misc_feature
<222> 25..31
<223> /note="n is a, c, g, or u"

<220>
<221> misc_feature
<222> 82..87
<223> /note="n is a, c, g, or u"

<400> 79
ucuguuaaaa uaaaaacguu gcucnnnnnn ngagucaacc cuugagccuu cuucccuuug      60

cucaagcaua uuaaacgcga cnnnnnnucg ccaaaggaga guauuuug                108

<210> 80
<211> 99
<212> RNA
<213> Lactobacillus casei

<220>
<221> source
<222> 1..99
<223> /mol_type="RNA"
      /organism="Lactobacillus casei"

<220>
<221> unsure
<222> 1..99

<220>
<221> misc_feature
<222> 24..27
<223> /note="n is a, c, g, or u"

<220>
<221> misc_feature
<222> 75..77
<223> /note="n is a, c, g, or u"

<400> 80
acaccaaaac ugaaaacgga aggnnnnccu uccacgacga uacuuacuuu ccuuugaucg      60

ucguuacuac uggcnnngcc acaaaggaga acaaacaug                          99

<210> 81
<211> 24
<212> RNA
<213> Unknown

<220>
<221> source
<222> 1..24
<223> /mol_type="RNA"
      /note="Consensus sequence"
      /organism="Unknown"

<220>
<221> stem_loop
<222> 1..24

<220>
<221> misc_feature
<222> 4..5
<223> /note="n is a, c, g, or u"

<220>
<221> misc_feature
<222> 7..8
<223> /note="n is a, c, g, or u"

<220>
<221> misc_feature
<222> 10..10
<223> /note="n is a, c, g, or u"

<220>
<221> misc_feature
<222> 12..12
<223> /note="n is a, c, g, or u"

<220>
<221> misc_feature
<222> 16..16
<223> /note="n is a, c, g, or u"

<220>
<221> misc_feature
<222> 19..19
<223> /note="n is a, c, g, or u"

<220>
<221> misc_feature
<222> 21..21
<223> /note="n is a, c, g, or u"

<220>
<221> misc_feature
<222> 24..24
<223> /note="n is a, c, g, or u"

<400> 81
ggunngnnan anurrnaang nugn                                              24


<210> 82
<211> 46
<212> RNA
<213> Unknown

<220>
<221> source
<222> 1..46
<223> /mol_type="RNA"
      /note="Consensus sequence"
      /organism="Unknown"

<220>
<221> stem_loop
<222> 1..46

<220>
<221> misc_feature
<222> 1..1
<223> /note="n is a, c, g, or u"

<220>
<221> misc_feature
<222> 12..12
<223> /note="n is a, c, g, or u"

<220>
<221> misc_feature
<222> 18..18
<223> /note="n is a, c, g, or u"

<220>
<221> misc_feature
<222> 26..27
<223> /note="n is a, c, g, or u"

<220>
<221> misc_feature
<222> 42..44
<223> /note="n is a, c, g, or u"

<220>
<221> misc_feature
<222> 46..46
<223> /note="n is a, c, g, or u"

<400> 82
nyracccuug rnrcuurnuy ycuuunnyca agcauauuay annngn                46


<210> 83
<211> 19
<212> RNA
<213> Unknown

<220>
<221> source
<222> 1..19
<223> /mol_type="RNA"
      /note="Consensus sequence"
      /organism="Unknown"

<220>
<221> stem_loop
<222> 1..19

<220>
<221> misc_feature
<222> 1..1
<223> /note="n is a, c, g, or u"

<220>
<221> misc_feature
<222> 3..4
<223> /note="n is a, c, g, or u"

<220>
<221> misc_feature
<222> 14..16
<223> /note="n is a, c, g, or u"

<400> 83
ncnnyaaagg gaannnaug                                                    19


<210> 84
<211> 104
<212> RNA
<213> Streptococcus pneumoniae

<220>
<221> source
<222> 1..104
<223> /mol_type="RNA"
      /organism="Streptococcus pneumoniae"

<220>
<221> unsure
<222> 1..104

<400> 84
ggugugcuau acuaguaagg uugaaugaau caacccuugg ugcuuagcuu cuuucaccaa      60

gcauauuaca cgcggauaac cgccaaagga gaaaagaugu uuuu                      104


<210> 85
<211> 74
<212> RNA
<213> Unknown

<220>
<221> source
<222> 1..74
<223> /mol_type="RNA"
      /note="Consensus sequence"
      /organism="Unknown"

<220>
<221> unsure
<222> 1..74

<400> 85
gguugaauga aucaacccuu ggugcuuagc uucuuucacc aagcauauua cacgcggaua      60

accgccaaag gaga                                                        74


<210> 86
<211> 10
<212> RNA
<213> Unknown

<220>
<221> source

<222> 1..10
<223> /mol_type="RNA"
      /note="Consensus sequences"
      /organism="Unknown"

<220>
<221> stem_loop
<222> 1..10

<400> 86
ucguuugagy                                                            10


<210> 87
<211> 13
<212> RNA
<213> Pseudoalteromonas syringae

<220>
<221> source
<222> 1..13
<223> /mol_type="RNA"
      /organism="Pseudoalteromonas syringae"

<220>
<221> 5'UTR
<222> 1..13

<400> 87
gucccauuug gac                                                        13


<210> 88
<211> 18
<212> RNA
<213> Unknown

<220>
<221> source
<222> 1..18
<223> /mol_type="RNA"
      /note="Consensus sequence"
      /organism="Unknown"

<220>
<221> stem_loop
<222> 1..18

<220>
<221> misc_feature
<222> 1..1
<223> /note="n is a, c, g, or u"

<220>
<221> misc_feature
<222> 10..10
<223> /note="n is a, c, g, or u"

<220>
<221> misc_feature
<222> 18..18
<223> /note="n is a, c, g, or u"

<400> 88
nrgcrgayun aucygcyn                                                   18

<210> 89
<211> 29
<212> RNA
<213> Unknown

<220>
<221> source
<222> 1..29
<223> /mol_type="RNA"
      /note="Consensus sequence"
      /organism="Unknown"

<220>
<221> unsure
<222> 1..27

<220>
<221> unsure
<222> 1..29

<220>
<221> misc_feature
<222> 1..3
<223> /note="n is a, c, g, or u"

<220>
<221> misc_feature
<222> 13..17
<223> /note="n is a, c, g, or u"

<220>
<221> misc_feature
<222> 23..23
<223> /note="n is a, c, g, or u"

<220>
<221> misc_feature
<222> 25..29
<223> /note="n is a, c, g, or u"

<400> 89
nnncuccgag cynnnnnryc uanrnnnnn                                          29


<210> 90
<211> 25
<212> RNA
<213> Unknown

<220>
<221> source
<222> 1..25
<223> /mol_type="RNA"
      /note="Consensus sequence"
      /organism="Unknown"

<220>
<221> unsure
<222> 1..25

<220>
<221> misc_feature
<222> 1..5

<223> /note="n is a, c, g, or u"

<220>
<221> misc_feature
<222> 11..14
<223> /note="n is a, c, g, or u"

<220>
<221> misc_feature
<222> 16..16
<223> /note="n is a, c, g, or u"

<220>
<221> misc_feature
<222> 21..25
<223> /note="n is a, c, g, or u"

<400> 90
nnnnnyaygg nnnnynrgcc nnnnn                                          25


<210> 91
<211> 54
<212> RNA
<213> Unknown

<220>
<221> source
<222> 1..54
<223> /mol_type="RNA"
      /note="Consensus sequence"
      /organism="Unknown"

<220>
<221> unsure
<222> 1..54

<220>
<221> misc_feature
<222> 1..7
<223> /note="n is a, c, g, or u"

<220>
<221> misc_feature
<222> 12..17
<223> /note="n is a, c, g, or u"

<220>
<221> misc_feature
<222> 25..29
<223> /note="n is a, c, g, or u"

<220>
<221> misc_feature
<222> 37..41
<223> /note="n is a, c, g, or u"

<220>
<221> misc_feature
<222> 50..50
<223> /note="n is a, c, g, or u"

<220>
<221> misc_feature
<222> 52..54

<223> /note="n is a, c, g, or u"

<400> 91
nnnnnnnggg unnnnnnrga aayynnnnng ccucccnnnn nuggaaaggn gnnn          54


<210> 92
<211> 140
<212> RNA
<213> Escherichia coli

<220>
<221> source
<222> 1..140
<223> /mol_type="RNA"
      /organism="Escherichia coli"

<220>
<221> unsure
<222> 1..140

<400> 92
ggaaccacua aacaccuagc cucugcaccu gggucaacug auacggugcu uuggccguga      60

caaugcucgu aaagauugcc accagggcga aggaagaaau gaacuucgcc ucccguaucu     120

ggaaaggugu acauggcuuc                                                 140


<210> 93
<211> 26
<212> RNA
<213> Escherichia coli

<220>
<221> source
<222> 1..26
<223> /mol_type="RNA"
      /organism="Escherichia coli"

<220>
<221> unsure
<222> 1..26

<400> 93
gugacaaugc ucguaaagau ugccac                                           26


<210> 94
<211> 47
<212> RNA
<213> Unknown

<220>
<221> source
<222> 1..47
<223> /mol_type="RNA"
      /note="Consensus sequence"
      /organism="Unknown"

<220>
<221> stem_loop
<222> 1..47

<220>

<221> misc_feature
<222> 7..7
<223> /note="n is a, c, g, or u"

<220>
<221> misc_feature
<222> 12..12
<223> /note="n is a, c, g, or u"

<220>
<221> misc_feature
<222> 16..16
<223> /note="n is a, c, g, or u"

<220>
<221> misc_feature
<222> 24..24
<223> /note="n is a, c, g, or u"

<220>
<221> misc_feature
<222> 30..30
<223> /note="n is a, c, g, or u"

<220>
<221> misc_feature
<222> 35..35
<223> /note="n is a, c, g, or u"

<220>
<221> misc_feature
<222> 39..39
<223> /note="n is a, c, g, or u"

<400> 94
cuuaucnaga gnggyngagg gaynggcccn ryganrccnc rgcaacc                47


<210> 95
<211> 15
<212> RNA
<213> Unknown

<220>
<221> source
<222> 1..15
<223> /mol_type="RNA"
      /note="Consensus sequence"
      /organism="Unknown"

<220>
<221> stem_loop
<222> 1..15

<220>
<221> misc_feature
<222> 9..9
<223> /note="n is a, c, g, or u"

<220>
<221> misc_feature
<222> 13..13
<223> /note="n is a, c, g, or u"

<400> 95

ggugcyaanu ccnrc                                                        15


<210> 96
<211> 10
<212> RNA
<213> Unknown

<220>
<221> source
<222> 1..10
<223> /mol_type="RNA"
      /note="Consensus sequence"
      /organism="Unknown"

<220>
<221> stem_loop
<222> 1..10

<400> 96
grragaurag                                                             10


<210> 97
<211> 84
<212> RNA
<213> Unknown

<220>
<221> source
<222> 1..84
<223> /mol_type="RNA"
      /note="Consensus sequence"
      /organism="Unknown"

<220>
<221> stem_loop
<222> 1..84

<220>
<221> misc_feature
<222> 18..18
<223> /note="n is a, c, g, or u"

<220>
<221> misc_feature
<222> 31..31
<223> /note="n is a, c, g, or u"

<220>
<221> misc_feature
<222> 50..51
<223> /note="n is a, c, g, or u"

<220>
<221> misc_feature
<222> 59..59
<223> /note="n is a, c, g, or u"

<220>
<221> misc_feature
<222> 75..75
<223> /note="n is a, c, g, or u"

<220>

<221> misc_feature
<222> 80..80
<223> /note="n is a, c, g, or u"

<400> 97
ggucaygagy rycagcrnya agccccggcu ngcugrycgg caacccuccn nycgcggyng     60

ggugccccgg gugangaccn ggyy                                            84


<210> 98
<211> 14
<212> RNA
<213> Unknown

<220>
<221> source
<222> 1..14
<223> /mol_type="RNA"
      /note="Consensus sequence"
      /organism="Unknown"

<220>
<221> stem_loop
<222> 1..14

<220>
<221> misc_feature
<222> 12..12
<223> /note="n is a, c, g, or u"

<400> 98
ggcaagygcg rnyy                                                       14


<210> 99
<211> 132
<212> RNA
<213> Streptomyces coelicolor

<220>
<221> source
<222> 1..132
<223> /mol_type="RNA"
      /organism="Streptomyces coelicolor"

<220>
<221> unsure
<222> 1..132

<400> 99
gguuuuucga caggucauga gugacaguca ugaggccccg gccgacuguc cggcaacccu     60

ccguccgugg cggggugccc cgggugaaga ccaggucgug gacagcaagg uccacggcaa    120

gcgcggaccc cu                                                        132


<210> 100
<211> 132
<212> RNA
<213> Unknown

<220>
<221> source

<222> 1..132
<223> /mol_type="RNA"
      /note="Consesus sequence"
      /organism="Unknown"

<220>
<221> unsure
<222> 1..132

<400> 100
gguuuuucga caggucauga gugacaguca ugaggccccg gccgacuguc cggcaacccu      60

ccguccgugg cggggugccc cgggugaaga ccaggucgug gacagcaagg uccacggcaa     120

gcgcggaccc cu                                                         132


<210> 101
<211> 49
<212> RNA
<213> Unknown

<220>
<221> source
<222> 1..49
<223> /mol_type="RNA"
      /note="Concensus sequence"
      /organism="Unknown"

<220>
<221> unsure
<222> 1..49

<220>
<221> misc_feature
<222> 1..2
<223> /note="n is a, c, g, or u"

<220>
<221> misc_feature
<222> 7..7
<223> /note="n is a, c, g, or u"

<220>
<221> misc_feature
<222> 14..14
<223> /note="n is a, c, g, or u"

<220>
<221> misc_feature
<222> 20..21
<223> /note="n is a, c, g, or u"

<220>
<221> misc_feature
<222> 29..30
<223> /note="n is a, c, g, or u"

<220>
<221> misc_feature
<222> 45..47
<223> /note="n is a, c, g, or u"

<220>
<221> misc_feature

<222> 49..49
<223> /note="n is a, c, g, or u"

<400> 101
nnyracnccu ugrnrcuurn nuyycuuunn ycaagcauau uayannngn                49


<210> 102
<211> 20
<212> RNA
<213> Unknown

<220>
<221> source
<222> 1..20
<223> /mol_type="RNA"
      /note="Consensus sequence"
      /organism="Unknown"

<220>
<221> unsure
<222> 1..20

<220>
<221> misc_feature
<222> 1..1
<223> /note="n is a, c, g, or u"

<220>
<221> misc_feature
<222> 3..4
<223> /note="n is a, c, g, or u"

<220>
<221> misc_feature
<222> 15..17
<223> /note="n is a, c, g, or u"

<400> 102
ncnnyaaagg agaannnaug                                                20

## Description

### CROSS-REFERENCE TO RELATED APPLICATIONS

This application claims benefit of U.S. Provisional Application No. 60/932,112, filed May 29, 2007. U.S. Provisional Application No. 60/932,112, filed May 29, 2007, is hereby incorporated herein by reference in its entirety.

### STATEMENT REGARDING FEDERALLY SPONSORED RESEARCH

This invention was made with government support under Grant Nos. GM 068819, RR19895-02, and R33 DK07027 awarded by the NIH, under Grant No. HV28186 awarded by NHLBI, under Grant No. T32GM007223 awarded by the National Institute of General Medical Sciences, and under Grant No. EIA-0323510 awarded by NSF. The government has certain rights in the invention.

### FIELD OF THE INVENTION

The disclosed invention is generally in the field of gene expression and specifically in the area of regulation of gene expression.

### BACKGROUND OF THE INVENTION

Precision genetic control is an essential feature of living systems, as cells must respond to a multitude of biochemical signals and environmental cues by varying genetic expression patterns. Most known mechanisms of genetic control involve the use of protein factors that sense chemical or physical stimuli and then modulate gene expression by selectively interacting with the relevant DNA or messenger RNA sequence. Proteins can adopt complex shapes and carry out a variety of functions that permit living systems to sense accurately their chemical and physical environments. Protein factors that respond to metabolites typically act by binding DNA to modulate transcription initiation (e.g. the lac repressor protein; Matthews, K.S., and Nichols, J.C., 1998, Prog. Nucleic Acids Res. Mol. Biol. 58, 127-164) or by binding RNA to control either transcription termination (e.g. the PyrR protein; Switzer, R.L., et al., 1999, Prog. Nucleic Acids Res. Mol. Biol. 62, 329-367) or translation (e.g. the TRAP protein; Babitzke, P., and Gollnick, P., 2001, J. Bacteriol. 183, 5795-5802). Protein factors respond to environmental stimuli by various mechanisms such as allosteric modulation or post-translational modification, and are adept at exploiting these mechanisms to serve as highly responsive genetic switches (*e.g*. see Ptashne, M., and Gann, A. (2002). Genes and Signals. Cold Spring Harbor Laboratory Press, Cold Spring Harbor, NY).

In addition to the widespread participation of protein factors in genetic control, it is also known that RNA can take an active role in genetic regulation. Recent studies have begun to reveal the substantial role that small non-coding RNAs play in selectively targeting mRNAs for destruction, which results in down-regulation of gene expression (*e.g.* see Hannon, G.J. 2002, Nature 418, 244-251 and references therein). This process of RNA interference takes advantage of the ability of short RNAs to recognize the intended mRNA target selectively via Watson-Crick base complementation, after which the bound mRNAs are destroyed by the action of proteins. RNAs are ideal agents for molecular recognition in this system because it is far easier to generate new target-specific RNA factors through evolutionary processes than it would be to generate protein factors with novel but highly specific RNA binding sites.

Although proteins fulfill most requirements that biology has for enzyme, receptor and structural functions, RNA also can serve in these capacities. For example, RNA has sufficient structural plasticity to form numerous ribozyme domains (Cech & Golden, Building a catalytic active site using only RNA. In: The RNA World R. F. Gesteland, T. R. Cech, J. F. Atkins, eds., pp.321-350 (1998); Breaker, In vitro selection of catalytic polynucleotides. Chem. Rev. 97, 371-390 (1997)) and receptor domains (Osborne & Ellington, Nucleic acid selection and the challenge of combinatorial chemistry. Chem. Rev. 97, 349-370 (1997); Hermann & Patel, Adaptive recognition by nucleic acid aptamers. Science 287, 820-825 (2000)) that exhibit considerable enzymatic power and precise molecular recognition. Furthermore, these activities can be combined to create allosteric ribozymes (Soukup & Breaker, Engineering precision RNA molecular switches. Proc. Natl. Acad. Sci. USA 96, 3584-3589 (1999); Seetharaman et al., Immobilized riboswitches for the analysis of complex chemical and biological mixtures. Nature Biotechnol. 19, 336-341 (2001)) that are selectively modulated by effector molecules.

Bacterial riboswitch RNAs are genetic control elements that are located primarily within the 5'-untranslated region (5'-UTR) of the main coding region of a particular mRNA. Structural probing studies (discussed further below) reveal that riboswitch elements are generally composed of two domains: a natural aptamer (T. Hermann, D. J. Patel, Science 2000, 287, 820; L. Gold, et al., Annual Review of Biochemistry 1995, 64, 763) that serves as the ligand-binding domain, and an 'expression platform' that interfaces with RNA elements that are involved in gene expression (*e.g*. Shine-Dalgarno (SD) elements; transcription terminator stems).

### BRIEF SUMMARY OF THE INVENTION

Disclosed herein is a regulatable gene expression construct comprising a nucleic acid molecule encoding an RNA comprising a riboswitch operably linked to a coding region, wherein the riboswitch regulates expression of the RNA, wherein the riboswitch and coding region are heterologous. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. The riboswitch can comprise an aptamer domain and an expression platform domain, wherein the aptamer domain and the expression platform domain are heterologous. The riboswitch can also comprise two or more aptamer domains and an expression platform domain, wherein at least one of the aptamer domains and the expression platform domain are heterologous. At least two of the aptamer domains can exhibit cooperative binding.

Also disclosed is a riboswitch, wherein the riboswitch is a non-natural derivative of a naturally-occurring riboswitch. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. The riboswitch can comprise an aptamer domain and an expression platform domain, wherein the aptamer domain and the expression platform domain are heterologous. The riboswitch can further comprise one or more additional aptamer domains. At least two of the aptamer domains can exhibit cooperative binding. The riboswitch can be activated by a trigger molecule, wherein the riboswitch produces a signal when activated by the trigger molecule.

Further disclosed is a method of detecting a compound of interest, the method comprising: bringing into contact a sample and a riboswitch, wherein the riboswitch is activated by the compound of interest, wherein the riboswitch produces a signal when activated by the compound of interest, wherein the riboswitch produces a signal when the sample contains the compound of interest, wherein the riboswitch comprises a riboswitch or a derivative of a riboswitch. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. The riboswitch can change conformation when activated by the compound of interest, wherein the change in conformation produces a signal via a conformation dependent label. The riboswitch can also change conformation when activated by the compound of interest, wherein the change in conformation causes a change in expression of an RNA linked to the riboswitch, wherein the change in expression produces a signal. The signal can be produced by a reporter protein expressed from the RNA linked to the riboswitch.

Also disclosed is a method comprising: (a) testing a compound for inhibition of gene expression of a gene encoding an RNA comprising a riboswitch, wherein the inhibition is via the riboswitch, wherein the riboswitch comprises a riboswitch or a derivative of a riboswitch, (b) inhibiting gene expression by bringing into contact a cell and a compound that inhibited gene expression in step (a), wherein the cell comprises a gene encoding an RNA comprising a riboswitch, wherein the compound inhibits expression of the gene by binding to the riboswitch. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch.

Also disclosed is a method comprising: (a) testing a compound for derepression of gene expression of a gene encoding an RNA comprising a riboswitch, wherein the derepression is via the riboswitch, wherein the riboswitch comprises a riboswitch or a derivative of a riboswitch, (b) derepressing gene expression by bringing into contact a cell and a compound that derepressed gene expression in step (a), wherein the cell comprises a gene encoding an RNA comprising a riboswitch, wherein the compound derepresses expression of the gene by binding to the riboswitch. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch.

Disclosed is a method of identifying riboswitches, the method comprising assessing in-line spontaneous cleavage of an RNA molecule in the presence and absence of trigger molecule, wherein the RNA molecule is encoded by a gene regulated by the trigger molecule, wherein a change in the pattern of in-line spontaneous cleavage of the RNA molecule indicates a riboswitch responsive to the trigger molecule. The trigger molecule can be cyclic di-GMP, S-adenosylhomocysteine, preQ₁, Moco, or SAM.

Also disclosed is a method of altering gene expression, the method comprising bringing into contact a compound and a cell, wherein the compound is cyclic di-GMP, pGpG, GpG, or GpGpG. The compound can also be a derivative of cyclic di-GMP, pGpG, GpG, or GpGpG that can activate a cyclic di-GMP-responsive riboswitch. Also disclosed is a method of altering gene expression, the method comprising bringing into contact a compound and a cell, wherein the compound is S-adenosylhomocysteine. The compound can also be a derivative of S-adenosylhomocysteine that can activate an S-adenosylhomocysteine-responsive riboswitch. For example, the compound can be S-adenosyl-L-cysteine (SAC). Also disclosed is a method of altering gene expression, the method comprising bringing into contact a compound and a cell, wherein the compound is preQ₁. The compound can also be a derivative of preQ₁ that can activate a preQ₁-responsive riboswitch. Also disclosed is a method of altering gene expression, the method comprising bringing into contact a compound and a cell, wherein the compound is Moco. The compound can also be a derivative of Moco that can activate a Moco-responsive riboswitch. Also disclosed is a method of altering gene expression, the method comprising bringing into contact a compound and a cell, wherein the compound is SAM. The compound can also be a derivative of SAM that can activate a SAM-responsive riboswitch. The compound for activating a SAM-IV riboswitch can also be MeAzaAdoMet.

Compounds useful with preQ₁-responsive riboswitches (and riboswitches derived from preQ₁-responsive riboswitches) include derivatives of preQ₁ where the N at position 1 can be substituted with C, O or S, where the amino group at position 2 can be substituted with a hydrogen bond donor, where the oxygen at position 6 can be substituted with a hydrogen bond acceptor, where the amino group at position 7 can be substituted with a hydrogen bond acceptor, and where the nitrogen can be substituted or derivatized with a hydrogen bond donor.

Examples of hydrogen bond donors and acceptors include NH, NH₂⁺, NH₃⁺, O, OH, S, SH, C-R₅, CH-R₅, N-R₅, NH-R₅, O-R₅, or S-R₅, wherein R₅ is NH₂⁺, NH₃⁺, CO₂H, B(OH)₂, CH(NH₂)₂, C(NH₂)₂⁺, CNH₂NH₃⁺, C(NH₃⁺)₃, hydroxymethyl, 1-hydroxyethyl, 2-hydroxyethyl, 1,2-dihydroxyethyl, 2-hydroxy-1-methylethyl,1-hydroxypropyl, 2-hydroxypropyl, 3-hydroxypropyl, 1,3-dihydroxypropyl, 2,3-dihydroxypropyl, 1-hydroxybutyl, 2-hydroxybutyl, 3-hydroxybutyl, 4-hydroxybutyl, 1, 4 dihydroxybutyl, 2,4-dihydroxybutyl, 1-hydroxy-2-methylpropyl, 2-hydroxy-2-methylpropyl, 3-hydroxy-2-methylpropyl, 1-hydroxymethyl-1-methylethyl, trishydroxymethylmethyl, thiolmethyl, 1-thiolethyl, 2-thiolethyl, 1,2-dithiolethyl, 2-thiol-1-methylethyl,1-thiolpropyl, 2-thiolpropyl, 3-thiolpropyl, 1,3-dithiolpropyl, 2,3-dithiolpropyl, 1-thiolbutyl, 2-thiolbutyl, 3-thiolbutyl, 4-thiolbutyl, 1, 4 dithiolbutyl, 2,4-dithiolbutyl, 1-thiol-2-methylpropyl, 2-thiol-2-methylpropyl, 3-thiol-2-methylpropyl, 1-thiolmethyl-1-methylethyl, tristhiolmethylmethyl, aminomethyl, 1-aminoethyl, 2-aminoethyl, 1,2-diaminoethyl, 2-amino-1-methylethyl,1-aminopropyl, 2-aminopropyl, 3-aminopropyl, 1,3-diaminopropyl, 2,3-diaminopropyl, 1-aminobutyl, 2-aminobutyl, 3-aminobutyl, 4-aminobutyl, 1, 4 diaminobutyl, 2,4-diaminobutyl, 1-amino-2-methylpropyl, 2-amino-2-methylpropyl, 3-amino-2-methylpropyl, 1-aminomethyl-1-methylethyl, and trisaminomethylmethyl.

The cell can be identified as being in need of altered gene expression. The cell can be a bacterial cell. The compound can kill or inhibit the growth of the bacterial cell. The compound and the cell can be brought into contact by administering the compound to a subject. The cell can be a bacterial cell in the subject, wherein the compound kills or inhibits the growth of the bacterial cell. The subject can have a bacterial infection. The cell can contain a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. The compound can be administered in combination with another antimicrobial compound. The compound can inhibit bacterial growth in a biofilm.

Also disclosed is a method of altering the physiological state of a cell, the method comprising bringing into contact a compound and a cell, wherein the compound is cyclic di-GMP, pGpG, GpG, or GpGpG. The compound can also be a derivative of cyclic di-GMP, pGpG, GpG, or GpGpG that can activate a cyclic di-GMP-responsive riboswitch.

Also disclosed is a method of producing a trigger molecule, the method comprising: cultivating a mutant bacterial cell capable of producing the trigger molecule, wherein the mutant bacterial cell comprises a mutation in a riboswitch responsive to the trigger molecule, which mutation increases production of the trigger molecule by the mutant bacterial cell in comparison to a cell not having the mutation; and isolating trigger molecule from the cell culture, thereby producing the trigger molecule. The trigger molecule can be, for example, cyclic di-GMP, S-adenosylhomocysteine, preQ₁, Moco, or SAM. This method can yield at least a 10% increase in production of the trigger molecule compared to cultivating a bacterial cell that does not comprise the mutation in the riboswitch. This method can yield at least a 15% increase in production of the trigger molecule compared to cultivating a bacterial cell that does not comprise the mutation in the riboswitch. This method can yield at least a 25% increase in production of the trigger molecule compared to cultivating a bacterial cell that does not comprise the mutation in the riboswitch. The riboswitch can comprise a knockout mutation. Further disclosed is a bacterial cell comprising a mutation in a riboswitch, which mutation measurably increases production of the trigger molecule for the riboswitch by the cell when compared to a cell that does not have the mutation.

Also disclosed is a method of inhibiting bacterial cell growth, the method comprising: bringing into contact a cell and a compound that binds a riboswitch, wherein the cell comprises a gene encoding an RNA comprising a riboswitch responsive to the compound, wherein the compound inhibits bacterial cell growth by binding to the riboswitch, thereby altering expression of the gene. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. This method can yield at least a 10% decrease in bacterial cell growth compared to a cell that is not in contact with the compound. The compound and the cell can be brought into contact by administering the compound to a subject. The cell can be a bacterial cell in the subject, wherein the compound kills or inhibits the growth of the bacterial cell. The subject can have a bacterial infection. The compound can be administered in combination with another antimicrobial compound.

Disclosed is a method of detecting a compound in a sample comprising: bringing a riboswitch responsive to the compound in contact with the sample; and detecting interaction between compound and the riboswitch, wherein interaction between compound and the riboswitch indicates the presence of the compound. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. The riboswitch can be labeled.

Also disclosed is a method comprising inhibiting gene expression of a gene encoding an RNA comprising a riboswitch by bringing into contact a cell and a compound that was identified as a compound that inhibits gene expression of the gene by testing the compound for inhibition of gene expression of the gene, wherein the inhibition was via the riboswitch. The riboswitch can be a cyclic di-GMP-responsive riboswitch, an S-adenosylhomocysteine-repsonsive riboswitch, a preQ₁-responsive riboswitch, a Moco-responsive riboswitch, a SAM-responsive riboswitch, or a derivative of such riboswitches.

Also disclosed is a method comprising derepressing gene expression of a gene encoding an RNA comprising a riboswitch by bringing into contact a cell and a compound that was identified as a compound that derepresses gene expression of the gene by testing the compound for derepression of gene expression of the gene, wherein the derepression was via the riboswitch. The riboswitch can be a cyclic di-GMP-responsive riboswitch, an S-adenosylhomocysteine-repsonsive riboswitch, a preQ₁-responsive riboswitch, a Moco-responsive riboswitch, a SAM-responsive riboswitch, or a derivative of such riboswitches.

A first object of the present invention is a regulatable gene expression construct comprising a nucleic acid molecule encoding an RNA comprising a riboswitch operably linked to a coding region, wherein the riboswitch regulates expression of the RNA, wherein the riboswitch and coding region are heterologous, wherein the riboswitch is a cyclic di-GMP-responsive riboswitch, an S-adenosylhomocysteine-repsonsive riboswitch, a preQ₁-responsive riboswitch, a Moco-responsive riboswitch, or a SAM-responsive riboswitch.

In a preferred embodiment, the riboswitch comprises an aptamer domain and an expression platform domain, wherein the aptamer domain and the expression platform domain are heterologous.

In a preferred embodiment, the riboswitch comprises two or more aptamer domains and an expression platform domain, wherein at least one of the aptamer domains and the expression platform domain are heterologous.

In a preferred embodiment, at least two of the aptamer domains exhibit cooperative binding.

In a preferred embodiment, the riboswitch is a non-natural derivative of a naturally-occurring riboswitch, wherein the riboswitch is a cyclic di-GMP-responsive riboswitch, an S-adenosylhomocysteine-repsonsive riboswitch, a preQ₁-responsive riboswitch, a Moco-responsive riboswitch, or a SAM-responsive riboswitch.

In a preferred embodiment, the riboswitch comprises an aptamer domain and an expression platform domain, wherein the aptamer domain and the expression platform domain are heterologous.

In a preferred embodiment, the riboswitch comprises a GEMM motif, a SAH motif, a preQ₁-II motif, a Moco motif or a SAM-IV motif.

In a preferred embodiment, the riboswitch is activated by a trigger molecule, wherein the riboswitch produces a signal when activated by the trigger molecule.

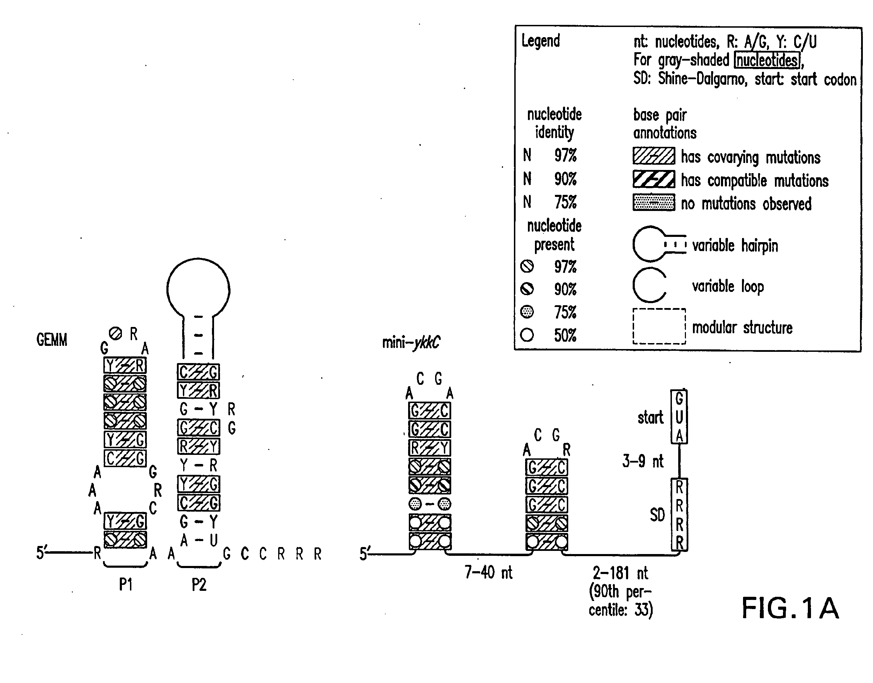

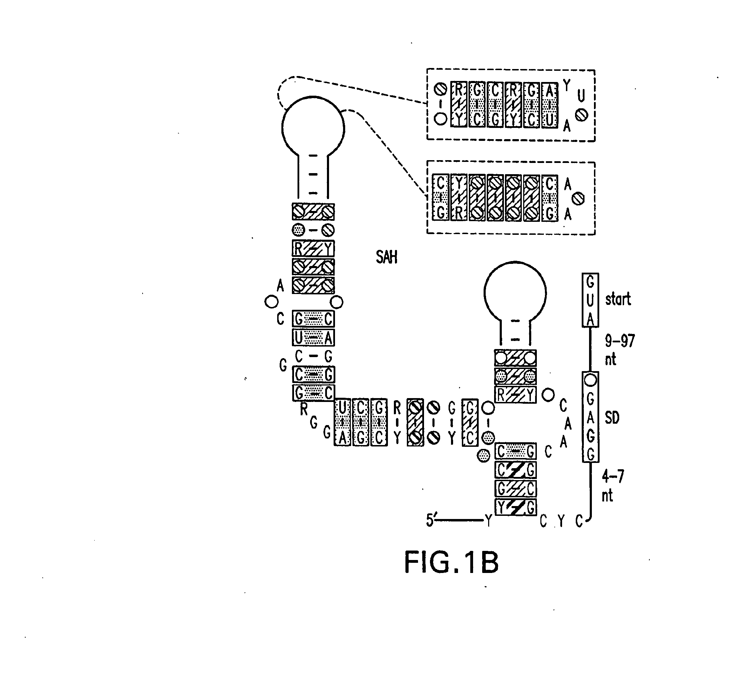

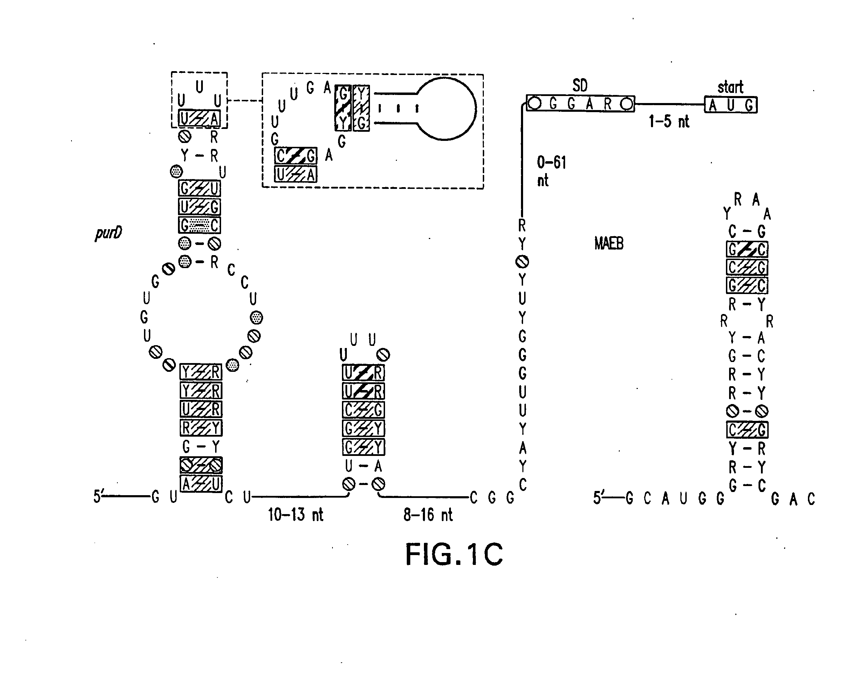

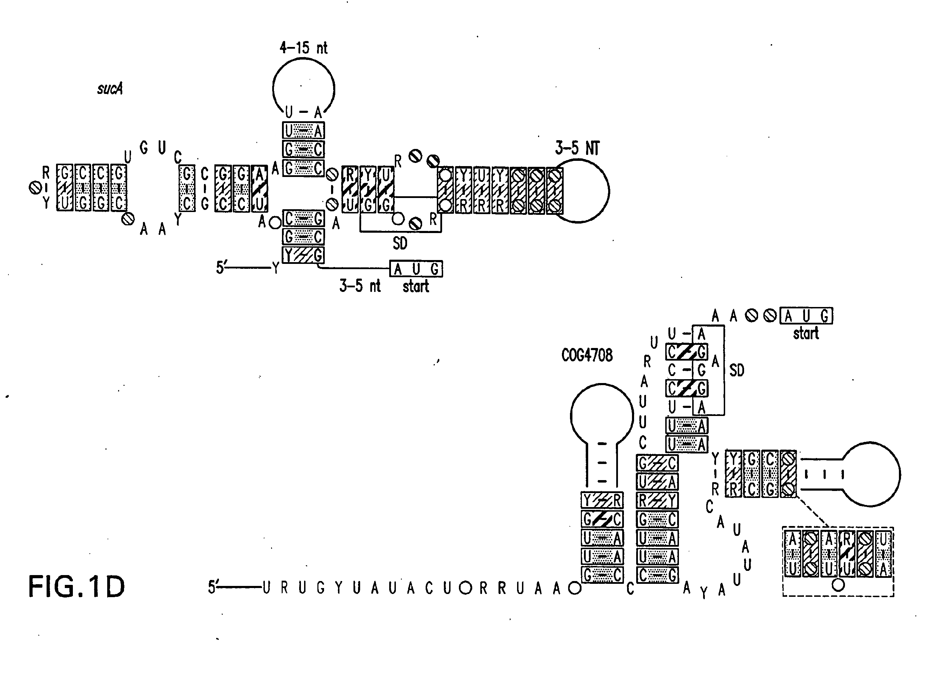

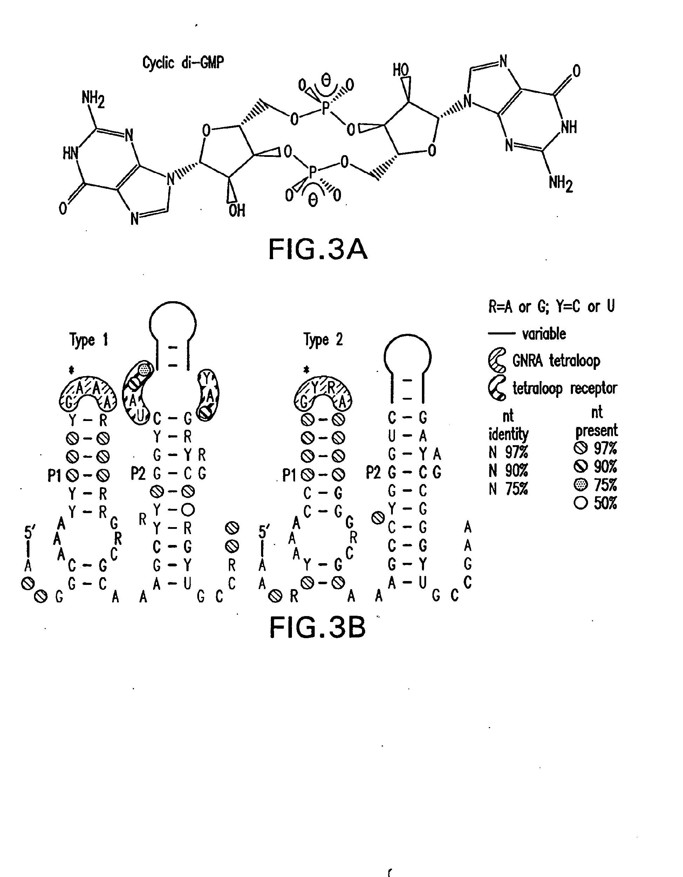

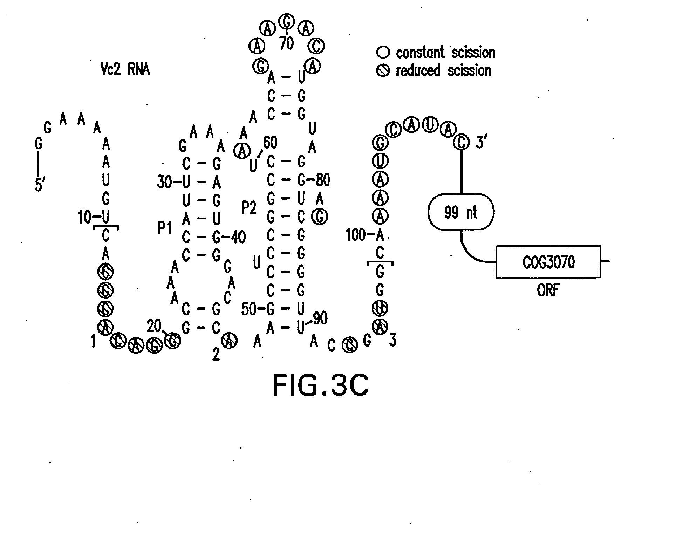

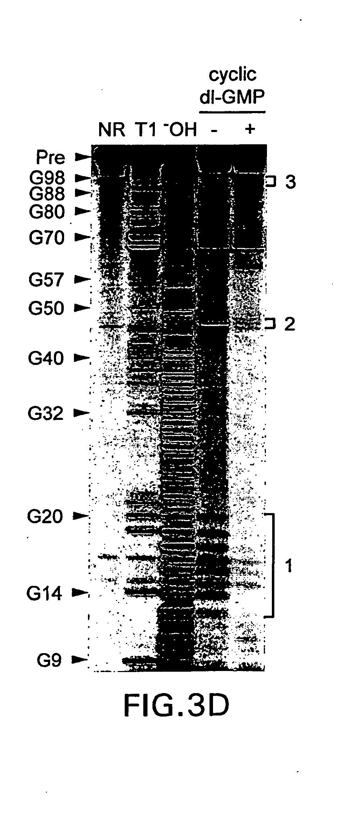

In a preferred embodiment, the riboswitch has one of the consensus structures of Figure 1 or Figure 3.

In a preferred embodiment, the riboswitch comprises an aptamer domain and an expression platform domain wherein the aptamer domain is derived from a naturally-occurring cyclic di-GMP-responsive riboswitch, S-adenosylhomocysteine-repsonsive riboswitch, preQ₁-responsive riboswitch, a Moco-responsive riboswitch, or a SAM-responsive riboswitch.

In a preferred embodiment, the aptamer domain is the aptamer domain of a naturally-occurring cyclic di-GMP-responsive riboswitch, S-adenosylhomocysteine-repsonsive riboswitch, preQ₁-responsive riboswitch, Moco-responsive riboswitch, or SAM-responsive riboswitch.

In a preferred embodiment, the aptamer domain has the consensus structure of an aptamer domain of the naturally-occurring riboswitch.

In a preferred embodiment, the aptamer domain consists of only base pair conservative changes of the naturally-occurring riboswitch.

A second object of the present invention is a method of detecting a compound of interest, the method comprising bringing into contact a sample and a riboswitch, wherein the riboswitch is activated by the compound of interest, wherein the riboswitch produces a signal when activated by the compound of interest, wherein the riboswitch produces a signal when the sample contains the compound of interest, wherein the riboswitch is a cyclic di-GMP-responsive riboswitch or a derivative of a cyclic di-GMP-responsive riboswitch, an S-adenosylhomocysteine-repsonsive riboswitch or a derivative of an S-adenosylhomocysteine-repsonsive riboswitch, a preQ₁-responsive riboswitch or a derivative of a preQ₁-responsive riboswitch, a Moco-responsive riboswitch or a derivative of a Moco-responsive riboswitch, or a SAM-responsive riboswitch or a derivative of a SAM-responsive riboswitch.

In a preferred embodiment, the riboswitch changes conformation when activated by the compound of interest, wherein the change in conformation produces a signal via a conformation dependent label.

In a preferred embodiment, the riboswitch changes conformation when activated by the compound of interest, wherein the change in conformation causes a change in expression of an RNA linked to the riboswitch, wherein the change in expression produces a signal.

In a preferred embodiment, the signal is produced by a reporter protein expressed from the RNA linked to the riboswitch.

A third object of the present invention is a method comprising (a) testing a compound for altering gene expression of a gene encoding an RNA comprising a riboswitch, wherein the alteration is via the riboswitch, wherein the riboswitch is a cyclic di-GMP-responsive riboswitch or a derivative of a cyclic di-GMP-responsive riboswitch, an S-adenosylhomocysteine-repsonsive riboswitch or a derivative of an S-adenosylhomocysteine-repsonsive riboswitch, a preQ₁-responsive riboswitch or a derivative of a preQ₁-responsive riboswitch, a Moco-responsive riboswitch or a derivative of a Moco-responsive riboswitch, or a SAM-responsive riboswitch or a derivative of a SAM-responsive riboswitch, (b) altering gene expression by bringing into contact a cell and a compound that altered gene expression in step (a), wherein the cell comprises a gene encoding an RNA comprising a riboswitch, wherein the compound inhibits expression of the gene by binding to the riboswitch.

A further object of the present invention is a method of identifying riboswitches, the method comprising assessing in-line spontaneous cleavage of an RNA molecule in the presence and absence of a compound, wherein the RNA molecule is encoded by a gene regulated by the compound, wherein a change in the pattern of in-line spontaneous cleavage of the RNA molecule indicates a riboswitch, wherein (a) the RNA comprises a cyclic di-GMP-responsive riboswitch or a derivative of a cyclic di-GMP-responsive riboswitch and the compound is cyclic di-GMP, (b) the RNA comprises an S-adenosylhomocysteine-repsonsive riboswitch or a derivative of an S-adenosylhomocysteine-repsonsive riboswitch and the compound is S-adenosylhomocysteine, (c) the RNA comprises a preQ₁-responsive riboswitch or a derivative of a preQ₁-responsive riboswitch and the compound is preQ₁, or (d) the RNA comprises a Moco-responsive riboswitch or a derivative of a Moco-responsive riboswitch, or a SAM-responsive riboswitch or a derivative of a SAM-responsive riboswitch.

A further object of the present invention is a method of altering gene expression, the method comprising bringing into contact a compound and a cell, wherein the cell comprises a gene encoding an RNA comprising a cyclic di-GMP-responsive riboswitch, an S-adenosylhomocysteine-repsonsive riboswitch, a preQ₁-responsive riboswitch, a Moco-responsive riboswitch, or a SAM-responsive riboswitch, wherein the compound alters expression of the gene by binding to the a cyclic di-GMP-responsive riboswitch, an S-adenosylhomocysteine-repsonsive riboswitch, a preQ₁-responsive riboswitch, a Moco-responsive riboswitch, or a SAM-responsive riboswitch.

In a preferred embodiment, the cell has been identified as being in need of altered gene expression.

In a preferred embodiment, the cell is a bacterial cell.

In a preferred embodiment, the compound kills or inhibits the growth of the bacterial cell.

In a preferred embodiment, the compound and the cell are brought into contact by administering the compound to a subject.

In a preferred embodiment, the cell is a bacterial cell in the subject, wherein the compound kills or inhibits the growth of the bacterial cell.

In a preferred embodiment, the subject has a bacterial infection.

In a preferred embodiment, the compound is administered in combination with another antimicrobial compound.

In a preferred embodiment, the compound inhibits bacterial growth in a biofilm.

Additional advantages of the disclosed method and compositions will be set forth in part in the description which follows, and in part will be understood from the description, or can be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.
Figure 1 shows consensus sequences and structures are depicted for seven of the 22 motifs identified. Calculations for conservation of nucleotide identity/presence and evidence of covariation are described in Example 1. Proposed pairs with more than 5% non-canonical or missing nucleotides are not classified as covarying. Note that the levels of nucleotide conservation are affected both by biochemical constraints on the motif and by phylogenetic diversity. Motifs with limited range (e.g., the preQ₁-II or COG4708 motif) will appear more conserved, and some covarying positions in variable-length stems are not shown.

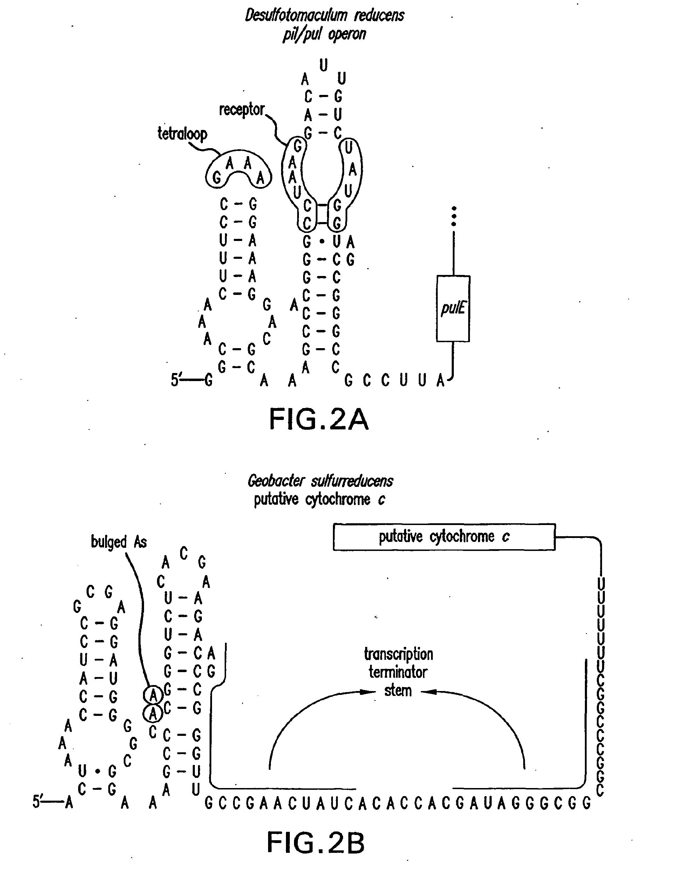

Figure 2 shows common featured of GEMM motifs. Two GEMM instances were selected to illustrate common features, although these two examples do not represent the full 322 GEMMs. (A) This putative RNA contains a canonical GNRA tetraloop and receptor (gray regions). Almost 50% of GEMM instances contain a likely tetraloop receptor. Only the first gene in the downstream operon is shown. (B) Some GEMM RNAs lack the tetraloop receptor, but there are two extra bulged A residues (gray shading) that are found in roughly half of the sequences lacking a receptor. Gray overlined nucleotides can fold to form a stem of a rho-independent transcription terminator (followed by 3'-trailing Us). This terminator appears to compete with the 3' part of the P2 stem (right-most hairpin). 78 of 322 GEMM instances have predicted transcription terminators overlapping P2.
Figure 3 shows cyclic di-GMP aptamers. **(A)** Chemical structure of the second messenger cyclic di-GMP. **(B)** Consensus sequences and structures of Type 1 and Type 2 GEMM RNAs. **(C)** Sequence and structure of the Vc2 RNA from *V. cholerae* chromosome 2, and its proximity to the ORF of VC1722. Nucleotides shown correspond to the 110 Vc2 RNA construct. Bold numbers identify regions of ligand-mediated structure modulation as observed in D. Brackets identify the minimal 5' or 3' terminus (when the opposing terminus for 110 Vc2 RNA is retained) that exhibits structural modulation when tested with 10 nM cyclic di-GMP. The sequence of nucleotides 13-20 is CGCACAGG. **(D)** PAGE separation of RNA products generated by in-line probing of 5' ³²P-labeled 110 Vc2 RNA. NR (no reaction); T1 (partial digest with RNase T1); ⁻OH (partial digest with alkali). RNA was incubated in the absence (-) or presence (+) of 100 µM cyclic di-GMP.

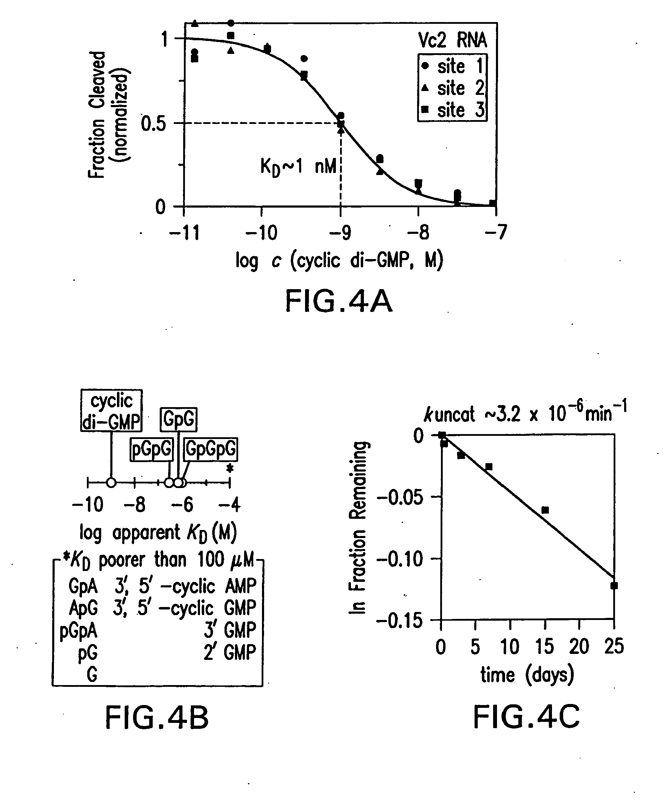

Figure 4 shows affinity and specificity of the Vc2 aptamer for cyclic di-GMP. **(A)** Plot of the normalized fraction of 110 Vc2 aptamer cleaved versus cyclic di-GMP concentration. Sites of structural modulation are as depicted in Figure 3. **(B)** Comparison of K_{D} values exhibited by 110 Vc2 aptamer for cyclic di-GMP and various analogs. G (guanosine); pG, pGpG, pGpA (5' phosphorylated mono- and dinucleotides); GpGpG (trinucleotide), AMP and GMP (adenosine- and guanosine monophosphate, respectively). **(C)** Plot of the natural logarithm of the fraction of cyclic di-GMP remaining intact versus incubation time under in-line probing conditions. Negative slope of the line reflects the uncatalyzed rate constant (*k*_{uncat}) for cleavage of the second messenger.

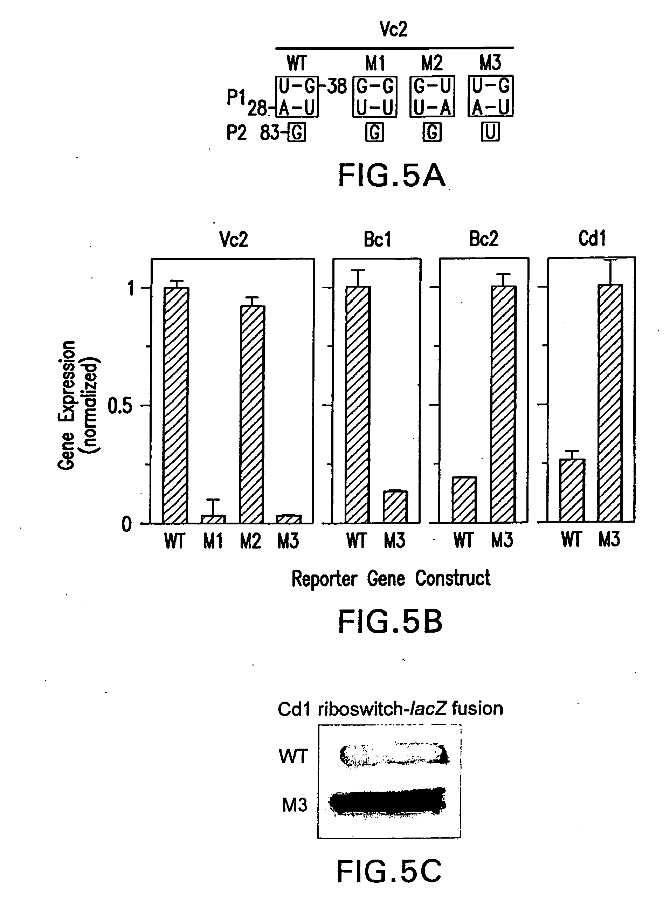

Figure 5 shows representative cyclic di-GMP aptamers are components of gene control elements. **(A)** Reporter fusion constructs carry wild-type (WT) or mutant (M1 through M3) riboswitches from *V*. *cholerae* (Vc2), or carry the equivalent WT and M3 riboswitches from *B. cereus* (Bc1 and Bc2) or *C*. *difficile* (Cd1). **(B)** β-galactosidase reporter gene assays for the constructs depicted in A when transformed into *E*. *coli.* Maximum Miller units measured for the four representatives were 436, 47, 5 and 51, respectively. **(C)** *B. subtilis* cells carrying a β-galactosidase reporter gene (*lacZ*) fused downstream of a WT or a M3 Cd1 riboswitch and grown on agar containing X-gal.

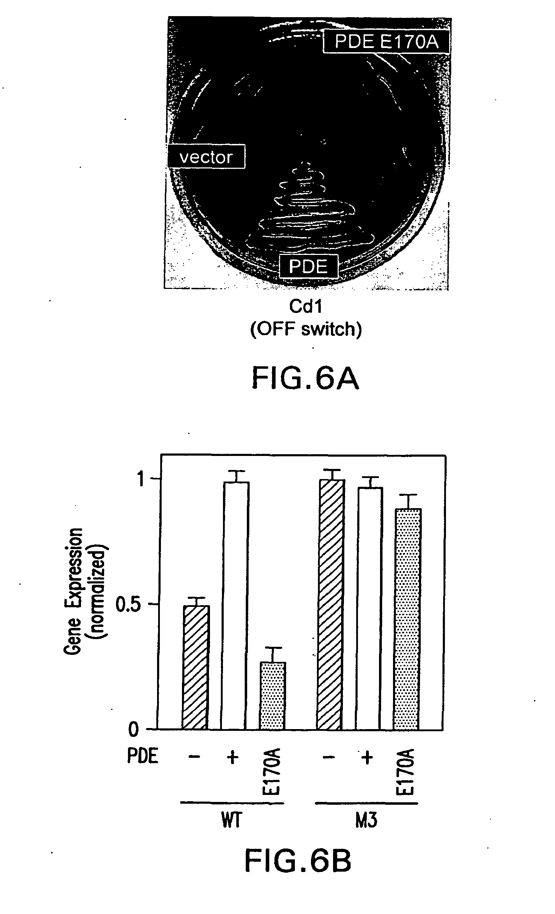

Figure 6 shows that expressing a cyclic di-GMP phosphodiesterase alters expression of a reporter gene regulated by the Cd1 riboswitch. **(A)** *B. subtilis* cells carrying a β-galactosidase reporter construct fused to a wild-type (WT) Cd1 riboswitch and transformed with a plasmid lacking (-) or carrying a normal (+) or mutant (E170A) *V. cholerae vieA* gene encoding an EAL phosphodiesterase (PDE). **(B)** β-galactosidase reporter assays of *B. subtilis* cells carrying the reporter gene fused to WT or M3 riboswitches as indicated, and transformed with plasmids as described in A. Normalized gene expression value of 1 for Cd1 represents 102 Miller units.

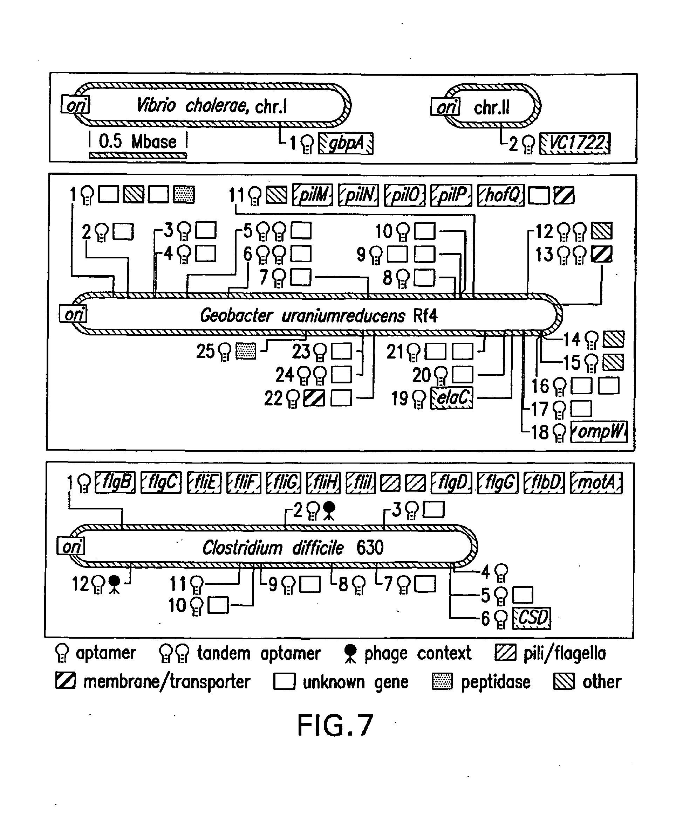

Figure 7 shows genomic locations of cyclic di-GMP riboswitches or aptamers for representative organisms. Genes residing immediately downstream of representatives are depicted, where multiple genes indicate predicted operons. Absence of genes indicates that the cyclic di-GMP aptamer is not located 5' proximal to any ORF. COG3070 is joined to a pfam04994 domain, which is a protein arrangement similar to TfoX (93, 94). Chromosomes are represented by shaded lines; ori (origin of replication).

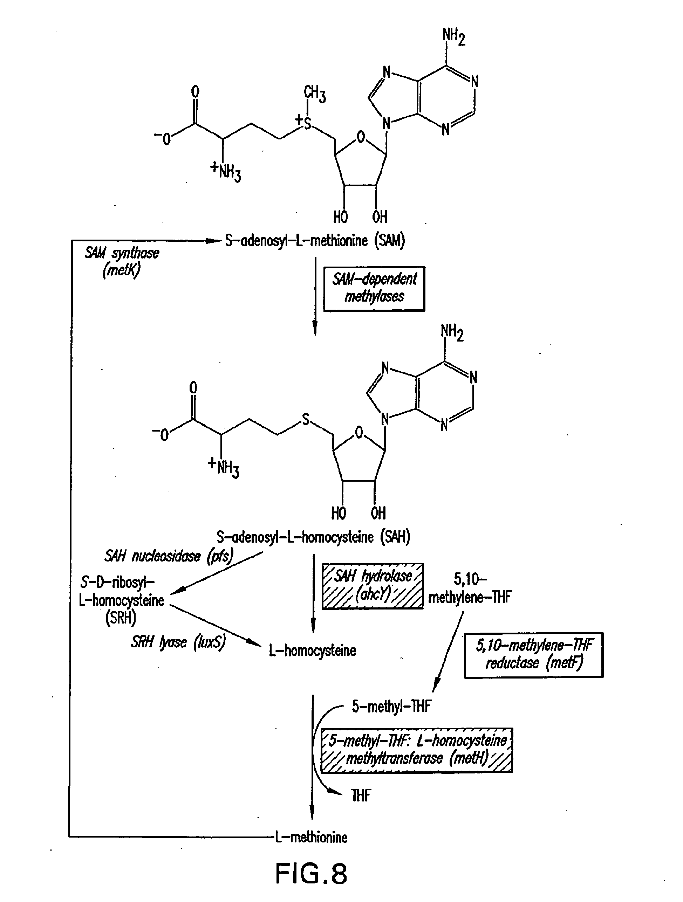

Figure 8 shows typical SAM Metabolic Cycle in Eubacteria. Names of genes from *Escherichia coli* (strain K-12) or *Pseudomonas syringae* are given in parentheses following each enzyme. Shaded boxes identify ORFs that are located immediately downstream of an SAH element, while open boxes identify ORFs that sometimes reside downstream of (and possibly in the same operon as) the SAH hydrolase gene *ahcY* when an SAH element is present. THF is tetrahydrofolate.

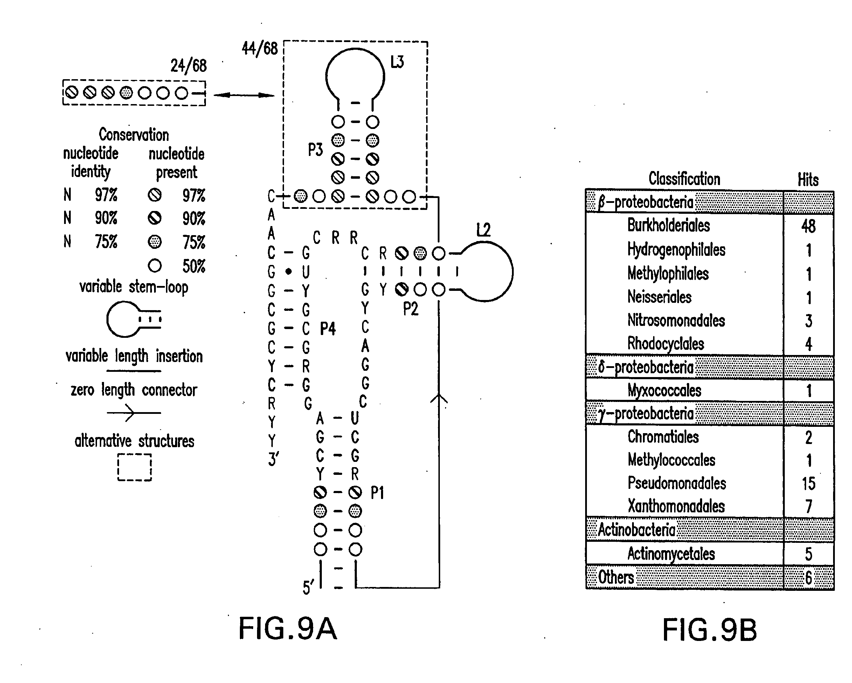

Figure 9 shows the SAH Element is a Highly Conserved RNA Motif Found in Many Gram-positive and Gram-negative Bacteria. (A) Consensus nucleotide sequence and secondary structure model of the conserved RNA element located in the 5' UTRs of genes for SAH recycling. The consensus sequence and structural model were determined by examining 68 representatives of the motif excluding duplicates identified from near identical genomic DNAs. P1 through P4 identify predicted base-paired regions, and the numbers of non-duplicative representatives that carry or exclude the optional P3 stem are indicated. (B) Phylogenetic distribution of SAH elements identified by bioinformatics searching and verified by genomic context, including duplicative hits of the same sequence in closely related bacterial strains. "Others" category refers to hits found in environmental sequences.

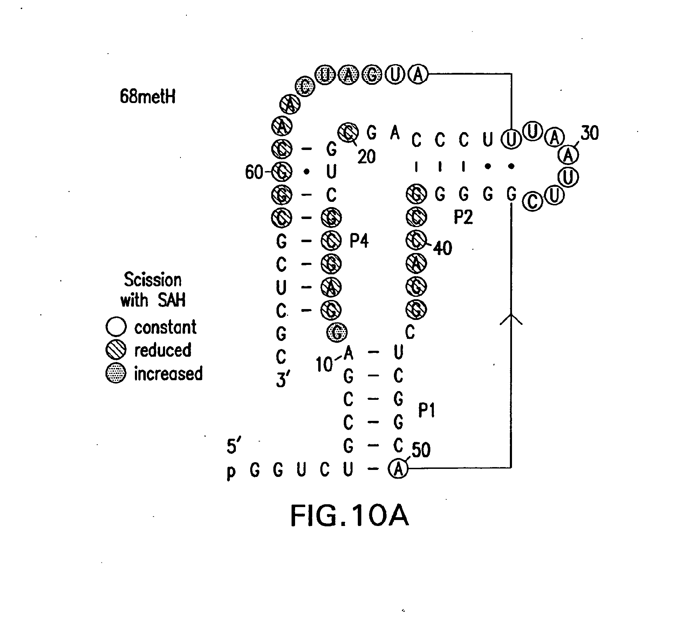

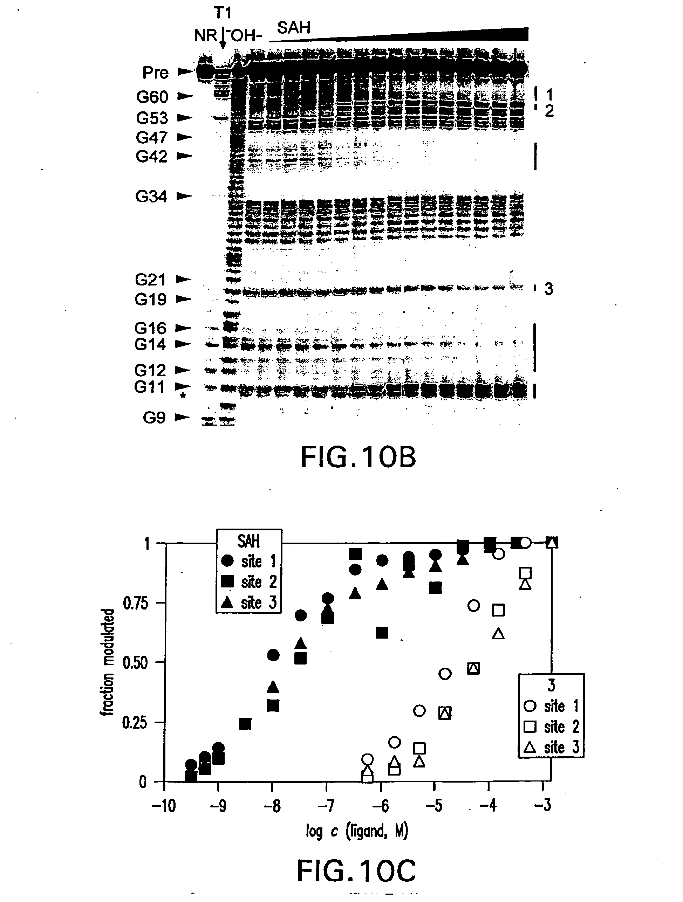

Figure 10 shows the SAH Element from the *metH* 5'-UTR of *Dechloromonas aromatica* Undergoes Structural Changes When Selectively Binding SAH. (A) Sequence and secondary structure model of the 68 *metH* RNA, which contains the conserved SAH aptamer. Sites of spontaneous RNA cleavage during in-line probing of 5' ³²P-labeled RNAs were identified from the data presented in B. Two guanosyl residues not present in the natural RNAs were added to the 5' end of the construct to facilitate *in vitro* transcription by T7 RNA polymerase. Nucleotides in regions 1 through 8 and 63 through 68 could not be visualized on the gel depicted in B, and therefore strand scission was not quantitated for these residues. (B) Spontaneous cleavage patterns resulting from in-line probing of the 68 *metH* RNA in the absence (-) and presence of SAH at concentrations ranging from 100 pM to 300 µM. NR, T1, and ⁻OH lanes identify precursor RNAs without treatment, partially digested with RNase T1, or partially digested at elevated pH, respectively. Some bands generated by RNase T1 digestion (cleavage after G residues) are identified (arrowheads), and Pre identifies the uncleaved precursor RNA bands. Regions of modulation of spontaneous RNA cleavage are identified with vertical bars, and sites that were quantitated to estimate binding affinity are labeled 1 through 3. The asterisk identifies a product band that corresponds to cleavage after G11, but where the 2', 3'-cyclic phosphate intermediate has been hydrolyzed to yield a mixture of 2'- and 3'-phosphate products that exhibit slightly faster mobility during PAGE. (C) Plot depicts the dependence of the 68 *metH* RNA spontaneous cleavage at the sites designated in B on the concentration (*c*) of SAH and compound 3. Apparent *K*_{D} values are estimated for each compound by using a best fit curve for the collective data and assuming a two-state binding model. The concentration of ligand needed to induce half-maximal structural modulation reflects the apparent *K*_{D}.

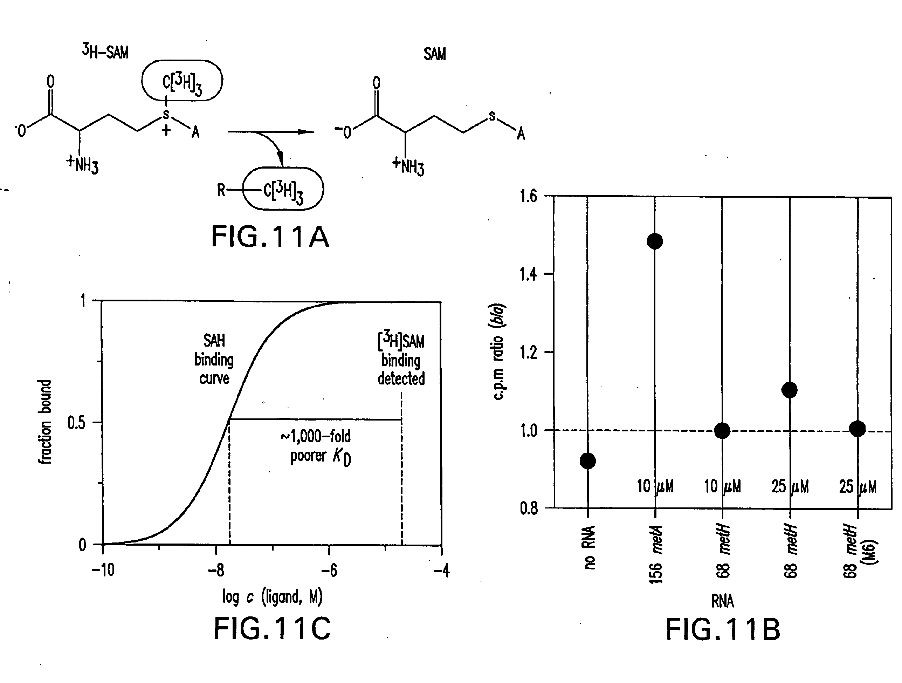

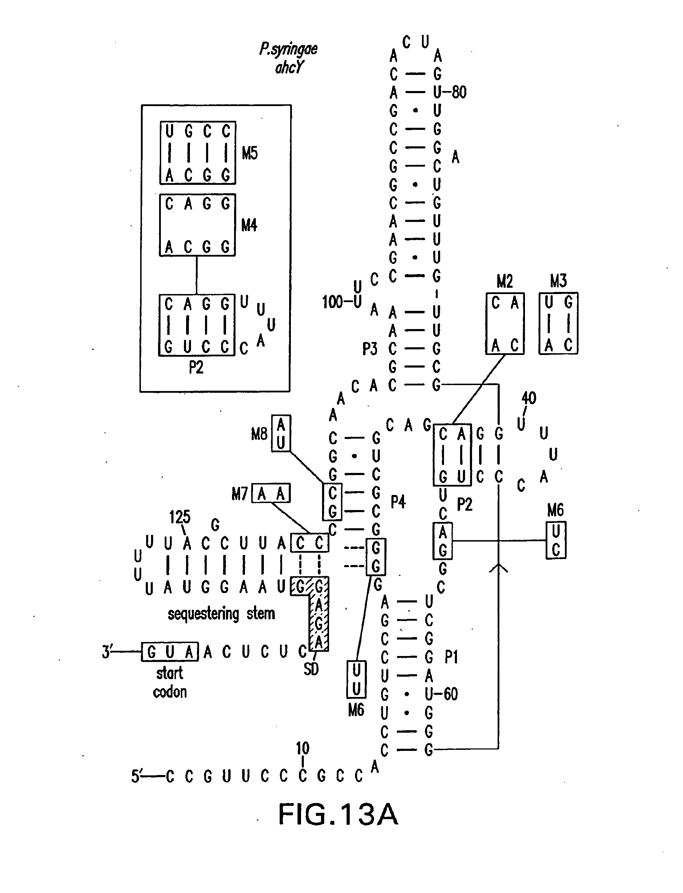

Figure 11 shows the 68 *metH* RNA Discriminates against SAM by Several Orders of Magnitude. (A) Breakdown of radioactive [³H]SAM by demethylation yields non-radioactive SAH. (B) Equilibrium dialysis was used to compare SAM binding affinity of a known SAM aptamer (156 *metA*) (Corbino et al., 2005) with 68 *metH* RNA. Chambers *a* and *b* of each equilibrium dialysis system are separated by a permeable membrane with a molecular weight cut-off (MWCO) of 5000 Daltons. [³H]SAM (100 nM) or RNA as indicated are added to chamber *a* and *b*, respectively. The apparent *K*_{D} of the SAM-II aptamer 156 *metA* for SAM is∼1 µM (Corbino et al., 2005), therefore the shift in tritium to chamber *b* should be maximal under the conditions used. Since 68 *metH* RNA is present in excess, the effect of non-radioactive SAH (from spontaneous demethylation of [³H]SAM) on tritium distribution between the chambers should be negligible. Data shown are representative of several repeats. (C) Comparison of the expected curve for binding of SAH with 68 *metH* RNA and the concentration of 68 *metH* RNA used for equilibrium dialysis. The observation that 68 *metH* RNA does not maximally shift [³H]SAM when the RNA is present at 25 µM suggests that the apparent *K*_{D} for SAM∼1,000-fold poorer or worse. M6 carries two mutations (G12U and A13U) that are equivalent in location and nucleotide identity to construct M6 depicted in Figure 13A.

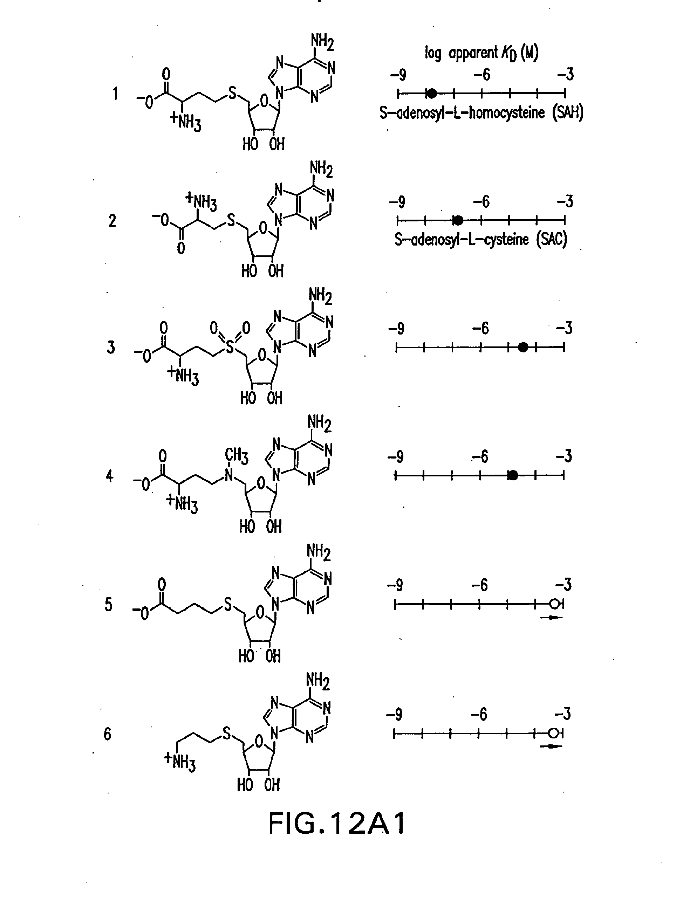

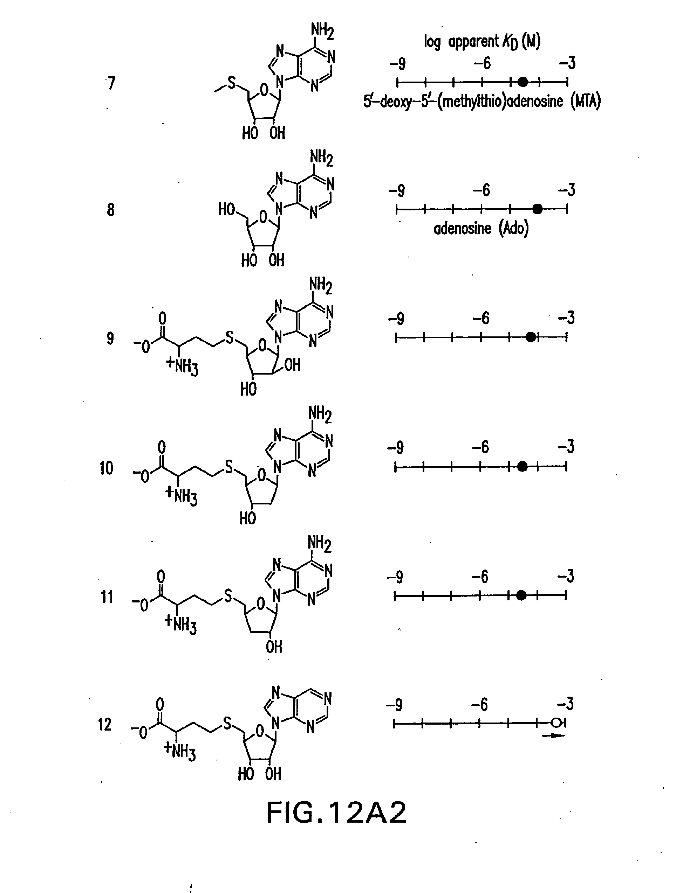

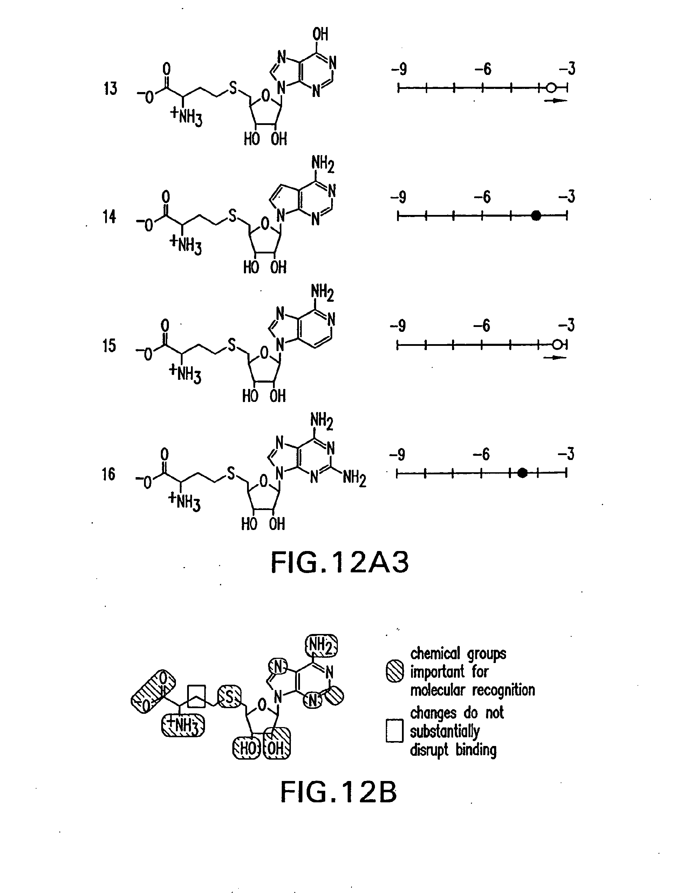

Figure 12 shows molecular Recognition Characteristics of the *68 metH* SAH Aptamer. (A) Chemical structures and apparent *K*_{D} values of compounds whose ligand-binding affinities were established by in-line probing with 68 *metH RNA.* Apparent *K*_{D} values were estimated as described for SAH in Figure 10C. (B) Summary of the known molecular recognition features of 68 *metH RNA* for SAH.

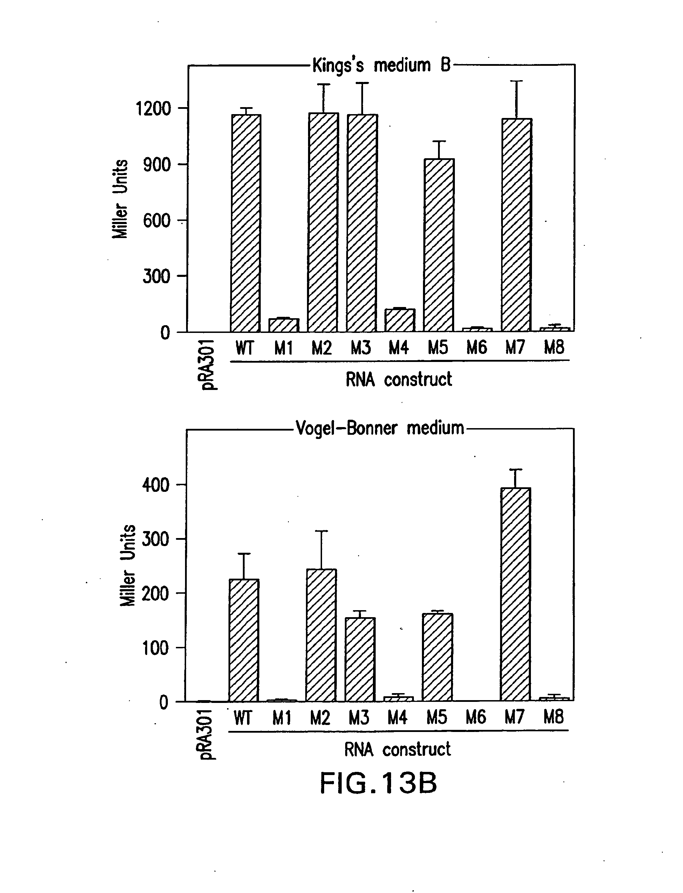

Figure 13 shows an SAH Riboswitch Controls Reporter Gene Expression. (A) Sequence and secondary structure model of the *ahcY* 5'-UTR of *P. syringae* and various mutations introduced for gene expression studies. (B) Plots comparing expression levels for a β-galactosidase reporter gene (*lacZ*) fused downstream of wild-type (WT) and mutant *ahcY* leader sequences as defined in A. pRA301 denotes the reporter strain transformed by the original plasmid pRA301 without any insertion in front of *lacZ.* Different growth media used are defined in Experimental Procedures. Values are averages from three independent experiments and error bars represent standard deviation.

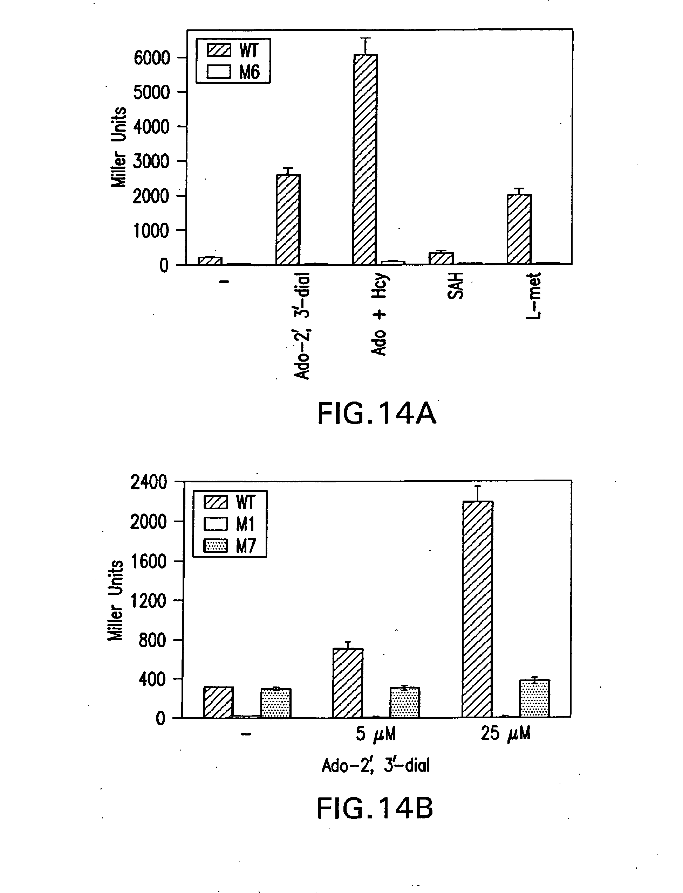

Figure 14 shows an elevated SAH Levels Increase Expression of the Riboswitch-Reporter Fusion. (A) Plotted are β-galactosidase expression levels of wild-type (WT) and mutant (M6) reporter strains (Figure 13A) grown in Vogel-Bonner medium without supplementation (-), or supplemented with 25 µM of Ado-2',3'-dial or 250 µM of the other compounds listed. Compound abbreviations: Ado-2',3'-dial (adenosine-2',3'-dialdehyde); Ado (adenosine); Hcy (homocysteine); L-met (L-methionine). (B) WT, M1 and M7 constructs subjected to reporter assays as described in A with Ado-2',3'-dial at indicated concentrations. Values are averages from three independent experiments and error bars represent standard deviation.

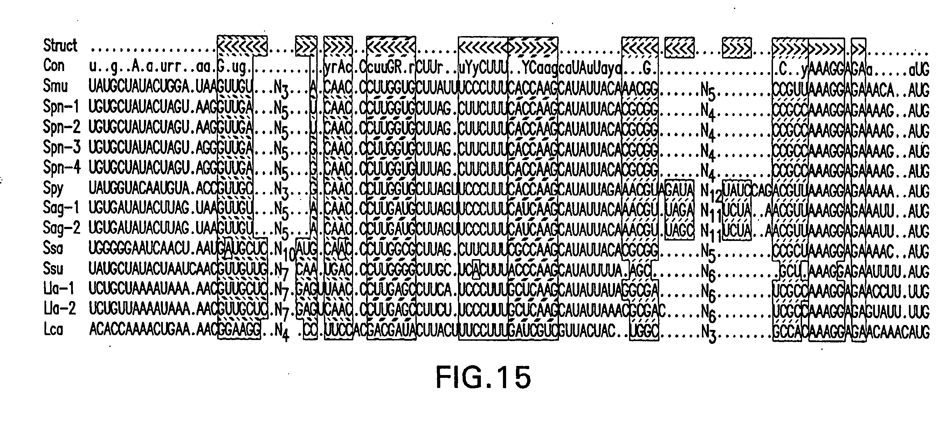

Figure 15 shows alignment of COG4708 RNA motif sequences. Nucleotides that are present in greater than 97% and 75% of the representatives are in uppercase and lowercase, respectively (R = A, G; Y = C, U). Colored regions marked by brackets indicated secondary structure elements P1 (blue), P2 (green), P3 (orange), and P4 (violet). All the sequences depicted are unique. However, some sequences only differ in the loop regions represented by Nₓ and thus appear identical here. Organism abbreviations: (Smu) *Streptococcus mutants,* (Spn-1) *S. pneumoniae* R6, (Spn-2) *S. pneumoniae* SP18-BS74, (Spn-3) *S. pneumoniae* SP19-BS75, (Spn-4) *S. pneumoniae* SP6-BS73, (Spy) *S*. *pyogenes,* (Sag-1) *S. agalactiae* 2630V/R, (Sag-2) *S. agalactiae* COH1, (Ssa) *S*. *sanguinis,* (Ssu) *S. suis,* (Lla-1) *Lactococcus lactis* subsp. lactis, (Lla-2) *L. lactis* subsp. cremoris SK11, (Lca) *Lactobacillus casei.* There are 12 additional examples of the Spy sequence, all in differing *S*. *pyogenes* strains. There are seven additional examples of Spn-1 all in differing *S*. *pneumoniae* strains. There are six additional examples of Sag-1 all in differing *S. agalactiae* strains. There is one additional example of Ssu, found in a different strain of *S*. *suis,* and there is an additional example of Lla-1 found in a different strain of *L. lactis.*

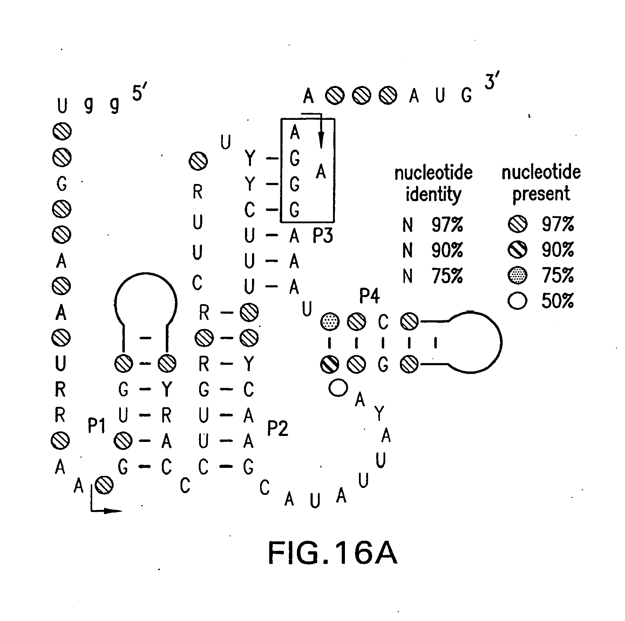

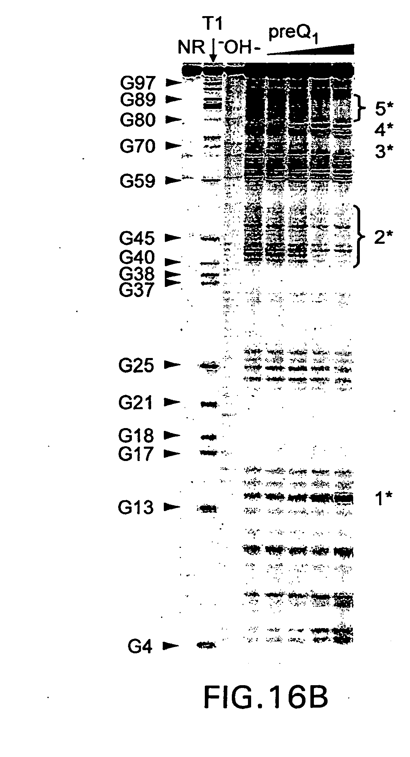

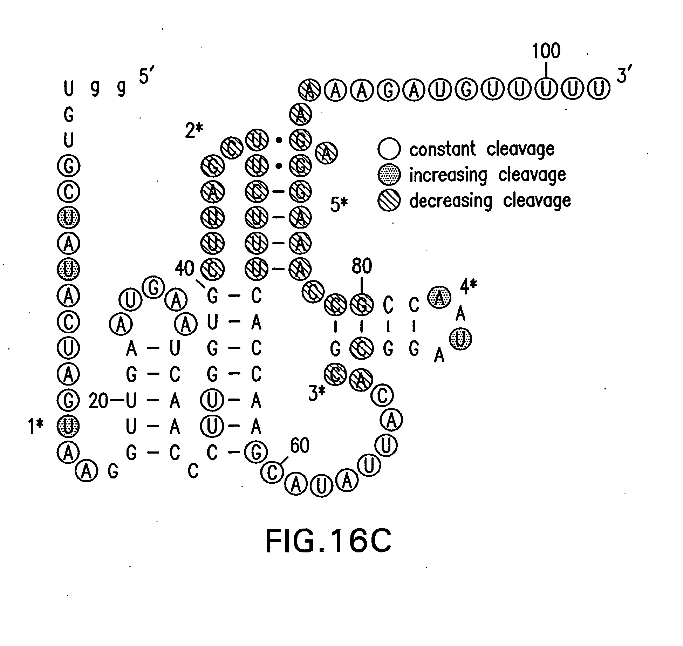

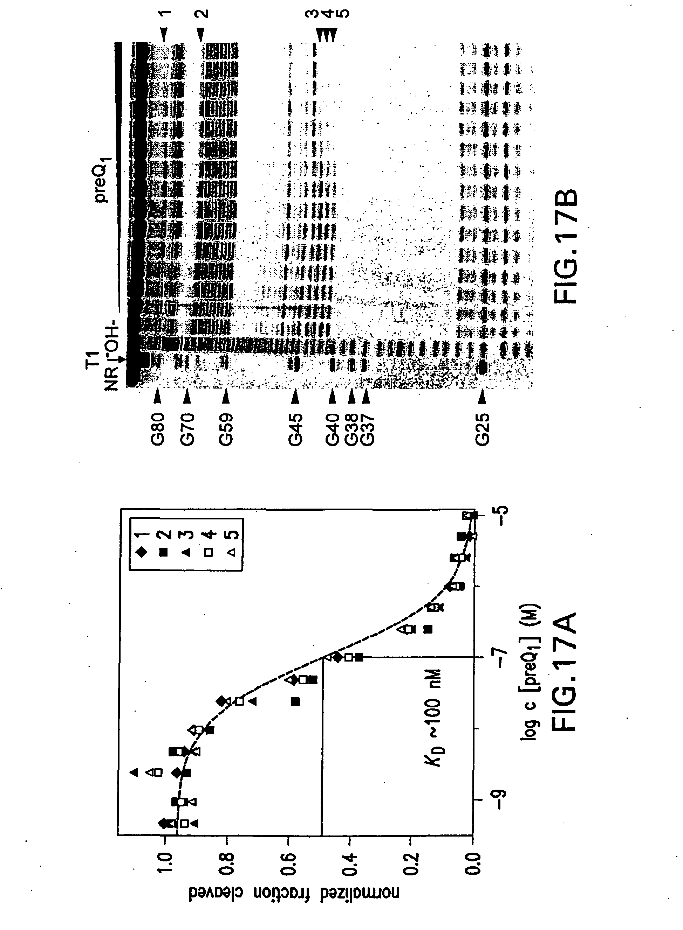

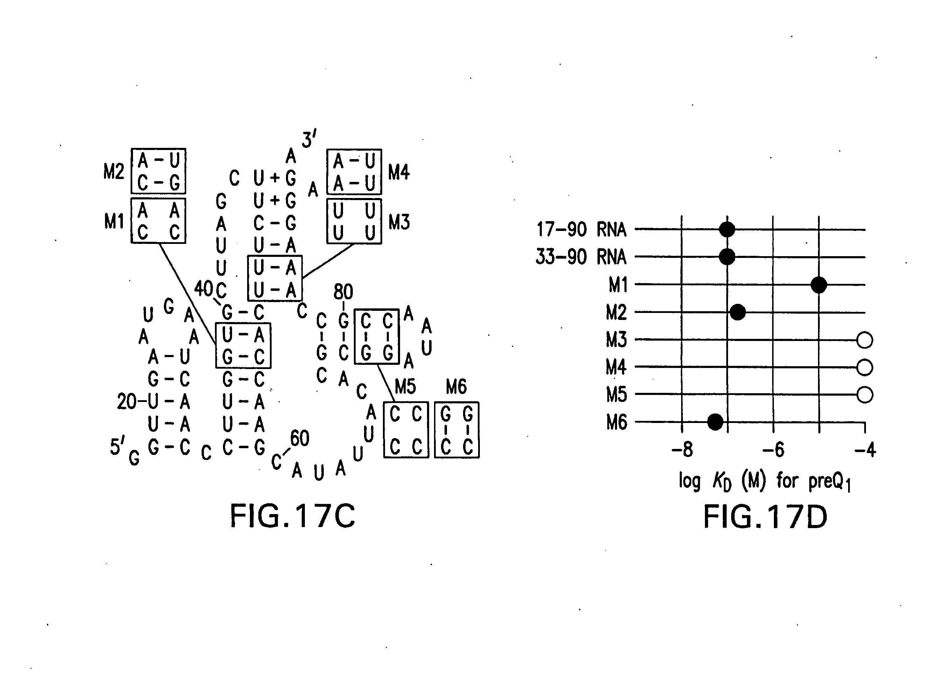

Figure 16 shows structural modulation of COG4708 RNA by preQ₁. (*A*) Consensus sequence and secondary structure model for the COG4708 RNA motif. Non-conserved nucleotides are represented by a solid black line or open circles, and conserved pairing elements P1-P4 are indicated. Arrows identify nucleotides present in a truncated construct that retain aptamer function (see Fig. 17). The shaded nucleotides comprise a conserved Shine-Dalgarno (SD) sequence. (*B*) Spontaneous cleavage products of the *S*. *pneumoniae* R6 preQ₁-II aptamer representative (1-103 RNA) separated by denaturing PAGE. NR, T1, ⁻OH, and (-) represent no reaction, partial RNase T1 digest, partial alkaline digest, and no ligand, respectively. Selected RNase T1 cleavage products (cleaves 3' of G residues) are identified on the left. Numbered asterisks on the right indicate locations of structural modulation in response to preQ₁. (*C*) Patterns of spontaneous cleavage of the 1-103 RNA mapped onto the predicted secondary structure. Lowercase g notations identify guanosine residues added to improve transcription and numbered asterisks identify the numbered regions depicted in *B*.
Figure 17 shows ligand binding affinity of wild-type and mutant 17-90 RNAs. (*A*) Plot of the modulation of spontaneous RNA cleavage during in-line probing at several internucleotide linkages of 17-90 RNA versus the logarithm of the molar concentration of preQ₁. (*B*) In-line probing gel of 17-90 RNA incubated with concentrations of preQ₁ from left (0.5 nM) to right (10 µM). Arrows on the right point to bands used to generate binding curve in *A*. Other notations are as described in the legend to Fig. 16B. (C) The sequence and structure of 17-90 RNA with disruptive and compensatory mutations M1 through M6 identified. (*D*) *K*_{D} values for preQ₁ exhibited by 17-90 RNA, 33-90 RNA and mutant 17-90 RNAs M1 through M6 as designated in *C*. Open circles indicate that the actual *K*_{D} value is higher than 100 µM.

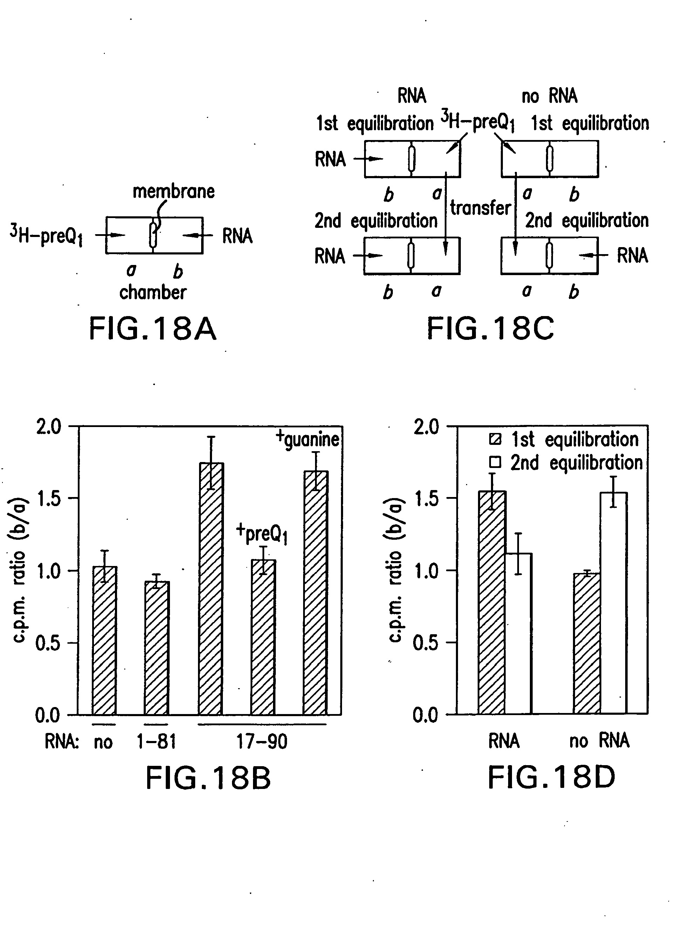

Figure 18 shows analysis of metabolite binding by the 17-90 RNA aptamer using equilibrium dialysis. (*A*) Loading of the equilibrium dialysis apparatus with ³H-preQ₁ and RNA. Chambers *a* and *b* are separated by a 5,000 MOCO membrane. (*B*) Results of equilibrium dialysis with chamber *b* lacking RNA (-), a truncated and inactive derivative of 17-90 RNA (1-81) and 17-90 RNA. For the competition experiments, excess unlabeled preQ₁ and guanine were added to previously equilibrated apparatuses containing 17-90 RNA and ³H-preQ₁. Error bars represent the standard deviation of three independent experiments. (*C*) Method of equilibrium dialysis to determine whether unshifted ³H-labeled material can be bound by RNA. For the 1^{st} equilibration, H³-preQ₁ is equilibrated with 17-90 RNA (left) or in the absence of RNA (right). Aliquots removed from each side of the apparatus to assess tritium distribution. For the 2^{nd} equilibration, buffer from the chamber lacking RNA (chamber a that contains unshifted H³-label) was transferred to a new apparatus and reequilibrated with chamber *b* containing 17-90 RNA. Aliquots again are removed to reassess tritium distribution. (*D*) Results of the equilibrium dialysis experiment described in C. Error bars represent the standard deviation of three independent experiments.

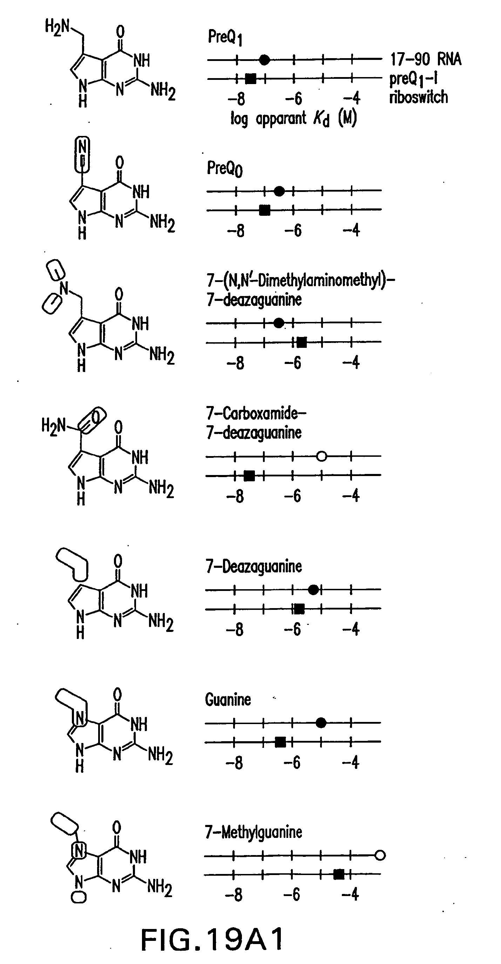

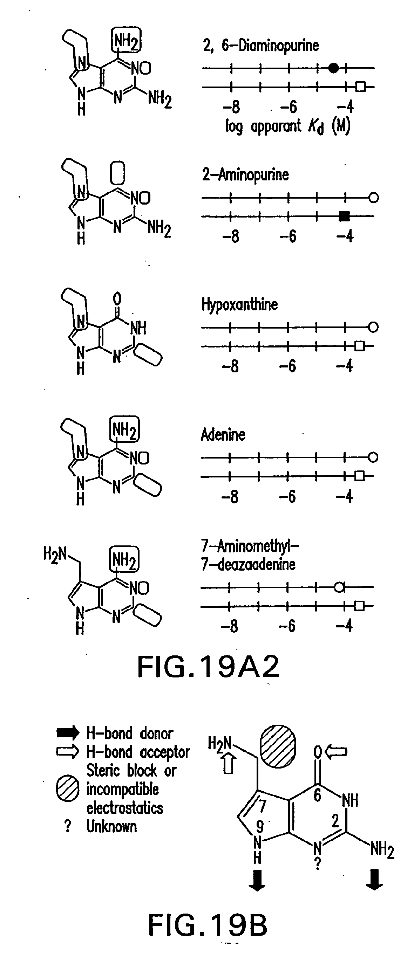

Figure 19 shows molecular recognition analysis of the 17-90 preQ₁-II aptamer. (*A*) Comparison of the molecular recognition characteristics of 17-90 RNA (circles) and the preQ₁-I riboswitch (squares) for preQ₁ and ligand analogs. *K*_{D} values for the preQ₁-I riboswitch were published previously (Roth et al. 2007). Shading on chemical structures identifies differences between the analog and preQ₁. Open symbols designate the highest concentrations of ligand tested in in-line probing assays, indicating that the *K*_{D} values are higher than this concentration. (*B*) Summary of the molecular recognition contacts inferred from the data in *A*.

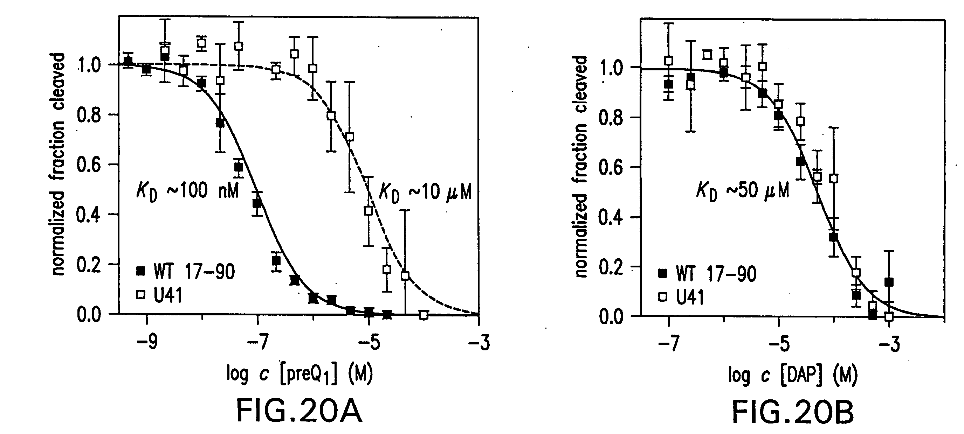

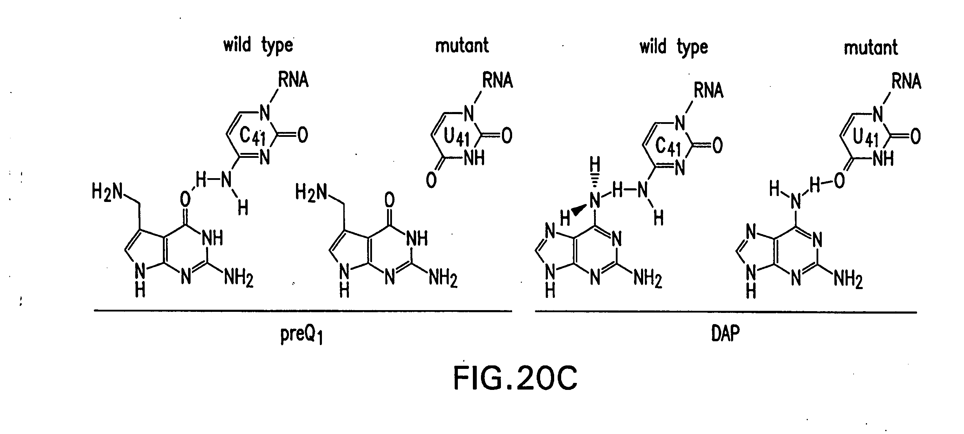

Figure 20 shows altered ligand discrimination by a mutant 17-90 aptamer. Plot depicting the modulation of RNA structure by in-line probing of wild-type (C41) and mutant (U41) 17-90 RNA at various concentrations of (*A*) preQ₁ and (*B*) DAP. Points represent average normalized fraction cleaved for several modulating bands and error bars represent the standard deviation of this average. (*C*) Possible aptamer-ligand interaction that is consistent with the molecular recognition model and mutational analysis data for the preQ₁-II aptamer from *S*. *pneumoniae* R6.

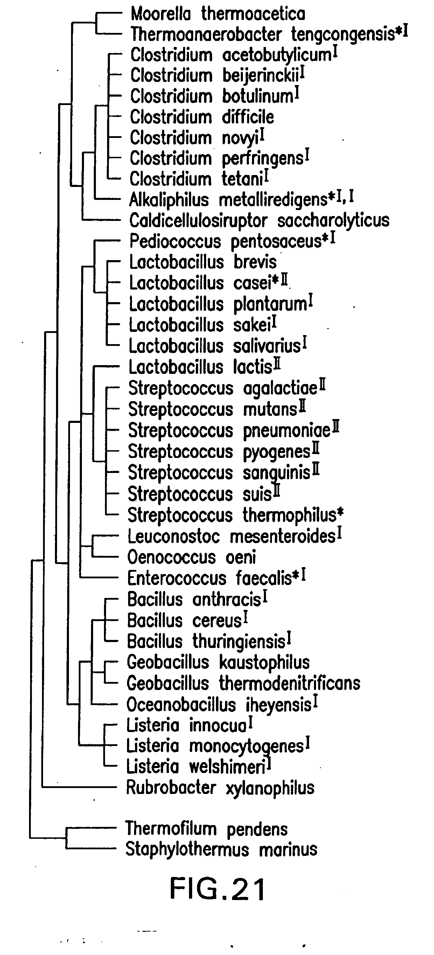

Figure 21 shows taxonomy tree of species carrying genes for COG4708 proteins. Species containing two copies of the gene are marked by an asterisk. Species where the gene encoding COG4708 is preceded by the preQ₁-I riboswitch are indicated by I, species where the gene is preceded by a preQ₁-II motif are indicated by II.

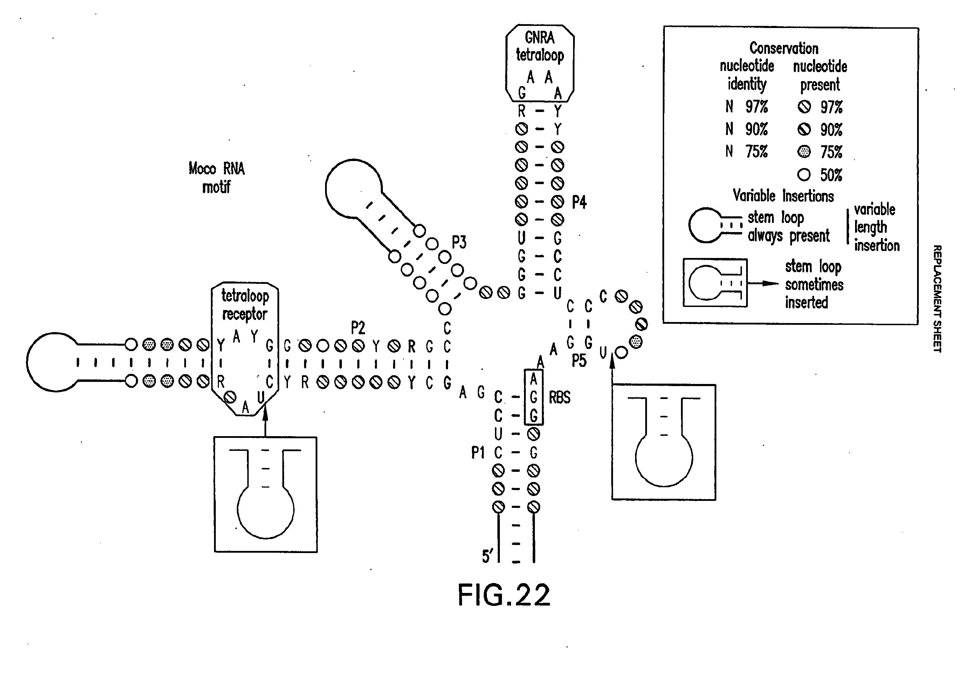

Figure 22 shows consensus sequence and secondary structure model of the most common form of Moco RNA motif derived from 176 representatives. R represents A or G and Y represents C or U. Boxed nucleotides denoted RBS are predicted to be the ribosome binding site for the adjacent ORF in some Moco RNA representatives.

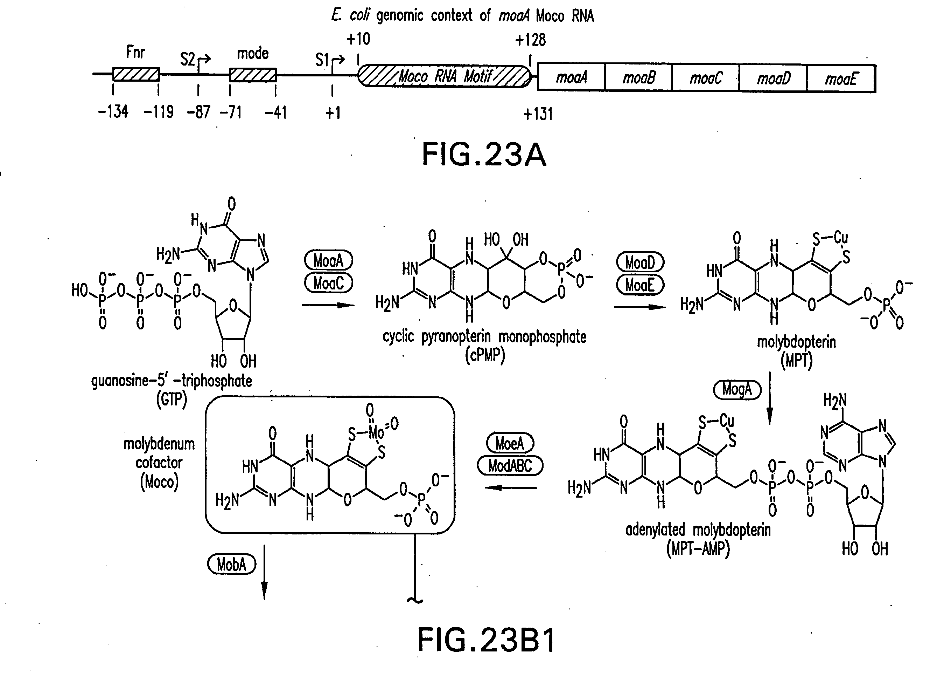

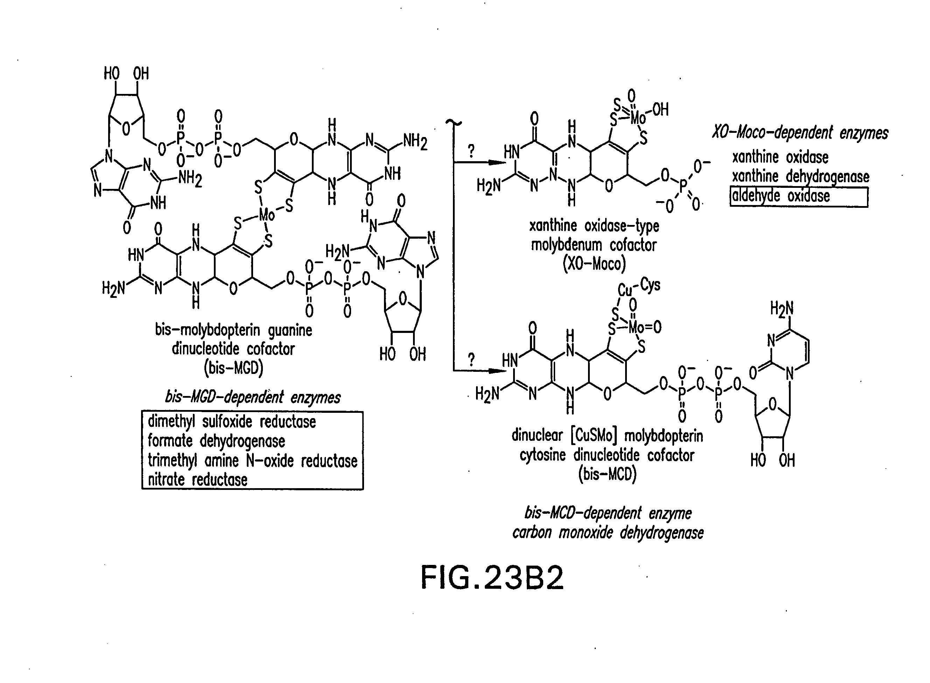

Figure 23 shows (a) schematic representation of the *moaABCDE* operon of *E*. *coli.* Nucleotide numbers for the genome region upstream of the *moaA* ORF are established by defining the first transcription start site (S1) as +1 and a second transcription start site (S2) as -87. The approximate locations of the Fnr and ModE binding sites are indicated with filled boxes, and the region designated as the Moco RNA motif begins and ends with the terminal nucleotides of P1 (Fig. 22). Numbering system and locations of various features are as reported previously (Anderson *et al.,* 2000). (b) Pathway for molybdenum cofactor biosynthesis in eubacteria (Schearz, 2005). Proteins with abbreviated designations are enzymes in the pathway except for ModABC, which is an ABC-type molybdate transporter. Structural studies (Sanihvili *et al.,* 2004; Bader *et al.,* 2004) indicate that it is similar to MogA and therefore is likely to be involved in molybdopterin biosynthesis. Question marks indicate that the biosynthetic enzymes for the conversion are not known. Other proteins listed are enzymes that use Moco derivatives as coenzymes. Proteins whose coding regions are located downstream and near a Moco RNA motif in at least one organism are highlighted with black shading.

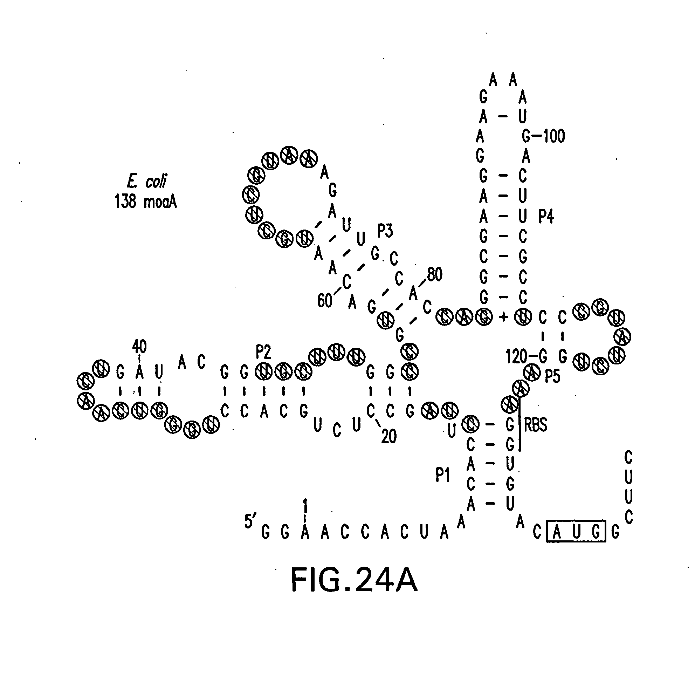

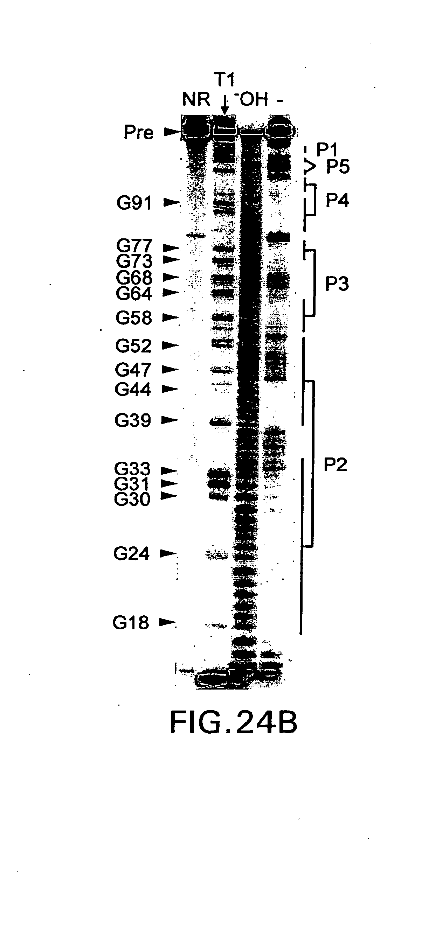

Figure 24 shows in-line probing assay of a Moco RNA. (a) Sequence of the 138 *moaA* RNA of *E. coli* depicted to conform to the secondary structure model predicted from comparative sequence analysis data (Fig. 22a). Nucleotides shaded in gray undergo greater rates of spontaneous 3' phosphodiester cleavage (from data depicted in b), which typically indicates an elevated level of structural flexibility relative to internucleotide linkages present in stable secondary or tertiary structures. The bar identifies purine nucleotides predicted to serve as a ribosome binding site and the translation start codon for *moaA* is boxed. (b) Gel image of the products generated during an in-line probing assay of the 138 *moaA* RNA from *E. coli.* Lanes 1-3 contain the 5' ³²P-labeled precursor RNA (Pre) loaded on the gel either without incubation (no reaction, NR), partially digested by T1 RNase (T1), or subjected to partial alkaline digestion (⁻OH). Lane 4 was loaded with labeled precursor RNA after incubation under in-line probing conditions in the absence (-) of a possible ligand compound. Indicated are regions of the gel containing radiolabeled RNA fragments cleaved after nucleotides predicted to be base paired.

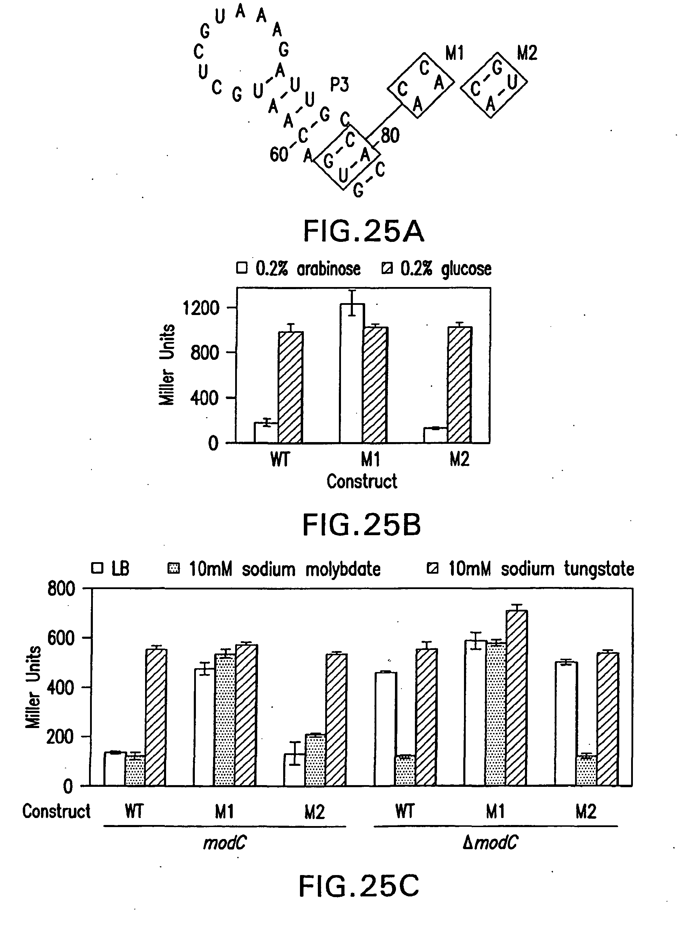

Figure 25 shows Moco RNA from *E. coli* requires Moco biosynthesis for gene repression. (a) Depiction of the P3 region of the *E. coli* 149 *moaA* RNA whose corresponding DNA template was fused to a β-galactosidase reporter gene. M1 and M2 carry nucleotide changes as indicated relative to the wild-type (WT) RNA. (b) Expression of β-galactosidase reporter mRNAs fused to the WT and mutant Moco RNAs under different Moco concentrations. Moco concentrations are elevated or reduced by the presence or absence of arabinose in the growth medium, respectively, due to the presence of a transgenic *moa* operon controlled by an arabinose-inducible promoter. (c) Dependence of expression of the reporter genes described above on the availability of molybdate or tungstate in cells. The presence (*modC*) or absence (Δ*modC*) of functional molybdate transporter protein in the *E. coli* strain used for the analysis is indicated. Cells were grown in LB containing 0.2% arabinose. Reporter gene expression assays were performed in triplicate and their mean values were plotted, with error bars indicating standard deviation for the replicates.

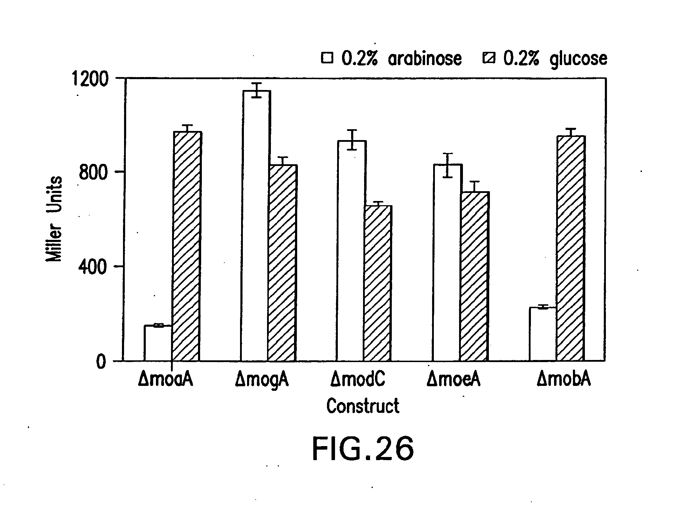

Figure 26 shows the effects of knockouts of various genes in the *E. coli* Moco biosynthetic pathway on expression of a β-galactosidase reporter gene fused to a DNA template for the WT 149 *moaA* RNA. Each transgenic strain carries a plasmid encoding *E. coli moaABCDE* under the control of an arabinose-inducible promoter.

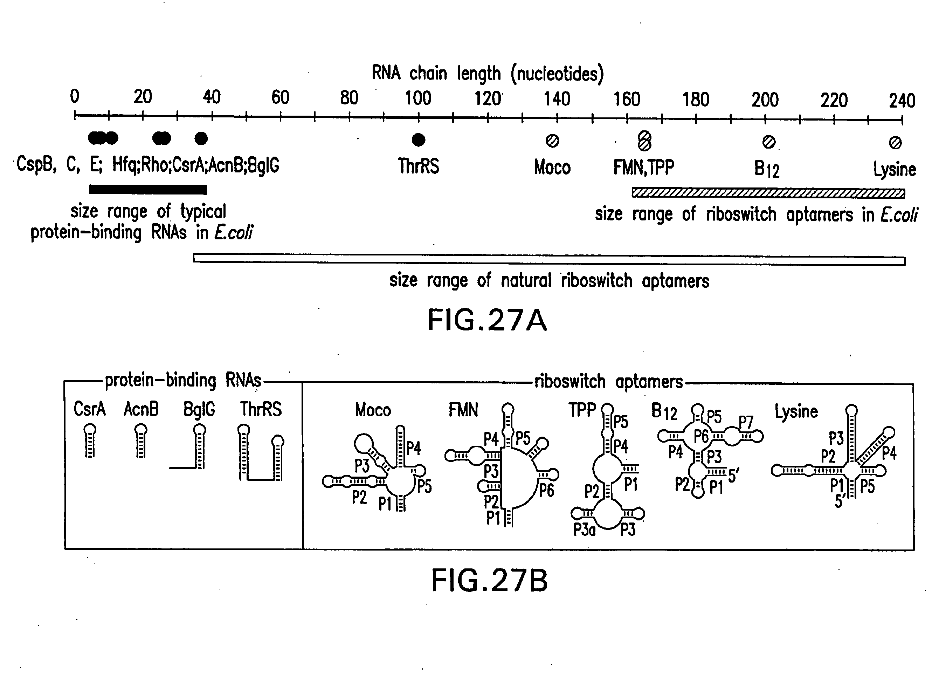

Figure 27 shows Moco RNA size and structure is similar to other riboswitch aptamers from *E. coli.* (a) Comparison of the length of the *E. coli moaA* Moco aptamer with the lengths of regulatory RNA elements from *E. coli* that either are known to be bound by proteins or function as riboswitch aptamers. RNA targets for ribosomal proteins are not included in this analysis. (b) Secondary structure models for the RNAs depicted in a. RNAs not shown do not have secondary structures established.

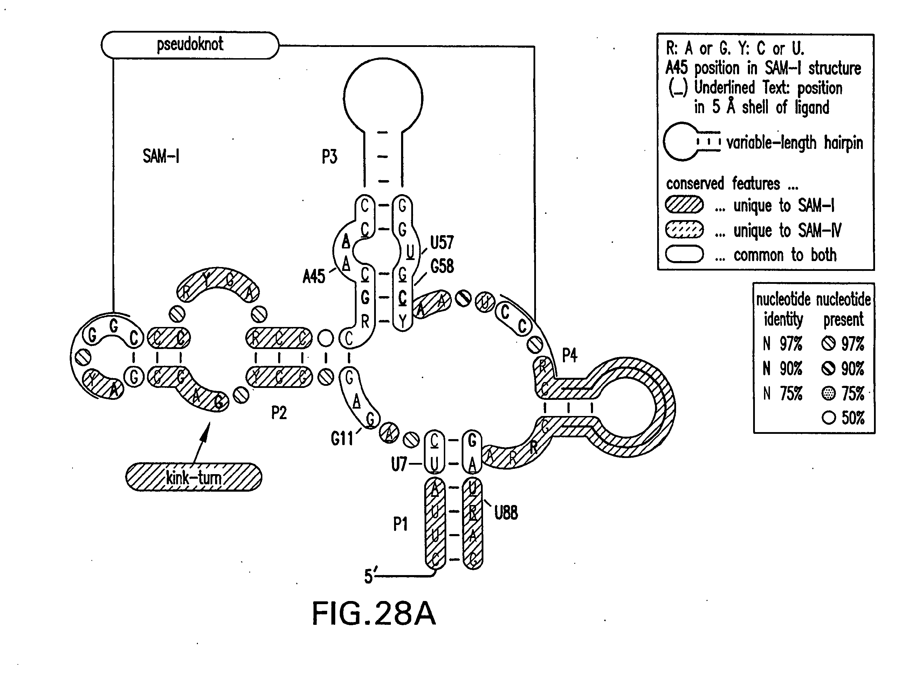

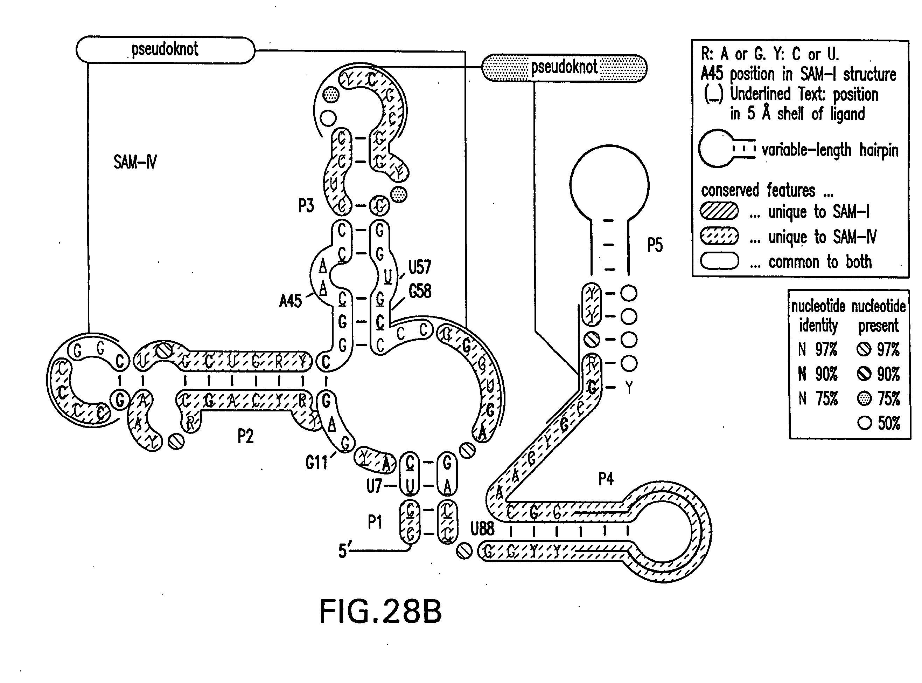

Figure 28 shows comparison of SAM-I and SAM-IV motifs. Stems are labeled P1-P5. P5 in SAM-IV is often missing, but its 5' side involved in the pseudoknot is always present. Nucleotide positions in the published SAM-I 3-D structure (Montange and Batey 2006) within 5Å of the ligand are depicted in bold, as are their putative corresponding positions in SAM-IV. Six positions proposed to directly contact the ligand (e.g., A45) are labeled in both motifs according to their SAM-I numbering (Montange and Batey 2006). Conserved features (nucleotide identities, bulges and stems) are shaded to indicate that they are common to both motifs (yellow), unique to SAM-I (pink) or unique to SAM-IV (blue).

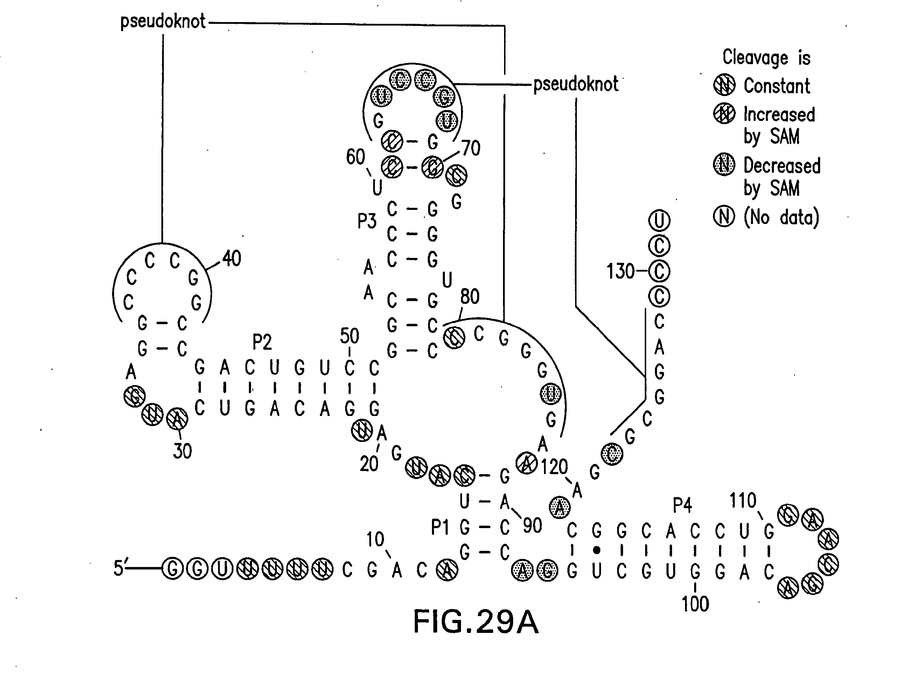

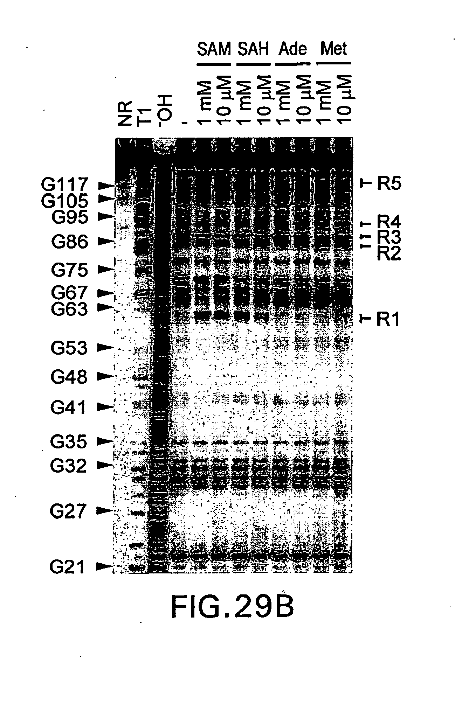

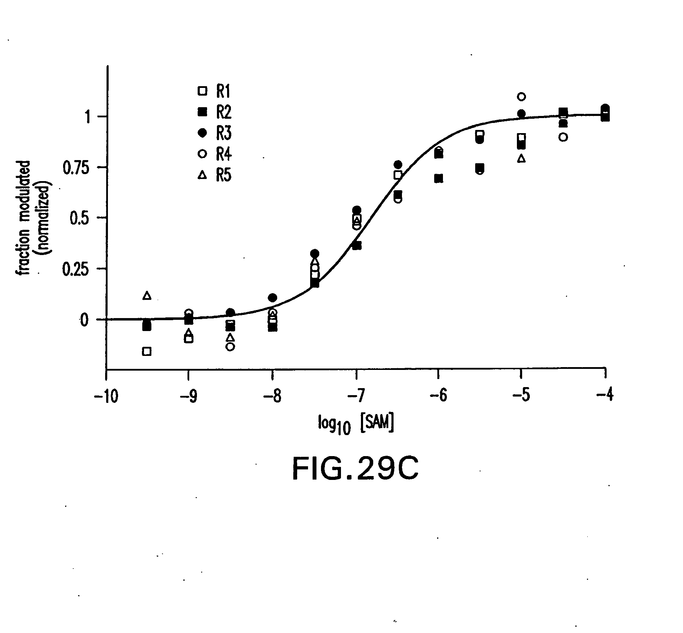

Figure 29 shows SAM-IV RNA selectively binds SAM. *(A)* Sequence and inferred secondary structure of 132 *Sc* RNA. *(B)* In-line probing gel. NR = no reaction (RNA only), T1 = partial RNase T1 digest (cleaves 3' to guanosyl residues), ⁻OH = partial alkaline digest (cleaves all internucleotide linkages), (-) = no compound was added to reaction. SAM, SAH, Ade (adenosine) and Met (methionine) were added as designated. G21-G117: identification of selected bands corresponding to cleavage agter G residues. R1-R5: regions undergoing ligand-mediated modulation. *(C)* Modulating regions (R1-R5) were quantitated from a gel (not shown) with multiple SAM concentrations, and normalized to the range 0 (no SAM) to 1 (saturating SAM concentration). The apparent K_{D} is that whose theoretical two-state binding curve best fits the data shown. The theoretical curve for *K*_{D}= 150 nM is shown.

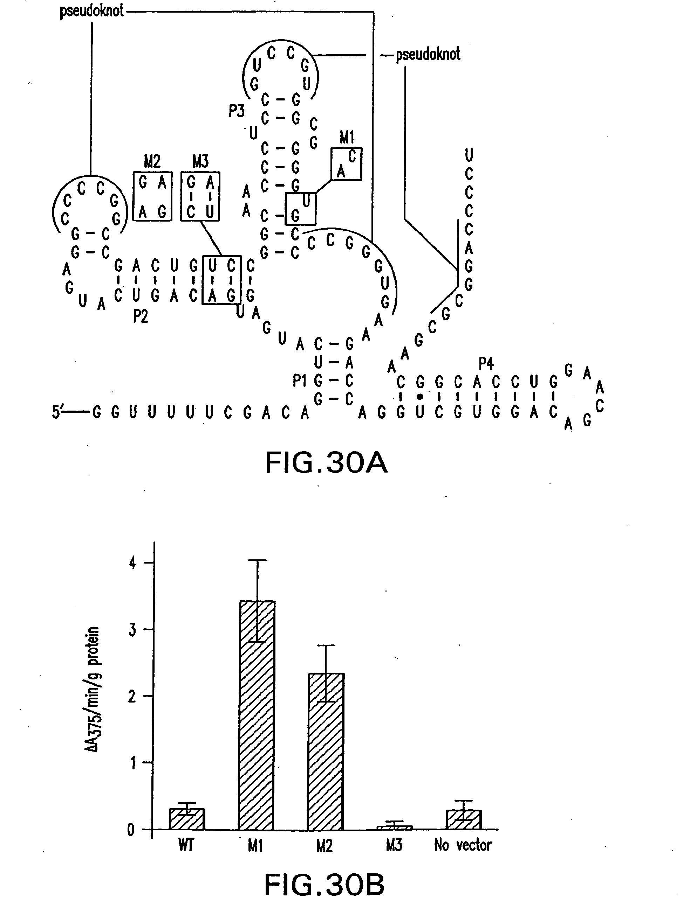

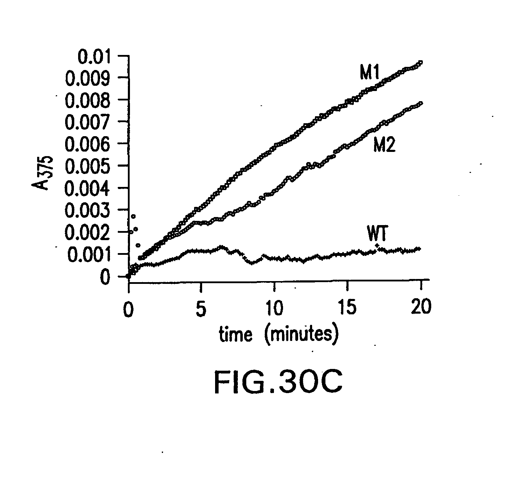

Figure 30 shows SAM-IV is a genetic regulatory element. *(A)* The mutations tested (M1, M2, M3) are shown in the aptamer secondary structure, although the entire intergenic region was used in the reporter assay (see Materials and methods). *(B)* XylE reporter activity was quantitated as change in A₃₇₅ per minute (measured over 20 minutes) per gram of total protein. Cell strains were: "WT" = wild-type IGR, "M1"-"M3" = mutated IGR, "no vector" = cells lack the vector containing the *xylE* reporter gene. (C) Absorbance vs. time plots for three typical experiments selected from B.

### DETAILED DESCRIPTION OF THE INVENTION

The disclosed methods and compositions can be understood more readily by reference to the following detailed description of particular embodiments and the Examples included therein and to the Figures and their previous and following description.

Messenger RNAs are typically thought of as passive carriers of genetic information that are acted upon by protein- or small RNA-regulatory factors and by ribosomes during the process of translation. It was discovered that certain mRNAs carry natural aptamer domains and that binding of specific metabolites directly to these RNA domains leads to modulation of gene expression. Natural riboswitches exhibit two functions that are not typically associated with natural RNAs. First, the mRNA element can adopt distinct structural states wherein one structure serves as a precise binding pocket for its target metabolite. Second, the metabolite-induced allosteric interconversion between structural states causes a change in the level of gene expression by one of several distinct mechanisms. Riboswitches typically can be dissected into two separate domains: one that selectively binds the target (aptamer domain) and another that influences genetic control (expression platform). It is the dynamic interplay between these two domains that results in metabolite-dependent allosteric control of gene expression.

Distinct classes of riboswitches have been identified and are shown to selectively recognize activating compounds (referred to herein as trigger molecules). For example, coenzyme B₁₂, glycine, thiamine pyrophosphate (TPP), and flavin mononucleotide (FMN) activate riboswitches present in genes encoding key enzymes in metabolic or transport pathways of these compounds. The aptamer domain of each riboswitch class conforms to a highly conserved consensus sequence and structure. Thus, sequence homology searches can be used to identify related riboswitch domains. Riboswitch domains have been discovered in various organisms from bacteria, archaea, and eukarya.

### A. General Organization of Riboswitch RNAs

Bacterial riboswitch RNAs are genetic control elements that are located primarily within the 5'-untranslated region (5'-UTR) of the main coding region of a particular mRNA. Structural probing studies (discussed further below) reveal that riboswitch elements are generally composed of two domains: a natural aptamer (T. Hermann, D. J. Patel, Science 2000, 287, 820; L. Gold, et al., Annual Review of Biochemistry 1995, 64, 763) that serves as the ligand-binding domain, and an 'expression platform' that interfaces with RNA elements that are involved in gene expression (*e.g*. Shine-Dalgarno (SD) elements; transcription terminator stems). These conclusions are drawn from the observation that aptamer domains synthesized *in vitro* bind the appropriate ligand in the absence of the expression platform (see Examples 2, 3 and 6 of U.S. Application Publication No. 2005-0053951). Moreover, structural probing investigations suggest that the aptamer domain of most riboswitches adopts a particular secondary- and tertiary-structure fold when examined independently, that is essentially identical to the aptamer structure when examined in the context of the entire 5' leader RNA. This indicates that, in many cases, the aptamer domain is a modular unit that folds independently of the expression platform (see Examples 2, 3 and 6 of U.S. Application Publication No. 2005-0053951).

Ultimately, the ligand-bound or unbound status of the aptamer domain is interpreted through the expression platform, which is responsible for exerting an influence upon gene expression. The view of a riboswitch as a modular element is further supported by the fact that aptamer domains are highly conserved amongst various organisms (and even between kingdoms as is observed for the TPP riboswitch), (N. Sudarsan, et al., RNA 2003, 9, 644) whereas the expression platform varies in sequence, structure, and in the mechanism by which expression of the appended open reading frame is controlled. For example, ligand binding to the TPP riboswitch of the *tenA* mRNA of *B. subtilis* causes transcription termination (A. S. Mironov, et al., Cell 2002, 111, 747). This expression platform is distinct in sequence and structure compared to the expression platform of the TPP riboswitch in the *thiM* mRNA from *E*. *coli,* wherein TPP binding causes inhibition of translation by a SD blocking mechanism (see Example 2 of U.S. Application Publication No. 2005-0053951). The TPP aptamer domain is easily recognizable and of near identical functional character between these two transcriptional units, but the genetic control mechanisms and the expression platforms that carry them out are very different.

Aptamer domains for riboswitch RNAs typically range from ∼70 to 170 nt in length (Figure 11 of U.S. Application Publication No. 2005-0053951). This observation was somewhat unexpected given that *in vitro* evolution experiments identified a wide variety of small molecule-binding aptamers, which are considerably shorter in length and structural intricacy (T. Hermann, D. J. Patel, Science 2000, 287, 820; L. Gold, et al., Annual Review of Biochemistry 1995, 64, 763; M. Famulok, Current Opinion in Structural Biology 1999, 9, 324). Although the reasons for the substantial increase in complexity and information content of the natural aptamer sequences relative to artificial aptamers remains to be proven, this complexity is believed required to form RNA receptors that function with high affinity and selectivity. Apparent K_{D} values for the ligand-riboswitch complexes range from low nanomolar to low micromolar. It is also worth noting that some aptamer domains, when isolated from the appended expression platform, exhibit improved affinity for the target ligand over that of the intact riboswitch. (∼10 to 100-fold) (see Example 2 of U.S. Application Publication No. 2005-0053951). Presumably, there is an energetic cost in sampling the multiple distinct RNA conformations required by a fully intact riboswitch RNA, which is reflected by a loss in ligand affinity. Since the aptamer domain must serve as a molecular switch, this might also add to the functional demands on natural aptamers that might help rationalize their more sophisticated structures.

### B. Cyclic di-GMP Riboswitch (GEMM Motif)

Cyclic di-GMP-responsive riboswitches containing a GEMM motif (which can also be referred to as cyclic di-GMP riboswitches or GEMM riboswitches) include two adjacent hairpins (paired regions) designated P1 and P2 (Figures 1 and 3). P1 is highly conserved in sequence and structure, and consists of 2- and 6-base-pair stems separated by a 3-nucleotide internal loop and capped by a terminal loop. The internal loop is highly conserved, and the terminal loop is almost always a GNRA tetraloop (Hendrix 1997). The P1 stem exhibits considerable evidence of covariation at several positions, and is highly conserved in structure over a wide range of bacteria. This fact, and the more modest covariation and variable-length stems of P2, provide strong evidence that GEMM functions as a structured RNA. Example 2 demonstrates that GEMM motifs function in riboswitches. The sequence linking P1 and P2 is virtually always AAA, with only 2 exceptions in 322 examples.

The P2 hairpin shows more modest conservation than P1. When the P1 tetraloop is GAAA, a GNRA tetraloop receptor usually appears in P2. This receptor is often the well-known 11-nt motif, which might be favored by GAAA loops, but some sequences could be novel tetraloop receptors. When P1 has a GYRA tetraloop, the receptor-like sequence is almost never present, although a bulge nearer the P2 base is sometimes found (Figure 2).

GEMM RNAs conform to one of two similar architectures termed Type 1 and Type 2 (Figure 3B). Both types carry two base paired regions (P1 and P2) that exhibit extensive covariation in the 503 representatives identified (Weinberg 2007). Type 1 RNAs (303 examples) have a GNRA tetraloop (Heus 1991) on P1 with a purine (R) at the second position. The presence of a GRRA sequence correlates with the presence of a tetraloop receptor at the extremity of P2, which is known to dock with this tetraloop type (Costa 1995). In contrast, Type 2 RNAs (171 examples) have a pyrimidine (Y) at the second position of the tetraloop, and in some instances include a structure on P2 that favors docking with GYRA tetraloops (Costa 1995). There are an additional 29 unassigned representatives, of which 24 carry a GNRA tetraloop flanked by non-paired nucleotides and five that lack a tetraloop sequence.

GNRA tetraloops and their receptors commonly occur in structured RNAs, and have previously been observed in riboswitch aptamers (Regulski 2008). However, the variability in sequence and structure at the tips of P1 and P2 hairpins suggests that any binding site for cyclic di-GMP likely is located in the central or basal portions that carry the most conserved features.

Many instances of GEMM include a rho-independent transcription terminator hairpin. The 5' side of the terminator stem often overlaps (and presumably competes with) the 3' side of the P2 stem (Figure 2B). If GEMM is a riboswitch, ligand binding can stabilize the proposed P1 and P2 structure, thus preventing the competing transcription terminator from forming. In this model, higher ligand concentrations increase gene expression. One-third of GEMM representatives in δ-proteobacteria, and some in other taxa, are in a "tandem" arrangement, wherein one instance appears 3' and nearby to another in the same UTR. Such arrangements of regulatory RNAs are implicated in more sophisticated control of gene expression than is permitted by a simple regulatory RNA configuration (Sudarsan 2006; Welz 2007; Mandal 2006).

GEMM is widespread in bacteria and appears to have a highly conserved sequence and structure suggestive of a function that imposes substantial biochemical constraints on the putative RNA. 322 GEMM sequences were found in both Gram-positive and Gram-negative bacteria. It is common in δ-proteobacteria, particularly in Geobacter and related genera. Within γ-proteobacteria, it is ubiquitous in the Alteromonadales and Vibrionales. It is also common in certain orders of the phyla Firmicutes and Plantomycetes. Prominent pathogens with GEMM include the causative agents of cholera and anthrax.

Out of 309 GEMM instances where sequence data includes gene annotations, GEMM is in a 5' regulatory configuration to a gene in 297 cases, implying a *cis-*regulatory role. Genes presumably regulated by GEMM display a wide range of functions, but most genes relate to the extracellular environment or to the membrane, and many are related to motility.

An understanding of the biological role of GEMM can shed light on the broad variety of microbial processes that it appears to regulate. In fact, GEMM is implicated in two systems that are already the object of several studies, in the species *Vibrio cholerae* and in *Geobacter sulfurreducens. Vibrio cholerae* causes cholera in humans, but spends much of its lifecycle in water, where it can adhere to chitin-containing exoskeletons of many crustaceans. Chitin, a polymer of GlcNAc (N-acetylglucosamine), has been shown to affect expression of many *V. cholerae* genes (Meibom 2004). GEMM appears to regulate two of these chitin-induced genes. The first, *gbpA,* is important for adhering to chitin beads (Meibom 2004) and human epithelial cells (Kirn 2005), as well as infection of mice (Kirn 2005).

The second chitin-induced gene is *tfoX*^{VC}. Remarkably, chitin induces natural competence in *V. cholerae* (Meibom 2005), and *tfoX*^{VC} expression is essential for this competence. *V. cholerae* has two genes that match the CDD models COG3070 and pfam04994 that correspond to separate *tfoX* domains. Both domains yield RPSBLAST (Schaffer 2001) E-values better than 10⁻²⁵. One of these is *tfoX*^{VC} (locus VC1153). GEMM appears to regulate the other, which is referred to as *tfoX*^{GEMM} (VC1722). Thus, in *V. cholerae* and related bacteria, GEMM might participate in chitin-induced competence, or even regulate competence in environments not containing elevated chitin concentrations.

*Geobacteria sulfurreducens* and related δ-proteobacteria can generate ATP by oxidizing organic compounds, using metal ions such as Fe(III) as electron acceptors (Methé 2003). GEMM is associated with pili assembly genes in *Geobacter* species. Pili in *G. sulfurreducens* have been shown to conduct electricity (Reguera 2005), and are thus a part of the process of reducing metal ions.

Moreover, GEMM appears to regulate seven cytochrome c genes in G. *sulfurreducens.* Although this bacteria has 111 putative cytochrome c genes, 5 of the 7 GEMM-associated genes have been identified in previous studies, and might have special roles. OmcS (Outer-Membrane Cytochrome S) is one of two proteins that are highly abundant on the outer membrane of *G*. *sulfurreducens,* and is required for reducing insoluble Fe(III) oxide, but not for soluble Fe(III) citrate (Mehta 2005). OmcG and OmcH are necessary for production of OmcB, an essential cytochrome c in many conditions (Kimm 2006). OmcA and OmcT are associated with OmcG, OmcH or OmcS. Only four other Omc annotations remain in *G*. *sulfurreducens* that have no direct GEMM association: OmcB, OmcC, OmcE and OmcF.

Unlike known riboswitches, GEMM is associated with a great diversity of gene functions (Table 2 in Example 1). This observation indicates that the GEMM riboswitch is not serving as a typical feedback sensor for control of a metabolic pathway. Rather, GEMM senses a second-messenger molecule involved in signal transduction or possibly cell-cell communication (Bassler 2006). Example 2 demonstrates that the secondary-messenger trigger molecule of the GEMM motif id cyclic di-GMP. In this way, different bacteria use GEMM and its signaling molecule to control different processes. The fact that many GEMM-associated genes encode signal transduction domains indicates a mechanism by which many of the signal transduction proteins are regulated. Example 2 demonstrates that GEMM RNAs indeed serve as aptamer components of a new-found riboswitch class.

### C. S-adenosylhomocysteine Riboswitch

S-adenosylhomocysteine-responsive riboswitches containing an SAH motif (which can also be referred to as SAH riboswitches) include a branched stem structure (Figures 1 and 9A). The SAH motif is highly conserved in sequence and structure (Figures 1 and 9A), showing covariation within predicted stem regions, including modular and variable-length stems. The SAH riboswitch is described in Examples 1 and 3. The SAH motif is found in a 5' regulatory configuration to genes related to SAH (*S-*adenosylhomocysteine) metabolism, primarily in β- and some γ-proteobacteria, and especially the genus *Pseudomonas.* SAH is a part of the *S*-adenosylmethionine (SAM) metabolic cycle, whose main components include the amino acid methionine and its derivative homocysteine. SAH is a byproduct of enzymes that use SAM as a cofactor for methylation reactions. Typically, SAH is hydrolyzed into homocysteine and adenosine. Homocysteine is then used to synthesize methionine, and ultimately SAM.

High levels of SAH are toxic to cells because SAH inhibits many SAM-dependent methyltransferases (Ueland 1982). Therefore cells likely need to sense rising SAH concentrations and dispose of this compound before it reaches toxic levels. The genes that the SAH motif associates with are *S*-adenosylhomocysteine hydrolase (*ahcY*), cobalamin-dependent methionine synthase (*metH*) and methylenetetrahydrofolate reductase (*metF*), which synthesizes a methyl donor used in methionine synthesis. This genetic arrangement of the SAH motif and its high degree of conservation are consistent with a role in sensing SAH and activating the expression of genes whose products are required for SAH destruction. Indeed, biochemical and genetic evidence indicate that this motif is an SAH-sensing riboswitch.

### D. PreQ₁ Riboswitch (preQ₁-II or COG4708 Motif)

PreQ₁-responsive riboswitches containing a preQ₁-II (which can also be referred to as a COG4708 motif). Such riboswitches can also be referred to as preQ₁ riboswitches, preQ₁-II riboswitches, or COG4708 riboswitches) are found upstream of COG4708 genes in some species of *Streptococcus* and in *Lactococcus lactis,* although some instances of the COG4708 gene family in *Streptococcus* lack the putative RNA motif. COG4708 genes are predicted to encode membrane proteins. The preQ₁-II riboswitch is described in Examples 1 and 4.

Although the COG4708 motif is highly constrained phylogenetically and has only six unique sequences, it shows covariation, modular stems, and variable-length stems (Figures 1, 15 and 16A). The motif has a pseudoknot that overlaps the Shine-Dalgarno sequences of COG4708 genes, which shows that the motif encodes a cis-regulator of these genes.

A riboswitch that senses the modified nucleobase preQ₁ was recently characterized (Roth 2007). Since this riboswitch is associated with COG4708, it was proposed that COG4708 is a transporter of a metabolite related to preQ₁. Therefore, it was considered that the COG4708 motif is also a preQ₁-sensing riboswitch. Experiments have supported this. The COG4708 motif shares no similarity in sequence or structure with the previously characterized preQ₁-sensing riboswitch (Roth 2007).

### E. Riboswitch Regulation of Transcription Termination in Bacteria

Bacteria primarily make use of two methods for termination of transcription. Certain genes incorporate a termination signal that is dependent upon the Rho protein, (J. P. Richardson, Biochimica et Biophysica Acta 2002, 1577, 251). while others make use of Rho-independent terminators (intrinsic terminators) to destabilize the transcription elongation complex (I. Gusarov, E. Nudler, Molecular Cell 1999, 3, 495; E. Nudler, M. E. Gottesman, Genes to Cells 2002, 7, 755). The latter RNA elements are composed of a GC-rich stem-loop followed by a stretch of 6-9 uridyl residues. Intrinsic terminators are widespread throughout bacterial genomes (F. Lillo, et al., 2002, 18, 971), and are typically located at the 3'-termini of genes or operons. Interestingly, an increasing number of examples are being observed for intrinsic terminators located within 5'-UTRs.

Amongst the wide variety of genetic regulatory strategies employed by bacteria there is a growing class of examples wherein RNA polymerase responds to a termination signal within the 5'-UTR in a regulated fashion (T. M. Henkin, Current Opinion in Microbiology 2000, 3, 149). During certain conditions the RNA polymerase complex is directed by external signals either to perceive or to ignore the termination signal. Although transcription initiation might occur without regulation, control over mRNA synthesis (and of gene expression) is ultimately dictated by regulation of the intrinsic terminator. One of at least two mutually exclusive mRNA conformations results in the formation or disruption of the RNA structure that signals transcription termination. A trans-acting factor, which in some instances is a RNA (F. J. Grundy, et al., Proceedings of the National Academy of Sciences of the United States of America 2002, 99, 11121; T. M. Henkin, C. Yanofsky, Bioessays 2002, 24, 700) and in others is a protein (J. Stulke, Archives of Microbiology 2002, 177, 433), is generally required for receiving a particular intracellular signal and subsequently stabilizing one of the RNA conformations. Riboswitches offer a direct link between RNA structure modulation and the metabolite signals that are interpreted by the genetic control machinery.

Most clinical antibacterial compounds target one of only four cellular processes (Wolfson 2006). Since bacteria have well developed resistance mechanisms to protect these processes (D'Costa 2006), it is useful to discover new targets that are vulnerable to drug intervention. One type of vulnerable process is the regulation of gene expression by riboswitches (Winkler 2005). Typically found in the 5'-UTRs of certain bacterial mRNAs, members of each known riboswitch class form a structured receptor (or "aptamer")

(Mandal 2004) that has evolved to bind a specific fundamental metabolite. In most cases, ligand binding regulates the expression of a gene or group of genes involved in the synthesis or transport of the bound metabolite. Because the biochemical pathways regulated by riboswitches are often essential for bacterial survival, repression of these pathways through riboswitch targeting can be lethal.

Several antibacterial metabolite analogs function by targeting riboswitches (Sudarsan 2003; Sudarsan 2005; Woolley 1943). For example, the antibacterial thiamine analog pyrithiamine (Woolley 1943) most likely functions by targeting a thiamine pyrophosphate-binding riboswitch (Sudarsan 2005). Similarly, the antibacterial lysine analog L-aminoethylcysteine (Shiota 1958) (AEC, Fig. 1b) binds to the *lysC* riboswitch from *B. subtilis* and represses the expression of a lysC-regulated reporter gene (Sudarsan 2006). Moreover, the *lysC* riboswitch is mutated in *B. subtilis* (Lu 1991) and *Escherichia coli* (Patte 1998) strains resistant to AEC.

It is to be understood that the disclosed method and compositions are not limited to specific synthetic methods, specific analytical techniques, or to particular reagents unless otherwise specified, and, as such, can vary. It is also to be understood that the terminology used herein is for the purpose of describing particular embodiments only and is not intended to be limiting.

### Materials

Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference to each of various individual and collective combinations and permutation of these compounds can not be explicitly disclosed, each is specifically contemplated and described herein. For example, if a riboswitch or aptamer domain is disclosed and discussed and a number of modifications that can be made to a number of molecules including the riboswitch or aptamer domain are discussed, each and every combination and permutation of riboswitch or aptamer domain and the modifications that are possible are specifically contemplated unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

### A. Riboswitches

Riboswitches are expression control elements that are part of an RNA molecule to be expressed and that change state when bound by a trigger molecule. Riboswitches typically can be dissected into two separate domains: one that selectively binds the target (aptamer domain) and another that influences genetic control (expression platform domain). It is the dynamic interplay between these two domains that results in metabolite-dependent allosteric control of gene expression. Disclosed are isolated and recombinant riboswitches, recombinant constructs containing such riboswitches, heterologous sequences operably linked to such riboswitches, and cells and transgenic organisms harboring such riboswitches, riboswitch recombinant constructs, and riboswitches operably linked to heterologous sequences. The heterologous sequences can be, for example, sequences encoding proteins or peptides of interest, including reporter proteins or peptides. Preferred riboswitches are, or are derived from, naturally occurring riboswitches, such as naturally occurring cyclic di-GMP riboswitches. The riboswitch can include or, optionally, exclude, artificial aptamers. For example, artificial aptamers include aptamers that are designed or selected via in vitro evolution and/or in vitro selection. The riboswitches can comprise the consensus sequence of naturally occurring riboswitches, such as the consensus sequence of cyclic di-GMP riboswitches, SAH riboswitches, preQ₁ riboswitches, Moco riboswitches, and SAM-IV riboswitches. Consensus sequences of cyclic di-GMP riboswitches are shown in Figure 1 and Figure 3B. Examples of cyclic di-GMP riboswitches are shown in Figures 2 and 3C. Consensus sequences of SAH riboswitches are shown in Figures 1 and 9A. Consensus sequences of preQ₁-II (preQ₁-II or COG4708 motif) riboswitches are shown in Figures 1, 15 (sequence) and 16A (structure).

Disclosed is a riboswitch, wherein the riboswitch is a non-natural derivative of a naturally-occurring riboswitch. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. The disclosed riboswitches, including the derivatives and recombinant forms thereof, generally can be from any source, including naturally occurring riboswitches and riboswitches designed de novo. Any such riboswitches can be used in or with the disclosed methods. However, different types of riboswitches can be defined and some such sub-types can be useful in or with particular methods (generally as described elsewhere herein). Types of riboswitches include, for example, naturally occurring riboswitches, derivatives and modified forms of naturally occurring riboswitches, consensus riboswitches, chimeric riboswitches, and recombinant riboswitches. A naturally occurring riboswitch is a riboswitch having the sequence of a riboswitch as found in nature. Such a naturally occurring riboswitch can be an isolated or recombinant form of the naturally occurring riboswitch as it occurs in nature. That is, the riboswitch has the same primary structure but has been isolated or engineered in a new genetic or nucleic acid context. A consensus riboswitch has the sequence and features of the consensus structure of naturally occurring riboswitches. Chimeric riboswitches can be made up of, for example, part of a riboswitch of any or of a particular class or type of riboswitch and part of a different riboswitch of the same or of any different class or type of riboswitch; part of a riboswitch of any or of a particular class or type of riboswitch and any non-riboswitch sequence or component. Recombinant riboswitches are riboswitches that have been isolated or engineered in a new genetic or nucleic acid context.

Riboswitches can have single or multiple aptamer domains. Aptamer domains in riboswitches having multiple aptamer domains can exhibit cooperative binding of trigger molecules or can not exhibit cooperative binding of trigger molecules (that is, the aptamers need not exhibit cooperative binding). In the latter case, the aptamer domains can be said to be independent binders. Riboswitches having multiple aptamers can have one or multiple expression platform domains. For example, a riboswitch having two aptamer domains that exhibit cooperative binding of their trigger molecules can be linked to a single expression platform domain that is regulated by both aptamer domains. Riboswitches having multiple aptamers can have one or more of the aptamers joined via a linker. Where such aptamers exhibit cooperative binding of trigger molecules, the linker can be a cooperative linker.

Aptamer domains can be said to exhibit cooperative binding if they have a Hill coefficient n between x and x-1, where x is the number of aptamer domains (or the number of binding sites on the aptamer domains) that are being analyzed for cooperative binding. Thus, for example, a riboswitch having two aptamer domains can be said to exhibit cooperative binding if the riboswitch has Hill coefficient between 2 and 1. It should be understood that the value of x used depends on the number of aptamer domains being analyzed for cooperative binding, not necessarily the number of aptamer domains present in the riboswitch. This makes sense because a riboswitch can have multiple aptamer domains where only some exhibit cooperative binding.

Disclosed are chimeric riboswitches containing heterologous aptamer domains and expression platform domains. That is, chimeric riboswitches are made up an aptamer domain from one source and an expression platform domain from another source. The heterologous sources can be from, for example, different specific riboswitches, different types of riboswitches, or different classes of riboswitches. The heterologous aptamers can also come from non-riboswitch aptamers. The heterologous expression platform domains can also come from non-riboswitch sources.

Modified or derivative riboswitches can be produced using *in vitro* selection and evolution techniques. In general, *in vitro* evolution techniques as applied to riboswitches involve producing a set of variant riboswitches where part(s) of the riboswitch sequence is varied while other parts of the riboswitch are held constant. Activation, deactivation or blocking (or other functional or structural criteria) of the set of variant riboswitches can then be assessed and those variant riboswitches meeting the criteria of interest are selected for use or further rounds of evolution. Useful base riboswitches for generation of variants are the specific and consensus riboswitches disclosed herein. Consensus riboswitches can be used to inform which part(s) of a riboswitch to vary for *in vitro* selection and evolution.

Also disclosed are modified riboswitches with altered regulation. The regulation of a riboswitch can be altered by operably linking an aptamer domain to the expression platform domain of the riboswitch (which is a chimeric riboswitch). The aptamer domain can then mediate regulation of the riboswitch through the action of, for example, a trigger molecule for the aptamer domain. Aptamer domains can be operably linked to expression platform domains of riboswitches in any suitable manner, including, for example, by replacing the normal or natural aptamer domain of the riboswitch with the new aptamer domain. Generally, any compound or condition that can activate, deactivate or block the riboswitch from which the aptamer domain is derived can be used to activate, deactivate or block the chimeric riboswitch.

Also disclosed are inactivated riboswitches. Riboswitches can be inactivated by covalently altering the riboswitch (by, for example, crosslinking parts of the riboswitch or coupling a compound to the riboswitch). Inactivation of a riboswitch in this manner can result from, for example, an alteration that prevents the trigger molecule for the riboswitch from binding, that prevents the change in state of the riboswitch upon binding of the trigger molecule, or that prevents the expression platform domain of the riboswitch from affecting expression upon binding of the trigger molecule.

Also disclosed are biosensor riboswitches. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo* or *in vitro.* For example, biosensor riboswitches operably linked to a reporter RNA that encodes a protein that serves as or is involved in producing a signal can be used *in vivo* by engineering a cell or organism to harbor a nucleic acid construct encoding the riboswitch/reporter RNA. An example of a biosensor riboswitch for use *in vitro* is a riboswitch that includes a conformation dependent label, the signal from which changes depending on the activation state of the riboswitch. Such a biosensor riboswitch preferably uses an aptamer domain from or derived from a naturally occurring riboswitch. Biosensor riboswitches can be used in various situations and platforms. For example, biosensor riboswitches can be used with solid supports, such as plates, chips, strips and wells.

Also disclosed are modified or derivative riboswitches that recognize new trigger molecules. New riboswitches and/or new aptamers that recognize new trigger molecules can be selected for, designed or derived from known riboswitches. This can be accomplished by, for example, producing a set of aptamer variants in a riboswitch, assessing the activation of the variant riboswitches in the presence of a compound of interest, selecting variant riboswitches that were activated (or, for example, the riboswitches that were the most highly or the most selectively activated), and repeating these steps until a variant riboswitch of a desired activity, specificity, combination of activity and specificity, or other combination of properties results.

In general, any aptamer domain can be adapted for use with any expression platform domain by designing or adapting a regulated strand in the expression platform domain to be complementary to the control strand of the aptamer domain. Alternatively, the sequence of the aptamer and control strands of an aptamer domain can be adapted so that the control strand is complementary to a functionally significant sequence in an expression platform. For example, the control strand can be adapted to be complementary to the Shine-Dalgarno sequence of an RNA such that, upon formation of a stem structure between the control strand and the SD sequence, the SD sequence becomes inaccessible to ribosomes, thus reducing or preventing translation initiation. Note that the aptamer strand would have corresponding changes in sequence to allow formation of a P1 stem in the aptamer domain. In the case of riboswitches having multiple aptamers exhibiting cooperative binding, only the P1 stem of the activating aptamer (the aptamer that interacts with the expression platform domain) need be designed to form a stem structure with the SD sequence.

As another example, a transcription terminator can be added to an RNA molecule (most conveniently in an untranslated region of the RNA) where part of the sequence of the transcription terminator is complementary to the control strand of an aptamer domain (the sequence will be the regulated strand). This will allow the control sequence of the aptamer domain to form alternative stem structures with the aptamer strand and the regulated strand, thus either forming or disrupting a transcription terminator stem upon activation or deactivation of the riboswitch. Any other expression element can be brought under the control of a riboswitch by similar design of alternative stem structures.

For transcription terminators controlled by riboswitches, the speed of transcription and spacing of the riboswitch and expression platform elements can be important for proper control. Transcription speed can be adjusted by, for example, including polymerase pausing elements (e.g., a series of uridine residues) to pause transcription and allow the riboswitch to form and sense trigger molecules.

Disclosed are regulatable gene expression constructs comprising a nucleic acid molecule encoding an RNA comprising a riboswitch operably linked to a coding region, wherein the riboswitch regulates expression of the RNA, wherein the riboswitch and coding region are heterologous. The riboswitch can be a cyclic di-GMP-responsive riboswitch, an S-adenosylhomocysteine-repsonsive riboswitch, a preQ₁-responsive riboswitch, a Moco-responsive riboswitch, a SAM-responsive riboswitch, or a derivative of such riboswitches. The riboswitch can comprise an aptamer domain and an expression platform domain, wherein the aptamer domain and the expression platform domain are heterologous. The riboswitch can comprise two or more aptamer domains and an expression platform domain, wherein at least one of the aptamer domains and the expression platform domain are heterologous. At least two of the aptamer domains can exhibit cooperative binding.

Disclosed are RNA molecules comprising heterologous riboswitch and coding region. That is, such RNA molecules are made up of a riboswitch from one source and a coding region from another source. The heterologous sources can be from, for example, different RNA molecules, different transcripts, RNA or transcripts from different genes, RNA or transcripts from different cells, RNA or transcripts from different organisms, RNA or transcripts from different species, natural sequences and artificial or engineered sequences, specific riboswitches, different types of riboswitches, or different classes of riboswitches.

As disclosed herein, the term "coding region" refers to any region of a nucleic acid that codes for amino acids. This can include both a nucleic acid strand that contains the codons or the template for codons and the complement of such a nucleic acid strand in the case of double stranded nucleic acid molecules. Regions of nucleic acids that are not coding regions can be referred to as noncoding regions. Messenger RNA molecules as transcribed typically include noncoding regions at both the 5' and 3' ends. Eukaryotic mRNA molecules can also include internal noncoding regions such as introns. Some types of RNA molecules do not include functional coding regions, such as tRNA and rRNA molecules. The expression of RNA molecules that do not include functional coding regions, which can be referred to as noncoding RNA molecules, can also be regulated or affected by the disclosed riboswitches. Thus, the disclosed riboswitches can be operably linked to a noncoding RNA molecule in any manner as disclosed herein for operable linkage of a riboswitch to a coding region. The riboswitch can regulate expression of such RNA as disclosed herein for regulation of RNA comprising a riboswitch operably linked to a coding region. The function of any nucleic acid molecule can also regulated or affected by the disclosed riboswitches. Examples include, but are not limited to, RNA, DNA, and artificial nucleic acids, including peptide nucleic acid (PNA), morpholino and locked nucleic acid (LNA), as well as glycol nucleic acid (GNA) and threose nucleic acid (TNA). In the disclosed method, the riboswitch can regulate expression of the coding region, expression of the encoded protein, expression of the noncoding RNA molecule, transcription of the RNA or of the coding region, or translation of the encoded protein, for example.

### 1. Aptamer Domains

Aptamers are nucleic acid segments and structures that can bind selectively to particular compounds and classes of compounds. Riboswitches have aptamer domains that, upon binding of a trigger molecule result in a change in the state or structure of the riboswitch. In functional riboswitches, the state or structure of the expression platform domain linked to the aptamer domain changes when the trigger molecule binds to the aptamer domain. Aptamer domains of riboswitches can be derived from any source, including, for example, natural aptamer domains of riboswitches, artificial aptamers, engineered, selected, evolved or derived aptamers or aptamer domains. Aptamers in riboswitches generally have at least one portion that can interact, such as by forming a stem structure, with a portion of the linked expression platform domain. This stem structure will either form or be disrupted upon binding of the trigger molecule.

Consensus aptamer domains of a variety of natural riboswitches are shown in Figure 11 of U.S. Application Publication No. 2005-0053951 and elsewhere herein. The consensus sequence and structure for cyclic di-GMP riboswitches can be found in Figures 1 and 3. Consensus sequences of SAH riboswitches are shown in Figures 1 and 9A. Consensus sequences of preQ₁-II (preQ₁-II or COG4708 motif) riboswitches are shown in Figures 1, 15 (sequence) and 16A (structure). These aptamer domains (including all of the direct variants embodied therein) can be used in riboswitches. The consensus sequences and structures indicate variations in sequence and structure. Aptamer domains that are within the indicated variations are referred to herein as direct variants. These aptamer domains can be modified to produce modified or variant aptamer domains. Conservative modifications include any change in base paired nucleotides such that the nucleotides in the pair remain complementary. Moderate modifications include changes in the length of stems or of loops (for which a length or length range is indicated) of less than or equal to 20% of the length range indicated. Loop and stem lengths are considered to be "indicated" where the consensus structure shows a stem or loop of a particular length or where a range of lengths is listed or depicted. Moderate modifications include changes in the length of stems or of loops (for which a length or length range is not indicated) of less than or equal to 40% of the length range indicated. Moderate modifications also include and functional variants of unspecified portions of the aptamer domain.

Aptamer domains of the disclosed riboswitches can also be used for any other purpose, and in any other context, as aptamers. For example, aptamers can be used to control ribozymes, other molecular switches, and any RNA molecule where a change in structure can affect function of the RNA.

### 2. Expression Platform Domains

Expression platform domains are a part of riboswitches that affect expression of the RNA molecule that contains the riboswitch. Expression platform domains generally have at least one portion that can interact, such as by forming a stem structure, with a portion of the linked aptamer domain. This stem structure will either form or be disrupted upon binding of the trigger molecule. The stem structure generally either is, or prevents formation of, an expression regulatory structure. An expression regulatory structure is a structure that allows, prevents, enhances or inhibits expression of an RNA molecule containing the structure. Examples include Shine-Dalgarno sequences, initiation codons, transcription terminators, stability signals, and processing signals, such as RNA splicing junctions and control elements.

### B. Trigger Molecules

Trigger molecules are molecules and compounds that can activate a riboswitch. This includes the natural or normal trigger molecule for the riboswitch and other compounds that can activate the riboswitch. Natural or normal trigger molecules are the trigger molecule for a given riboswitch in nature or, in the case of some non-natural riboswitches, the trigger molecule for which the riboswitch was designed or with which the riboswitch was selected (as in, for example, *in vitro* selection or *in vitro* evolution techniques).

### C. Compounds

Also disclosed are compounds, and compositions containing such compounds, that can activate, deactivate or block a riboswitch. Riboswitches function to control gene expression through the binding or removal of a trigger molecule. Compounds can be used to activate, deactivate or block a riboswitch. The trigger molecule for a riboswitch (as well as other activating compounds) can be used to activate a riboswitch. Compounds other than the trigger molecule generally can be used to deactivate or block a riboswitch. Riboswitches can also be deactivated by, for example, removing trigger molecules from the presence of the riboswitch. A riboswitch can be blocked by, for example, binding of an analog of the trigger molecule that does not activate the riboswitch.

Also disclosed are compounds for altering expression of an RNA molecule, or of a gene encoding an RNA molecule, where the RNA molecule includes a riboswitch. This can be accomplished by bringing a compound into contact with the RNA molecule. Riboswitches function to control gene expression through the binding or removal of a trigger molecule. Thus, subjecting an RNA molecule of interest that includes a riboswitch to conditions that activate, deactivate or block the riboswitch can be used to alter expression of the RNA. Expression can be altered as a result of, for example, termination of transcription or blocking of ribosome binding to the RNA. Binding of a trigger molecule can, depending on the nature of the riboswitch, reduce or prevent expression of the RNA molecule or promote or increase expression of the RNA molecule.

Also disclosed are compounds for regulating expression of an RNA molecule, or of a gene encoding an RNA molecule. Also disclosed are compounds for regulating expression of a naturally occurring gene or RNA that contains a riboswitch by activating, deactivating or blocking the riboswitch. If the gene is essential for survival of a cell or organism that harbors it, activating, deactivating or blocking the riboswitch can in death, stasis or debilitation of the cell or organism.

Also disclosed are compounds for regulating expression of an isolated, engineered or recombinant gene or RNA that contains a riboswitch by activating, deactivating or blocking the riboswitch. If the gene encodes a desired expression product, activating or deactivating the riboswitch can be used to induce expression of the gene and thus result in production of the expression product. If the gene encodes an inducer or repressor of gene expression or of another cellular process, activation, deactivation or blocking of the riboswitch can result in induction, repression, or de-repression of other, regulated genes or cellular processes. Many such secondary regulatory effects are known and can be adapted for use with riboswitches. An advantage of riboswitches as the primary control for such regulation is that riboswitch trigger molecules can be small, non-antigenic molecules.

Also disclosed are methods of identifying compounds that activate, deactivate or block a riboswitch. For example, compounds that activate a riboswitch can be identified by bringing into contact a test compound and a riboswitch and assessing activation of the riboswitch. If the riboswitch is activated, the test compound is identified as a compound that activates the riboswitch. Activation of a riboswitch can be assessed in any suitable manner. For example, the riboswitch can be linked to a reporter RNA and expression, expression level, or change in expression level of the reporter RNA can be measured in the presence and absence of the test compound. As another example, the riboswitch can include a conformation dependent label, the signal from which changes depending on the activation state of the riboswitch. Such a riboswitch preferably uses an aptamer domain from or derived from a naturally occurring riboswitch. As can be seen, assessment of activation of a riboswitch can be performed with the use of a control assay or measurement or without the use of a control assay or measurement. Methods for identifying compounds that deactivate a riboswitch can be performed in analogous ways.

Identification of compounds that block a riboswitch can be accomplished in any suitable manner. For example, an assay can be performed for assessing activation or deactivation of a riboswitch in the presence of a compound known to activate or deactivate the riboswitch and in the presence of a test compound. If activation or deactivation is not observed as would be observed in the absence of the test compound, then the test compound is identified as a compound that blocks activation or deactivation of the riboswitch.

Disclosed herein are compounds that interact with the cyclic di-GMP, SAH, preQ₁, Moco, and SAM-IV riboswitches. Further modified versions of these compounds can have improved binding to the riboswitch by making new contacts to other functional groups in the RNA structure. Furthermore, modulation of bioavailability, toxicity, and synthetic ease (among other characteristics) can be tunable by making modifications in these two regions of the scaffold, as the structural model for the riboswitch shows many modifications are possible at these sites.

High-throughput screening can also be used to reveal entirely new chemical scaffolds that also bind to riboswitch RNAs either with standard or non-standard modes of molecular recognition. Multiple different approaches can be used to detect metabolite binding RNAs, including allosteric ribozyme assays using gel-based and chip-based detection methods, and in-line probing assays. Also disclosed are compounds made by identifying a compound that activates, deactivates or blocks a riboswitch and manufacturing the identified compound. This can be accomplished by, for example, combining compound identification methods as disclosed elsewhere herein with methods for manufacturing the identified compounds. For example, compounds can be made by bringing into contact a test compound and a riboswitch, assessing activation of the riboswitch, and, if the riboswitch is activated by the test compound, manufacturing the test compound that activates the riboswitch as the compound.

Also disclosed are compounds made by checking activation, deactivation or blocking of a riboswitch by a compound and manufacturing the checked compound. This can be accomplished by, for example, combining compound activation, deactivation or blocking assessment methods as disclosed elsewhere herein with methods for manufacturing the checked compounds. For example, compounds can be made by bringing into contact a test compound and a riboswitch, assessing activation of the riboswitch, and, if the riboswitch is activated by the test compound, manufacturing the test compound that activates the riboswitch as the compound. Checking compounds for their ability to activate, deactivate or block a riboswitch refers to both identification of compounds previously unknown to activate, deactivate or block a riboswitch and to assessing the ability of a compound to activate, deactivate or block a riboswitch where the compound was already known to activate, deactivate or block the riboswitch.

As used herein, the term "substituted" is contemplated to include all permissible substituents of organic compounds. In a broad aspect, the permissible substituents include acyclic and cyclic, branched and unbranched, carbocyclic and heterocyclic, and aromatic and nonaromatic substituents of organic compounds. Illustrative substituents include, for example, those described below. The permissible substituents can be one or more and the same or different for appropriate organic compounds. For the purposes of this disclosure, the heteroatoms, such as nitrogen, can have hydrogen substituents and/or any permissible substituents of organic compounds described herein which satisfy the valences of the heteroatoms. This disclosure is not intended to be limited in any manner by the permissible substituents of organic compounds. Also, the terms "substitution" or "substituted with" include the implicit proviso that such substitution is in accordance with permitted valence of the substituted atom and the substituent, and that the substitution results in a stable compound, e.g., a compound that does not spontaneously undergo transformation such as by rearrangement, cyclization, elimination, etc.

"A¹," "A²," "A³," and "A⁴" are used herein as generic symbols to represent various specific substituents. These symbols can be any substituent, not limited to those disclosed herein, and when they are defined to be certain substituents in one instance, they can, in another instance, be defined as some other substituents.

The term "alkyl" as used herein is a branched or unbranched saturated hydrocarbon group of 1 to 24 carbon atoms, such as methyl, ethyl, n-propyl, isopropyl, n-butyl, isobutyl, t-butyl, pentyl, hexyl, heptyl, octyl, nonyl, decyl, dodecyl, tetradecyl, hexadecyl, eicosyl, tetracosyl, and the like. The alkyl group can also be substituted or unsubstituted. The alkyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol, as described below. The term "lower alkyl" is an alkyl group with 6 or fewer carbon atoms, e.g., methyl, ethyl, propyl, isopropyl, butyl, sec-butyl, iso-butyl, tert-butyl, pentyl, hexyl, and the like.

Throughout the specification "alkyl" is generally used to refer to both unsubstituted alkyl groups and substituted alkyl groups; however, substituted alkyl groups are also specifically referred to herein by identifying the specific substituent(s) on the alkyl group. For example, the term "halogenated alkyl" specifically refers to an alkyl group that is substituted with one or more halide, e.g., fluorine, chlorine, bromine, or iodine. The term "alkoxyalkyl" specifically refers to an alkyl group that is substituted with one or more alkoxy groups, as described below. The term "alkylamino" specifically refers to an alkyl group that is substituted with one or more amino groups, as described below, and the like. When "alkyl" is used in one instance and a specific term such as "halogenated alkyl" is used in another, it is not meant to imply that the term "alkyl" does not also refer to specific terms such as "halogenated alkyl" and the like.

This practice is also used for other groups described herein. That is, while a term such as "cycloalkyl" refers to both unsubstituted and substituted cycloalkyl moieties, the substituted moieties can, in addition, be specifically identified herein; for example, a particular substituted cycloalkyl can be referred to as, e.g., an "alkylcycloalkyl." Similarly, a substituted alkoxy can be specifically referred to as, e.g., a "halogenated alkoxy," a particular substituted alkenyl can be, e.g., an "alkenylalcohol," and the like. Again, the practice of using a general term, such as "cycloalkyl," and a specific term, such as "alkylcycloalkyl," is not meant to imply that the general term does not also include the specific term.

The term "alkoxy" as used herein is an alkyl group bonded through a single, terminal ether linkage; that is, an "alkoxy" group can be defined as —OA¹ where A² is alkyl as defined above.

The term "alkenyl" as used herein is a hydrocarbon group of from 2 to 24 carbon atoms with a structural formula containing at least one carbon-carbon double bond. Asymmetric structures such as (A¹A²)C=C(A³A⁴) are intended to include both the E and Z isomers. This can be presumed in structural formulae herein wherein an asymmetric alkene is present, or it can be explicitly indicated by the bond symbol C=C. The alkenyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol, as described below.

The term "alkynyl" as used herein is a hydrocarbon group of 2 to 24 carbon atoms with a structural formula containing at least one carbon-carbon triple bond. The alkynyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol, as described below.

The term "aryl" as used herein is a group that contains any carbon-based aromatic group including, but not limited to, benzene, naphthalene, phenyl, biphenyl, phenoxybenzene, and the like. The term "aryl" also includes "heteroaryl," which is defined as a group that contains an aromatic group that has at least one heteroatom incorporated within the ring of the aromatic group. Examples of heteroatoms include, but are not limited to, nitrogen, oxygen, sulfur, and phosphorus. Likewise, the term "non-heteroaryl," which is also included in the term "aryl," defines a group that contains an aromatic group that does not contain a heteroatom. The aryl group can be substituted or unsubstituted. The aryl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol as described herein. The term "biaryl" is a specific type of aryl group and is included in the definition of aryl. Biaryl refers to two aryl groups that are bound together *via* a fused ring structure, as in naphthalene, or are attached *via* one or more carbon-carbon bonds, as in biphenyl.

The term "cycloalkyl" as used herein is a non-aromatic carbon-based ring composed of at least three carbon atoms. Examples of cycloalkyl groups include, but are not limited to, cyclopropyl, cyclobutyl, cyclopentyl, cyclohexyl, etc. The term "heterocycloalkyl" is a cycloalkyl group as defined above where at least one of the carbon atoms of the ring is substituted with a heteroatom such as, but not limited to, nitrogen, oxygen, sulfur, or phosphorus. The cycloalkyl group and heterocycloalkyl group can be substituted or unsubstituted. The cycloalkyl group and heterocycloalkyl group can be substituted with one or more groups including, but not limited to, alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol as described herein.

The term "cycloalkenyl" as used herein is a non-aromatic carbon-based ring composed of at least three carbon atoms and containing at least one double bound, *i.e*., C=C. Examples of cycloalkenyl groups include, but are not limited to, cyclopropenyl, cyclobutenyl, cyclopentenyl, cyclopentadienyl, cyclohexenyl, cyclohexadienyl, and the like. The term "heterocycloalkenyl" is a type of cycloalkenyl group as defined above, and is included within the meaning of the term "cycloalkenyl," where at least one of the carbon atoms of the ring is substituted with a heteroatom such as, but not limited to, nitrogen, oxygen, sulfur, or phosphorus. The cycloalkenyl group and heterocycloalkenyl group can be substituted or unsubstituted. The cycloalkenyl group and heterocycloalkenyl group can be substituted with one or more groups including, but not limited to, alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol as described herein.

The term "cyclic group" is used herein to refer to either aryl groups, non-aryl groups (*i.e*., cycloalkyl, heterocycloalkyl, cycloalkenyl, and heterocycloalkenyl groups), or both. Cyclic groups have one or more ring systems that can be substituted or unsubstituted. A cyclic group can contain one or more aryl groups, one or more non-aryl groups, or one or more aryl groups and one or more non-aryl groups.

The term "aldehyde" as used herein is represented by the formula —C(O)H. Throughout this specification "C(O)" is a short hand notation for C=O.

The terms "amine" or "amino" as used herein are represented by the formula NA¹A²A³, where A¹, A², and A³ can be, independently, hydrogen, an alkyl, halogenated alkyl, alkenyl, alkynyl, aryl, heteroaryl, cycloalkyl, cycloalkenyl, heterocycloalkyl, or heterocycloalkenyl group described above.

The term "carboxylic acid" as used herein is represented by the formula
—C(O)OH. A "carboxylate" as used herein is represented by the formula — C(O)O⁻.
The term "ester" as used herein is represented by the formula —OC(O)A¹ or
—C(O)OA¹, where A¹ can be an alkyl, halogenated alkyl, alkenyl, alkynyl, aryl, heteroaryl, cycloalkyl, cycloalkenyl, heterocycloalkyl, or heterocycloalkenyl group described above.

The term "ether" as used herein is represented by the formula A¹OA², where A¹ and A² can be, independently, an alkyl, halogenated alkyl, alkenyl, alkynyl, aryl, heteroaryl, cycloalkyl, cycloalkenyl, heterocycloalkyl, or heterocycloalkenyl group described above.

The term "ketone" as used herein is represented by the formula A¹C(O)A², where A¹ and A² can be, independently, an alkyl, halogenated alkyl, alkenyl, alkynyl, aryl, heteroaryl, cycloalkyl, cycloalkenyl, heterocycloalkyl, or heterocycloalkenyl group described above.

The term "halide" as used herein refers to the halogens fluorine, chlorine, bromine, and iodine.

The term "hydroxyl" as used herein is represented by the formula —OH.

The term "sulfo-oxo" as used herein is represented by the formulas —S(O)A¹ (*i.e*., "sulfonyl"), A¹S(O)A² (*i.e*., "sulfoxide"), —S(O)₂A¹, A¹SO₂A² (*i.e*., "sulfone"),
—OS(O)₂A¹, or —OS(O)₂OA¹, where A₁ and A² can be hydrogen, an alkyl, halogenated alkyl, alkenyl, alkynyl, aryl, heteroaryl, cycloalkyl, cycloalkenyl, heterocycloalkyl, or heterocycloalkenyl group described above. Throughout this specification "S(O)" is a short hand notation for S=O.

The term "sulfonylamino" or "sulfonamide" as used herein is represented by the formula —S(O)₂NH—.

The term "thiol" as used herein is represented by the formula —SH.

As used herein, "Rⁿ" where n is some integer can independently possess one or more of the groups listed above. Depending upon the groups that are selected, a first group can be incorporated within second group or, alternatively, the first group can be pendant (*i.e*., attached) to the second group. For example, with the phrase "an alkyl group comprising an amino group," the amino group can be incorporated within the backbone of the alkyl group. Alternatively, the amino group can be attached to the backbone of the alkyl group. The nature of the group(s) that is (are) selected will determine if the first group is embedded or attached to the second group.

Unless stated to the contrary, a formula with chemical bonds shown only as solid lines and not as wedges or dashed lines contemplates each possible isomer, e.g., each enantiomer and diastereomer, and a mixture of isomers, such as a racemic or scalemic mixture.

Certain materials, compounds, compositions, and components disclosed herein can be obtained commercially or readily synthesized using techniques generally known to those of skill in the art. For example, the starting materials and reagents used in preparing the disclosed compounds and compositions are either available from commercial suppliers such as Aldrich Chemical Co., (Milwaukee, Wis.), Acros Organics (Morris Plains, N.J.), Fisher Scientific (Pittsburgh, Pa.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); March's Advanced Organic Chemistry, (John Wiley and Sons, 4th Edition); and Larock's Comprehensive Organic Transformations (VCH Publishers Inc., 1989).

Compounds for activating the disclosed riboswitches include cyclic di-GMP, pGpG, GpG, or GpGpG, derivatives of cyclic di-GMP, pGpG, GpG, or GpGpG that can activate a cyclic di-GMP-responsive riboswitch, S-adenosylhomocysteine, derivatives of S-adenosylhomocysteine that can activate an S-adenosylhomocysteine-responsive riboswitch, preQ₁, derivatives of preQ₁ that can activate a preQ₁-responsive riboswitch, Moco, derivatives of Moco that can activate a Moco-responsive riboswitch, SAM, and derivatives of SAM that can activate a SAM-responsive riboswitch. The compound for activating a SAM-IV riboswitch can also be MeAzaAdoMet.

For activation of an S-adenosylhomocysteine-responsive riboswitch, the compound can be S-adenosylhomocysteine. The compound can also be a derivative of S-adenosylhomocysteine that can activate an S-adenosylhomocysteine-responsive riboswitch. For example, the compound can be S-adenosyl-L-cysteine (SAC). Figure 12B shows structural elements that are important for a compound that can activate an S-adenosylhomocysteine-responsive riboswitch.

For activation of preQ₁-responsive riboswitches (and riboswitches derived from preQ₁-responsive riboswitches), the compound can be derivatives of preQ₁ where the N at position 1 can be substituted with C, O or S, where the amino group at position 2 can be substituted with a hydrogen bond donor, where the oxygen at position 6 can be substituted with a hydrogen bond acceptor, where the amino group at position 7 can be substituted with a hydrogen bond acceptor, and where the nitrogen can be substituted or derivatized with a hydrogen bond donor.

It is to be understood that while a particular moiety or group can be referred to herein as a hydrogen bond donor or acceptor, this terminology is used to merely categorize the various substituents for ease of reference. Such language should not be interpreted to mean that a particular moiety actually participates in hydrogen bonding with the riboswitch or some other compound. It is possible that, for example, a moiety referred to herein as a hydrogen bond acceptor (or donor) could solely or additionally be involved in hydrophobic, ionic, van de Waals, or other type of interaction with the riboswitch or other compound.

It is also understood that certain groups disclosed herein can be referred to herein as both a hydrogen bond acceptor and a hydrogen bond donor. For example, -OH can be a hydrogen bond donor by donating the hydrogen atom; -OH can also be a hydrogen bond acceptor through one or more of the nonbonded electron pairs on the oxygen atom. Thus, throughout the specification various moieties can be a hydrogen bond donor and acceptor and can be referred to as such.

Every compound within the above definition is intended to be and should be considered to be specifically disclosed herein. Further, every subgroup that can be identified within the above definition is intended to be and should be considered to be specifically disclosed herein. As a result, it is specifically contemplated that any compound, or subgroup of compounds can be either specifically included for or excluded from use or included in or excluded from a list of compounds. As an example, a group of compounds is contemplated where each compound is as defined above and is able to activate a cyclic di-GMP-responsive riboswitch.

It should be understood that particular contacts and interactions (such as hydrogen bond donation or acceptance) described herein for compounds interacting with riboswitches are preferred but are not essential for interaction of a compound with a riboswitch. For example, compounds can interact with riboswitches with less affinity and/or specificity than compounds having the disclosed contacts and interactions. Further, different or additional functional groups on the compounds can introduce new, different and/or compensating contacts with the riboswitches. Such functional groups can have, and can be designed to have, contacts and interactions with other part of the riboswitch. Such contacts and interactions can compensate for contacts and interactions of the trigger molecules and core structure.

### D. Constructs, Vectors and Expression Systems

The disclosed riboswitches can be used with any suitable expression system. Recombinant expression is usefully accomplished using a vector, such as a plasmid. The vector can include a promoter operably linked to riboswitch-encoding sequence and RNA to be expression (e.g., RNA encoding a protein). The vector can also include other elements required for transcription and translation. As used herein, vector refers to any carrier containing exogenous DNA. Thus, vectors are agents that transport the exogenous nucleic acid into a cell without degradation and include a promoter yielding expression of the nucleic acid in the cells into which it is delivered. Vectors include but are not limited to plasmids, viral nucleic acids, viruses, phage nucleic acids, phages, cosmids, and artificial chromosomes. A variety of prokaryotic and eukaryotic expression vectors suitable for carrying riboswitch-regulated constructs can be produced. Such expression vectors include, for example, pET, pET3d, pCR2.1, pBAD, pUC, and yeast vectors. The vectors can be used, for example, in a variety of *in vivo* and *in vitro* situation.

Viral vectors include adenovirus, adeno-associated virus, herpes virus, vaccinia virus, polio virus, AIDS virus, neuronal trophic virus, Sindbis and other RNA viruses, including these viruses with the HIV backbone. Also useful are any viral families which share the properties of these viruses which make them suitable for use as vectors. Retroviral vectors, which are described in Verma (1985), include Murine Maloney Leukemia virus, MMLV, and retroviruses that express the desirable properties of MMLV as a vector. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA.

A "promoter" is generally a sequence or sequences of DNA that function when in a relatively fixed location in regard to the transcription start site. A "promoter" contains core elements required for basic interaction of RNA polymerase and transcription factors and can contain upstream elements and response elements.

"Enhancer" generally refers to a sequence of DNA that functions at no fixed distance from the transcription start site and can be either 5' (Laimins, 1981) or 3' (Lusky et al., 1983) to the transcription unit. Furthermore, enhancers can be within an intron (Banerji et al., 1983) as well as within the coding sequence itself (Osborne et al., 1984). They are usually between 10 and 300 bp in length, and they function in cis. Enhancers function to increase transcription from nearby promoters. Enhancers, like promoters, also often contain response elements that mediate the regulation of transcription. Enhancers often determine the regulation of expression.

Expression vectors used in eukaryotic host cells (yeast, fungi, insect, plant, animal, human or nucleated cells) can also contain sequences necessary for the termination of transcription which can affect mRNA expression. These regions are transcribed as polyadenylated segments in the untranslated portion of the mRNA encoding tissue factor protein. The 3' untranslated regions also include transcription termination sites. It is preferred that the transcription unit also contains a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs.

The vector can include nucleic acid sequence encoding a marker product. This marker product is used to determine if the gene has been delivered to the cell and once delivered is being expressed. Preferred marker genes are the *E*. *Coli* lacZ gene which encodes β-galactosidase and green fluorescent protein.

In some embodiments the marker can be a selectable marker. When such selectable markers are successfully transferred into a host cell, the transformed host cell can survive if placed under selective pressure. There are two widely used distinct categories of selective regimes. The first category is based on a cell's metabolism and the use of a mutant cell line which lacks the ability to grow independent of a supplemented media. The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern and Berg, 1982), mycophenolic acid, (Mulligan and Berg, 1980) or hygromycin (Sugden et al., 1985).

Gene transfer can be obtained using direct transfer of genetic material, in but not limited to, plasmids, viral vectors, viral nucleic acids, phage nucleic acids, phages, cosmids, and artificial chromosomes, or via transfer of genetic material in cells or carriers such as cationic liposomes. Such methods are well known in the art and readily adaptable for use in the method described herein. Transfer vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)). Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991).

### 1. Viral Vectors

Preferred viral vectors are Adenovirus, Adeno-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Preferred retroviruses include Murine Maloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, they are not useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. A preferred embodiment is a viral vector which has been engineered so as to suppress the immune response of the host organism, elicited by the viral antigens. Preferred vectors of this type will carry coding regions for Interleukin 8 or 10.

Viral vectors have higher transaction (ability to introduce genes) abilities than do most chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans.

### i. Retroviral Vectors

A retrovirus is an animal virus belonging to the virus family of Retroviridae, including any types, subfamilies, genus, or tropisms. Retroviral vectors, in general, are described by Verma, I.M., Retroviral vectors for gene transfer. In Microbiology-1985, American Society for Microbiology, pp. 229-232, Washington, (1985), which is incorporated by reference herein. Examples of methods for using retroviral vectors for gene therapy are described in U.S. Patent Nos. 4,868,116 and 4,980,286; PCT applications WO 90/02806 and WO 89/07136; and Mulligan, (Science 260:926-932 (1993)); the teachings of which are incorporated herein by reference.

A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome, contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serve as the priming site for the synthesis of the second strand of DNA synthesis, and specific sequences near the ends of the LTRs that enable the insertion of the DNA state of the retrovirus to insert into the host genome. The removal of the gag, pol, and env genes allows for about 8 kb of foreign sequence to be inserted into the viral genome, become reverse transcribed, and upon replication be packaged into a new retroviral particle. This amount of nucleic acid is sufficient for the delivery of a one to many genes depending on the size of each transcript. It is preferable to include either positive or negative selectable markers along with other genes in the insert.

Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery, but lacks any packaging signal. When the vector carrying the DNA of choice is transfected into these cell lines, the vector containing the gene of interest is replicated and packaged into new retroviral particles, by the machinery provided in cis by the helper cell. The genomes for the machinery are not packaged because they lack the necessary signals.

### ii. Adenoviral Vectors

The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, *in vivo* delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Biol. Chem. 267:25129-25134 (1992); Rich, Human Gene Therapy 4:461-476 (1993); Zabner, Nature Genetics 6:75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287-1291 (1993); and Ragot, J. Gen. Virology 74:501-507 (1993)). Recombinant adenoviruses achieve gene transduction by binding to specific cell surface receptors, after which the virus is internalized by receptor-mediated endocytosis, in the same manner as wild type or replication-defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol. 4:1528-1533 (1984); Varga et al., J. Virology 65:6061-6070 (1991); Wickham et al., Cell 73:309-319 (1993)).

A preferred viral vector is one based on an adenovirus which has had the E1 gene removed and these virons are generated in a cell line such as the human 293 cell line. In another preferred embodiment both the E1 and E3 genes are removed from the adenovirus genome.

Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An especially preferred embodiment of this type of vector is the P4.1 C vector produced by Avigen, San Francisco, CA, which can contain the herpes simplex virus thymidine kinase gene, HSV-tk, and/or a marker gene, such as the gene encoding the green fluorescent protein, GFP.

The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and can contain upstream elements and response elements.

### 2. Viral Promoters and Enhancers

Preferred promoters controlling transcription from vectors in mammalian host cells can be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a HindIII E restriction fragment (Greenway, P.J. et al., Gene 18: 355-360 (1982)). Of course, promoters from the host cell or related species also are useful herein.

Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, α-fetoprotein and insulin), typically one will use an enhancer from a eukaryotic cell virus. Preferred examples are the SV40 enhancer on the late side of the replication origin (bp 100-270), the cytomegalovirus early promoter enhancer, the polyoma enhancer on the late side of the replication origin, and adenovirus enhancers.

The promoter and/or enhancer can be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

It is preferred that the promoter and/or enhancer region be active in all eukaryotic cell types. A preferred promoter of this type is the CMV promoter (650 bases). Other preferred promoters are SV40 promoters, cytomegalovirus (full length promoter), and retroviral vector LTF.

It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

Expression vectors used in eukaryotic host cells (yeast, fungi, insect, plant, animal, human or nucleated cells) can also contain sequences necessary for the termination of transcription which can affect mRNA expression. These regions are transcribed as polyadenylated segments in the untranslated portion of the mRNA encoding tissue factor protein. The 3' untranslated regions also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In a preferred embodiment of the transcription unit, the polyadenylation region is derived from the SV40 early polyadenylation signal and consists of about 400 bases. It is also preferred that the transcribed units contain other standard sequences alone or in combination with the above sequences improve expression from, or stability of, the construct.

### 3. Markers

The vectors can include nucleic acid sequence encoding a marker product. This marker product is used to determine if the gene has been delivered to the cell and once delivered is being expressed. Preferred marker genes are the E. Coli lacZ gene which encodes β-galactosidase and green fluorescent protein.

In some embodiments the marker can be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR⁻ cells and mouse LTK⁻ cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

The second category is dominant selection which refers to a selection scheme used in any cell type and does not require the use of a mutant cell line. These schemes typically use a drug to arrest growth of a host cell. Those cells which would express a protein conveying drug resistance and would survive the selection. Examples of such dominant selection use the drugs neomycin, (Southern P. and Berg, P., J. Molec. Appl. Genet. 1: 327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### E. Biosensor Riboswitches

Also disclosed are biosensor riboswitches. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo* or *in vitro.* For example, cyclic di-GMP biosensor riboswitches operably linked to a reporter RNA that encodes a protein that serves as or is involved in producing a signal can be used *in vivo* by engineering a cell or organism to harbor a nucleic acid construct encoding the cyclic di-GMP riboswitch/reporter RNA. An example of a biosensor riboswitch for use *in vitro* is a riboswitch that includes a conformation dependent label, the signal from which changes depending on the activation state of the riboswitch. Such a biosensor riboswitch preferably uses an aptamer domain from or derived from a naturally occurring riboswitch, such as cyclic di-GMP.

### F. Reporter Proteins and Peptides

For assessing activation of a riboswitch, or for biosensor riboswitches, a reporter protein or peptide can be used. The reporter protein or peptide can be encoded by the RNA the expression of which is regulated by the riboswitch. The examples describe the use of some specific reporter proteins. The use of reporter proteins and peptides is well known and can be adapted easily for use with riboswitches. The reporter proteins can be any protein or peptide that can be detected or that produces a detectable signal. Preferably, the presence of the protein or peptide can be detected using standard techniques (e.g., radioimmunoassay, radio-labeling, immunoassay, assay for enzymatic activity, absorbance, fluorescence, luminescence, and Western blot). More preferably, the level of the reporter protein is easily quantifiable using standard techniques even at low levels. Useful reporter proteins include luciferases, green fluorescent proteins and their derivatives, such as firefly luciferase (FL) from Photinus pyralis, and Renilla luciferase (RL) from Renilla reniformis.

### G. Conformation Dependent Labels

Conformation dependent labels refer to all labels that produce a change in fluorescence intensity or wavelength based on a change in the form or conformation of the molecule or compound (such as a riboswitch) with which the label is associated. Examples of conformation dependent labels used in the context of probes and primers include molecular beacons, Amplifluors, FRET probes, cleavable FRET probes, TaqMan probes, scorpion primers, fluorescent triplex oligos including but not limited to triplex molecular beacons or triplex FRET probes, fluorescent water-soluble conjugated polymers, PNA probes and QPNA probes. Such labels, and, in particular, the principles of their function, can be adapted for use with riboswitches. Several types of conformation dependent labels are reviewed in Schweitzer and Kingsmore, Curr. Opin. Biotech. 12:21-27(2001).

Stem quenched labels, a form of conformation dependent labels, are fluorescent labels positioned on a nucleic acid such that when a stem structure forms a quenching moiety is brought into proximity such that fluorescence from the label is quenched. When the stem is disrupted (such as when a riboswitch containing the label is activated), the quenching moiety is no longer in proximity to the fluorescent label and fluorescence increases. Examples of this effect can be found in molecular beacons, fluorescent triplex oligos, triplex molecular beacons, triplex FRET probes, and QPNA probes, the operational principles of which can be adapted for use with riboswitches.

Stem activated labels, a form of conformation dependent labels, are labels or pairs of labels where fluorescence is increased or altered by formation of a stem structure. Stem activated labels can include an acceptor fluorescent label and a donor moiety such that, when the acceptor and donor are in proximity (when the nucleic acid strands containing the labels form a stem structure), fluorescence resonance energy transfer from the donor to the acceptor causes the acceptor to fluoresce. Stem activated labels are typically pairs of labels positioned on nucleic acid molecules (such as riboswitches) such that the acceptor and donor are brought into proximity when a stem structure is formed in the nucleic acid molecule. If the donor moiety of a stem activated label is itself a fluorescent label, it can release energy as fluorescence (typically at a different wavelength than the fluorescence of the acceptor) when not in proximity to an acceptor (that is, when a stem structure is not formed). When the stem structure forms, the overall effect would then be a reduction of donor fluorescence and an increase in acceptor fluorescence. FRET probes are an example of the use of stem activated labels, the operational principles of which can be adapted for use with riboswitches.

### H. Detection Labels

To aid in detection and quantitation of riboswitch activation, deactivation or blocking, or expression of nucleic acids or protein produced upon activation, deactivation or blocking of riboswitches, detection labels can be incorporated into detection probes or detection molecules or directly incorporated into expressed nucleic acids or proteins. As used herein, a detection label is any molecule that can be associated with nucleic acid or protein, directly or indirectly, and which results in a measurable, detectable signal, either directly or indirectly. Many such labels are known to those of skill in the art. Examples of detection labels suitable for use in the disclosed method are radioactive isotopes, fluorescent molecules, phosphorescent molecules, enzymes, antibodies, and ligands.

Examples of suitable fluorescent labels include fluorescein isothiocyanate (FITC), 5,6-carboxymethyl fluorescein, Texas red, nitrobenz-2-oxa-1,3-diazol-4-yl (NBD), coumarin, dansyl chloride, rhodamine, amino-methyl coumarin (AMCA), Eosin, Erythrosin, BODIPY^{®}, Cascade Blue^{®}, Oregon Green^{®}, pyrene, lissamine, xanthenes, acridines, oxazines, phycoerythrin, macrocyclic chelates of lanthanide ions such as quantum dye^{™}, fluorescent energy transfer dyes, such as thiazole orange-ethidium heterodimer, and the cyanine dyes Cy3, Cy3.5, Cy5, Cy5.5 and Cy7. Examples of other specific fluorescent labels include 3-Hydroxypyrene 5,8,10-Tri Sulfonic acid, 5-Hydroxy Tryptamine (5-HT), Acid Fuchsin, Alizarin Complexon, Alizarin Red, Allophycocyanin, Aminocoumarin, Anthroyl Stearate, Astrazon Brilliant Red 4G, Astrazon Orange R, Astrazon Red 6B, Astrazon Yellow 7 GLL, Atabrine, Auramine, Aurophosphine, Aurophosphine G, BAO 9 (Bisaminophenyloxadiazole), BCECF, Berberine Sulphate, Bisbenzamide, Blancophor FFG Solution, Blancophor SV, Bodipy F1, Brilliant Sulphoflavin FF, Calcien Blue, Calcium Green, Calcofluor RW Solution, Calcofluor White, Calcophor White ABT Solution, Calcophor White Standard Solution, Carbostyryl, Cascade Yellow, Catecholamine, Chinacrine, Coriphosphine O, Coumarin-Phalloidin, CY3.1 8, CY5.1 8, CY7, Dans (1-Dimethyl Amino Naphaline 5 Sulphonic Acid), Dansa (Diamino Naphtyl Sulphonic Acid), Dansyl NH-CH3, Diamino Phenyl Oxydiazole (DAO), Dimethylamino-5-Sulphonic acid, Dipyrrometheneboron Difluoride, Diphenyl Brilliant Flavine 7GFF, Dopamine, Erythrosin ITC, Euchrysin, FIF (Formaldehyde Induced Fluorescence), Flazo Orange, Fluo 3, Fluorescamine, Fura-2, Genacryl Brilliant Red B, Genacryl Brilliant Yellow 10GF, Genacryl Pink 3G, Genacryl Yellow 5GF, Gloxalic Acid, Granular Blue, Haematoporphyrin, Indo-1, Intrawhite Cf Liquid, Leucophor PAF, Leucophor SF, Leucophor WS, Lissamine Rhodamine B200 (RD200), Lucifer Yellow CH, Lucifer Yellow VS, Magdala Red, Marina Blue, Maxilon Brilliant Flavin 10 GFF, Maxilon Brilliant Flavin 8 GFF, MPS (Methyl Green Pyronine Stilbene), Mithramycin, NBD Amine, Nitrobenzoxadidole, Noradrenaline, Nuclear Fast Red, Nuclear Yellow, Nylosan Brilliant Flavin E8G, Oxadiazole, Pacific Blue, Pararosaniline (Feulgen), Phorwite AR Solution, Phorwite BKL, Phorwite Rev, Phorwite RPA, Phosphine 3R, Phthalocyanine, Phycoerythrin R, Polyazaindacene Pontochrome Blue Black, Porphyrin, Primuline, Procion Yellow, Pyronine, Pyronine B, Pyrozal Brilliant Flavin 7GF, Quinacrine Mustard, Rhodamine 123, Rhodamine 5 GLD, Rhodamine 6G, Rhodamine B, Rhodamine B 200, Rhodamine B Extra, Rhodamine BB, Rhodamine BG, Rhodamine WT, Serotonin, Sevron Brilliant Red 2B, Sevron Brilliant Red 4G, Sevron Brilliant Red B, Sevron Orange, Sevron Yellow L, SITS (Primuline), SITS (Stilbene Isothiosulphonic acid), Stilbene, Snarf 1, sulpho Rhodamine B Can C, Sulpho Rhodamine G Extra, Tetracycline, Thiazine Red R, Thioflavin S, Thioflavin TCN, Thioflavin 5, Thiolyte, Thiozol Orange, Tinopol CBS, True Blue, Ultralite, Uranine B, Uvitex SFC, Xylene Orange, and XRITC.

Useful fluorescent labels are fluorescein (5-carboxyfluorescein-N-hydroxysuccinimide ester), rhodamine (5,6-tetramethyl rhodamine), and the cyanine dyes Cy3, Cy3.5, Cy5, Cy5.5 and Cy7. The absorption and emission maxima, respectively, for these fluors are: FITC (490 nm; 520 nm), Cy3 (554 nm; 568 nm), Cy3.5 (581 nm; 588 nm), Cy5 (652 nm: 672 nm), Cy5.5 (682 nm; 703 nm) and Cy7 (755 nm; 778 nm), thus allowing their simultaneous detection. Other examples of fluorescein dyes include 6-carboxyfluorescein (6-FAM), 2',4',1,4,-tetrachlorofluorescein (TET), 2',4',5',7',1,4-hexachlorofluorescein (HEX), 2',7'-dimethoxy-4', 5'-dichloro-6-carboxyrhodamine (JOE), 2'-chloro-5'-fluoro-7',8'-fused phenyl-1,4-dichloro-6-carboxyfluorescein (NED), and 2'-chloro-7'-phenyl-1,4-dichloro-6-carboxyfluorescein (VIC). Fluorescent labels can be obtained from a variety of commercial sources, including Amersham Pharmacia Biotech, Piscataway, NJ; Molecular Probes, Eugene, OR; and Research Organics, Cleveland, Ohio.

Additional labels of interest include those that provide for signal only when the probe with which they are associated is specifically bound to a target molecule, where such labels include: "molecular beacons" as described in Tyagi & Kramer, Nature Biotechnology (1996) 14:303 and EP 0 070 685 B1. Other labels of interest include those described in U.S. Pat. No. 5,563,037; WO 97/17471 and WO 97/17076.

Labeled nucleotides are a useful form of detection label for direct incorporation into expressed nucleic acids during synthesis. Examples of detection labels that can be incorporated into nucleic acids include nucleotide analogs such as BrdUrd (5-bromodeoxyuridine, Hoy and Schimke, Mutation Research 290:217-230 (1993)), aminoallyldeoxyuridine (Henegariu et al., Nature Biotechnology 18:345-348 (2000)), 5-methylcytosine (Sano et al., Biochim. Biophys. Acta 951:157-165 (1988)), bromouridine (Wansick et al., J. Cell Biology 122:283-293 (1993)) and nucleotides modified with biotin (Langer et al., Proc. Natl. Acad. Sci. USA 78:6633 (1981)) or with suitable haptens such as digoxygenin (Kerkhof, Anal. Biochem. 205:359-364 (1992)). Suitable fluorescence-labeled nucleotides are Fluorescein-isothiocyanate-dUTP, Cyanine-3-dUTP and Cyanine-5-dUTP (Yu et al., Nucleic Acids Res., 22:3226-3232 (1994)). A preferred nucleotide analog detection label for DNA is BrdUrd (bromodeoxyuridine, BrdUrd, BrdU, BUdR, Sigma-Aldrich Co). Other useful nucleotide analogs for incorporation of detection label into DNA are AA-dUTP (aminoallyl-deoxyuridine triphosphate, Sigma-Aldrich Co.), and 5-methyl-dCTP (Roche Molecular Biochemicals). A useful nucleotide analog for incorporation of detection label into RNA is biotin-16-UTP (biotin-16-uridine-5'-triphosphate, Roche Molecular Biochemicals). Fluorescein, Cy3, and Cy5 can be linked to dUTP for direct labeling. Cy3.5 and Cy7 are available as avidin or anti-digoxygenin conjugates for secondary detection of biotin- or digoxygenin-labeled probes.

Detection labels that are incorporated into nucleic acid, such as biotin, can be subsequently detected using sensitive methods well-known in the art. For example, biotin can be detected using streptavidin-alkaline phosphatase conjugate (Tropix, Inc.), which is bound to the biotin and subsequently detected by chemiluminescence of suitable substrates (for example, chemiluminescent substrate CSPD: disodium, 3-(4-methoxyspiro-[1,2,-dioxetane-3-2'-(5'-chloro)tricyclo [3.3.1.1^{3,7}]decane]-4-yl) phenyl phosphate; Tropix, Inc.). Labels can also be enzymes, such as alkaline phosphatase, soybean peroxidase, horseradish peroxidase and polymerases, that can be detected, for example, with chemical signal amplification or by using a substrate to the enzyme which produces light (for example, a chemiluminescent 1,2-dioxetane substrate) or fluorescent signal.

Molecules that combine two or more of these detection labels are also considered detection labels. Any of the known detection labels can be used with the disclosed probes, tags, molecules and methods to label and detect activated or deactivated riboswitches or nucleic acid or protein produced in the disclosed methods. Methods for detecting and measuring signals generated by detection labels are also known to those of skill in the art. For example, radioactive isotopes can be detected by scintillation counting or direct visualization; fluorescent molecules can be detected with fluorescent spectrophotometers; phosphorescent molecules can be detected with a spectrophotometer or directly visualized with a camera; enzymes can be detected by detection or visualization of the product of a reaction catalyzed by the enzyme; antibodies can be detected by detecting a secondary detection label coupled to the antibody. As used herein, detection molecules are molecules which interact with a compound or composition to be detected and to which one or more detection labels are coupled.

### I. Sequence Similarities

It is understood that as discussed herein the use of the terms homology and identity mean the same thing as similarity. Thus, for example, if the use of the word homology is used between two sequences (non-natural sequences, for example) it is understood that this is not necessarily indicating an evolutionary relationship between these two sequences, but rather is looking at the similarity or relatedness between their nucleic acid sequences. Many of the methods for determining homology between two evolutionarily related molecules are routinely applied to any two or more nucleic acids or proteins for the purpose of measuring sequence similarity regardless of whether they are evolutionarily related or not.

In general, it is understood that one way to define any known variants and derivatives or those that might arise, of the disclosed riboswitches, aptamers, expression platforms, genes and proteins herein, is through defining the variants and derivatives in terms of homology to specific known sequences. This identity of particular sequences disclosed herein is also discussed elsewhere herein. In general, variants of riboswitches, aptamers, expression platforms, genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent homology to a stated sequence or a native sequence. Those of skill in the art readily understand how to determine the homology of two proteins or nucleic acids, such as genes. For example, the homology can be calculated after aligning the two sequences so that the homology is at its highest level.

Another way of calculating homology can be performed by published algorithms. Optimal alignment of sequences for comparison can be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989 which are herein incorporated by reference for at least material related to nucleic acid alignment. It is understood that any of the methods typically can be used and that in certain instances the results of these various methods can differ, but the skilled artisan understands if identity is found with at least one of these methods, the sequences would be said to have the stated identity.

For example, as used herein, a sequence recited as having a particular percent homology to another sequence refers to sequences that have the recited homology as calculated by any one or more of the calculation methods described above. For example, a first sequence has 80 percent homology, as defined herein, to a second sequence if the first sequence is calculated to have 80 percent homology to the second sequence using the Zuker calculation method even if the first sequence does not have 80 percent homology to the second sequence as calculated by any of the other calculation methods. As another example, a first sequence has 80 percent homology, as defined herein, to a second sequence if the first sequence is calculated to have 80 percent homology to the second sequence using both the Zuker calculation method and the Pearson and Lipman calculation method even if the first sequence does not have 80 percent homology to the second sequence as calculated by the Smith and Waterman calculation method, the Needleman and Wunsch calculation method, the Jaeger calculation methods, or any of the other calculation methods. As yet another example, a first sequence has 80 percent homology, as defined herein, to a second sequence if the first sequence is calculated to have 80 percent homology to the second sequence using each of calculation methods (although, in practice, the different calculation methods will often result in different calculated homology percentages).

### J. Hybridization and Selective Hybridization

The term hybridization typically means a sequence driven interaction between at least two nucleic acid molecules, such as a primer or a probe and a riboswitch or a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.

Parameters for selective hybridization between two nucleic acid molecules are well known to those of skill in the art. For example, in some embodiments selective hybridization conditions can be defined as stringent hybridization conditions. For example, stringency of hybridization is controlled by both temperature and salt concentration of either or both of the hybridization and washing steps. For example, the conditions of hybridization to achieve selective hybridization can involve hybridization in high ionic strength solution (6X SSC or 6X SSPE) at a temperature that is about 12-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art (Sambrook et al., Molecular Cloning: A Laboratory Manual, 2nd Ed., Cold Spring Harbor Laboratory, Cold Spring Harbor, New York, 1989; Kunkel et al. Methods Enzymol. 1987:154:367, 1987 which is herein incorporated by reference for material at least related to hybridization of nucleic acids). A preferable stringent hybridization condition for a DNA:DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or A-T richness of any area wherein high homology is desired, all as known in the art.

Another way to define selective hybridization is by looking at the amount (percentage) of one of the nucleic acids bound to the other nucleic acid. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting nucleic acid is in for example, 10 or 100 or 1000 fold excess. This type of assay can be performed at under conditions where both the limiting and non-limiting nucleic acids are for example, 10 fold or 100 fold or 1000 fold below their k_{d}, or where only one of the nucleic acid molecules is 10 fold or 100 fold or 1000 fold or where one or both nucleic acid molecules are above their k_{d}.

Another way to define selective hybridization is by looking at the percentage of nucleic acid that gets enzymatically manipulated under conditions where hybridization is required to promote the desired enzymatic manipulation. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the nucleic acid is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the nucleic acid molecules are extended. Preferred conditions also include those suggested by the manufacturer or indicated in the art as being appropriate for the enzyme performing the manipulation.

Just as with homology, it is understood that there are a variety of methods herein disclosed for determining the level of hybridization between two nucleic acid molecules. It is understood that these methods and conditions can provide different percentages of hybridization between two nucleic acid molecules, but unless otherwise indicated meeting the parameters of any of the methods would be sufficient. For example if 80% hybridization was required and as long as hybridization occurs within the required parameters in any one of these methods it is considered disclosed herein.

It is understood that those of skill in the art understand that if a composition or method meets any one of these criteria for determining hybridization either collectively or singly it is a composition or method that is disclosed herein.

### K. Nucleic Acids

There are a variety of molecules disclosed herein that are nucleic acid based, including, for example, riboswitches, aptamers, and nucleic acids that encode riboswitches and aptamers. The disclosed nucleic acids can be made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if a nucleic acid molecule is introduced into a cell or cell environment through for example exogenous delivery, it is advantageous that the nucleic acid molecule be made up of nucleotide analogs that reduce the degradation of the nucleic acid molecule in the cellular environment.

So long as their relevant function is maintained, riboswitches, aptamers, expression platforms and any other oligonucleotides and nucleic acids can be made up of or include modified nucleotides (nucleotide analogs). Many modified nucleotides are known and can be used in oligonucleotides and nucleic acids. A nucleotide analog is a nucleotide which contains some type of modification to either the base, sugar, or phosphate moieties. Modifications to the base moiety would include natural and synthetic modifications of A, C, G, and T/U as well as different purine or pyrimidine bases, such as uracil-5-yl, hypoxanthin-9-yl (I), and 2-aminoadenin-9-yl. A modified base includes but is not limited to 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Additional base modifications can be found for example in U.S. Pat. No. 3,687,808, Englisch et al., Angewandte Chemie, International Edition, 1991, 30, 613, and Sanghvi, Y. S., Chapter 15, Antisense Research and Applications, pages 289-302, Crooke, S. T. and Lebleu, B. ed., CRC Press, 1993. Certain nucleotide analogs, such as 5-substituted pyrimidines, 6-azapyrimidines and N-2, N-6 and O-6 substituted purines, including 2-aminopropyladenine, 5-propynyluracil and 5-propynylcytosine. 5-methylcytosine can increase the stability of duplex formation. Other modified bases are those that function as universal bases. Universal bases include 3-nitropyrrole and 5-nitroindole. Universal bases substitute for the normal bases but have no bias in base pairing. That is, universal bases can base pair with any other base. Base modifications often can be combined with for example a sugar modification, such as 2'-O-methoxyethyl, to achieve unique properties such as increased duplex stability. There are numerous United States patents such as 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941, which detail and describe a range of base modifications. Each of these patents is herein incorporated by reference in its entirety, and specifically for their description of base modifications, their synthesis, their use, and their incorporation into oligonucleotides and nucleic acids.

Nucleotide analogs can also include modifications of the sugar moiety. Modifications to the sugar moiety would include natural modifications of the ribose and deoxyribose as well as synthetic modifications. Sugar modifications include but are not limited to the following modifications at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted C1 to C10, alkyl or C2 to C10 alkenyl and alkynyl. 2' sugar modifications also include but are not limited to -O[(CH₂)n O]m CH₃, - O(CH₂)n OCH₃, -O(CH₂)n NH₂, -O(CH₂)n CH₃, -O(CH₂)n -ONH₂, and - O(CH₂)nON[(CH₂)n CH₃)]₂, where n and m are from 1 to about 10.

Other modifications at the 2' position include but are not limited to: C1 to C10 lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂ CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications can also be made at other positions on the sugar, particularly the 3' position of the sugar on the 3' terminal nucleotide or in 2'-5' linked oligonucleotides and the 5' position of 5' terminal nucleotide. Modified sugars would also include those that contain modifications at the bridging ring oxygen, such as CH₂ and S. Nucleotide sugar analogs can also have sugar mimetics such as cyclobutyl moieties in place of the pentofuranosyl sugar. There are numerous United States patents that teach the preparation of such modified sugar structures such as 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, each of which is herein incorporated by reference in its entirety, and specifically for their description of modified sugar structures, their synthesis, their use, and their incorporation into nucleotides, oligonucleotides and nucleic acids.

Nucleotide analogs can also be modified at the phosphate moiety. Modified phosphate moieties include but are not limited to those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. It is understood that these phosphate or modified phosphate linkages between two nucleotides can be through a 3'-5' linkage or a 2'-5' linkage, and the linkage can contain inverted polarity such as 3'-5' to 5'-3' or 2'-5' to 5'-2'. Various salts, mixed salts and free acid forms are also included. Numerous United States patents teach how to make and use nucleotides containing modified phosphates and include but are not limited to, 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, each of which is herein incorporated by reference its entirety, and specifically for their description of modified phosphates, their synthesis, their use, and their incorporation into nucleotides, oligonucleotides and nucleic acids.

It is understood that nucleotide analogs need only contain a single modification, but can also contain multiple modifications within one of the moieties or between different moieties.

Nucleotide substitutes are molecules having similar functional properties to nucleotides, but which do not contain a phosphate moiety, such as peptide nucleic acid (PNA). Nucleotide substitutes are molecules that will recognize and hybridize to (base pair to) complementary nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

Nucleotide substitutes are nucleotides or nucleotide analogs that have had the phosphate moiety and/or sugar moieties replaced. Nucleotide substitutes do not contain a standard phosphorus atom. Substitutes for the phosphate can be for example, short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts. Numerous United States patents disclose how to make and use these types of phosphate replacements and include but are not limited to 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, each of which is herein incorporated by reference its entirety, and specifically for their description of phosphate replacements, their synthesis, their use, and their incorporation into nucleotides, oligonucleotides and nucleic acids.

It is also understood in a nucleotide substitute that both the sugar and the phosphate moieties of the nucleotide can be replaced, by for example an amide type linkage (aminoethylglycine) (PNA). United States patents 5,539,082; 5,714,331; and 5,719,262 teach how to make and use PNA molecules, each of which is herein incorporated by reference. (See also Nielsen et al., Science 254:1497-1500 (1991)).

Oligonucleotides and nucleic acids can be comprised of nucleotides and can be made up of different types of nucleotides or the same type of nucleotides. For example, one or more of the nucleotides in an oligonucleotide can be ribonucleotides, 2'-O-methyl ribonucleotides, or a mixture of ribonucleotides and 2'-O-methyl ribonucleotides; about 10% to about 50% of the nucleotides can be ribonucleotides, 2'-O-methyl ribonucleotides, or a mixture of ribonucleotides and 2'-O-methyl ribonucleotides; about 50% or more of the nucleotides can be ribonucleotides, 2'-O-methyl ribonucleotides, or a mixture of ribonucleotides and 2'-O-methyl ribonucleotides; or all of the nucleotides are ribonucleotides, 2'-O-methyl ribonucleotides, or a mixture of ribonucleotides and 2'-O-methyl ribonucleotides. Such oligonucleotides and nucleic acids can be referred to as chimeric oligonucleotides and chimeric nucleic acids.

### L. Solid Supports

Solid supports are solid-state substrates or supports with which molecules (such as trigger molecules) and riboswitches (or other components used in, or produced by, the disclosed methods) can be associated. Riboswitches and other molecules can be associated with solid supports directly or indirectly. For example, analytes (e.g., trigger molecules, test compounds) can be bound to the surface of a solid support or associated with capture agents (e.g., compounds or molecules that bind an analyte) immobilized on solid supports. As another example, riboswitches can be bound to the surface of a solid support or associated with probes immobilized on solid supports. An array is a solid support to which multiple riboswitches, probes or other molecules have been associated in an array, grid, or other organized pattern.

Solid-state substrates for use in solid supports can include any solid material with which components can be associated, directly or indirectly. This includes materials such as acrylamide, agarose, cellulose, nitrocellulose, glass, gold, polystyrene, polyethylene vinyl acetate, polypropylene, polymethacrylate, polyethylene, polyethylene oxide, polysilicates, polycarbonates, teflon, fluorocarbons, nylon, silicon rubber, polyanhydrides, polyglycolic acid, polylactic acid, polyorthoesters, functionalized silane, polypropylfumerate, collagen, glycosaminoglycans, and polyamino acids. Solid-state substrates can have any useful form including thin film, membrane, bottles, dishes, fibers, woven fibers, shaped polymers, particles, beads, microparticles, or a combination. Solid-state substrates and solid supports can be porous or non-porous. A chip is a rectangular or square small piece of material. Preferred forms for solid-state substrates are thin films, beads, or chips. A useful form for a solid-state substrate is a microtiter dish. In some embodiments, a multiwell glass slide can be employed.

An array can include a plurality of riboswitches, trigger molecules, other molecules, compounds or probes immobilized at identified or predefined locations on the solid support. Each predefined location on the solid support generally has one type of component (that is, all the components at that location are the same). Alternatively, multiple types of components can be immobilized in the same predefined location on a solid support. Each location will have multiple copies of the given components. The spatial separation of different components on the solid support allows separate detection and identification.

Although useful, it is not required that the solid support be a single unit or structure. A set of riboswitches, trigger molecules, other molecules, compounds and/or probes can be distributed over any number of solid supports. For example, at one extreme, each component can be immobilized in a separate reaction tube or container, or on separate beads or microparticles.

Methods for immobilization of oligonucleotides to solid-state substrates are well established. Oligonucleotides, including address probes and detection probes, can be coupled to substrates using established coupling methods. For example, suitable attachment methods are described by Pease et al., Proc. Natl. Acad. Sci. USA 91(11):5022-5026 (1994), and Khrapko et al., Mol Biol (Mosk) (USSR) 25:718-730 (1991). A method for immobilization of 3'-amine oligonucleotides on casein-coated slides is described by Stimpson et al., Proc. Natl. Acad Sci. USA 92:6379-6383 (1995). A useful method of attaching oligonucleotides to solid-state substrates is described by Guo et al., Nucleic Acids Res. 22:5456-5465 (1994).

Each of the components (for example, riboswitches, trigger molecules, or other molecules) immobilized on the solid support can be located in a different predefined region of the solid support. The different locations can be different reaction chambers. Each of the different predefined regions can be physically separated from each other of the different regions. The distance between the different predefined regions of the solid support can be either fixed or variable. For example, in an array, each of the components can be arranged at fixed distances from each other, while components associated with beads will not be in a fixed spatial relationship. In particular, the use of multiple solid support units (for example, multiple beads) will result in variable distances.

Components can be associated or immobilized on a solid support at any density. Components can be immobilized to the solid support at a density exceeding 400 different components per cubic centimeter. Arrays of components can have any number of components. For example, an array can have at least 1,000 different components immobilized on the solid support, at least 10,000 different components immobilized on the solid support, at least 100,000 different components immobilized on the solid support, or at least 1,000,000 different components immobilized on the solid support.

### M. Kits

The materials described above as well as other materials can be packaged together in any suitable combination as a kit useful for performing, or aiding in the performance of, the disclosed method. It is useful if the kit components in a given kit are designed and adapted for use together in the disclosed method. For example disclosed are kits for detecting compounds, the kit comprising one or more biosensor riboswitches. The kits also can contain reagents and labels for detecting activation of the riboswitches.

### N. Mixtures

Disclosed are mixtures formed by performing or preparing to perform the disclosed method. For example, disclosed are mixtures comprising riboswitches and trigger molecules.

Whenever the method involves mixing or bringing into contact compositions or components or reagents, performing the method creates a number of different mixtures. For example, if the method includes 3 mixing steps, after each one of these steps a unique mixture is formed if the steps are performed separately. In addition, a mixture is formed at the completion of all of the steps regardless of how the steps were performed. The present disclosure contemplates these mixtures, obtained by the performance of the disclosed methods as well as mixtures containing any disclosed reagent, composition, or component, for example, disclosed herein.

### O. Systems

Disclosed are systems useful for performing, or aiding in the performance of, the disclosed method. Systems generally comprise combinations of articles of manufacture such as structures, machines, devices, and the like, and compositions, compounds, materials, and the like. Such combinations that are disclosed or that are apparent from the disclosure are contemplated. For example, disclosed and contemplated are systems comprising biosensor riboswitches, a solid support and a signal-reading device.

### P. Data Structures and Computer Control

Disclosed are data structures used in, generated by, or generated from, the disclosed method. Data structures generally are any form of data, information, and/or objects collected, organized, stored, and/or embodied in a composition or medium. Riboswitch structures and activation measurements stored in electronic form, such as in RAM or on a storage disk, is a type of data structure.

The disclosed method, or any part thereof or preparation therefor, can be controlled, managed, or otherwise assisted by computer control. Such computer control can be accomplished by a computer controlled process or method, can use and/or generate data structures, and can use a computer program. Such computer control, computer controlled processes, data structures, and computer programs are contemplated and should be understood to be disclosed herein.

### Methods

Disclosed are methods for activating, deactivating or blocking a riboswitch. Such methods can involve, for example, bringing into contact a riboswitch and a compound or trigger molecule that can activate, deactivate or block the riboswitch. Riboswitches function to control gene expression through the binding or removal of a trigger molecule. Compounds can be used to activate, deactivate or block a riboswitch. The trigger molecule for a riboswitch (as well as other activating compounds) can be used to activate a riboswitch. Compounds other than the trigger molecule generally can be used to deactivate or block a riboswitch. Riboswitches can also be deactivated by, for example, removing trigger molecules from the presence of the riboswitch. Thus, the disclosed method of deactivating a riboswitch can involve, for example, removing a trigger molecule (or other activating compound) from the presence or contact with the riboswitch. A riboswitch can be blocked by, for example, binding of an analog of the trigger molecule that does not activate the riboswitch.

Also disclosed are methods for altering expression of an RNA molecule, or of a gene encoding an RNA molecule, where the RNA molecule includes a riboswitch, by bringing a compound into contact with the RNA molecule. Riboswitches function to control gene expression through the binding or removal of a trigger molecule. Thus, subjecting an RNA molecule of interest that includes a riboswitch to conditions that activate, deactivate or block the riboswitch can be used to alter expression of the RNA. Expression can be altered as a result of, for example, termination of transcription or blocking of ribosome binding to the RNA. Binding of a trigger molecule can, depending on the nature of the riboswitch, reduce or prevent expression of the RNA molecule or promote or increase expression of the RNA molecule.

Also disclosed are methods for regulating expression of a naturally occurring gene or RNA that contains a riboswitch by activating, deactivating or blocking the riboswitch. If the gene is essential for survival of a cell or organism that harbors it, activating, deactivating or blocking the riboswitch can result in death, stasis or debilitation of the cell or organism. For example, activating a naturally occurring riboswitch in a naturally occurring gene that is essential to survival of a microorganism can result in death of the microorganism (if activation of the riboswitch turns off or represses expression). This is one basis for the use of the disclosed compounds and methods for antimicrobial and antibiotic effects. The compounds that have these antimicrobial effects are considered to be bacteriostatic or bacteriocidal.

Also disclosed are methods for selecting and identifying compounds that can activate, deactivate or block a riboswitch. Activation of a riboswitch refers to the change in state of the riboswitch upon binding of a trigger molecule. A riboswitch can be activated by compounds other than the trigger molecule and in ways other than binding of a trigger molecule. The term trigger molecule is used herein to refer to molecules and compounds that can activate a riboswitch. This includes the natural or normal trigger molecule for the riboswitch and other compounds that can activate the riboswitch. Natural or normal trigger molecules are the trigger molecule for a given riboswitch in nature or, in the case of some non-natural riboswitches, the trigger molecule for which the riboswitch was designed or with which the riboswitch was selected (as in, for example, *in vitro* selection or *in vitro* evolution techniques). Non-natural trigger molecules can be referred to as non-natural trigger molecules.

Also disclosed are methods of killing or inhibiting bacteria, comprising contacting the bacteria with a compound disclosed herein or identified by the methods disclosed herein.

Also disclosed are methods of identifying compounds that activate, deactivate or block a riboswitch. For example, compounds that activate a riboswitch can be identified by bringing into contact a test compound and a riboswitch and assessing activation of the riboswitch. If the riboswitch is activated, the test compound is identified as a compound that activates the riboswitch. Activation of a riboswitch can be assessed in any suitable manner. For example, the riboswitch can be linked to a reporter RNA and expression, expression level, or change in expression level of the reporter RNA can be measured in the presence and absence of the test compound. As another example, the riboswitch can include a conformation dependent label, the signal from which changes depending on the activation state of the riboswitch. Such a riboswitch preferably uses an aptamer domain from or derived from a naturally occurring riboswitch. As can be seen, assessment of activation of a riboswitch can be performed with the use of a control assay or measurement or without the use of a control assay or measurement. Methods for identifying compounds that deactivate a riboswitch can be performed in analogous ways.

In addition to the methods disclosed elsewhere herein, identification of compounds that block a riboswitch can be accomplished in any suitable manner. For example, an assay can be performed for assessing activation or deactivation of a riboswitch in the presence of a compound known to activate or deactivate the riboswitch and in the presence of a test compound. If activation or deactivation is not observed as would be observed in the absence of the test compound, then the test compound is identified as a compound that blocks activation or deactivation of the riboswitch.

Also disclosed are methods of detecting compounds using biosensor riboswitches. The method can include bringing into contact a test sample and a biosensor riboswitch and assessing the activation of the biosensor riboswitch. Activation of the biosensor riboswitch indicates the presence of the trigger molecule for the biosensor riboswitch in the test sample. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo* or *in vitro.* For example, cyclic di-GMP biosensor riboswitches operably linked to a reporter RNA that encodes a protein that serves as or is involved in producing a signal can be used *in vivo* by engineering a cell or organism to harbor a nucleic acid construct encoding the riboswitch/reporter RNA. An example of a biosensor riboswitch for use *in vitro* is a cyclic di-GMP riboswitch that includes a conformation dependent label, the signal from which changes depending on the activation state of the riboswitch. Such a biosensor riboswitch preferably uses an aptamer domain from or derived from a naturally occurring cyclic di-GMP riboswitch.

Also disclosed are compounds made by identifying a compound that activates, deactivates or blocks a riboswitch and manufacturing the identified compound. This can be accomplished by, for example, combining compound identification methods as disclosed elsewhere herein with methods for manufacturing the identified compounds. For example, compounds can be made by bringing into contact a test compound and a riboswitch, assessing activation of the riboswitch, and, if the riboswitch is activated by the test compound, manufacturing the test compound that activates the riboswitch as the compound.

Also disclosed are compounds made by checking activation, deactivation or blocking of a riboswitch by a compound and manufacturing the checked compound. This can be accomplished by, for example, combining compound activation, deactivation or blocking assessment methods as disclosed elsewhere herein with methods for manufacturing the checked compounds. For example, compounds can be made by bringing into contact a test compound and a riboswitch, assessing activation of the riboswitch, and, if the riboswitch is activated by the test compound, manufacturing the test compound that activates the riboswitch as the compound. Checking compounds for their ability to activate, deactivate or block a riboswitch refers to both identification of compounds previously unknown to activate, deactivate or block a riboswitch and to assessing the ability of a compound to activate, deactivate or block a riboswitch where the compound was already known to activate, deactivate or block the riboswitch. Further disclosed is a method of detecting a compound of interest, the method comprising: bringing into contact a sample and a riboswitch, wherein the riboswitch is activated by the compound of interest, wherein the riboswitch produces a signal when activated by the compound of interest, wherein the riboswitch produces a signal when the sample contains the compound of interest. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. The riboswitch can change conformation when activated by the compound of interest, wherein the change in conformation produces a signal via a conformation dependent label. The riboswitch can also change conformation when activated by the compound of interest, wherein the change in conformation causes a change in expression of an RNA linked to the riboswitch, wherein the change in expression produces a signal. The signal can be produced by a reporter protein expressed from the RNA linked to the riboswitch.

Further disclosed is a method of detecting a compound of interest, the method comprising: bringing into contact a sample and a riboswitch, wherein the riboswitch is activated by the compound of interest, wherein the riboswitch produces a signal when activated by the compound of interest, wherein the riboswitch produces a signal when the sample contains the compound of interest, wherein the riboswitch comprises a riboswitch or a derivative of a riboswitch. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. The riboswitch can change conformation when activated by the compound of interest, wherein the change in conformation produces a signal via a conformation dependent label. The riboswitch can also change conformation when activated by the compound of interest, wherein the change in conformation causes a change in expression of an RNA linked to the riboswitch, wherein the change in expression produces a signal. The signal can be produced by a reporter protein expressed from the RNA linked to the riboswitch.

Specifically, disclosed is a method of detecting cyclic di-GMP in a sample comprising: bringing a cyclic di-GMP-responsive riboswitch in contact with the sample; and detecting interaction between cyclic di-GMP and the cyclic di-GMP-responsive riboswitch, wherein interaction between cyclic di-GMP and the cyclic di-GMP-responsive riboswitch indicates the presence of cyclic di-GMP. The cyclic di-GMP-responsive riboswitch can be labeled.

Also disclosed is a method comprising: (a) testing a compound for inhibition of gene expression of a gene encoding an RNA comprising a riboswitch, wherein the inhibition is via the riboswitch, wherein the riboswitch comprises a riboswitch or a derivative of a riboswitch, (b) inhibiting gene expression by bringing into contact a cell and a compound that inhibited gene expression in step (a), wherein the cell comprises a gene encoding an RNA comprising a riboswitch, wherein the compound inhibits expression of the gene by binding to the riboswitch. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch.

Also disclosed is a method comprising: (a) testing a compound for derepression of gene expression of a gene encoding an RNA comprising a riboswitch, wherein the derepression is via the riboswitch, wherein the riboswitch comprises a riboswitch or a derivative of a riboswitch, (b) derepressing gene expression by bringing into contact a cell and a compound that derepressed gene expression in step (a), wherein the cell comprises a gene encoding an RNA comprising a riboswitch, wherein the compound derepresses expression of the gene by binding to the riboswitch. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch.

Also disclosed is a method of altering gene expression, the method comprising bringing into contact a compound and a cell, wherein the compound is cyclic di-GMP, pGpG, GpG, or GpGpG. The compound can also be a derivative of cyclic di-GMP, pGpG, GpG, or GpGpG that can activate a cyclic di-GMP-responsive riboswitch. Also disclosed is a method of altering gene expression, the method comprising bringing into contact a compound and a cell, wherein the compound is S-adenosylhomocysteine. The compound can also be a derivative of S-adenosylhomocysteine that can activate an S-adenosylhomocysteine-responsive riboswitch. Also disclosed is a method of altering gene expression, the method comprising bringing into contact a compound and a cell, wherein the compound is preQ₁. The compound can also be a derivative of preQ₁ that can activate a preQ₁-responsive riboswitch. Also disclosed is a method of altering gene expression, the method comprising bringing into contact a compound and a cell, wherein the compound is Moco. The compound can also be a derivative of Moco that can activate a Moco-responsive riboswitch. Also disclosed is a method of altering gene expression, the method comprising bringing into contact a compound and a cell, wherein the compound is SAM. The compound can also be a derivative of SAM that can activate a SAM-responsive riboswitch. The compound for activating a SAM-IV riboswitch can also be MeAzaAdoMet.

For activation of an S-adenosylhomocysteine-responsive riboswitch, the compound can be S-adenosylhomocysteine. The compound can also be a derivative of S-adenosylhomocysteine that can activate an S-adenosylhomocysteine-responsive riboswitch. For example, the compound can be S-adenosyl-L-cysteine (SAC). Figure 12B shows structural elements that are important for a compound that can activate an S-adenosylhomocysteine-responsive riboswitch.

For activation of preQ₁-responsive riboswitches (and riboswitches derived from preQ₁-responsive riboswitches), the compound can be derivatives of preQ₁ where the N at position 1 can be substituted with C, O or S, where the amino group at position 2 can be substituted with a hydrogen bond donor, where the oxygen at position 6 can be substituted with a hydrogen bond acceptor, where the amino group at position 7 can be substituted with a hydrogen bond acceptor, and where the nitrogen can be substituted or derivatized with a hydrogen bond donor.

The cell can be identified as being in need of altered gene expression. The cell can be a bacterial cell. The compound can kill or inhibit the growth of the bacterial cell. The compound and the cell can be brought into contact by administering the compound to a subject. The cell can be a bacterial cell in the subject, wherein the compound kills or inhibits the growth of the bacterial cell. The subject can have a bacterial infection. The cell can contain a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. The compound can be administered in combination with another antimicrobial compound. The compound can inhibit bacterial growth in a biofilm. Also disclosed is a method of altering the physiological state of a cell, the method comprising bringing into contact a compound and a cell, wherein the compound is cyclic di-GMP, pGpG, GpG, or GpGpG. The compound can also be a derivative of cyclic di-GMP, pGpG, GpG, or GpGpG that can activate a cyclic di-GMP-responsive riboswitch.

Disclosed is a method of identifying riboswitches, the method comprising assessing in-line spontaneous cleavage of an RNA molecule in the presence and absence of trigger molecule, wherein the RNA molecule is encoded by a gene regulated by the trigger molecule, wherein a change in the pattern of in-line spontaneous cleavage of the RNA molecule indicates a riboswitch responsive to the trigger molecule. The trigger molecule can be cyclic di-GMP, S-adenosylhomocysteine, preQ₁, Moco, or SAM.

Disclosed is a method comprising: (a) testing a compound for inhibition of gene expression of a gene encoding an RNA comprising a riboswitch, wherein the inhibition is via the riboswitch, wherein the riboswitch comprises a cyclic di-GMP-responsive riboswitch or a derivative of a cyclic di-GMP-responsive riboswitch, (b) inhibiting gene expression by bringing into contact a cell and a compound that inhibited gene expression in step (a), wherein the cell comprises a gene encoding an RNA comprising a riboswitch, wherein the compound inhibits expression of the gene by binding to the riboswitch.

Also disclosed is a method comprising inhibiting gene expression of a gene encoding an RNA comprising a riboswitch by bringing into contact a cell and a compound that was identified as a compound that inhibits gene expression of the gene by testing the compound for inhibition of gene expression of the gene, wherein the inhibition was via the riboswitch, wherein the riboswitch comprises a cyclic di-GMP-responsive riboswitch or a derivative of a cyclic di-GMP-responsive riboswitch.

### A. Identification of Antimicrobial Compounds

Riboswitches are a new class of structured RNAs that have evolved for the purpose of binding small organic molecules. The natural binding pocket of riboswitches can be targeted with metabolite analogs or by compounds that mimic the shape-space of the natural metabolite. The small molecule ligands of riboswitches provide useful sites for derivitization to produce drug candidates. Distribution of some riboswitches is shown in Table 1 of U.S. Application Publication No. 2005-0053951. Once a class of riboswitch has been identified and its potential as a drug target assessed, such as the cyclic di-GMP riboswitch, candidate molecules can be identified.

The emergence of drug-resistant stains of bacteria highlights the need for the identification of new classes of antibiotics. Anti-riboswitch drugs represent a mode of anti-bacterial action that is of considerable interest for the following reasons. Riboswitches control the expression of genes that are critical for fundamental metabolic processes. Therefore manipulation of these gene control elements with drugs yields new antibiotics. These antimicrobial agents can be considered to be bacteriostatic, or bacteriocidal. Riboswitches also carry RNA structures that have evolved to selectively bind metabolites, and therefore these RNA receptors make good drug targets as do protein enzymes and receptors.

A compound can be identified as activating a riboswitch or can be determined to have riboswitch activating activity if the signal in a riboswitch assay is increased in the presence of the compound by at least 1 fold, 2 fold, 3 fold, 4 fold, 5 fold, 50%, 75%, 100%, 125%, 150%, 175%, 200%, 250%, 300%, 400%, or 500% compared to the same riboswitch assay in the absence of the compound (that is, compared to a control assay). The riboswitch assay can be performed using any suitable riboswitch construct. Riboswitch constructs that are particularly useful for riboswitch activation assays are described elsewhere herein. The identification of a compound as activating a riboswitch or as having a riboswitch activation activity can be made in terms of one or more particular riboswitches, riboswitch constructs or classes of riboswitches. For convenience, compounds identified as activating a class of riboswitch or having riboswitch activating activity for a class of riboswitch can be so identified for particular riboswitches, such as the riboswitches of that class found in particular species. For example, compounds identified as activating a cyclic di-GMP riboswitch or having riboswitch activating activity for a cyclic di-GMP riboswitch can be so identified for particular cyclic di-GMP riboswitches, such as the cyclic di-GMP riboswitches found in, for example, *Vibrio cholerae or Gluconacetobacter xylinus.*

### B. Methods of Using Antimicrobial Compounds

Disclosed herein are *in vivo* and *in vitro* anti-bacterial methods. By "anti-bacterial" is meant inhibiting or preventing bacterial growth, killing bacteria, or reducing the number of bacteria. Thus, disclosed is a method of inhibiting or preventing bacterial growth comprising contacting a bacterium with an effective amount of one or more compounds disclosed herein. Additional structures for the disclosed compounds are provided herein.

Disclosed is a method of inhibiting bacterial cell growth, the method comprising: bringing into contact a cell and a compound that binds a riboswitch, wherein the cell comprises a gene encoding an RNA comprising a riboswitch responsive to the compound, wherein the compound inhibits bacterial cell growth by binding to the riboswitch, thereby altering expression of the gene. The riboswitch can be a cyclic di-GMP-responsive riboswitch, an S-adenosylhomocysteine-repsonsive riboswitch, a preQ₁-responsive riboswitch, a Moco-responsive riboswitch, or a SAM-responsive riboswitch. This method can yield at least a 10% decrease in bacterial cell growth compared to a cell that is not in contact with the compound. The compound and the cell can be brought into contact by administering the compound to a subject. The cell can be a bacterial cell in the subject, wherein the compound kills or inhibits the growth of the bacterial cell. The subject can have a bacterial infection. The compound can be administered in combination with another antimicrobial compound.

The bacteria can be any bacteria, such as bacteria from the genus *Bacillus, Acinetobacter, Actinobacillus, Burkholderia, Campylobacter, Clostridium, Desulfitobacterium, Enterococcus, Erwinia, Escherichia, Exiguobacterium, Fusobacterium, Geobacillus, Geobacter, Gluconacetobacter, Haemophilus, Heliobacter, Klebsiella, Idiomarina, Lactobacillus, Lactococcus, Leuconostoc, Listeria, Moorella, Mycobacterium, Oceanobacillus, Oenococcus, Pasteurella, Pediococcus, Pseudomonas, Shewanella, Shigella, Solibacter, Staphylococcus, Streptococcus, Thermoanaerobacter, Thermotoga, and Vibrio,* for example. The bacteria can be, for example, *Actinobacillus pleuropneumoniae, Bacillus anthracis, Bacillus cereus, Bacillus clausii, Bacillus halodurans, Bacillus licheniformis, Bacillus subtilis, Bacillus thuringiensis, Clostridium acetobutylicum, Clostridium dificile, Clostridium perfringens, Clostridium tetani, Clostridium thermocellum, Desulfitobacterium hafniense, Enterococcus faecalis, Erwinia carotovora, Escherichia coli, Exiguobacterium sp., Fusobacterium nucleatum, Geobacillus kaustophilus, Geobacter sulfurreducens, Gluconacetobacter xylinus, Haemophilus ducreyi, Haemophilus influenzae, Haemophilus somnus, Idiomarina loihiensis, Lactobacillus acidophilus, Lactobacillus casei, Lactobacillus delbrueckii, Lactobacillus gasseri, Lactobacillus johnsonii, Lactobacillus plantarum, Lactococcus lactis, Leuconostoc mesenteroides, Listeria innocua, Listeria monocytogenes, Moorella thermoacetica, Oceanobacillus iheyensis, Oenococcus oeni, Pasteurella multocida, Pediococcus pentosaceus, Pseudomonas aeruginosa, Shewanella oneidensis, Shigella flexneri, Solibacter usitatus, Staphylococcus aureus, Staphylococcus epidermidis, Thermoanaerobacter tengcongensis, Thermotoga maritima, Vibrio cholerae, Vibrio fischeri, Vibrio parahaemolyticus,* or *Vibrio vulnificus.* Bacterial growth can also be inhibited in any context in which bacteria are found. For example, bacterial growth in fluids, biofilms, and on surfaces can be inhibited. The compounds disclosed herein can be administered or used in combination with any other compound or composition. For example, the disclosed compounds can be administered or used in combination with another antimicrobial compound. "Inhibiting bacterial growth" is defined as reducing the ability of a single bacterium to divide into daughter cells, or reducing the ability of a population of bacteria to form daughter cells. The ability of the bacteria to reproduce can be reduced by about 10%, about 20%, about 30%, about 40%, about 50%, about 60%, about 70%, about 80%, about 90%, or 100% or more.

Also provided is a method of inhibiting the growth of and/or killing a bacterium or population of bacteria comprising contacting the bacterium with one or more of the compounds disclosed and described herein.

"Killing a bacterium" is defined as causing the death of a single bacterium, or reducing the number of a plurality of bacteria, such as those in a colony. When the bacteria are referred to in the plural form, the "killing of bacteria" is defined as cell death of a given population of bacteria at the rate of 10% of the population, 20% of the population, 30% of the population, 40% of the population, 50% of the population, 60% of the population, 70% of the population, 80% of the population, 90% of the population, or less than or equal to 100% of the population.

The compounds and compositions disclosed herein have anti-bacterial activity *in vitro* or *in vivo,* and can be used in conjunction with other compounds or compositions, which can be bactericidal as well.

By the term "therapeutically effective amount" of a compound as provided herein is meant a nontoxic but sufficient amount of the compound to provide the desired reduction in one or more symptoms. As will be pointed out below, the exact amount of the compound required will vary from subject to subject, depending on the species, age, and general condition of the subject, the severity of the disease that is being treated, the particular compound used, its mode of administration, and the like. Thus, it is not possible to specify an exact "effective amount." However, an appropriate effective amount may be determined by one of ordinary skill in the art using only routine experimentation.

The compositions and compounds disclosed herein can be administered *in vivo* in a pharmaceutically acceptable carrier. By "pharmaceutically acceptable" is meant a material that is not biologically or otherwise undesirable, i.e., the material may be administered to a subject without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

The compositions or compounds disclosed herein can be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

Parenteral administration of the composition or compounds, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795, which is incorporated by reference herein.

The compositions and compounds disclosed herein can be used therapeutically in combination with a pharmaceutically acceptable carrier. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

Compositions for oral administration include powders or granules, suspensions or solutions in water or non-aqueous media, capsules, sachets, or tablets. Thickeners, flavorings, diluents, emulsifiers, dispersing aids or binders may be desirable.

Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

Therapeutic compositions as disclosed herein may also be delivered by the use of monoclonal antibodies as individual carriers to which the compound molecules are coupled. The therapeutic compositions of the present disclosure may also be coupled with soluble polymers as targetable drug carriers. Such polymers can include, but are not limited to, polyvinyl-pyrrolidone, pyran copolymer, polyhydroxypropylmethacryl-amidephenol, polyhydroxyethylaspartamidephenol, or polyethyl-eneoxidepolylysine substituted with palmitoyl residues. Furthermore, the therapeutic compositions of the present disclosure may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.

Preferably at least about 3%, more preferably about 10%, more preferably about 20%, more preferably about 30%, more preferably about 50%, more preferably 75% and even more preferably about 100% of the bacterial infection is reduced due to the administration of the compound. A reduction in the infection is determined by such parameters as reduced white blood cell count, reduced fever, reduced inflammation, reduced number of bacteria, or reduction in other indicators of bacterial infection. To increase the percentage of bacterial infection reduction, the dosage can increase to the most effective level that remains non-toxic to the subject.

As used throughout, "subject" refers to an individual. Preferably, the subject is a mammal such as a non-human mammal or a primate, and, more preferably, a human. "Subjects" can include domesticated animals (such as cats, dogs, etc.), livestock (e.g., cattle, horses, pigs, sheep, goats, etc.), laboratory animals (e.g., mouse, rabbit, rat, guinea pig, etc.) and fish.

A "bacterial infection" is defined as the presence of bacteria in a subject or sample. Such bacteria can be an outgrowth of naturally occurring bacteria in or on the subject or sample, or can be due to the invasion of a foreign organism.

The compounds disclosed herein can be used in the same manner as antibiotics. Uses of antibiotics are well established in the art. One example of their use includes treatment of animals. When needed, the disclosed compounds can be administered to the animal via injection or through feed or water, usually with the professional guidance of a veterinarian or nutritionist. They are delivered to animals either individually or in groups, depending on the circumstances such as disease severity and animal species. Treatment and care of the entire herd or flock may be necessary if all animals are of similar immune status and all are exposed to the same disease-causing microorganism.

Another example of a use for the compounds includes reducing a microbial infection of an aquatic animal, comprising the steps of selecting an aquatic animal having a microbial infection, providing an antimicrobial solution comprising a compound as disclosed, chelating agents such as EDTA, TRIENE, adding a pH buffering agent to the solution and adjusting the pH thereof to a value of between about 7.0 and about 9.0, immersing the aquatic animal in the solution and leaving the aquatic animal therein for a period that is effective to reduce the microbial burden of the animal, removing the aquatic animal from the solution and returning the animal to water not containing the solution. The immersion of the aquatic animal in the solution containing the EDTA, a compound as disclosed, and TRIENE and pH buffering agent may be repeated until the microbial burden of the animal is eliminated. (US Patent 6,518,252).

Other uses of the compounds disclosed herein include, but are not limited to, dental treatments and purification of water (this can include municipal water, sewage treatment systems, potable and non-potable water supplies, and hatcheries, for example).

### C. Methods of Producing Trigger Molecules

Disclosed herein is a method of producing a trigger molecule, the method comprising: cultivating a mutant bacterial cell capable of producing the trigger molecule_{.} wherein the mutant bacterial cell comprises a mutation in a riboswitch responsive to the trigger molecule, which mutation increases production of the trigger molecule by the mutant bacterial cell in comparison to a cell not having the mutation; and isolating trigger molecule from the cell culture, thereby producing the trigger molecule. The trigger molecule can be cyclic di-GMP, S-adenosylhomocysteine, preQ₁, Moco, or SAM.

The mutant bacterial cell can be a knockout mutant, wherein the cell cannot produce the riboswitch responsive to the trigger molecule. The cell can have the region coding for the riboswitch removed completely. Production of the trigger molecule can be increased by 10, 20, 30, 40, 50, 60, 70, 80, 90% or more. This can be measured by the overall amount of trigger molecule compared to that produced by a cell that has an intact riboswitch.

Further disclosed is a bacterial cell comprising a mutation in a riboswitch responsive to a trigger molecule, which mutation measurably increases production of the trigger molecule by the cell when compared to a cell that does not have the mutation. By "measurably increases" is meant a 10, 20, 30, 40, 50, 60, 70, 80, 90% or more increase in production of the trigger molecule.

### Examples

### A. Example 1: Identification of 22 candidate structured RNAs in bacteria using the CMfinder comparative genomics pipeline

A computational pipeline based on comparative genomics was applied to bacteria, and 22 candidate RNA motifs were identified. Six were believed to be riboswitches, which are mRNA elements that regulate gene expression on binding a specific metabolite. In separate studies, it was confirmed that two of these are novel riboswitches. Three other riboswitch candidates are upstream of either a putative transporter gene in the order Lactobacillales, citric acid cycle genes in Burkholderiales, or molybdenum cofactor biosynthesis genes in several phyla. The remaining riboswitch candidate, the widespread GEMM (Genes for the Environment, for Membranes, and for Motility) motif is associated with genes important for natural competence in *Vibrio cholerae* and the use of metal ions as electron acceptors in *Geobacter sulfurreducens.* Among the other motifs, one has a genetic distribution similar to a previously published candidate riboswitch, *ykkC*/*yxkD,* but has a different structure. Possible non-coding RNAs were identified in five phyla, and several additional cis-regulatory RNAs, including one in ε-proteobacteria (upstream of *purD,* involved in purine biosynthesis), and one in Cyanobacteria (within an ATP synthase operon). These candidate RNAs add to the growing list of RNA motifs involved in multiple cellular processes, and show that many additional RNAs remain to be discovered.

Recent discoveries of novel structured RNAs (He 2006; Storz 2005; Kima 2006; Claverie 2005) indicate that such RNAs are common in cells. To assist in discovering additional structured RNAs, an automated pipeline has been developed that can identify conserved RNAs within bacteria (Yao 2007). This pipeline assembles the potential 5' UTRs (untranslated regions) ofmRNAs of homologous genes, and uses the CMfinder (Yao 2006) program to predict conserved RNA structures, or "motifs", within each set of UTRs. Automated homology searches are then employed to find additional examples of these motifs, which CMfinder uses to improve the secondary structure model and sequence alignment of each motif. The output is a set of alignments with a predicted RNA secondary structure, and these alignments are subsequently analyzed manually to make improvements to the model and to assess which motifs merit further study.

Although other automated searches for RNAs have been performed (Barrick 2004; Corbino 2005; Axmann 2005; Seliverstov 2005; McCutcheon 2003; Coventry 2004; Washietl 2005; Pedersen 2006; Torarinsson 2006), this pipeline (Yao 2007) is distinguished from these by three features. First, this pipeline uses CMfinder, which can discover a motif even when some input sequences either do not contain the motif or include motif representatives carrying unrelated sequence domains. CMfinder also can produce a useful alignment even with low sequence conservation, however, the algorithm will exploit whatever sequence similarity is present. Second, this pipeline integrates homology searches to automatically refine the alignment and structural model for each motif. Third, since this pipeline aligns UTRs of homologous genes, it is well suited to find cis-regulatory RNAs, and its dependence on sequence conservation is further reduced.

Indeed, using the bacterial phylum Firmicutes as a test case, it was previously demonstrated that this pipeline makes useful predictions for virtually all known *cis-*regulatory RNAs (Yao 2007). The pipeline also finds motifs that are likely to be *trans-*encoded, or "non-coding RNAs" (ncRNAs), when these happen to be upstream of homologous genes.

This pipeline can be used to find structured RNAs amongst all bacteria whose genomes have been sequenced. 22 motifs that are likely to be conserved, structured RNAs are described. Riboswitches, a type of structured RNA usually found in mRNAs that directly senses a specific small molecule and regulates gene expression, were the main focus of the study (Winkler 2005; Batey 2006). Subsequent experiments have confirmed that two of these motifs are novel riboswitches. The first binds SAH (*S-*adenosylhomocysteine) (Figure 1) and the second is a SAM (S-adenosylmethionine)-binding riboswitch in *Streptomyces coelicolor* and related species. Experimental evidence with another riboswitch candidate indicates that it senses molybdenum cofactor or "Moco".

A candidate of particular interest is the GEMM (Genes for the Environment, for Membranes, and for Motility) motif, which has properties that are typical of riboswitches. For example, GEMM is a widespread and highly conserved genetic element that, in 297 out of 309 cases, is positioned such that it is likely to be present in the 5' UTR of the adjacent open reading frame (ORF). The genes putatively regulated by GEMM are typically related to sensing and reacting to extracellular conditions, which suggests that GEMM can sense a metabolite produced for signal transduction or for cell-cell communication.

### 1. MATERIALS AND METHODS

### i. Identification of candidate RNA motifs

The potential 5'UTRs of genes classified in the Conserved Domain Database (Marchler-Bauer 2005) version 2.08 were used as input for the computational pipeline (Yao 2007). Completed genome sequences and gene positions were taken from RefSeq (Pruitt 2005) version 14, but genomes whose gene content was highly similar to other genomes was eliminated. The UTR extraction algorithm (Neph 2006) accounted for the fact that a UTR might not always be immediately upstream of the gene due to operon structure.

For each conserved domain, the collected sequences of potential UTRs were given as input to CMfinder version 0.2, which produced local multiple sequence alignments of structurally conserved motifs within the UTR sets. These alignments were then used to search for additional homologs within annotated intergenic regions, except that intergenic regions were extended by 50 nucleotides on both ends to account for misannotated ORFs. This homology search was performed with the RAVENNA program version 0.2f (Weinberg 2004; Weinberg 2004ii; Weinberg 2006) with ML-heuristic filters (Weinberg 2006), Covariance Model (Eddy 1994) in global mode implemented by Infernal (Eddy 2005) version 0.7, and an E-value (Klein 2003) cutoff of 10. CMfinder then refined its initial alignment using these new homologs. Known RNAs were detected based on the Rfam Database (Griffiths-Jones 2005) version 7.0. Predictions were scored based on phylogenetic conservation of short sequences, diversity of species and structural criteria. The pipeline algorithm is described in more detail elsewhere (Yao 2007).

Bacterial genomic sequence data was split into groups, and UTR extractions were performed, and a motif prediction and homology search was performed on each group separately. This was motivated by the fact that UTRs in different phyla often do not contain the same motif. However, phyla with few sequenced members were coalesced based on taxonomy to try to assemble sufficient sequence data to predict a motif. Phyla with only one or two sequenced members (e.g., Chloroflexi) were ignored. The following eight groups were used: (1) Firmicutes, (2) Actinobacteria, (3) α-proteobacteria, (4) β-proteobacteria, (5) γ-proteobacteria, (6) δ- and ε-proteobacteria, (7) Cyanobacteria and (8) Bacteroidetes, Chlorobi, Chlamydiae, Verrucomicrobia and Spirochaetes.

### ii. Analysis of candidate motifs and homology search strategies

Promising motifs were then selected, further analyzed them by performing additional homology searches, and edited their alignments with RALEE (Griffiths-Jones 2005). NCBI BLAST (Altschul 1997), Mfold (Zuker 2003), Rnall (Wan 2004) was used (to identify rho-independent transcription terminators), CMfinder and RAVENNA to assist in these analyses. Information on metabolic pathways associated with motifs was retrieved from KEGG (Kanehisa 2006). To expand alignments by identifying additional structured elements, alignments were extended on their 5' and 3' ends by 50-100 nucleotides, and realigned as necessary using either CMfinder or by manual inspection.

The additional searches for homologs used RAVENNA in several ways. Both global- and local-mode (Eddy 2005) Covariance Model searches gave complementary results. Sequence databases used in manually directed searches were the "microbial" subset of RefSeq version 19 (Pruitt 2005), and environmental shotgun sequences from acid mine drainage (Tyson 2004) (GenBank accession AADL01000000) and Sargasso Sea (Venter, 2004) (AACY01000000).

When appropriate, four kinds of subsets of these sequences were searched. First, environmental sequence data was not always used. Second, only genomes in the bacterial group were searched (e.g., β-proteobacteria) from which the motif was originally derived. Third, only intergenic regions (extended by 50 nucleotides as before) were searched. Fourth, the BLAST program tblastn was used to search for genes homologous to those associated with the motif. RAVENNA searches were then conducted on the 2 Kb upstream of these matches (which is expected to contain the 5' UTR), and 200 nucleotides downstream (since apparent coding homology might extend upstream of the true ORF, causing BLAST to misidentify the start codon). Using the motifs bacterial group as the BLAST database facilitated the discovery of highly diverged homologs. For example, a search upstream of *purD* genes in c-proteobacteria revealed homologs of the *purD* motif (see below) with a truncated stem. Additionally, with such small databases, the ML-heuristic filters were foregone in RAVENNA. When the full sequence set is used as the BLAST database, it can help to find homologs in other phyla.

### iii. Rejection of motifs

Motifs were rejected from further study when they failed to show features that are characteristic of structured RNAs. To help reject motifs with spurious predictions of structure, homology searches were performed using sequence information only, by removing all base pairs in the predicted structure. Sequence-based matches that do not conserve the structure indicate that the predicted structure is incorrect. However, such homologs can be missed by Covariance Models, which assume the structure is conserved. Several motifs were rejected using this strategy when sequence homologs revealed that the proposed structure was, in fact, poorly conserved.

### iv. Establishing the extent of Conservation and covariation for consensus diagrams

To establish the extent of conservation reflected in consensus diagrams (e.g., in Figure 1), sequences were weighted to de-emphasize highly similar homologs. Weighting used the GSC algorithm (Gerstein 1994), as implemented by Infernal (Eddy 2005), and weighted nucleotide frequencies were then calculated at each position in the multiple sequence alignment. To classify base pairs as covarying, the weighted frequency of Watson-Crick or G-U pairs was calculated. However, aligned sequences in which both nucleotides were missing or where the identity of either nucleotide was uncertain (e.g., was "N", signifying any of the four bases) were discarded. Classification as a covarying position was made if two sequences had Watson-Crick or G-U pairs that differ at both positions amongst sequences that carry the motif. If only one position differed, the occurrence was classified as a compatible mutation. However, if the frequency of non-Watson-Crick or G-U pairs was more than 5%, these positions were not annotated as covarying or as compatible mutations.

### 2. RESULTS

### i. Evaluation and analysis of novel RNA motifs

Promising structured RNA motifs predicted by the CMfinder pipeline were examined manually to refine the consensus sequence and structural models (see MATERIALS AND METHODS) and to provide information on possible function. Key findings for each candidate are summarized in Table 1. Of the 22 motifs identified, seven are depicted in Figure 1.

**Table 1.** Summary of putative structured RNA motifs. "RNA" = functions as RNA (as opposed to dsDNA), "Cis" = *cis*-regulatory, "Switch" = riboswitch. Evaluation: "Y" = certainly true, "y" = probably true, "?" = possible, "n" = probably not, "N" = certainly not. These evaluations were conducted prior to experimental examinations. Remaining columns are "Phylum/class" (phylum containing the motif, or class for Proteobacteria), "M,V" ("M" = has modular stems, which are stems that are only sometimes present, "V" = variable-length stems), "Cov." = number of covarying paired positions (see Methods; note that it is not advisable to rank motifs solely by this number, but rather the alignment as a whole should be evaluated), "#" = number of representatives, "Non cis" = X/Y where X is number of representatives that are *not* in a 5' regulatory configuration to a gene and Y is the number of representatives within sequences that have annotated genes (some RefSeq sequences lack annotations). Moco and SAM-IV riboswitch data will be presented in future reports. Gamma-150 and coccus-1 are only in the supplement.

| Motif | RNA? | Cis? | Switch? | Phylum/class | M,V | Cov. | # | Non cis |
|---|---|---|---|---|---|---|---|---|
| GEMM | Y | Y | y | Widespread | V | 21 | 322 | 12/309 |
| Moco | Y | Y | Y | Widespread | M,V | 15 | 105 | 3/81 |
| SAH | Y | Y | Y | Proteobacteria | M,V | 22 | 42 | 0/41 |
| SAM-IV | Y | Y | Y | Actinobacteria | V | 28 | 54 | 2/54 |
| COG4708 | Y | Y | y | Firmicutes | M,V | 8 | 23 | 0/23 |
| *sucA* | Y | Y | y | β-proteobacteria | | 9 | 40 | 0/40 |
| 23S-methyl | Y | Y | n | Firmicutes | | 12 | 38 | 1/37 |
| *hemB* | Y | ? | | β-proteobacteria | V | 12 | 50 | 2/50 |
| *(anti-hemB)* | | (n) | (n) | | | | (37) | (31/37) |
| MAEB | ? | Y | n | β-proteobacteria | | 3 | 662 | 15/646 |
| *mini-ykkc* | Y | Y | ? | Widespread | V | 17 | 208 | 1/205 |
| *purD* | Y | Y | ? | ε-proteobacteria | M | 16 | 21 | 0/20 |
| 6C | Y | ? | n | Actinobacteria | | 21 | 27 | 1/27 |
| alpha- | ? | N | N | α-proteobacteria | | 16 | 102 | 39/99 |
| transposases | | | | | | | | |
| excisionase | ? | ? | n | Actinobacteria | | 7 | 27 | 0/27 |
| ATPC | y | ? | ? | Cyanobacteria | | 11 | 29 | 0/23 |
| cyano-30S | Y | Y | n | Cyanobacteria | | 7 | 26 | 0/23 |
| lacto-1 | ? | ? | n | Firmicutes | | 10 | 97 | 18/95 |
| lacto-2 | y | N | n | Firmicutes | | 14 | 357 | 67/355 |
| TD-1 | y | ? | n | Spirochaetes | M,V | 25 | 29 | 2/29 |
| TD-2 | y | N | n | Spirochaetes | V | 11 | 36 | 17/36 |
| coccus-1 | ? | N | N | Firmicutes | | 6 | 246 | 112/189 |
| gamma-150 | ? | N | N | γ-proteobacteria | | 9 | 27 | 6/27 |

The decision to select a candidate RNA motif for further study was based on a qualitative evaluation of conservation of both sequence and structure, covariation, and gene context (e.g., whether or not the motif is consistently upstream of a specific gene family). Conserved sequence is important because structured RNAs usually have many conserved nucleotides in regions that form complex tertiary structures or are under other constraints. Structured RNAs sometimes have variable-length stems or "modular stems" (stems that are present in some but not all representatives). The fact that both sides of a stem either appear together or neither appear is analogous to covariation, and is evidence that the structure is conserved.

Cis-regulatory RNAs such as riboswitches are regions of mRNAs that regulate mRNA genes. In bacteria, most cis-regulatory RNAs occur in the 5' UTR of the mRNA under regulation. Although it is not possible to reliably predict the transcription start site, representatives of a motif are declared as positioned in a "5' regulatory configuration" to a gene when the element could be in the 5' UTR of an mRNA (if the transcription start site is 5' to the element). When most or all representatives of a motif are in a 5' regulatory configuration to a gene, this is evidence that the motif can have a *cis* regulatory function.

*Cis*-regulatory RNAs often have one of two noteworthy structural features: rho-independent transcription terminators, or stems that overlap the Shine-Dalgarno sequence (bacterial ribosome binding site) (Winkler 2005). Rho-independent transcription terminators consist of a strong hairpin followed by four or more U residues (Henkin 2002). Regulatory RNA domains can control gene expression by conditionally forming the terminator stem. Similarly, conditionally formed stems can overlap the Shine-Dalgamo sequence, thereby regulating genes at the translational level (Winkler 2005).

Some motifs identified in this study consist of single or tandem hairpins. It is possible that some of these are protein-binding motifs in which a homodimeric protein binds to a given DNA-based element in opposite strands. For convenience, such motifs are also described as having a 5' regulatory configuration, even though they might not form structured RNAs or function at the mRNA level.

### ii. The GEMM motif

GEMM is widespread in bacteria and appears to have a highly conserved sequence and structure suggestive of a function that imposes substantial biochemical constraints on the putative RNA. 322 GEMM sequences were found in both Gram-positive and Gram-negative bacteria. It is common in δ-proteobacteria, particularly in Geobacter and related genera. Within γ-proteobacteria, it is ubiquitous in the Alteromonadales and Vibrionales. It is also common in certain orders of the phyla Firmicutes and Plantomycetes. Prominent pathogens with GEMM include the causative agents of cholera and anthrax.

Out of 309 GEMM instances where sequence data includes gene annotations, GEMM is in a 5' regulatory configuration to a gene in 297 cases, implying a *cis-*regulatory role. Genes presumably regulated by GEMM display a wide range of functions, but most genes relate to the extracellular environment or to the membrane, and many are related to motility.

GEMM consists of two adjacent hairpins (paired regions) designated P1 and P2 (Figures 1 and 3). P1 is highly conserved in sequence and structure, and consists of 2- and 6-base-pair stems separated by a 3-nucleotide internal loop and capped by a terminal loop. The internal loop is highly conserved, and the terminal loop is almost always a GNRA tetraloop (Hendrix 1997). The P1 stem exhibits considerable evidence of covariation at several positions, and is highly conserved in structure over a wide range of bacteria. This fact, and the more modest covariation and variable-length stems of P2, provide strong evidence that GEMM functions as a structured RNA. Example 2 demonstrates that GEMM motifs function in riboswitches. The sequence linking P1 and P2 is virtually always AAA, with only 2 exceptions in 322 examples.

The P2 hairpin shows more modest conservation than P1. When the P1 tetraloop is GAAA, a GNRA tetraloop receptor usually appears in P2. This receptor is often the well-known 11-nt motif, which might be favored by GAAA loops, but some sequences could be novel tetraloop receptors. When P1 has a GYRA tetraloop, the receptor-like sequence is almost never present, although a bulge nearer the P2 base is sometimes found (Figure 2).

Many instances of GEMM include a rho-independent transcription terminator hairpin. The 5' side of the terminator stem often overlaps (and presumably competes with) the 3' side of the P2 stem (Figure 2B). If GEMM is a riboswitch, ligand binding can stabilize the proposed P1 and P2 structure, thus preventing the competing transcription terminator from forming. In this model, higher ligand concentrations increase gene expression. One-third of GEMM representatives in δ-proteobacteria, and some in other taxa, are in a "tandem" arrangement, wherein one instance appears 3' and nearby to another in the same UTR. Such arrangements of regulatory RNAs are implicated in more sophisticated control of gene expression than is permitted by a simple regulatory RNA configuration (Sudarsan 2006; Welz 2007; Mandal 2006).

An understanding of the biological role of GEMM can shed light on the broad variety of microbial processes that it appears to regulate. In fact, GEMM is implicated in two systems that are already the object of several studies, in the species *Vibrio cholerae* and in *Geobacter sulfurreducens. Vibrio cholerae* causes cholera in humans, but spends much of its lifecycle in water, where it can adhere to chitin-containing exoskeletons of many crustaceans. Chitin, a polymer of GlcNAc (N-acetylglucosamine), has been shown to affect expression of many *V. cholerae* genes (Meibom 2004). GEMM appears to regulate two of these chitin-induced genes. The first, *gbpA,* is important for adhering to chitin beads (Meibom 2004) and human epithelial cells (Kirn 2005), as well as infection of mice (Kirn 2005).

The second chitin-induced gene is *tfoX*^{VC}. Remarkably, chitin induces natural competence in *V. cholerae* (Meibom 2005), and *tfoX*^{VC} expression is essential for this competence. *V. cholerae* has two genes that match the CDD models COG3070 and pfam04994 that correspond to separate *tfoX* domains. Both domains yield RPSBLAST (Schaffer 2001) E-values better than 10⁻²⁵. One of these is *tfoX*^{VC} (locus VC1153). GEMM appears to regulate the other, which is referred to as *tfoX*^{GEMM} (VC1722). Thus, in *V. cholerae* and related bacteria, GEMM might participate in chitin-induced competence, or even regulate competence in environments not containing elevated chitin concentrations.

*Geobacteria sulfurreducens* and related δ-proteobacteria can generate ATP by oxidizing organic compounds, using metal ions such as Fe(III) as electron acceptors (Methé 2003). GEMM is associated with pili assembly genes in *Geobacter* species. Pili in *G. sulfurreducens* have been shown to conduct electricity (Reguera 2005), and are thus a part of the process of reducing metal ions.

Moreover, GEMM appears to regulate seven cytochrome c genes in *G*. *sulfurreducens.* Although this bacteria has 111 putative cytochrome *c* genes, 5 of the 7 GEMM-associated genes have been identified in previous studies, and might have special roles. OmcS (Outer-Membrane Cytochrome S) is one of two proteins that are highly abundant on the outer membrane of *G. sulfurreducens,* and is required for reducing insoluble Fe(III) oxide, but not for soluble Fe(III) citrate (Mehta 2005). OmcG and OmcH are necessary for production of OmcB, an essential cytochrome c in many conditions (Kimm 2006). OmcA and OmcT are associated with OmcG, OmcH or OmcS. Only four other Omc annotations remain in *G*. *sulfurreducens* that have no direct GEMM association: OmcB, OmcC, OmcE and OmcF.

Unlike known riboswitches, GEMM is associated with a great diversity of gene functions (Table 2). This observation indicates that the GEMM riboswitch is not serving as a typical feedback sensor for control of a metabolic pathway. Rather, GEMM senses a second-messenger molecule involved in signal transduction or possibly cell-cell communication (Bassler 2006). Example 2 demonstrates that the secondary-messenger trigger molecule of the GEMM motif id cyclic di-GMP. In this way, different bacteria use GEMM and its signaling molecule to control different processes. The fact that many GEMM-associated genes encode signal transduction domains indicates a mechanism by which many of the signal transduction proteins are regulated. Example 2 demonstrates that GEMM RNAs indeed serve as aptamer components of a new-found riboswitch class.

**Table 2. Gene families that appear to be regulated by GEMM in more than one instance.**

| Functional role | Gene families |
|---|---|
| Pili and flagella | *cpaA, flgB, flgC, flgG, fliE, fliG, fliM, motA, motB, papC, papD, pilM, pilO, pilQ, pulF.* |
| Secretion (related | *fhaC, fliF, fliI, hofQ* |
| to pili/flagella) | |
| Chemotaxis | *cheW, cheY,* methyl-accepting chemotaxis protein, Cache domain |
| regulator | (classically associated with chemotaxis receptors in bacteria). |
| Signal | PAS domain, histidine kinase, HAMP (Histidine kinases, Adenylyl |
| transduction | cyclases, Methyl binding proteins, Phosphatases), HD-GYP domain, GGDEF domain |
| Chitin | chitin/cellulose binding domain, chitinase, carbohydrate-binding protein |
| Membranes | lysin domain (involved in cell wall remodeling, but might have general peptidoglycan binding function), uncharacterized outer membrane proteins and lipoproteins, putative collagen binding protein |
| Peptides | non-ribosomal peptide synthase, condensation domain (synthesis of peptide antibiotics), transglutaminase-like cysteine protease, subtilase (superfamily of extracellular peptidases). |
| Other | *tfoX* (regulator of competence), cytochrome c |

### iii. The SAH motif

The SAH motif is highly conserved in sequence and structure (Figure 1), showing covariation within predicted stem regions, including modular and variable-length stems. The SAH motif is found in a 5' regulatory configuration to genes related to SAH (*S-*adenosylhomocysteine) metabolism, primarily in β- and some γ-proteobacteria, and especially the genus *Pseudomonas.* SAH is a part of the *S*-adenosylmethionine (SAM) metabolic cycle, whose main components include the amino acid methionine and its derivative homocysteine. SAH is a byproduct of enzymes that use SAM as a cofactor for methylation reactions. Typically, SAH is hydrolyzed into homocysteine and adenosine. Homocysteine is then used to synthesize methionine, and ultimately SAM.

High levels of SAH are toxic to cells because SAH inhibits many SAM-dependent methyltransferases (Ueland 1982). Therefore cells likely need to sense rising SAH concentrations and dispose of this compound before it reaches toxic levels. The genes that the SAH motif associates with are *S*-adenosylhomocysteine hydrolase (*ahcY*), cobalamin-dependent methionine synthase (*metH*) and methylenetetrahydrofolate reductase (*metF*), which synthesizes a methyl donor used in methionine synthesis. This genetic arrangement of the SAH motif and its high degree of conservation are consistent with a role in sensing SAH and activating the expression of genes whose products are required for SAH destruction. Indeed, biochemical and genetic evidence supports the hypothesis that this motif is an SAH-sensing riboswitch.

### iv. The COG4708 motif

This motif is found upstream of COG4708 genes in some species of *Streptococcus* and in *Lactococcus lactis,* although some instances of the COG4708 gene family in *Streptococcus* lack the putative RNA motif. COG4708 genes are predicted to encode membrane proteins.

Although the COG4708 motif (which can also be referred to as the preQ₁-II motif) is highly constrained phylogenetically and has only six unique sequences, it shows covariation, modular stems, and variable-length stems (Figure 1). The motif has a pseudoknot that overlaps the Shine-Dalgarno sequences of COG4708 genes, which shows that the motif encodes a *cis*-regulator of these genes.

A riboswitch that senses the modified nucleobase preQ₁ was recently characterized (Roth 2007). Since this riboswitch is associated with COG4708, it was proposed that COG4708 is a transporter of a metabolite related to preQ₁. Therefore, it was believed that the COG4708 motif is also a preQ₁-sensing riboswitch. Experiments have supported this. The COG4708 motif shares no similarity in sequence or structure with the previously characterized preQ₁-sensing riboswitch (Roth 2007).

### v. The sucA motif

The *sucA* motif is only found in a 5' regulatory configuration to *sucA* genes, which are likely co-transcribed with the related downstream genes *sucB*/*aceF* and *lpd.* The products of these three genes synthesize succinyl-CoA from 2-oxoglutarate in the citric acid cycle. All detected instances of the *sucA* motifs are in β-proteobacteria in the order Burkholderiales. Although many nucleotides in the *sucA* motif are strictly conserved, those that are not show covariation and contain very few non-canonical base pairs (Figure 1). The motif has stems that overlap the putative Shine-Dalgarno sequence, so the *sucA* motif can correspond to a *cis*-regulatory RNA. The relatively complex structure of the *sucA* motif shows that it can be a riboswitch.

### vi. The 23S-methyl motif

This motif is consistently upstream of genes annotated as rRNA methyltransferases that can act on 23S rRNA. The one exception occurs when 23S-methyl RNA is roughly 3 Kb from the 23S rRNA methyltransferase ORF, with other genes on the opposite strand in the intervening sequence. The 23S-methyl motif is confined to the Lactobacillales, which is an order of Firmicutes.

The 23S-methyl motif consists of two large hairpins. The second hairpin ends in a run of Us and can be a rho-independent transcription terminator. Both stems have considerable covariation, providing strong evidence that they are part of a functional RNA. Although the structural model shows that many paired positions sometimes have non-canonical base pairs, each instance of the motif consists predominantly of energetically favorable pairs. The presence of a putative transcription terminator shows that this is a *cis*-regulatory RNA. Since 23S rRNA methyltransferase interacts with an RNA substrate, it might autoregulate its expression using the 23S-methyl motif, in a manner similar to autoregulation of ribosomal protein genes (Zengel 1994).

### vii. The hemB/anti-hemB motif

This motif is found in a variety of β-proteobacteria, especially *Burkholderia.* There is some ambiguity as to the DNA strand from which it might be transcribed, because its structure exhibits comparable covariation and conservation in both directions. In one direction, it is often upstream of *hemB* genes. It could be a *cis*-regulatory RNA in this direction, but there are two genes, not homologous to each other, that are immediately downstream of *hemB* motif representatives and these are positioned on the wrong strand to be controlled in the usual manner of *cis*-regulatory RNAs. In the other direction (anti-*hem*B), the motif is not typically in the 5'UTRs of genes. The *anti-hemB* motif ends in a transcription terminator hairpin. Many genes downstream of *anti-hemB* instances are on the opposite strand, thus indicating that *anti-hemB* could encode a non-coding RNA.

### viii. MAEB (Metabolism-Associated Element in Burkholderia)

This motif consists of a single hairpin with several conserved positions (Figure 1). It is widespread in *Burkholderia,* a genus of β-proteobacteria. It typically occurs multiple times in succession (2-6 copies) with conserved linker sequences, but ranges to as many as 12 copies in two instances. In 141 occurrences of single or repetitive MAEB motifs, 132 are in a 5' regulatory configuration to a gene. In fact, many of these genes are directly involved in primary metabolism (e.g., genes involved in biosynthesis, catabolism or transport of small molecules), and not genes such as DNA repair, replication, signaling or motility. Out of the 46 conserved domains (excluding hypothetical genes) downstream of MAEB in more than one instance, at least 42 are annotated as participating in primary metabolism. There are many bacterial genes not involved in primary metabolism, so these data show a functional association with metabolic gene control.

There can exist a relationship between MAEB and cellular response to abundant glycine. MAEB is frequently associated with *gcvP* and *gcvT,* which are part of the glycine cleavage system, wherein excess glycine feeds into the citric acid cycle. MAEB is also associated with several citric acid cycle genes. However, MAEB is associated with some other genes with a more tenuous relationship to glycine or the citric acid cycle. A relationship to the glycine cleavage system can exist because the highest number of MAEB repeats are associated with the gcv genes in this system. Moreover, there exists at least one riboswitch class that binds glycine, but this class is present in only one copy per genome in organisms with MAEB, which can leave a role in glycine regulation for

### MAEB.

Representatives of the MAEB motif exhibit covariation that preserves base pairing, but others carry mutations that disrupt pairing. This fact shows that it can, in fact, be a DNA-sequence that binds a protein dimer, such that each protein unit binds to opposite strands. Nucleotides at one pair of symmetric positions are conserved as purines (A or G) in both sides of the stem. Since purines are never Watson-Crick pairs, they can not have the same identity on opposite strands. Although it is expected that instances of a DNA binding motif can differ, the symmetric purines imply that the motif itself (and not merely the instances) has a distinct pattern on opposite strands.

### ix. The mini-ykkc motif

The mini-*ykkc* motif consists of two tandem hairpins whose stems show considerable covariation and whose loops have characteristic ACGR motifs (Figure 1). Mini-*ykkC* is widespread in α-, β- and γ-proteobacteria, with additional examples in other taxa. This motif was named mini-*ykkc* because it appears to be a *cis*-regulator of a set of genes similar to that of the previously described *ykkClyxkD motif* (*Barrick* 2004) (hereafter termed "*ykkC*"). All eight conserved domains common to *ykkC* and mini-*ykkc* are listed below. The structures of *ykkC* and mini-*ykkC* appear to be unrelated.

The *ykkC* motif is a highly structured and broadly conserved motif. The simple structure of mini-*ykkC*, however, is uncharacteristic of most other riboswitches, though its broad phylogeny suggests a function that dictates broad conservation. Mini-*ykkC* appears to be a *cis*-regulatory element because it is associated with a relatively narrow set of gene functions and it is near to their coding sequences (90% are within 33 nucleotides of the Shine-Dalgarno sequence). Mini-*ykkC* can serve the same role as the *ykkC* motif, although the mechanisms used to control gene expression can be different. It is noted that there can be instances of mini-*ykkc* with only one hairpin.

### x. The purD motif

The *purD* motif is found upstream of all *purD* genes in fully sequenced ε-proteobacteria (e.g., *Campylobacter* and *Helicobacter*). The *purD* gene encodes GAR (phosphoribosylglycinamide) synthetase, which is involved in purine biosynthesis. The *purD* motif shows covariation and modular stems, although it also exhibits some mutations that disrupt base pairing (Figure 1).

### xi. The 6C motif

This motif is widespread among Actinobacteria, and consists of two hairpins, where the loop of each contains a run of at least 6 Cs. The 6C motif exhibits significant covariation in its stems. 6C motif instances are usually moderately close (200-300 nucleotides) to genes predicted to be related to chromosome partitioning and pilus assembly.

### xii. Transposon- and excisionase-associated motifs

One transposase-associated motif in α-proteobacteria and another associated with Xis excisionases in Actinobacteria were also found. Both motifs consist primarily of a single hairpin with a 10-to-15-base-pair stem. Both motifs also exhibit much covariation, which suggests they form functional, structured RNAs. The excisionase motif is in a 5' regulatory configuration to the excisionase gene.

### xiii. The ATPC motif

The ATPC motif occurs in some Cyanobacteria in an ATP synthase operon, between genes encoding the A and C subunits. ATPC motif instances are found in all sequenced strains of *Prochlorococcus marinus,* and certain species of *Synechococcus.* The motif consists primarily of a three-stem junction. Previous studies have proposed hairpin-like structures in Cyanobacterial ATP synthase operons, but not more complicated shapes, and in different locations from the ATPC motif Curtis 1988).

### xiv. The cyano-30S motif

This motif occurs in some Cyanobacteria and is in a 5' regulatory configuration to genes encoding 30S ribosomal protein S1. It consists of two hairpins that are dissimilar to each other, and a pseudoknot wherein five nucleotides in the P1 loop base-pair with nucleotides just beyond the 3' end of P2. Although there are several mutations that disrupt pairing in P1 and P2, there are also many compensatory mutations in these stems. Moreover, the pairing in the pseudoknot covaries and is present in all representatives.

Given the gene context, this motif can mimic the ligand of the downstream ribosomal protein gene (Zengel 1994), and that the product of this gene thereby controls its own expression. The identification of the cyano-30S motif supports the view that such RNAs are found in a wide variety of phyla.

### xv. Lactobacillales motifs

The lacto-1 and lacto-2 motifs are confined to the order Lactobacillales. The lacto-1 motif has some covariation, but some mutations disrupt base pairing. Some instances intersect a variable region of the S(MK) (or SAM-III) (Fuchs 2006) riboswitch between the main hairpin and the Shine-Dalgarno sequence. The lacto-2 motif consists of a large hairpin with many internal loops, some of which have highly conserved sequences. Although some mutations disrupt pairing, there is a considerable amount of covariation, which suggests that the lacto-2 motif instances are structured RNAs.

### xvi. TD (Treponema denticola) motifs

Two predicted motifs have several instances in *Treponema denticola,* but are not found in any other sequenced bacteria. The motifs, TD-1 and TD-2 have 28 and 36 representatives, respectively. Seven TD-1 motif representatives overlap reverse complements of instances of TD-2, and share the two 5'-most hairpins.

Both motifs show covariation and either variable-length or modular stems. The TD-1 motif is usually in a 5' regulatory configuration to genes. The TD-2 motif does not share the 5' regulatory configuration, so it can correspond to a non-coding RNA.

### 3. DISCUSSION

Using the CMfinder-based comparative genomics pipeline, 22 novel putative RNA motifs were found. Two have already been experimentally confirmed as riboswitches. For several others, covariation and other characteristics show that they are functional structured RNAs, and possible functions for many of the motifs have been proposed. Thus, the pipeline appears to be useful for discovering novel RNAs.

The findings show that the CMfinder-based pipeline is able to recover RNAs that are widespread, possess a highly conserved and extensive secondary structure, are roughly 60 nucleotides or more in length, and are associated with homologous genes. Three candidate riboswitches have these characteristics (GEMM, Moco and SAH). The remaining three candidates, SAM-IV, the COG4708 motif and the *sucA* motif are more narrowly distributed than most known riboswitches in that none of these motifs is found outside a single order in taxonomy level.

### B. Example 2: Riboswitch Class in Eubacteria Senses the Second Messenger Cyclic Di-GMP

Cyclic di-GMP is a circular RNA dinucleotide that functions as a second messenger to trigger wide-ranging physiological changes in diverse species of bacteria. Processes controlled include cell differentiation, conversion between motile and biofilm lifestyles, and virulence gene expression. However, the mechanisms used by cyclic di-GMP to regulate gene expression have remained a mystery since the discovery of the compound more than two decades ago. Data indicate that cyclic di-GMP in many bacterial species is sensed by a riboswitch class that controls the expression of genes involved in numerous fundamental cellular processes. A variety of cyclic di-GMP regulons are revealed, including some riboswitches associated with virulence gene expression, pili formation, and flagellar organelle biosynthesis. In addition, sequences matching the consensus for cyclic di-GMP riboswitches are present in the genome of a bacteriophage.

The second messenger cyclic di-GMP (Figure 3A) was discovered as an activator of cellulose synthase from the bacterium *Gluconacetobacter xylinus* (Ross 1985; Ross 1987). Subsequent studies revealed that cyclic di-GMP is nearly ubiquitous in bacteria, where it has wide-ranging effects on cell physiology (Jenal 2006; Ryan 2006; Tamayo 2007). Cyclic di-GMP is a circular RNA dinucleotide formed from two guanosine-5'-triphosphate molecules by diguanylate cyclase (DGC) enzymes that are characterized by GGDEF amino acid domain. Once formed, the compound is degraded selectively by phosphodiesterase (PDE) enzymes that contain either EAL or HD-GYP amino acid domains (Jenal 2006; Ryan 2006; Tamayo 2007; Simm 2004 and references therein). Many species of bacteria, including the pathogen *Vibrio cholerae,* have dozens of distinct DGC and PDE proteins (Galperin 2001; Ulrich 2005; Römling 2005). The activities of these proteins are triggered by specific stimuli to modulate cellular cyclic di-GMP concentrations (Römling 2005), and changes in the concentration of this second messenger in *V. cholerae* are known to control rugosity, biofilm formation, and virulence (Lim 2006c; Beyhan 2007; Tischler 2005; Waters 2008).

The mechanisms used by cyclic di-GMP to bring about diverse physiological changes in bacteria have long remained obscure. In addition to being targeted by cyclic di-GMP-cleaving PDEs, this second messenger can be bound by some DGC proteins to allosterically represses its own synthesis (Chan 2004; Wassman 2007). The only other protein targets known are *G*. *xylinus* cellulose synthase (Ross 1985; Ross 1987), *Pseudomonas aeruginosa* PelD protein (Lee 2007) and PilZ domain proteins (Ryjenkov 2006). However, cyclic di-GMP binding to these proteins does not fully explain its global cellular effects (Tamayo 2007; Wolfe 2008). Thus, there are key regulatory targets for cyclic di-GMP that remain to be discovered.

**A riboswitch for cyclic di-GMP**. It has been hypothesized (Tamayo 2007) that the existence of cyclic di-GMP riboswitches could explain how this second messenger controls the transcription and translation of many genes. The identification of a highly-conserved RNA domain called GEMM that resides immediately upstream of the open reading frames (ORFs) for DGC and PDE proteins in some organisms was recently reported (Weinberg, 2007). This RNA domain also is frequently associated with genes that are likely controlled by cyclic di-GMP. The high conservation and genomic distributions of GEMM RNAs are characteristic of metabolite-sensing riboswitches (Winkler 2005; Breaker 2008 (Science)) that control gene expression in response to changing concentrations of their target ligand.

GEMM RNAs conform to one of two similar architectures termed Type 1 and Type 2 (Figure 3B). Both types carry two base paired regions (P1 and P2) that exhibit extensive covariation in the 503 representatives identified (Weinberg, 2007). Type 1 RNAs (303 examples) have a GNRA tetraloop (Heus 1991) on P1 with a purine (R) at the second position. The presence of a GRRA sequence correlates with the presence of a tetraloop receptor at the extremity of P2, which is known to dock with this tetraloop type (Costa 1995). In contrast, Type 2 RNAs (171 examples) have a pyrimidine (Y) at the second position of the tetraloop, and in some instances include a structure on P2 that favors docking with GYRA tetraloops (Costa 1995). There are an additional 29 unassigned representatives, of which 24 carry a GNRA tetraloop flanked by non-paired nucleotides and five that lack a tetraloop sequence.

GNRA tetraloops and their receptors commonly occur in structured RNAs, and have previously been observed in riboswitch aptamers (Regulski 2008). However, the variability in sequence and structure at the tips of P1 and P2 hairpins suggests that any binding site for cyclic di-GMP likely is located in the central or basal portions that carry the most conserved features.

**GEMM RNAs selectively bind cyclic di-GMP.** The genome of the pathogenic bacterium *V*. *cholerae* carries two GEMM RNA sequences on separate chromosomes. On chromosome I, a Type 1 GEMM RNA (termed Vc1) resides upstream of the *V*. *cholerae gbpA* gene, which codes for a protein factor that binds to chitin or its primary monomeric unit, *N*-acetyl-D-glucosamine. GbpA has been implicated in cholera infections in humans (Kirn, 2005). On chromosome II, another Type 1 RNA (termed Vc2) resides upstream of a gene (VC1722) homologous to *tfoX* (Mandal 2003; Meibom 2005). The *tfoX* gene (termed *sxy* in some organisms; Cameron 2008) codes for a factor responsible for regulating genes that increase competence when *V. cholerae* is exposed to chitin. Given this similarity in sequence, the VC1722 protein might also function as a transcription factor.

Biochemical and genetic analyses were carried out with both GEMM RNAs to determine whether they function as aptamers for cyclic di-GMP. A 110-nucleotide Vc2 RNA construct (110 Vc2; Figure 3C) was subjected to in-line probing (Soukup 1999) in the absence or presence of 100 µM cyclic di-GMP (Figure 3D). The changing patterns of spontaneous cleavage at the base of stems P1 and P2 suggest that the conserved nucleotides near the regions undergoing structural modulation (labeled 1 through 3) are important for second messenger binding. Similar results were obtained for a construct encompassing the Vc1 RNA and for representative cyclic di-GMP aptamers from *Bacillus cereus* and *Clostridium difficile.*

By subjecting 110 Vc2 RNA to in-line probing with various cyclic di-GMP concentrations, an apparent dissociation constant (K_{D}) of approximately 1 nM was established. The resulting plot (Figure 4A) is consistent with a 1 to 1 saturable interaction between an RNA aptamer and its ligand (Welz 2007). Although the aptamer could bind one of the various multimeric cyclic di-GMP complexes that have been reported (Egli 1990; Zhang 2006), formation of these intermolecular cyclic di-GMP complexes occurs at concentrations far greater than the measured *K*_{D} value. Moreover, a change in *K*_{D} is not observed when K⁺ is replaced with Na⁺ in in-line probing reactions, even though these monovalent metals affect the type of cyclic di-GMP complexes formed.

The complex formed between the 110 Vc2 aptamer and cyclic di-GMP is nearly three orders of magnitude tighter than the affinity measured for cyclic di-GMP binding by an *Escherichia coli* PilZ protein domain (*K*_{D} = 840 nM; Ryjenkov 2006). Furthermore, various analogs of the second messenger are strongly discriminated against by the Vc2 aptamer (Figure 4B). From the analogs tested, it appears that both the circular structure of the second messenger and its guanine nucleobases contribute substantially to ligand recognition. For example, the linear breakdown product of cyclic di-GMP by EAL PDEs, termed pGpG, is bound by the aptamer nearly three orders of magnitude less tightly. Similarly, the pG (5' GMP) product of HD-GYP PDEs is not bound by the aptamer even at a concentration that is five orders of magnitude higher than the K_{D} for cyclic di-GMP. Therefore, the biologically relevant ligand for this aptamer class appears to be cyclic di-GMP and not the breakdown products of this second messenger.

Cyclic di-GMP is an RNA dinucleotide that includes two structurally constrained 3',5'-phosphodiester linkages (Figure 3A). In some cases, internucleotide linkages with constrained geometry exhibit accelerated RNA strand scission (Soukup 1999). To assess whether cyclic di-GMP is stable under in-line probing conditions, its rate constant for spontaneous degradation was determined. Under in-line probing conditions, cyclic di-GMP undergoes cleavage with a rate constant of 3.2 x 10⁻⁶ min⁻¹ (Figure 4C), which corresponds to a half life of ~150 days. This rate constant is similar to that predicted for RNA linkages that are present in unconstrained linear polynucleotides when incubated under similar conditions (Li 1999). Therefore, the second messenger is likely to be stable in in-line probing assays and even more stable in cells unless subjected to the action of PDE enzymes.

**Gene control by cyclic di-GMP riboswitches.** Bacterial riboswitches typically carry a conserved aptamer located immediately upstream of a less well-conserved expression platform. Most expression platforms in bacteria form structures that control transcription termination or translation initiation (Barrick 2007). Similar architectures are associated with many GEMM RNA representatives, indicating that cyclic di-GMP aptamers are likely to be components of riboswitches.

Four 5' UTRs from *V. cholerae, B. cereus,* and *C*. *difficile* were chosen to assess whether cyclic di-GMP aptamers function *in vivo* as sensory domains for riboswitches. For Vc2, DNAs encompassing the predicted native promoter, the aptamer or its variants (Figure 5A), and the adjoining expression platform were used to prepare translational fusions with the *E*. *coli lacZ* reporter gene. Transgenic *E*. *coli* cells carrying the aptamer-reporter fusion constructs were grown in liquid medium and assayed for β-galactosidase activity.

The reporter construct carrying the wild-type (WT) Vc2 aptamer exhibits a high level of gene expression (Figure 5B). In contrast, constructs M1 and M3 that carry mutations disrupting P1 or altering an otherwise strictly conserved nucleotide in the P2 bulge express the reporter gene at less than 10% of WT. Furthermore, an aptamer that carries four mutations (M2) that restore the structure of P1 exhibits near-WT gene expression. These results indicate that the riboswitch integrating the Vc2 aptamer functions as an "on" switch by activating translation of the adjoining ORF. Similarly, transcriptional fusions were prepared for cyclic di-GMP riboswitches from *B. cereus* (Bc1 and Bc2) and *C*. *difficile* (Cd1). The expression patterns for WT and M3 variants are consistent with "on" switch function for Bc1 and "off' switch function for Bc2 and Cd1 (Figure 5B).

**Changing cyclic di-GMP concentrations modulate riboswitch-mediated gene expression.** Modulation of gene expression by cyclic di-GMP riboswitches was established by examining "off" switch action of the Cd1 RNA while manipulating the cellular concentration of the second messenger. Cyclic di-GMP concentrations were modulated by expressing the *V. cholerae vieA* gene product. VieA is an EAL phosphodiesterase (PDE) that is expected to lower the cellular concentration of cyclic di-GMP by promoting hydrolysis of the second messenger to its linear pGpG form (Li 1999). Cells that carry an empty vector, or that express a VieA protein with an E170A mutation that eliminates PDE activity (Tamayo 2005), are expected to maintain cyclic di-GMP concentrations that are normal for cells under the conditions grown.

Transgenic *B. subtilis* cells carrying a transcriptional fusion of the WT Cd1 riboswitch with *lacZ,* and grown on agar plates with X-gal, exhibit low β-galactosidase activity when co-transformed with either the empty plasmid vector or the vector coding for the inactive E170A VieA mutant (Figure 6A). In contrast, robust β-galactosidase activity is evident in cells that are co-transformed with the reporter construct and the vector coding for VieA PDE. A similar trend is observed when these transgenic cells are assayed from liquid culture, whereas a reporter construct carrying the M3 mutation in the Cd1 aptamer remains largely unaffected by *vieA* expression (Figure 6B). These observations are again consistent with "off" switch function by the Cd1 riboswitch, wherein the action of the PDE relieves repression by lowering the cyclic di-GMP concentration in cells.

**Roles for cyclic di-GMP riboswitches in diverse bacteria.** Although *V. cholerae* has only two cyclic di-GMP riboswitch representatives (Figure 7), the physiological roles for these RNAs is substantial. The *gbpA* gene product associated with the Vc1 riboswitch is a sugar-binding protein that has been reported to be the key determinant permitting the bacterium to colonize mammalian intestines, leading to cholera disease (Kirn, 2005). It has also been shown that *V. cholerae* lowers its cyclic di-GMP levels when colonizing mammalian host intestines (Tamayo 2005). The *V. cholerae vieA* gene used in the study to demonstrate Cd1 riboswitch response to changing cyclic di-GMP levels (Figure 6A) is known to be essential for bacterial infection. Thus, it is possible that a reduction in cyclic di-GMP levels brought about by the action of VieA is sensed by the Vc1 riboswitch to facilitate expression of GbpA and *V. cholerae* infection.

The VC1722 gene associated with the Vc2 riboswitch codes for a protein similar to a known transcription factor that is important for competence (Meibom 2005). It has been previously shown that a *V. cholerae* mutant producing a rugose phenotype has elevated cyclic di-GMP levels and exhibits higher expression of the VC1722 mRNA (Lim 2006c; Beyhan 2007). This is consistent with the data indicating that the Vc2 riboswitch associated with the VC1722 mRNA is a genetic "on" switch that yields higher gene expression when cyclic di-GMP concentrations are elevated (Figure 5B).

Some organisms have a strikingly high number of cyclic di-GMP riboswitch representatives (Figure 7). *Geobacter uraniumreducens,* with 30 representatives identified, has the largest number of cyclic di-GMP aptamer RNAs among bacterial species whose genomes have been sequenced. These are distributed upstream of 25 different transcriptional units, with five RNAs carrying tandem cyclic di-GMP aptamers. Similar tandem arrangements have been observed for some glycine aptamers that bind two amino acids cooperatively (Mandal 2004c). However, more common tandem arrangements involve complete riboswitches that respond to the same ligand (Welz 2007; Sudarsan 2006). Both tandem architectural arrangements allow riboswitches to respond more sensitively to smaller changes in ligand concentration (Welz 2007) and function as more digital genetic switches.

Although the functions of the genes associated with many cyclic di-GMP riboswitches are unknown, several representatives in *C*. *difficile* are notable for their locations (Figure 7). The Cd1 riboswitch is a genetic "off" switch (Figures 5 and 6) that is located in the 5' UTR of a large operon that encodes for the proteins required to construct the entire flagella apparatus of this species. This arrangement reveals that some bacteria use a riboswitch to sense changes in cyclic di-GMP concentrations and regulate organelle biosynthesis to control motile versus sessile lifestyle transformations.

Also intriguing is the identification of cyclic di-GMP riboswitch representatives residing within PhiCD119 bacteriophage DNA that are integrated within the *C*. *difficile* genome. The riboswitch sequence, located within the lysis module of the bacteriophage genome, is also evident in DNA packaged into bacteriophage particles (Govind 2006). Although more than 20 metabolite-sensing riboswitch classes have been reported, and thousands of representatives of these classes have been identified, the cyclic di-GMP RNAs Cd2, Cd12, and the related RNA from phage are the only known examples of bacteriophage-associated riboswitches. Perhaps viruses have little need for sensing fundamental metabolic products, but might gain an evolutionary advantage by monitoring the physiological transformations of bacterial cells brought about by changing concentrations of the second messenger cyclic di-GMP.

### 1. Materials and methods

### i. Bioinformatics

The set of known cyclic di-GMP riboswitches was expanded by using the previously established multiple sequence alignment (Weinberg 2007) to perform additional homology searches. These searches were carried out using RAVENNA (Weinberg 2006) on RefSeq version 25 (Pruitt 2005) microbial sequences, as well as environmental sequences from acid mine drainage (Tyson 2004), soil and whale fall (Tringe 2005), human gut (Gill 2006), mouse gut (Turnbaugh 2006), gutless sea worms (Woyke 2006), sludge communities (Garcia Martin 2006), and the global ocean survey (Venter 2004 ; Rusch 2007). Conservation statistics reflected in the consensus diagrams (Figure 3) were calculated using previously established protocols (Weinberg 2007). Figure 7 was generated using gene annotations from RefSeq and the DOE Joint Genome Institute or global ocean survey. Genes were classified based on the Conserved Domain Database (Marchler-Bauer 2005) version 2.08.

### ii. Oligonucleotides, Chemicals, Plasmids and Bacteria Strains

DNA oligonucleotides were purchased from Sigma-Genosys or the W. M. Keck Foundation Biotechnology Resource Laboratory at Yale University. Cyclic di-GMP, pGpG, and 3', 5'-cyclic GMP were purchased from BioLog Life Science Institute (Germany), and 3'-guanosine monophosphate was purchased from MP Biomedicals. ApG, GpG, GpA, pGpA, and GpGpG were purchased from Oligos Etc. All other chemicals were purchased from Sigma-Aldrich.

pDG1661 and pDG 148 were obtained from the *Bacillus* Genetic Stock Center (BGSC; The Ohio State University). pRS414 was gift from Dr. R. Simons (UCLA). *V*. *cholerae* O1 biovar eltor strain N16961 genomic DNA was provided by Dr. B. Bassler (Princeton U.). *B. subtilis* strain 1A1, *B. cereus* strain 6A5 (ATCC 14579) and *B. cereus* strain 6A15 (ATCC 10987) also were obtained from the BGSC. *C*. *difficile* genomic DNA from ATCC strain 9689 was obtained from ATCC. Competent DH5-alpha E. *coli* were purchased from New England Biolabs.

### iii. Kinetics of Cyclic Di-GMP Degradation

A solution of 500 µM cyclic di-GMP was incubated in the presence of in-line probing buffer (50 mM Tris-HCl [pH 8.3 at 23°C], 100 mM KCl, 20 mM MgCl₂) for varying amounts of time. The reactions were initiated by the addition of cyclic di-GMP and quenched when a 10 µL aliquot was injected onto an Agilent Technologies 1200 series HPLC equipped with an Agilent Zorbax Oligo column (5 µM, 6.2 mm x 80 mm). The compounds were eluted using a gradient mobile phase composed of 95% 0.1 M NaHPO₄ [pH 6.0 at 23°C] and 5% acetonitrile to 100% 1 M NaCl. The absorbance was monitored at 254 nm. A rate constant for cyclic di-GMP was established by plotting the natural logarithm of the fraction of the cyclic di-GMP remaining uncleaved versus time, wherein the negative slope of the resulting line reflects the rate constant.

### iv. Purity Analyses and Integrity of Cyclic Di-GMP Analogs

The purities of the di- and tri- nucleotide analogs of cyclic di-GMP were examined using an Agilent Technologies 1200 series HPLC with monitoring of the absorbance of eluted compounds at 254 nm. The compounds were separated using an Agilent Extend C18 column (3.5 µM, 4.6 mm x 150 mm) using a gradient mobile phase of 95% water, 5% acetonitrile and 0.1 % trifluoroacetic to 100% acetonitrile and 0.1 % trifluoroacetic acid. HPLC traces revealed that 50% to 95% of the UV-absorbing material corresponded to the desired analogs.

High resolution mass spectrum analysis was performed on each analog to confirm their exact mass. HRMS (ES) calculated and (M-H)⁻ found for C₂₀H₂₅N₁₀O₁₁P (GpA): 612.1442, 611.1375; C₂₀H₂₅N₁₀O₁₁P (ApG): 612.1442, 611.13723; C₂₀H₂₅N₁₀O₁₁P (GpG): 628.1391, 627.13207; C₂₀H₂₆N₁₀O₁₄P₂ (pGpA): 692.1105, 691.10410; C₃₀H₃₇N₁₅O₁₉P₂ (GpGpG): 973.1865, 972.1825, and (M-2H)⁻² 485.58597.

### v. Preparation of RNAs

DNA templates were prepared by PCR amplification using wild-type or mutant pRS414 plasmid DNA with primers that added the promoter sequence for T7 RNA polymerase (T7 RNAP) plus two guanine residues at the start site of transcription to increase the yield of RNA. RNA molecules were prepared by *in vitro* transcription using the appropriate DNA template and T7 RNAP in 20 mM Tris-HCl (pH 8.0 at 23°C), 20 mM NaCl, 14 mM MgCl₂, and 100 µM EDTA. RNA was purified on a denaturing (8 M urea) 10% polyacrylamide gel and eluted from the gel in 10 mM Tris-HCl (pH 7.5 at 23°C), 200 mM NaCl and 1 mM EDTA (pH 8.0). RNAs were precipitated with ethanol, dephosphorylated using alkaline phosphatase (Roche Diagnostics), and 5' radiolabeled using [γ-³²P] ATP and T4 polynucleotide kinase (New England Biolabs) according to the manufacturer's protocols. Radiolabeled RNAs were gel purified as described above.

### vi. In-Line Probing

In-line probing assays were performed using methods similar to those described previously (Soukup 1999). Briefly, 5' ³²P-labeled RNAs (~50 pM for cyclic di-GMP K_{D} analysis, ~5 nM for all other analyses) were incubated at 23°C for approximately 40 hours in 20 mM MgCl₂, 100 mM KCl, and 50 mM Tris (pH 8.3 at 23°C) with the indicated compounds. RNA cleavage products were separated by denaturing 10% PAGE, visualized using a Phosphorimager (Molecular Dynamics), and analyzed using SAFA v1.1 software (Das 2005) or ImageQuant (Molecular Dynamics). SAFA software was used to normalize to correct for differences in the amounts of RNA loaded to each lane and to determine the intensity of each band. Bands that showed a significant change in intensity were identified as undergoing structural modulation.

To determine the K_{D} of 110 Vc2 for cyclic di-GMP, in-line probing was conducted as described above with various concentrations of cyclic di-GMP. Due to the use of an exceptionally low concentration of radiolabeled 110 Vc2 RNA, the samples loaded were supplemented with additional unlabeled RNA that had been separately subjected to in-line probing. This reduced the amount of radiolabeled RNA that bound non-specifically to the gel matrix and increased the resolution of bands during imaging. Dissociation constants (*K*_{D}) were determined using ImageQuant software (Molecular Dynamics) to compare individual band intensities at sites of structural modulation assuming that there was no modulation in the absence of ligand and complete modulation in the presence of the highest ligand concentration tested. Dissociation constants were determined by plotting the fraction modulated (F) versus the logarithm of the ligand concentration [L] at the indicated modulating sites and fitting the data to the equation F = [L]/([L]+ *K*_{D}) using SigmaPlot 9 (Systat Software).

### vii. Plasmid and Bacteria Strain Preparation

Vc2, the 5' UTR of COG3070 (Tfox-like gene), was amplified from *V. cholerae* genomic DNA as an EcoRI-BamHI fragment, cloned into the translational reporter vector pRS414, and its gene control function in *E*. *coli* was established using methods similar to those described previously (Nahvi 2002). Specifically, the region -348 nt to +21 nt with respect to the beginning of the ORF was cloned as a translation fusion in the *lacZ* reporter vector wherein the 7^{th} codon of the COG3070 ORF was fused in frame to the 9^{th} codon of the *lacZ* reporter gene. Mutations in the regulatory region were generated from this plasmid using the QuikChange Site-Directed Mutagenesis Kit (Stratagene) following the manufacturer's directions.

The 5' UTR region upstream of putative ORFs in *B. cereus* and *C*. *difficile* were amplified by PCR as EcoRI-BamHI fragments and cloned into the vector pDG1661 to yield transcriptional fusions with a promoterless *lacZ* gene. The amplified fragments included the predicted promoter elements upstream of the UTR and the transcription terminator associated with the riboswitch upstream of the corresponding gene. The resulting constructs were integrated into the *amyE* locus of *B. subtilis* as described previously (Sudarsan 2003). Mutant constructs were generated using site-directed mutagenesis as described above.

A cyclic di-GMP-specific phosphodiesterase gene *(vieA) from V. cholerae* was amplified from genomic DNA by PCR. The *vieA* gene was cloned into the stuI site located downstream of the *Pspac* promoter in a modified pDG148 vector using ligation-independent cloning as described previously (Joseph 2001). The *lacI* gene in this vector has been mutated so that the *vieA* gene is expected to give constitutive expression. The *vieA* knockout mutant E170A was created using site-directed mutagenesis as described above.

### viii. β-galactosidase Assays

Wild-type and mutant Vc2-pRS414 constructs were transformed into competent NEB 5-alpha (New England Biolabs) *E. coli* cells. Individual colonies were grown overnight at 37°C with shaking in Luria-Bertani broth containing 50 µg/mL carbenicillin. Cultures were diluted to an OD₆₀₀ of ~0.1 and grown to an OD₆₀₀ of ~0.6 before performing β-galactosidase assays following the standard protocol (Miller 1992). Overnight cultures of *B. subtilis* strains transformed with pDG1661 containing Bc1, Bc2 and Cd1 were subcultured to an OD₆₀₀ of ~0.2 and subsequently grown for approximately three hours to an OD₆₀₀ of ~1 before performing β-galactosidase assays as described above.

Gene expression assays conducted on solid agar plates using the transgenic B. *subtilis* cells described above were conducted by growing streaked cells for at least 16 hours at 37°C. Agar (TBAB) contained 400 µg mL⁻¹ X-gal and 5 µg mL⁻¹ of chloramphenicol and/or kanamycin when required.

### C. Example 3: Riboswitches that Sense S-adenosylhomocysteine and Activate Genes Involved in Coenzyme Recycling

A highly conserved RNA motif that occurs upstream of genes involved in S-adenosyl-L-methionine (SAM) recycling in many Gram-positive and Gram-negative species of bacteria has been identified. The phylogenetic distribution and the conserved structural features of representatives of this motif are indicative of riboswitch function. Riboswitches are widespread metabolite-sensing gene control elements that are typically found in the 5' untranslated regions (UTRs) of bacterial mRNAs. Examples of this RNA motif were identified that specifically recognize S-adenosylhomocysteine (SAH) in protein-free *in vitro* assays, and it was confirmed that these RNAs strongly discriminate against SAM and other closely related analogs. A representative SAH motif was found to activate expression of a downstream *gene in vivo* when the metabolite is bound. These observations confirm that SAH motif RNAs are distinct ligand-binding aptamers for a riboswitch class that selectively binds SAH and controls genes essential for recycling expended SAM coenzymes.

RNA aptamers are highly structured polynucleotides that form selective binding pockets for specific ligands (Gold et al., 1995; Osborne and Ellington, 1997). Engineered aptamers can be made to exhibit high affinity and selectivity for their targets, and can have considerable potential for applications in basic research, biotechnology and therapeutics (Breaker, 2004; Bunka and Stockley, 2006). RNA aptamers also can exist naturally in the 5' UTRs of many bacterial messenger RNAs, where they serve as the sensor domain of gene control elements called riboswitches (Mandal and Breaker, 2004a; Soukup and Soukup, 2004; Winkler and Breaker, 2005). Like their engineered counterparts, natural aptamers can bind their targets with high affinity and specificity, which is required for RNA aptamers that are used by cells to precisely regulate the expression of critical metabolic genes. However, natural aptamers are typically much larger (ranging between 34 and 200 nucleotides) than engineered aptamers (Breaker, 2006), which presumably allows natural aptamers to exhibit the high level of performance required to compete with gene control factors made of protein.

Each riboswitch usually harnesses metabolite binding by its aptamer domain to bring about structural changes in an adjoining expression platform. These structural changes establish the level of protein production from the open reading frame (ORF) or from multiple ORFs located downstream. However, folding changes are not always brought about by metabolite binding. For example, one riboswitch class uses the target metabolite as a coenzyme for a self-cleaving ribozyme whose action represses expression of the downstream ORF (Winkler et al., 2004; Cochrane et al., 2007). Regardless of the mechanism used, riboswitches often control genes whose protein products are involved in the synthesis, degradation, or transport of the metabolite or precursors of the metabolite whose cellular concentration is sensed by the aptamer domain.

Riboswitch aptamers must form precision binding pockets for their target metabolites using only the four common nucleotides, unless modified bases or supporting protein factors are involved. This places strong selective pressure on each aptamer to maintain certain structural characteristics throughout evolution, although some ligands such as SAM (Corbino et al., 2005; Fuchs et al., 2006) and preQ₁ (Weinberg et al., 2007) are detected by multiple riboswitch classes with entirely different aptamer structures. The representatives of riboswitch aptamer classes identified from even distantly related organisms exhibit considerable conservation in sequence and secondary structure. This conservation, coupled with the identity of the ligand bound by the aptamer, forms the basis for the organization of riboswitches into distinct classes.

The conserved features of riboswitch aptamers have been exploited by computer-aided search algorithms to expand the number of representatives of known riboswitch classes (*e.g.* Rodionov et al., 2002; Rodionov et al., 2003a; Nahvi et al., 2004; Rodionov et al., 2004;) and to discover previously unknown riboswitch classes (*e.g.* Rodionov et al., 2003b; Barrick et al., 2004; Corbino et al., 2005; Weinberg et al., 2007). In contrast, expression platforms vary widely in sequence and structure, even within the same riboswitch class. For example, TPP riboswitches integrate a consensus aptamer with various expression platform architectures to control transcription termination and translation initiation in bacteria (Winkler et al., 2002; Mironov et al., 2002; Rentmeister et al., 2007), and RNA splicing in eukaryotes (Sudarsan et al., 2003a; Kubodera et al., 2003; Bocobza et al., 2007; Cheah et al., 2007; Wachter et al., 2007).

As noted above, riboswitches with aptamers from different structural classes can recognize the same target metabolite. Four riboswitch classes have been discovered that specifically recognize the coenzyme SAM (or AdoMet), and these have been termed SAM-I (Epshtein et al., 2003; McDaniel et al., 2003; Winkler et al., 2003), SAM-II (Corbino et al., 2005, Lim et al., 2006b), SAM-III or S_{MK} (Fuchs et al., 2006) and SAM-IV (Weinberg et al., 2007; unpublished data). Among these classes, SAM-I riboswitches have the most widespread phylogenetic distribution known, with examples found in many bacterial groups (Rodionov et al., 2004). SAM-II riboswitches are largely found in Gram-negative proteobacteria and bacteroidetes (Corbino et al., 2005), while SAM-III riboswitches are reported only in Gram-positive lactic acid bacteria (Fuchs et al., 2006). The aptamers of all four classes preferentially bind SAM over SAH (or AdoHcy), which is a byproduct of SAM-dependent methyl group transfer (Takusagawa et al., 1998). For example, representatives of the SAM-I and SAM-II aptamers discriminate ~80-fold and more than 1000-fold against SAH, respectively (Lim et al., 2006b). This level of discrimination is striking considering the compounds differ by only a single methyl group.

The discovery of a bacterial RNA element with highly conserved primary sequence and secondary structure that is always found upstream of genes responsible for converting SAH to L-methionine is described herein. The RNA element represents a distinct class of aptamers that selectively binds SAH and discriminates at least 1000-fold against SAM, which is in stark contrast to the molecular recognition characteristics of the four known SAM aptamers. In the *ahcY* mRNA of the bacterium *Pseudomonas syringae,* the structural changes brought about by metabolite binding to the SAH aptamer activates expression of the downstream *gene in vivo,* thus demonstrating that the SAH aptamer forms the sensor component of a distinct class of SAH-responsive riboswitches.

### 1. Results

### i. Identification of a Conserved RNA Motif Associated with SAH Recycling

In an effort to identify additional riboswitch classes and other structured regulatory RNA motifs, systematic searches of the intergenic regions (IGRs) of gene families in numerous bacterial classes were carried out (Weinberg et al., 2007) using the CMfinder comparative genomics pipeline (Yao et al., 2007). Numerous RNA motifs were identified in bacteria using this search strategy, and several have the characteristics expected for RNAs that function as metabolite-sensing riboswitches.

One candidate riboswitch class was termed the SAH motif because the genes most commonly associated with representatives of this RNA are S-adenosylhomocysteine hydrolase *(ahcY,* COG0499), cobalamin-dependent methionine synthase (*metH,* COG1410) and methylenetetrahydrofolate reductase (*metF,* COG0685) (Figure 8). The genomic contexts of SAH motif representatives, and their high degree of conservation, are consistent with a role in sensing SAH and activating the expression of genes whose products are required for SAH recycling to resynthesize SAM. The SAH byproduct of SAM-dependent methylation reactions (Takusagawa et al., 1998) binds a number of SAM-dependent methyltransferases with similar or even better affinities compared to SAM, and therefore functions as a potent inhibitor of these enzymes (Ueland, 1982). During times of high methytransferase activity, cells can accumulate toxic levels of SAH unless the compound is converted into less toxic products. Thus, rapid sensing of SAH and activation of genes for recycling of SAH to reform SAM is important.

There are two major routes in eubacteria for SAH catabolism that both convert SAH to homocysteine (Figure 8). In many bacterial species, SAH nucleosidase (EC 3.2.2.9) catalyzes cleavage of the N-glycosidic bond of SAH to produce adenine and *S-*ribosylhomocysteine (Della Ragione et al., 1985). The latter compound can be subsequently hydrolyzed by the gene product of *luxS* to form homocysteine (Schauder et al., 2001). The alternative route involves the *ahcY* gene that is commonly associated with SAH motif representatives. SAH hydrolase, the product of the *ahcY gene,* catalyzes the breakdown of the thioether bond of SAH to produce adenosine and homocysteine (Shimizu et al., 1984). The gene product of *metH* subsequently methylates homocysteine to L-methionine using 5-methyltetrahydrofolic acid (5-methylTHF) as the methyl donor (Old et al., 1988), which is generated by the gene product of *metF* from 5,10-methylTHF (Sheppard, 1999). SAM synthase, encoded by the *metK* gene, regenerates SAM from methionine and ATP to complete the cycle (Tabor and Tabor, 1984). It is interesting to note that the *metK* gene resides in close proximity upstream of the SAH RNA motif in some species of□ β-proteobacteria and γ-proteobacteria, and can be in the same operon. No homologs of the *luxS* gene product (COG1854) were identified in genomes where the conserved RNA element was found, so in these organisms the catabolism of SAH can proceed exclusively by the route involving SAH hydrolase.

In total, 95 occurrences of the SAH RNA motif conforming to a revised structural model were found, and 68 of these matches are non-duplicative. All examples of the SAH RNA motif identified occur adjacent to SAH recycling genes except for those identified from environmental sequences or from incompletely-sequenced genomes where the gene identities adjacent to the RNA motif were not available. In most instances, SAH RNA representatives are located upstream of what appear to be operons that, almost without exception, code for SAH hydrolase (*ahcY*), methylenetetrahydrofolate reductase *(metE)* and occasionally methionine synthase *(metH).* In some species of *Burkholderia, Dechloromonas,* and *Ralstonia,* the operon includes a predicted membrane protein (COG1950) of unknown function. Other genes appearing rarely in SAH RNA-associated operons include those that encode an rRNA methylase (COG1189), a regulator of competence-specific genes (*tfoX,* COG3070) and an enzyme involved in guanosine polyphosphate metabolism (*spoT,* COG0317).

A consensus sequence and structural model of the RNA motif (Figure 9A) was constructed based on the 68 non-duplicative sequences in the alignment. The consensus RNA motif is composed of a central core of highly-conserved nucleotides flanked by base-paired regions designated P1 and P2, wherein P2 is capped by a variable-sequence loop (L2). The RNA also forms a pseudoknot (P4) between the central core and highly-conserved nucleotides at the 3' terminus of the RNA. A hairpin (P3 and L3) occurs in some representatives between the P1 and P4 stems. The secondary structure elements are all supported by covariation.

In addition to β-proteobacteria and γ-proteobacteria, examples of RNAs matching this consensus sequence and structure were also found in a species of □-proteobacteria and in some Gram-positive Actinobacteria (Figure 9B). Most organisms have one occurrence of the RNA element, except for *Acidovorax* sp., *Dechloromonas aromatica, Azoarcus* sp., *Polaromonas naphthalenivorans, Polaromonas* sp., *Rhodoferax ferrireducens,* and *Rubrivivax gelatinosus,* where two incidents were identified in front of both the predicted *ahcY* and *metH* coding sequences that are located in different regions of the genome.

### ii. Selective binding of SAH by the 68 metH RNA

The structural features and genomic arrangements of SAH RNAs show that they serve as aptamers for riboswitches that sense SAH. Examples of the RNA motif that can specifically recognize metabolites in a protein-free *in vitro* system and function as a gene control element, which are two defining characteristics of riboswitches, were sought. A 68-nucleotide RNA encompassing the SAH RNA identified upstream of the *metH* gene in *Dechloromonas aromatica* (designated 68 *metH,* Figure 10A) was chosen. The *D*. *aromatica* 68 *metH RNA* is nearly the shortest sequence that closely corresponds to the consensus sequence and structure for this motif. Its reduced length is due in part to the absence of the optional P3 stem (Figure 9A).

An in-line probing assay (Soukup and Breaker, 1999) was used to corroborate the predicted secondary structure (Figure 10A), and to establish that 68 *metH RNA* directly binds SAH. This assay exploits the fact that spontaneous cleavage of RNA occurs faster at internucleotide linkages that are unstructured, and therefore can reveal structural features of RNAs and the changes they undergo when binding ligands. 5' ³²P-labeled 68 *metH* RNA was incubated in reactions containing progressively higher concentrations of SAH and the resulting spontaneous cleavage products were separated by polyacrylamide gel electrophoresis (Figure 10B). In the absence of any ligand, higher band intensities were observed in regions of the RNA predicted to form L2, the internal loop separating P1 and P2, and the 3' terminal portion of the RNA. These observations suggest that P1 and P2 are formed, but that the vast majority of the highly conserved nucleotides in the core of the RNA and in the 3' tail (including the nucleotides of the P4 pseudoknot) are less structured in the absence of ligand.

In the presence of increasing concentrations of SAH, progressive changes in band intensities were observed at several locations throughout the RNA, confirming that SAH causes changes in RNA folding, as expected if the RNA serves as an aptamer for this metabolite. Moreover, the structural changes occur mostly in the phylogenetically conserved regions (Figure 9A, Figure 10A), as is typically observed for many other riboswitch aptamers. The most noteworthy changes in response to SAH binding are the stabilization of pseudoknot P4, and reduction of flexibility in other conserved nucleotides of the core. This shows that the highly-conserved nucleotides are instrumental in forming the binding pocket for SAH.

The apparent dissociation constant *K*_{D} of 68 *metH* RNA for SAH was estimated to be ~20 nM by quantifying the extent of spontaneous cleavage at several nucleotide positions over a range of SAH concentrations (Figure 10B and 10C). This value for apparent *K*_{D} is typical of riboswitch aptamers, which have been found to range from ~200 µM (Winkler et al., 2004) to ~200 pM (Welz and Breaker, 2007; Sudarsan et al., 2006). Similar SAH-mediated shape changes revealed by in-line probing assays were obtained with a 122-nucleotide SAH aptamer construct derived from the *ahcY gene* of *P. syringae.* Although 122 *ahcY* RNA carries the optional P3 stem, the RNA undergoes key structural changes in the core of the aptamer on ligand binding, and the apparent *K*_{D} value is similar to that measured for 68 *metH* RNA.

For the SAH aptamer to function as the sensor for an SAH-responsive riboswitch, it must be able to discriminate against biologically relevant compounds that are structurally similar to SAH. Perhaps most importantly, the aptamer must discriminate against SAM, which differs from SAH only by a single methyl group and the associated positive charge at the sulfur center. Using in-line probing and a commercial source of SAM, it was found that the 68 *metH* RNA, as well as other SAH aptamers, exhibited apparent *K*_{D} values that were no more than 10-fold higher than the apparent *K*_{D} values established for SAH. However, SAM spontaneously degrades to produce substantial levels of SAH contamination, and even freshly prepared commercial samples of SAM contain ~10% SAH (Sigma-Aldrich Technical Service). Thus, a greater level of discrimination against SAM by the 68 *metH* RNA can be obscured by the presence of SAH in SAM samples.

To obtain a more accurate estimate of the ability of SAH aptamers to discriminate against SAM, in-line probing using a sulfone analog of SAH that has been shown previously to closely mimic the binding properties of SAM with a SAM-I class riboswitch aptamer (Lim et al., 2006b) was done. Specifically, the sulfone analog of SAH lacks the methyl group on sulfur that is present in SAM, but carries two electronegative oxygen atoms that withdraw electron density from sulfur to increase the relative positive charge of this atom similar to that of the natural coenzyme. In-line probing results (Figure 10C) reveal that the sulfone analog is bound by the 68 *metH* RNA with an apparent *K*_{D} that is approximately three orders of magnitude poorer than SAH.

The K_{D} value of the 68 *metH* aptamer for SAM also was assessed by using equilibrium dialysis to compare the functions of the SAH aptamer with a previously-reported SAM-II aptamer termed 156 *metA* (Corbino et al., 2005). Either 68 *metH RNA* or 156 *metA* RNA (10 □M) were added to one chamber of an equilibrium dialysis apparatus and 100 nM of [³H]SAM (Figure 11A) was added to the other chamber. The chambers were separated by a permeable membrane with a molecular weight cut-off of 5000 Daltons. Since the methyl group at the sulfur center of [³H]SAM is radiolabeled, contaminating SAH is not radioactive and therefore does not affect the equilibrium distribution of radioactivity between the two chambers.

156 *metA* RNA shifts the distribution of [³H]SAM to favor the chamber containing the SAM-II aptamer by ~1.5-fold, compared to no shift when the 68 *metH* RNA is present under identical assay conditions (Figure 11B). The 156 *metA* RNA has an apparent *K*_{D} of ~1 □M for SAM as determined by in-line probing (Corbino et al., 2005). Therefore, the *K*_{D} value of 68 *metH for* SAM is higher than 1 □M. Furthermore, the absence of a shift in radioactivity equivalent to that observed for the SAM-II riboswitch when 25 µM 68 *metH* RNA is used indicates that its K_{D} for SAM is likely to be no better than 25 µM. This show that 68 *metH* RNA recognizes SAH with an affinity that is likely to be greater than 1000-fold better than for SAM (Figure 11C). This discrimination between SAH and SAM should be sufficient to precisely control the expression of SAH recycling genes in response to changing SAH levels even if the riboswitch were exposed to concentrations of SAM that are equivalent to SAH, or even in modest excess.

The molecular recognition characteristics of SAH aptamers were assessed in greater detail by establishing the apparent *K*_{D} values of 68 *metH RNA* for 15 analogs of SAH (Figure 12A). The carboxyl and amino groups of the amino acid, various positions on the sugar ring and the nucleobase, as well as the thioester linkage were modified. All analogs, except *S*-adenosylcysteine (SAC), exhibit decreases of at least 3 orders of magnitude in affinity. Interestingly, the shorter amino acid side chain of SAC caused only a modest loss of affinity, unlike that observed for SAM-sensing riboswitches (Lim et al., 2006b).

The changes at the sulfur atom in the sulfone (compound **3**) and the aza (compound **4**) derivatives of SAH also decrease affinity by three orders of magnitude, indicating the importance of this position for ligand recognition. Compound **4** is largely uncharged under the conditions used for in-line probing (Lim et al., 2006b). Therefore, its weaker interaction with the RNA might result from steric interference due to the presence of the methyl group. The higher positive charge density of compound **3** at the sulfur atom (Lim et al., 2006b) might contribute to the slightly weaker binding of this compound compared to compound **4.** In addition, the two set of lone pair electrons of the sulfur center in SAH might play a key role in ligand recognition of the RNA. The sulfur atom of SAH (and SAC) conceivably could make a productive interaction with the aptamer via charge transfer or van der Waals interactions. Finally, adenosine does interact weakly with the SAH aptamer (*K*_{d} ~100 □M), but neither homocysteine nor methionine cause any modulation of the RNA structure at concentrations as high as 1 mM.

These results have been used to create a model for molecular recognition of SAH by the 68 *metH RNA* (Figure 12B). This model indicates that essentially every functional group on the ligand serves as a critical molecular recognition determinant, as has been observed for several other riboswitch aptamer classes (e.g. Mandal et al., 2003; Sudarsan et al., 2003b; Mandal et al., 2004c; Lim et al., 2006a; Lim et al., 2006b).

### iii. SAH Binding Activates Expression of a Reporter Gene

The sequence between the SAH element and the start codon of the *ahcY mRNA* from *P. syringae* is predicted to form an extra stem-loop structure where the two nucleotides at the 3' end of the P4 stem alternatively could base pair with the ribosome binding site or Shine-Dalgarno (SD) sequence (Figure 13A). This latter interaction extends the extra stem-loop structure by two base pairs, and thus might function as a SD-sequestering stem. Therefore, this RNA functions as an SAH-responsive riboswitch that activates gene expression on ligand binding, perhaps by exposing the SD sequence to facilitate ribosome interaction with the mRNA. Although a similar SAH-mediated rearrangement is predicted for the SAH aptamer from the *D. aromatica metH* gene, ligand binding to this RNA appears to control the formation of a transcription terminator stem.

*An in vivo* gene control assay was conducted wherein the IGR encompassing the 5' UTR of the *ahcY* gene in *P. syringae* was fused immediately upstream of a *lacZ* coding sequence. This construct retained the start codon of the *ahcY* gene, which was fused in frame with the reporter gene ORF. The resulting translational fusion vector was transformed into *P. syringae* to produce the wild-type (WT) reporter strain. Similarly, a series of mutant reporter strains were constructed that are expected to disrupt or restore aptamer function (Figure 13A). Transformed *P. syringae* strains subsequently were grown in either King's medium B (rich) or Vogel-Bonner medium (minimal), and the reporter expression levels of the mutant constructs were compared to the results from the WT reporter construct (Figure 13B).

In rich medium, SAH concentrations are expected to be high because cells should be utilizing metabolic pathways that require the use of SAM as a cofactor. As expected, □-galactosidase reporter activity for the WT construct is elevated compared to construct M1 (Figure 13B, top) that carries mutations disrupting two conserved nucleotides in the junction between stems P1 and P2 or J1-2 (Figure 13A). This finding is consistent with the belief that SAH binding activates gene expression, and mutations that disrupt ligand binding cause the riboswitch to default to an off state. Unfortunately, both M2 and M3 exhibit WT-like gene expression, despite the fact that destabilization of the P2 stem by the mutations carried by M2 was expected to disrupt riboswitch function. However, in-line probing (with and without SAH present) revealed that the M2 construct is grossly misfolded, which can cause the reporter construct to default to high gene expression. Additional mutations in P2 that yielded constructs that did not excessively misfold were examined. As expected, mutants M4 (P2 disruption) and M5 (P2 restoration) exhibit a loss of gene expression and a subsequent restoration of gene expression, respectively. These findings again indicate that disruption of the aptamer causes the riboswitch to default to an off state for gene expression, and show that the M2 mutation indeed is abnormal in its misfolding and gene expression results.

Similarly, the effects of the mutations carried by constructs M6, M7 and M8 are consistent with a mechanism for gene control involving SD sequestration. These constructs each carry two mutations that reduce the stability of P4, and a loss of SAH binding affinity for such constructs was observed by using in-line probing. Furthermore, constructs with disrupted SAH binding can also to yield low □-galactosidase reporter activity. This result indeed is observed for M6 and M8, but M7 produces near WT expression (Figure 13A). Although the M7 mutations disrupt P4 formation, they also can disrupt the sequestering stem that overlaps the SD sequence. Therefore, the M7 riboswitch should no longer be capable of defaulting to an OFF state, and gene expression of the riboswitch-reporter fusion construct can be high as is observed. A similar set of reporter gene assays was conducted with cells grown in a minimal medium (Vogel-Bonner medium). Although the same trends are observed with the various constructs grown in minimal medium (Figure 13B, bottom), the absolute values for reporter expression were higher in rich medium. This reduction in overall reporter expression in minimal medium can be due to a reduction in general metabolic activity and protein synthesis.

In-line probing was used to determine whether the competition between formation of the P4 stem and the SD-sequestering stem could be observed. In-line probing using longer constructs based on a 151 *ahcY* RNA, similar to that depicted in Figure 13A, reveal that the SD-sequestering stem indeed is disrupted in the WT construct when SAH is present. In contrast, the M6 version of this construct forms the SD-sequestering stem regardless of the presence or absence of SAH, whereas the M7 variant cannot form this stem. Thus, the mutually-exclusive formation of the full P4 stem and the full SD-sequestering stem can be a critical component of the expression platform for the SAH riboswitch from the *P. syringae ahcY* gene.

To further establish SAH as the metabolite effector *in vivo,* reporter expression was measured for the WT strain growing in minimal medium supplemented with effectors that are known to increase cellular SAH levels (Figure 14). Adenosine-2',3'-dialdehyde (an inhibitor of SAH hydrolase) and adenosine plus homocysteine were both reported previously to substantially increase cellular SAH levels (Hermes et al., 2004). Medium supplemented with either the SAH hydrolase inhibitor or the adenosine/homocysteine mixture increase expression of the WT riboswitch-reporter fusion construct by ~10 to 20 fold (Figure 14A), and increasing adenosine-2',3'-dialdehyde concentrations cause increasing gene expression with the WT but not the defective variants M1 and M7 (Figure 14B). The addition of methionine to the medium causes a similar increase in riboswitch-mediated expression. Excess methionine in growth medium leads to higher cellular SAM concentrations (Winkler et al., 2003), and the cellular SAH concentration can be increased as well.

In contrast, the addition of 250 □M SAH to the medium only slightly increases reporter expression. SAH does not cross cell membranes as an intact molecule (Ueland, 1982), which explains why the addition of this compound has little effect on reporter expression levels. The up-regulation of reporter expression appears to be riboswitch dependent, since almost no gene expression was observed with an M6 riboswitch-reporter fusion construct carrying mutations that disrupt the formation of P4 and alter the core consensus sequence of the aptamer (Figure 13A).

### 2. Discussion

SAH is ubiquitous in living systems as the product of SAM-dependent methyl transfer reactions, but also functions as an inhibitor of these reactions when present in high concentrations (Ueland, 1982). Therefore, many cells have a need to selectively sense SAH and dispose of the excess. In some organisms, SAH is sensed by a protein genetic factor (Rey et al., 2005), which regulates genes involved in sulfur metabolism. These findings reveal that RNA molecules can serve in a similar capacity to sense SAH and control key genes required for its catabolism.

The identification of the SAH element provides the first example of a highly conserved and widely distributed bacterial RNA that selectively binds SAH. This RNA serves as the aptamer domain of a riboswitch class that always is located immediately upstream of ORFs whose predicted protein products are involved in SAH catabolism. This shows that the vast majority of SAH riboswitches are likely to turn gene expression on in response to ligand binding. Indeed, direct ligand binding of the RNA element stabilizes certain secondary and tertiary structures, which can make accessible the SD sequence for the adjoining ORF to permit ribosome binding and subsequent translation.

Although there are several distinct classes of SAM-sensing riboswitches known that strongly discriminate against SAH binding (Lim et al., 2006b; Fuchs, 2006), the SAH riboswitch is the first class of RNAs identified that reverses this discrimination to favor SAH binding and reject SAM. As with other riboswitch classes high selectivity of ligand binding can be required to prevent regulation of gene expression by other closely-related compounds. The strong molecular discrimination by SAH aptamers likely precludes other compounds such as SAM from unnecessarily activating SAH catabolic gene expression, which is required only when SAH accumulates to high levels. This discriminatory power of SAH aptamers is particularly important because SAM, the metabolic precursor of SAH, has a larger cellular pool than SAH under normal conditions (Ueland, 1982). Both the gene context of SAH riboswitches and their ability to discriminate against SAM by ~3 orders of magnitude show that SAH is the metabolite sensed by the RNA element *in vivo.* Therefore, SAH riboswitches provide cells with a class of sensor and gene control element that permits cells to expend resources for SAH degradation only when SAH concentrations merit expression of the appropriate genes.

Like natural SAM aptamers, the SAH aptamer examined in greatest detail in this study uses essentially every functional group on the ligand for molecular recognition. One notable exception is the observation that SAH aptamers tolerate the removal of a methylene group from the amino acid portion of the ligand without substantial loss of affinity. *S*-adenosylcysteine (SAC; Figure 12A, compound **2**) carries the same array of functional groups that are present in SAH, but some of these groups must be displaced by one or more angstroms in the binding pocket of the aptamer. A possible explanation for the tolerance of the shorter SAC analog is that the RNA aptamer might form two separate binding pockets wherein each interacts with one side of the ligand. This can allow for a certain level of adaptability that allows the binding site to recognize ligands of different lengths. Precedence for this type of riboswitch aptamer function has been reported for TPP-binding riboswitches that can bind the progressively shorter thiamin monophosphate and thiamin compounds, albeit with progressively poorer apparent K_{D} values (Winkler et al., 2002). Atomic-resolution models of TPP riboswitch aptamers (Edwards and Ferré-D'Amaré, 2006; Serganov et al., 2006; Thore et al., 2006) reveal a bipartite ligand-binding pocket that can adjust their positions to bind the shorter thiamin monophosphate ligand with relatively high affinity.

The most highly conserved nucleotides in the SAH aptamer are largely located in P4 and in the junctions bridging P1, P2 and P4 (Figure 9A). These nucleotides can be involved in forming the binding pocket for SAH, as is evident by the substantial modulation of structure of these residues observed by in-line probing (Figure 10A and 10B). These nucleotides form a pocket that binds a ligand lacking charge at the sulfur center, and therefore they form a structure that interacts with this atomic center in a way that is distinct from that of SAM-I aptamers. SAH aptamers reject both compounds tested in this study that carry modifications to the sulfur center (Figure 12A, compounds **3** and **4**). The discrimination against compound **4** is particularly notable, as the nitrogen center replacing the sulfur of SAH is similarly uncharged. Therefore, it appears that SAH riboswitches discriminate by steric exclusion of compounds like SAM, **3** and **4** that carry additional chemical moieties on the atom equivalent to the sulfur in SAH.

In addition to expanding the list of riboswitch classes, the identification of SAH aptamers leads to applications as highly selective SAH sensors. For example, SAH is a source of homocysteine, which can be used as a marker for cardiovascular disease (Nygård et al., 1999). The shortest example identified in sequenced bacterial genomes that conforms to the consensus of the SAH element is slightly more than 50 nucleotides in size (occurring upstream of ORF Daro_0186 in *Dechloromonas aromatica*). Coupled with nanomolar affinity for SAH and discrimination by several orders of magnitude against closely related analogs such as SAM, as well as readily available expression platforms, the SAH element possesses desired properties for *in vitro* and *in vivo* applications.

### 3. Experimental Procedures

### i. Oligonucleotides, Chemicals, and Bacteria

Synthetic DNA oligonucleotides were purchased from the W. M. Keck Foundation Biotechnology Resource Laboratory at Yale University or from Sigma-Genosys, and were used without further treatment unless otherwise stated. [³H]SAM or *S-*(5'-adenosyl)-L-methionine-(methyl-³H) was purchased from Sigma-Aldrich, and [γ-³²P]ATP was purchased from Amersham Biosciences. Compound **4** was the gift from Prof. G. Michael Blackburn at The University of Sheffield. All other chemicals were purchased from Sigma-Aldrich, except for SAH analogs **3, 5, 6,** and **9-16,** whose preparations were described elsewhere (Lim et al., 2006b). *Pseudomonas syringae* pv. *tomato* str. DC3000 was the gift of Prof. Gregory B. Martin at Cornell University.

### ii. RNA Preparation

The 68 *meth* and 122 *ahcY* RNAs were prepared by transcription *in vitro* using T7 RNA polymerase and the corresponding DNA templates. The DNA template for 68 *metH* was generated by extending a primer carrying the T7 promoter sequence hybridized to synthetic template DNA, using SuperScript II reverse transcriptase (Invitrogen) following the manufacturer's instructions. Synthetic template DNAs were purified by using denaturing (8 M urea) polyacrylamide gel electrophoresis (PAGE) prior to extension. The DNA templates for wild-type and mutant 122 *ahcY* RNAs were produced from PCR amplification using appropriate plasmids as described below. The RNA transcripts were purified by denaturing PAGE and eluted from gel fragments using a solution containing 10 mM Tris-HCl (pH 7.5 at 23°C), 200 mM NaCl and 1 mM EDTA. RNAs were concentrated by precipitation with ethanol, dephosphorylated with alkaline phosphatase (Roche Diagnostics) and 5'-radiolabeled using [γ-³²P]ATP and T4 polynucleotide kinase (New England Biolabs) following the manufacturer's instructions. Radiolabeled RNAs were purified by denaturing PAGE and isolated as described above.

The wild-type and mutant 68 *metH* RNAs used for equilibrium dialysis were prepared and purified as described above, but without dephosphorylation and radiolabeling. The 156 *metA* RNA (Corbino et al., 2005) used for equilibrium dialysis was prepared as described above using DNA templates generated by whole-cell PCR of *Agrobacterium tumefaciens* strain GV2260.

### iii. In-line Probing Analysis

In-line probing assays were carried out essentially as previously described (Soukup and Breaker, 1999). 5' ³²P-labeled RNAs were incubated at room temperature for approximately 40 h in 10 µl volumes containing 50 mM Tris-HCl (pH 8.3 at 25°C), 20 mM MgCl₂, 100 mM KCl, and included test compounds at the concentrations indicated. Spontaneous cleavage products were separated by denaturing 10% PAGE, visualized using a PhosphorImager (Molecular Dynamics), and quantified using SAFA v1.1 software (Das et al., 2005). The amount of RNA cleaved at each nucleotide position was obtained from SAFA analysis, which automatically determines reference nucleotide positions with invariant levels of spontaneous cleavage to correct for different amounts loaded in each lane. Individual band intensities at each modulated site were summed and normalized to obtain values for the fraction of RNA that was structurally modulated, assuming that there was no modulation in the absence of ligand and maximal modulation in the presence of the highest concentration of ligand. Values for the apparent dissociation constant (*K*_{D}) were determined by fitting the plot of the fraction modulated (F), versus the ligand concentration, [L], at all three indicated modulation sites to the equation F = [L]/([L] + *K*_{D}) using SigmaPlot 9 software (Systat Software, Inc.).

### iv. Equilibrium dialysis

Equilibrium dialysis was performed by delivering 20 µl samples to each side of a Dispo-Equilibrium Biodialyzer (The Nest Group, Inc., Southboro, MA, USA) wherein the separation between chambers is maintained by a dialysis membrane with a 5000 Dalton molecular weight cut-off. One chamber contained a buffer solution comprised of 100 mM Tris-HCl (pH 8.3 at 24°C), 20 mM MgCl₂, 100 mM KCl, plus 100 nM [³H]SAM. The opposing chamber contained the same buffer solution plus either 156 *metA* or 68 *metH* RNAs at the concentrations indicated. After incubation for 11.5 h at 25°C, aliquots of 5 µl were withdrawn from both chambers and radioactivity was measured by a liquid scintillation counter.

### v. In-vivo reporter gene expression assays

Nucleotides -367 to +6 relative to the predicted *ahcY* translation start site (GenBank accession: NC_004578.1; nucleotides 5,771,072 to 5,771,444) were amplified as a BamHI-HindIII fragment by whole-cell PCR of *Pseudomonas syringae* pv. *tomato* str. DC3000. The PCR product was cloned into the translational fusion vector pRA301 (Akakura and Winans, 2002) immediately upstream of the *lacZ* reporter gene. This reporter vector is stably maintained in pseudomonas since it harbors the origin of replication *rep* and stability region *sta* derived from the pseudomonas aeruginosa vector pVS1 (Itoh et al., 1984). Mutants M1-M8 were generated using the resulting plasmid as the template, appropriate mutagenic primers, and the QuikChange site-directed mutagenesis kit (Stratagene) according to the manufacturer's instructions. The integrity of all recombinant plasmids was confirmed by sequencing. Transformations of pRA301 variants into *Pseudomonas syringae* were accomplished by electroporation following a standard protocol (Ausubel et al., 1992). The correct transformants were identified by selecting for spectinomycin (75 µg ml⁻¹) and rifampicin (50 µg ml⁻¹) resistance.

To measure expression levels of the *lacZ* gene in wild-type (WT) and mutant *Pseudomonas syringae* reporter strains, cells were grown overnight with shaking at 28°C in either King's medium B (King et al., 1954) (20 g l⁻¹ Proteose Peptone no. 3, 10 g l⁻¹ glycerol, 1.5 g l⁻¹ K₂HPO₄, 1.5 g l⁻¹ MgSO₄·7H₂O, pH 7.2), 50 µg ml⁻¹ rifampicin and 75 µg ml⁻¹ spectinomycin, or Vogel-Bonner minimal medium (0.2 g l⁻¹ MgSO₄·7H₂O, 2 g l⁻¹ citric acid monohydrate, 10 g l⁻¹ K₂HPO₄, 3.5 g l⁻¹ NH₄NaHPO₄·4H₂O, pH 7.0), 0.5 % w/v glucose, 50 µg ml⁻¹ rifampicin and 75 µg ml⁻¹ spectinomycin. The overnight cultures were diluted to A₆₀₀ = 0.1 to 0.2 into the same medium, and supplemented with additional compounds as indicated. Cultures were incubated for an additional 4.5 h (King's medium B) or 6 h (Vogel-Bonner medium) before performing β-galactosidase assays following standard protocols (Miller, 1992). Miller units reported were averages of three repetitive experiments.

### D. Example 4: Confirmation of a second natural preQ₁ aptamer class in Streptococcaceae bacteria

Bioinformatics searches of eubacterial genomes have yielded many riboswitch candidates where the identity of the ligand is not immediately obvious on examination of associated genes. One of these motifs is found exclusively in the family Streptococcaceae within the 5' untranslated regions (UTRs) of genes encoding the hypothetical membrane protein classified as COG4708 or DUF988. A riboswitch binding the queuosine biosynthetic intermediate pre-queuosine₁ (preQ₁) has been identified in the 5' UTR of homologous genes in many Firmicute species of bacteria outside of Streptococcaceae. It is herein shown that a representative of the COG4708 RNA motif from *Streptococcus pneumoniae* R6 also binds preQ₁. Furthermore, representatives of this RNA have structural and molecular recognition characteristics that are distinct from those of the previously described preQ₁ riboswitch class. PreQ₁ is the second metabolite for which two or more distinct classes of natural aptamers exist, indicating that natural aptamers utilizing different structures to bind the same metabolite can be common. Additionally, the association of preQ₁ binding RNAs with most genes encoding proteins classified as COG4708 shows that these proteins function as transporters for preQ₁ or another queuosine biosynthetic intermediate.

Riboswitches are structured RNAs that bind small molecule metabolites to regulate gene expression (Mandal and Breaker 2004a; Soukup and Soukup 2004; Tucker and Breaker 2005; Winkler and Breaker 2005). They typically consist of a ligand-binding aptamer and an expression platform that translates ligand binding into genetic regulation. The aptamer of each riboswitch class usually is well conserved due to constraints imposed by the ligand binding site. This conservation of aptamer sequences and structures of aptamers makes bioinformatics analyses of genomic sequences an effective tool for identifying new riboswitch candidates (e.g. Rodionov et al. 2002; Rodionov et al. 2003; Barrick et al. 2004; Abreu-Goodger and Merino 2005; Corbino et al. 2005; Weinberg et al. 2007). In contrast, expression platforms can vary considerably and utilize diverse genetic regulatory mechanisms such as transcription termination and translation inhibition (Mandal and Breaker 2004a). Although expression platforms usually can be discerned by examining the sequences of individual riboswitches, their relative lack of sequence and structural conservation precludes their usefulness as a target for the bioinformatics discovery of new riboswitch classes.

Bioinformatics searches have been used to discover numerous riboswitches and riboswitch candidates (e.g. Barrick et al. 2004; Corbino et al. 2005). Most recently, the discovery of 22 RNA motifs using the CMfinder pipeline (Yao et al. 2006) that was applied to search several hundred bacterial genomes (Weinberg et al. 2007) was used. Six of the 22 motifs exhibit characteristics that are common of cis-regulatory RNAs such as riboswitches. For example, these motifs are consistently positioned immediately upstream of a gene or distinct set of genes. In addition, representatives of these motifs have one of two features: a rho-independent transcription terminator or a stem that overlaps the ribosomal binding site or Shine-Dalgarno sequence (SD) for the gene located immediately downstream. For several of the motifs described, ligand identity was inferred from the genomic context of the RNA (Weinberg et al. 2007; Wang et al. 2008; Regulski et al. submitted). For other motifs, the genomic context previously provided little or no information about ligand identity. One such RNA motif was found to be associated only with genes for a conserved hypothetical membrane protein found in Firmicutes that is classified as COG4708 or DUF988.

A riboswitch responding to the queuosine (Q) biosynthetic precursor preQ₁ (Roth et al. 2007) has been described. Q is a hyper-modified base found at the wobble position of GUN anticodons in tRNAs for Tyr, Asn, Asp, and His in most bacteria (Harada and Nishimura 1972). The Q modification is known to be important for translational fidelity (Meier et al. 1985; Bienz and Kubli 1981; Urbonavi 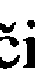 ius et al. 2001). Q biosynthesis starts with GTP and proceeds through the intermediate preQ₀ (7-cyano-7-deazaguanine) and preQ₁ (7-aminomethyl-7-deazaguanine) in a series of enzymatic steps (Kuchino et al. 1976; Okada et al. 1978; Iwata-Reuyl 2003; Gaur and Varshney 2005; Van Lanen et al. 2005). PreQ₁ is preferentially exchanged for a specific guanine tRNA, and the remaining biosynthetic steps take place *in situ* (Okada et al. 1979; Slany et al. 1993). Therefore, preQ₁ is the last Q precursor that exists in free form prior to insertion into the tRNA.

Representatives of the known preQ₁ riboswitch class (Roth et al. 2007) are often located upstream of a Q biosynthetic operon (Reader et al. 2004), but some also have been identified upstream of other genes encoding proteins otherwise not associated with Q, and one of these gene types is classified as COG4708. Thus, it was speculated that COG4708 proteins can be involved in transporting Q biosynthetic precursors.

In present study, it was determined that the RNA associated with genes encoding COG4708 proteins binds preQ₁ tightly and selectively, as well as displays characteristics consistent with a genetic 'off' switch. Thus it was proposed that representatives of this RNA motif indeed serve as a second class of preQ₁ riboswitches, hereafter termed preQ₁-II. The preQ₁-II riboswitch aptamer has a substantially different structure and molecular recognition profile than that of the previously described preQ₁ riboswitch (Roth et al. 2007), now renamed preQ₁-I. Thus, preQ₁ joins S-adenosylmethionine (SAM) (Corbino et al. 2005) as the second example of a metabolite recognized by at least two classes of natural RNA aptamers. Moreover, the discovery of new riboswitch classes can be aided by conducting searches in the non-coding regions of mRNAs whose homologs are controlled by known riboswitches.

### 1. Results And Discussion

### i. Bioinformatics of the COG4708 RNA motif

The original description of the COG4708 RNA motif included 22 sequences from the family Streptococcaceae, of which six were unique (Weinberg et al. 2007). To identify additional examples of the COG4708 RNA motif, homology searches were conducted using an updated sequence database release (RefSeq25). An additional 18 sequences matching the motif were identified, bringing the total number of sequences to 40 and the number of unique sequences to 13 (Fig. 15).

Most of the new representatives were identified in organisms such as S. *pneumoniae, S. pyogenes,* or *Lactococcus lactis,* where representative strains had previously been sequenced. Thus, some of the additional COG4708 RNAs are quite similar or identical to those already described. Sequences matching the motif in *S*. *suis, S. sanguinis,* and *Lactobacillus casei* were also identified. All representatives of the motif were found in the 5'-UTR of genes encoding the protein annotated as COG4708 or DUF988. The conserved features of the motif described previously (Weinberg et al. 2007) including the pseudoknot, loop sequences, and SD sequence within the aptamer are present in all new representatives (Fig. 16A). This class of structured RNAs is conserved over a wider range of bacterial species than had previously been determined. In addition, both the consensus sequence pattern and the secondary structure model for the RNA are substantially more complex that those observed for the preQ₁-I aptamer class (Roth et al. 2007).

### ii. COG4708 RNA motif representatives likely function as preQ₁-sensing riboswitches

Based on the association of genes encoding COG4708 proteins with the previously described preQ₁-I riboswitch (Roth et al. 2007), it was hypothesized that a second conserved RNA motif associated with genes for COG4708 also senses preQ₁. To determine whether COG4708 RNAs undergo structural changes in response to preQ₁ binding, in-line probing assays (Soukup and Breaker 1999; Winkler et al. 2002) were performed on a representative of this motif in the presence and absence of preQ₁. In-line probing assays exploit differing rates of spontaneous RNA cleavage due to internal phosphoester transfer. Unconstrained regions of an RNA chain typically have rates of cleavage that are faster than constrained regions so that RNA structure changes on metabolite binding generally result in changes to the RNA fragmentation pattern (Soukup and Breaker 1999; Li and Breaker 1999).

A 5' ³²P-labeled RNA corresponding to a 103-nucleotide construct encompassing the COG4708 RNA motif from *S*. *pneumoniae* R6 (hereafter termed 1-103 RNA) was incubated with varying concentrations of preQ₁ and the spontaneous cleavage products were separated by denaturing polyacrylamide gel electrophoresis (PAGE). The fragmentation pattern of the 1-103 RNA exhibits a concentration-dependent change, thus revealing preQ₁-dependent RNA structure changes occur (Fig. 16B). These findings also confirm that COG4708 RNAs are preQ₁ aptamers of a distinct class that we have named preQ₁-II. The cleavage pattern of the full-length 1-103 RNA construct is consistent with the secondary structure predicted based on phylogenetic analysis (Weinberg et al. 2007). The loops and joining regions show evidence of cleavage while the predicted base-paired regions remain largely protected from cleavage (Fig. 16C). Most nucleotides that exhibit reduced rates of cleavage correspond to nucleotides that are most highly conserved (Fig. 16C). The SD sequence and anti-SD that comprise P3 show some cleavage in the absence of preQ₁. This cleavage is reduced upon ligand binding, showing that the interaction of the SD and anti-SD is stabilized in the presence of preQ₁, which is expected to sequester the SD and prevent the initiation of translation. In contrast, the high level of spontaneous cleavage that occurs at nucleotides numbered 16 and lower indicates that the 5' region of the 1-103 RNA construct remains largely unstructured. This observation, coupled with the relatively sparse distribution of conserved nucleotides among preQ₁-II aptamer representatives, shows that this region is not involved in forming critical portions of the aptamer structure.

Based on bioinformatics and structural probing data, it has been shown that the preQ₁-II RNA motif from *S*. *pneumoniae* functions as a genetic off-switch, where ligand binding stabilizes the P3 secondary structure to sequester the SD sequence and prevent gene expression. This mechanism of gene control has previously been observed for several other riboswitches (Rodionov et al. 2002; Mandal and Breaker 2004a; Fuchs et al. 2006; Wang et al. 2008). Similarly, representatives of the preQ₁-I riboswitch are predicted to down-regulate expression of homologous COG4708 genes in response to preQ₁ (Roth et al. 2007).

### iii. The minimal preQ₁-II secondary structure is accurately predicted by bioinformatics

A series of 5' and 3' truncated RNAs and used in-line probing to determine whether truncated constructs retain preQ₁ binding activity were constructed. An RNA containing pairing elements P1 through P4 (nucleotides 17-90) has an apparent *K*_{D} for preQ₁ of approximately 100 nM (Fig. 17A, B). This affinity is within the range observed for other riboswitch aptamers, and therefore demonstrates that the 5'-most nucleotides that remain relatively unstructured during in-line probing (Fig. 16C) are not necessary for high-affinity metabolite binding. An even shorter RNA construct (nucleotides 33-90) missing P1 has approximately the same *K*_{D} as the 17-90 RNA construct, revealing that the conserved P1 element also is unlikely to be involved molecular recognition of the preQ₁ ligand, despite the co-variation observed at several positions (Weinberg et al. 2007). Perhaps P1 serves a role during RNA folding or function *in vivo* that is not captured by our *in vitro* in-line probing assays.

To further examine the proposed secondary structure model, constructs were created that carry disruptive and compensatory mutations in each pairing element of the 17-90 RNA (Fig. 17C) and the ligand-binding activities of these constructs were assessed by using in-line probing (Fig. 17D). As expected, the disruptive mutation in P2 (M1) decreases ligand binding affinity by ~100 fold (*K*_{D} ~10 µM), and the compensatory mutation (M2) partially rescues ligand binding affinity (*K*_{D} ~250 nM). The pattern of spontaneous cleavage products is consistent with structures wherein P2 is unpaired in M and paired in M2.

The disruptive mutations M3 and M5 located in P3 and P4, respectively, both abolish preQ₁ binding (Fig. 17D). Whereas the compensatory mutation M6 (in P4) fully restores ligand binding as expected, the M4 mutant carrying compensatory changes to restore P3 base pairing does not yield a restoration of preQ₁ binding. The failure of this compensatory mutation can be due to several reasons. M3 and M4 both exhibit patterns of spontaneous cleavage during in-line probing that differ from that of the wild-type RNA in the absence of ligand, showing that these mutations cause considerable misfolding. Additionally, the P3 nucleotides mutated are strictly conserved due to the presence of SD and anti-SD sequences, and this region modulates upon ligand binding (Fig. 16B, C). These characteristics suggest that altering the base identities in P3 causes extensive misfolding, and that molecular contacts that are (either directly or indirectly) important for preQ₁ binding can be lacking in the M4 construct.

### iv. Equilibrium dialysis confirms preQ₁ binding

Equilibrium dialysis experiments were preformed with ³H-preQ₁ and the 17-90 RNA construct to confirm ligand binding and the specificity of molecular recognition using a different methodology. For equilibrium dialysis, ³H-preQ₁ was added to chamber *a* of an equilibrium dialysis apparatus, and excess RNA is added to chamber *b*. The two chambers are separated by a 5,000 Dalton molecular weight cut off dialysis membrane (Fig. 18A). The solutions were allowed to equilibrate and the fraction of tritium in each chamber was subsequently measured by liquid scintillation counting. A shift of tritium toward the RNA when ³H-preQ₁ is equilibrated with the 17-90 RNA to give a ratio of radioactive signal for the two chambers (*b*/*a*) of ~1.75 (Fig. 18B) was observed, indicating that RNA is binding ³H-preQ₁ and sequestering it to chamber *b*. When ³H-preQ₁ (1 µM) is equilibrated without RNA, or with an RNA that lacks the ability to form the P3 stem (1-81 RNA), the ³H label distributes evenly *(b*/*a ~1).* When the 17-90 RNA is allowed to equilibrate with ³H-preQ₁ and establish an asymmetric distribution of radioactivity, the addition of excess unlabeled preQ₁ to chamber *a* again causes the ³H label to redistribute. However, the addition of excess unlabeled guanine has no effect on the distribution of ³H, indicating that guanine at the concentration tested cannot compete with ³H-preQ₁ for the ligand binding site in the 17-90 RNA.

Although the trends observed during equilibrium dialysis are consistent with the binding functions of the preQ₁-II aptamer observed by in-line probing, the extent of ³H-label shifted is not as large as expected. The concentration of RNA used should have provided excess binding sites for ³H-preQ₁ and, given the measured *K*_{D} of the aptamer for its ligand, should have been sufficient to saturate all preQ₁ molecules with RNA. Therefore, the *b*/*a* ratio should have been greater when equilibration occurred in the absence of unlabeled preQ₁ competitor.

To examine this discrepancy further, experiments were conducted where the contents of chamber *a* containing the unshifted ³H-labeled material remaining after equilibration with the 17-90 RNA was transferred to chamber *a* of a new apparatus opposite a newly-prepared sample of 17-90 RNA, and the two chambers were allowed to reequilibrated (Fig. 18C). As expected the ³H-labeled material distributed evenly between both chambers (Fig. 18D), indicating that the molecular source of the unshifted portion of the ³H is not bound by the RNA and is unlikely to be preQ₁. From this analysis, it was estimated that only approximately 25% of the ³H-labeled material is preQ₁. Consistent with this conclusion are the results of a purity analysis of the ³H-labeled preQ₁ sample used for equilibrium dialysis. Approximately 82% of the ³H-label is derived from solvent, and no other UV-absorbing compounds are present in the mixture. In summary, the equilibrium dialysis experiments also indicate that an RNA construct carrying the core of the COG4708 RNA motif functions as a selective aptamer for preQ₁.

### v. Selective recognition of preQ₁ by 17-90 RNA

The molecular recognition determinants of the preQ₁-II aptamer from S. *pneumoniae* were established by measuring the binding affinities of the 17-90 RNA for a series of preQ₁ analogs. To assess recognition of the aminomethyl group, we tested preQ₀ (7-cyano-7-deazaguanine), 7-(N,N'-dimethylaminomethyl)-7-deazaguanine, and 7-carboxamide-7-deazaguanine. Both the dimethylaminomethyl analog and preQ₀ exhibit *K*_{D} values of ~500 nM, indicating that there is no substantial steric occlusion occurring at the terminus of the alkylamine group, and that this group is unlikely to function as a hydrogen bond donor (Fig. 19A). Rather, the productive interaction to the aminoethyl group might involve the amino group as a hydrogen bond acceptor. Although rare, the nitrogen atom of an amino group can (Luisi et al. 1998) serve as a hydrogen bond acceptor in some other nucleic acid structures.

As was observed by using equilibrium dialysis (Fig. 18B), the 17-90 RNA strongly discriminates against guanine (*K*_{D} ~10 µM). The guanine analog 7-deazaguanine is bound with only a slightly better affinity, showing that little selectivity for preQ₁ is derived from the absence of a nitrogen atom at position 7. The lack of 7-methylguanine binding at concentrations as high as 1 mM is likely due to the absence of a hydrogen bond donor at position 9 rather than to any disruption of molecular recognition contacts at position 7.

From the remaining purine compounds tested, it is clear that the amine at position 2 is critical for ligand binding (Fig. 19A). 2,6-diaminopurine (DAP) is the only compound that lacks a guanine-like Watson-Crick base pairing edge but retains the ability to be measurably bound by the RNA at the concentrations tested. Interestingly, compounds carrying either a keto oxygen (guanine) or an amine (DAP) at position 6 of the purine ring yield near identical *K*_{D} values. However, no binding is detected for 2-aminopurine which differs from guanine and DAP by lacking an exocyclic functional group at position 6. Thus, the keto group of guanine and the amino group of DAP at position 6 appear to contribute equally to molecular recognition. Although there are more complex possibilities, the simplest explanation for this observation is that both the keto group of preQ₁ and the nitrogen atom of the DAP amine at position 6 serve as hydrogen bond acceptors.

A summary of the putative molecular recognition contacts made by the 17-90 RNA and its preQ₁ ligand (Fig. 19B) reveal differences compared to the molecular recognition contacts identified for preQ₁-I aptamers (Roth et al. 2007). Recognition of the aminomethyl moiety at the 7 position is substantially different, as the relative specificities of the 7-dimethylaminomethyl and 7-carboxymethyl analogs are reversed for the two aptamer classes. In addition, the preQ₁-I riboswitch forms a much tighter interaction with guanine, resulting in significantly less specificity for preQ₁ over guanine. Similar to the preQ₁-I riboswitch, the amine at position 2 appears to be critical for ligand binding for 17-90 RNA. However, the analog binding data show that the preQ₁-II aptamer does not form a contact with the N1 position of the purine ring. This differs from the Watson-Crick base pair proposed (Roth et al. 2007) to form between the ligand and the preQ₁-I aptamer. It is clear from the existing data that the distinctive sequences and structural elements of preQ₁-II aptamers fold to form a binding pocket that is distinct from that made by preQ₁-I aptamers.

### vi. Watson-Crick base-pairing is unlikely to account for ligand recognition

Riboswitches binding guanine and adenine have been found to use canonical Watson-Crick base pairs for selective ligand binding (Gilbert et al. 2006; Mandal and Breaker 2004b; Batey et al. 2004; Serganov et al. 2004; Noeske et al. 2005). A single point mutation of the purine riboswitch aptamer is sufficient to switch aptamer specificity between guanine and adenine (Mandal and Breaker 2004b). Similarly, the specificity of a riboswitch binding 2'-deoxyguanosine can be altered by a comparable mutation (Kim et al. 2007). An analogous mechanism can be used in preQ₁-I riboswitch aptamers, where mutation of a conserved cytidine to uridine alters ligand specificity from preQ₁ to DAP (Roth et al. 2007).

To assess whether the 17-90 RNA uses a similar mechanism to promote ligand specificity, the sole universally conserved unpaired cytidine (C33) (Fig. 15, Fig. 16A) was mutated to uridine. Although there are additional strictly conserved cytidine residues present in preQ₁-II aptamers (C32 and C51), these reside within predicted base-paired elements and thus are unlikely to be forming a canonical base pair with the ligand. However, it was observed that the mutant RNA carrying a U at position 33 behaves very similarly to the wild-type RNA, and exhibits a *K*_{D} for preQ₁ of ~250 nM. Therefore this residue is unlikely to form a canonical base pair with the ligand. This observation is not surprising given that this area of the RNA does not modulate on ligand binding (Fig. 16B, C), and that P1 is not necessary for ligand binding.

Nucleotide C41 was also observed as a candidate for base-pair formation with preQ₁. This nucleotide is proximal to the conserved core of the aptamer and modulates upon ligand binding (Fig. 16). Only one representative of the 40 known COG4708 RNAs was found wherein C41 is mutated to a U residue (Fig. 15). A 17-90 RNA construct carrying this C-to-U change at position exhibits ~2 orders of magnitude loss in affinity for preQ₁ (Fig. 20A), indicating that this residue indeed is important for ligand binding. However, the mutant construct does not undergo a change in specificity to favor analogs with an adenine base-pairing face. These results show that C41 also may not form a canonical base pair with the ligand.

Although the U41 mutant binds preQ₁ more poorly than the wild-type 17-90 RNA, this mutant binds DAP with essentially the same affinity as wild type (Fig. 20B). In contrast, guanine binding is no longer detectable with the U41 mutant. These observations show that the nucleotide at position 41 forms a contact with the ligand in a manner consistent with the molecular recognition model for preQ₁-II aptamers (Fig. 19B). The exocyclic amino group of C41 can donate a hydrogen to the keto group of preQ₁, but the keto group in place of this amine in U41 cannot form an equivalent hydrogen bond (Fig. 20C). Therefore, as is observed, the mutant RNA should bind preQ₁ more poorly than the wild-type RNA (Fig. 20A). In contrast, the exocyclic amino group of C41 can donate a hydrogen to the nitrogen atom of the exocyclic amine at the 6 position of DAP to form a hydrogen bond. Likewise, the corresponding keto group of U41 can serve as a hydrogen bond acceptor and allow formation of an equivalent hydrogen bond with the same exocyclic amine at the 6 position of DAP (Fig. 20C). This arrangement cam explain why near identical K_{D} values for DAP binding by the C41 and U41 RNAs (Fig. 20B) are observed.

### 2. Conclusions

PreQ₁ is the second example of a metabolite recognized by more than one class of natural aptamers. Four different aptamer classes recognizing *S*-adenosylmethionine (AdoMet or SAM) have been identified to date. SAM-I or S-box (Epshtein et al. 2003; McDaniel et al. 2003; Winkler et al. 2003), SAM-II (Corbino et al. 2006), SAM-III or SMK-box (Fuchs et al. 2006) and SAM-IV (Weinberg et al. 2007) often regulate the same genes in different organisms (e.g. SAM synthase, homoserine O-succinyltransferase). The discovery of a second natural preQ₁ aptamer class shows that SAM is not a special case and that additional metabolites may be bound by multiple distinct RNA architectures to control similar sets of genes in different organisms.

The association of two preQ₁-binding RNAs with the membrane protein classified as COG4708 or DUF988 shows this protein is a transporter for a queuosine biosynthetic intermediate. Membrane proteins of unknown function whose genes are associated with the TPP riboswitch have subsequently been shown to be involved with TPP and thiamin transport (Rodionov et al. 2002; Winkler et al. 2002; Schyns et al. 2005) illustrating how characterization of RNA genetic control elements can lead toward description of protein function. Likewise, knowledge of the function of a protein encoded by an mRNA can aid in establishing the ligand identity of an associated riboswitch.

Interestingly, in many bacteria there are numerous genes with homology to those associated with preQ₁ biosynthesis or transport, yet these genes have no established control mechanisms. Therefore, it was speculated that the mRNAs for such genes could carry other preQ₁ riboswitch classes. To assess the potential for new preQ₁ aptamer discovery, the intergenic regions upstream of several COG4708 genes in organisms that lack preQ₁-I and preQ₁-II aptamers and whose genomes have been sequenced was observed.

The COG4708 gene is narrowly distributed in bacteria, with all but three examples occurring in the classes Bacilli and Clostridia (Fig. 21). There is one example of COG4708 in a γ-proteobacteria *(Rubrobacter xylanophilus)* and there are two examples found in Archaea (*Thermophilum pendens* and *Staphylothermus marinus).* Genes corresponding to COG4708 proteins are associated with a preQ₁ aptamers in 30 of 46 instances (Fig. 21). Several organisms contain two copies of the gene, and a riboswitch is typically associated with only one of these copies, although in *Alkaliphilus metalliredigens* both copies of the gene are preceded by a preQ₁-I riboswitch. In many organisms where the gene for COG4708 is not associated with a known preQ₁-sensing RNA, the intergenic region directly upstream of the ORF is greater than 50 base pairs. This is sufficient space for an RNA aptamer to be located in the 5' UTR of the mRNA. However, such regions typically display little to no detectable homology to any other sequence, showing that they either lack structured RNA or that they do not carry an aptamer that has homologs in many other bacteria.

In-line probing assays were used to examine the intergenic regions upstream of ORFs encoding COG4708 proteins in *Moorella thermoacetica, Clostridium difficile, Oenococcus oeni, Geobacillus kaustophilus,* and upstream of both COG4708 copies in *Streptococcus thermophilus.* None of the RNAs tested show structural modulation in the presence of preQ₁, showing that they do not contain preQ₁-binding riboswitches. However, several different factors including RNA misfolding due to interference from extraneous 5' or 3' sequence, as well as differences between *in vivo* and *in vitro* conditions can complicate in-line probing assays.

In contrast to most other identified riboswitches, the preQ₁-II aptamer is very narrowly distributed, predominantly occurring in bacteria from the Streptococcaceae family. This narrow distribution has precedent, as SAM-III also appears limited to the same group of bacteria (Fuchs et al. 2006).

### 3. Materials And Methods

### i. Chemicals and DNA Oligonucleotides

The chemical syntheses of preQ₁, preQ₀, 7-(N,N'-dimethylaminomethyl)-7-deazaguanine, 7-carboxamide-7-deazaguanine, and 7-aminomethyl-7-deazaadenine were described previously (Roth et al. 2007). The remaining purine compounds and other chemicals were purchased from Sigma-Aldrich unless noted otherwise. ³H-preQ₁ was obtained as previously described (Hurt et al. 2007). Synthetic DNAs were prepared by Sigma-Genosys.

### ii. RNA preparation

The portion of the 5' UTR of the gene encoding COG4708 corresponding to the preQ₁-II (Cog4708 RNA) motif was PCR amplified from *Streptococcus pneumoniae* R6 using the following primers: 5'-GGAATTCAACAAGTCTAACAGAAAAGTAG AAAGGCGGGC-3' (SEQ ID NO: 1), 5'- TTTTGTCATTTTTTCTCCTTTAACGT CTGGATAACTCTCAAAAGC-3' (SEQ ID NO:2). The resulting double-stranded DNA was subsequently cloned into pCR2.1 using a TOPO TA cloning kit (Invitrogen). The plasmid insert was sequenced (The W. M. Keck Foundation Biotechnology Resource Center, Yale University) and used as template for PCR amplification of DNA fragments encoding the desired RNA preceded by a T7 promoter sequence and including two guanosine residues to improve transcription efficiency. Disruptive and compensatory mutations were introduced into PCR products using mutant primers for PCR. PCR products were transcribed *in vitro* using T7 RNA polymerase and the resulting RNA purified by denaturing 6% PAGE using methods similar to those described elsewhere (Seetharaman et al. 2001).

DNA fragments corresponding to the constructs described from *S. thermophilus* were constructed by PCR (Dillon and Rosen 1990) from sets of synthetic oligonucleotides based on the genomic sequence. DNA fragments corresponding to the intergenic regions upstream of genes encoding COG4708 proteins from *M. thermoacetica, O. oeni (PSU-1), C. difficile,* and *G. kaustophilus* were amplified by PCR using the respective genomic DNAs and primers containing the T7 promoter sequence in the appropriate position. *O. oeni* and *C. difficile* genomic DNA samples were obtained from ATCC. RNA molecules were prepared by in vitro transcription as described above.

### iii. In-line probing assays

RNA molecules prepared by *in vitro* transcription were dephosphorylated with alkaline phosphatase (Roche Diagnostics), radiolabeled with [γ-³²P]ATP (GE Biosciences) and T4 polynucleotide kinase (New England Biolabs) according to the manufacturers' instructions. The 5' ³²P-labeled RNAs were purified by PAGE as described above. In-line probing reactions containing ~1 nM of 5' ³²P-labeled RNAs were assembled with and without ligand as described (Soukup and Breaker, 1999). Reaction mixtures containing 50 mM Tris-HCl (pH 8.3 at 23°C), 20 mM MgCl₂, and 100 mM KCl were incubated at 25°C for approximately 40 hours. For RNAs originating from *M. thermoacetica* and *G. kaustophilus,* incubations were also conducted at 58°C for 30 minutes and 60°C for 15 minutes, respectively. Samples were separated by denaturing 10% PAGE and the imaging and quantification were carried out using a Molecular Dynamics PhosphorImager and ImageQuaNT software.

The K_{D} values were determined by conducting in-line probing assays using a series of ligand concentrations. The normalized fraction of RNA cleaved at the sites indicated for each analysis was calculated at each ligand concentration, and a standard binding curve was fit to the points. Concentrations of most compounds examined varied between 1 nM and 1 mM, with the exception of preQ₁, guanine, 7-carboxamide-7-deazaguanine, 7-aminomethyl-7-deazaadenine, where the highest concentrations tested were 100 µM, 100 µM, 10 µM, and 30 µM, respectively.

### iv. Equilibrium Dialysis

A 30 µl mixture containing 50 mM Tris-HCl (pH 8.3 at 23°C), 20 mM MgCl₂, 100 mM KCl, and 1 µM of ³H-labeled preQ₁ was placed in one chamber of a DispoEquilibrium Dialyzer (ED-1, Harvard Bioscience) and the same volume containing the same constituents minus the ³H-labeled preQ₁ was added to the opposing side. When noted, the latter solution also contained either 5 µM 17-90 RNA or 5 µM 1-81 RNA. After equilibrating for 14 hours, a 5 µl aliquot was removed from each side of the apparatus to determine the distribution of ³H-label in the two chambers by liquid scintillation counting. For competitive binding experiments, 3 µL of the buffer mixture described above containing 1 mM unlabeled compound was added to the chamber lacking RNA, and an equivalent volume of buffer was added to the RNA-containing chamber. The chambers were allowed to equilibrate for an additional 8 hours before the distribution of ³H-label was reassessed as described above.

HPLC of the ³H-labeled preQ₁ showed a single UV-active peak consistent with preQ₁. To assess where the ³H-label not incorporated into preQ₁ originated, a 40 µL aliquot of the ³H-preQ₁ solution (~2 mM) was mixed with 8 mg of decolorizing carbon and 200 µL water. The slurry was subsequently separated by centrifugation and the supernatant removed and filtered (0.22 micron). HPLC analysis of the supernatant revealed that no preQ₁ was present in the aqueous portion, indicating that the carbon had completely adsorbed the heterocycle. Tritium present in the supernatant portion and the carbon residue was measured by liquid scintillation counting.

### v. Bioinformatics

Homology searches were conducted on RefSeq25 (Pruitt et al. 2007) and the COG4708 RNA motif consensus sequence was determined as previously described (Weinberg et al. 2007). Instances of COG4708 (DUF988) genes were identified through the PFAM database (release 22.0) (Finn et al. 2006). A BLAST search of the COG4708 protein sequence from several organisms against the NCBI genomic database failed to yield any additional sequences. Examples were limited to fully sequenced genomes and determined whether the preQ₁-I or preQ₁-II riboswitches were present in the intergenic regions upstream of the ORF encoding COG4708 proteins. Highly similar strains or isolates of the same species were removed from the listing to reduce redundancy.

### E. Example 5: A widespread riboswitch candidate that controls bacterial genes involved in molybdenum cofactor and tungsten cofactor metabolism

A highly conserved RNA motif located upstream of genes encoding molybdate transporters, molybdenum cofactor (Moco) biosynthesis enzymes, and proteins that utilize Moco as a coenzyme have been identified. Bioinformatics searches have identified 176 representatives in γ-Proteobacteria, δ-Proteobacteria, Clostridia, Actinobacteria, Deinococcus-Thermus species, and DNAs from environmental samples. Using genetic assays, it has been demonstrated that a Moco RNA in *Escherichia coli* associated with the Moco biosynthetic operon controls gene expression in response to Moco production. In addition, evidence indicating that this conserved RNA discriminates against closely related analogs of Moco has been provided. These results, together with extensive phylogenetic conservation and typical gene control structures near some examples, indicate that representatives of this structured RNA represent a novel class of riboswitches that sense Moco. Furthermore, variants of this RNA that are likely to be triggered by the related tungsten cofactor (Tuco) have been identified, which carries tungsten in place of molybdenum as the metal constituent.

Riboswitches are structured RNA domains that selectively bind metabolites or metal ions and function as gene control elements (Mandal and Breaker, 2004; Soukup and Soukup, 2004; Winkler and Breaker, 2005; Winkler, 2005). Riboswitches are commonly found in the untranslated regions of eubacterial mRNAs, and usually exert control over gene expression by regulating transcription elongation or translation initiation (Breaker and Barrick, 2007). With few exceptions (Winkler *et al.*, 2004; Barick *et al.*, 2004), riboswitches are composed of two modular regions, an aptamer domain and an expression platform. The aptamer forms a selective binding pocket for the target metabolite, and this binding event allosterically regulates the folding of the adjoining expression platform to control gene expression. The metabolite-binding pocket of each riboswitch class is highly conserved in sequence and structure, even among representatives identified from distantly related organisms (Breaker and Barrick, 2007).

Riboswitches make excellent targets for discovery using bioinformatics searches. For example, the CMfinder comparative genomics pipeline (Yao *et al.,* 2007; Weinberg *et al.,* 2007) represents one bioinformatics approach that was used to identify novel functional RNA elements. It identifies the putative 5' untranslated regions (5' UTRs) of homologous genes, and subsequently examines these regions for evidence of sequence and secondary-structure conservation amongst the UTRs from various organisms. The patterns of sequence and structure conservation are used to identify additional homologs for refinement of the initial predicted motif The CMfinder pipeline has identified a number of promising riboswitch candidates that have recently been verified (Wang *et al.*, 2008) or that await verification. One novel motif that has been reported is a large and complex RNA domain that is commonly found upstream of open reading frames (ORFs) coding for proteins involved in molybdenum cofactor (Moco) metabolism (Weinberg *et al.*, 2007).

Moco is a tricyclic pyranopterin (Schwartz, 2005; Baugh *et al.*, 1998) that coordinates an atom of molybdenum (Mo), which is a transition metal located in the same column of the periodic table of the elements as chromium and tungsten. After biosynthesis, Moco is inserted into the active site of Mo-dependent enzymes, which harness the redox activity of Mo to catalyze key reactions in the carbon, nitrogen and sulfur metabolic cycles (Baugh *et al.,* 1998). 176 instances of this motif have been identified in a wide variety of eubacteria including γ-Proteobacteria, δ-Proteobacteria, Clostridia, Actinobacteria and Deinococcus-Thermus and environmental sequences (Weinberg *et al.*, 2007). This motif displays features typical of metabolite-sensing riboswitches, including extensive sequence conservation, evidence of nucleotide covariation within predicted base-paired elements, and an elaborate assembly of base-paired elements intermixed with conserved unpaired nucleotides.

Evidence is herein provided that a representative Moco RNA from *E. coli* selectively senses Moco and controls expression of adjacent genes in response to changing levels of this coenzyme. Furthermore, it is shown that the Moco RNA is involved in regulatory discrimination between Moco and the closely related analog Tuco, thus providing further evidence that Moco is specifically recognized by aptamers formed by Moco RNAs. These experimental findings, together with a correlation between certain RNA variants and coenzyme metabolism characteristics, show that this class of structured RNAs includes distinct Moco- and Tuco-sensing riboswitches.

### 1. RESULTS AND DISCUSSION

### i. The Moco motif is widespread and highly conserved

When a comparative genomics pipeline (Yao *et al.,* 2007) was used to examine UTRs of genes encoding molybdenum cofactor biosynthesis protein A in γ-proteobacteria, a motif was identified that exhibits covariation and other features suggestive of a structured RNA (Weinberg *et al.,* 2007). These searches resulted in the identification of 176 instances of this motif (termed "Moco RNA") in γ-Proteobacteria, δ-Proteobacteria, Clostridia, Actinobacteria, Deinococcus-Thermus species and environmental DNA sequences. These sequences were compared by manual alignment using RALEE (Griffiths-Jones, 2005) guided by the putative consensus structure. This alignment reveals that sequence conservation is highest near the central multi-stem junction of the motif (Fig. 22), which carries most of the nucleotides exhibiting 75 to 100% sequence conservation.

As many as five base-paired elements (labeled P1 through P5) form the secondary structure architecture of Moco RNAs. Within this conserved secondary structure is a GNRA tetraloop (Heus and Pardi, 1991) and a tetraloop receptor (Costa and Michel, 1995) found at the distal end of P4 and centered in a P2 bulge, respectively. The tetraloop sequence in all but five Moco RNAs is GAAA. However, all four Moco RNAs carrying GCAA tetraloops lack the receptor, while only two Moco RNAs with GAAA tetraloops are lacking the receptor. A similar correlation was previously observed for representatives of another new-found RNA motif called the GEMM motif (Weinberg *et al.,* 2007).

Representatives of the Moco motif can be categorized into two groups based on the presence or absence of the P3 stem. The alignment of all known representatives reveals an apparent aptamer region that is highly conserved, followed by sequences that appear to form different types of expression platforms that are typical of known metabolite-sensing riboswitches. For example, a possible expression platform of the *E. coli* Moco RNA upstream of the *moaABCDE* operon (Fig. 23a) appears to encompass the ribosome binding site for the downstream open reading frame (ORF) within the nucleotides forming the P1 stem. This arrangement can show that ligand binding by the aptamer domain represses gene expression. Examples also exist wherein the putative aptamer domain is found upstream of a stem-loop structure that conforms to the expected sequence and structure of a bacterial intrinsic transcription terminator (Gusarov and Nudler, 1999; Yarnell and Roberts, 1999). Moreover, some organisms carry more than one Moco RNA representative and manifest both types of expression platforms in the same organism.

Although most organisms carry one Moco RNA representative per genome, some organisms whose genomes have been fully sequenced have as many as four or five instances of the motif. The highest numbers of Moco RNA representatives are carried by *Desulfitobacterium hafniense*, which has eight, and *Syntrophomonas wolfei*, which has at least 15 versions. Interestingly, tandem arrangements of Moco RNA motifs exist in *S. wolfei* and *Geobacter metallireducens.* Although rare, tandem-arranged riboswitches or their subdomains have been identified with other metabolite-sensing riboswitch classes (Mandal and Breaker, 2004; Famulok, 2004; Sudarsan *et al.*, 2006; Stoddard and Batey, 2006). These more complex architectures are used by riboswitches to achieve sophisticated functions such as the ability to respond to multiple signaling inputs (Sudarsan *et al.,* 2006) or are more responsive to smaller changes in ligand concentrations (Mandal and Breaker, 2004; Welz and Breaker, 2007). One operon in *S. wotfei* carries three Moco RNAs in series.

### ii. The Moco motif is associated with Moco biosynthesis genes

Most known riboswitches function in *cis* to regulate expression of proximal genes in response to binding of their cognate ligand. The ligand sensed by the riboswitch aptamer is typically the final product of the pathway catalyzed by the enzyme or enzymes encoded by the mRNA under control. Alternatively, the small molecule ligand is sometimes required as a cofactor or substrate for the gene product whose expression is controlled by the riboswitch. Since riboswitches usually employ *cis* regulatory mechanism, the functions of the proteins encoded by the genes located downstream of riboswitches can provide much information about the identity of the ligand.

In an effort to establish the function of Moco RNAs, the functions of genes in the neighboring genomic locations that carry representatives of this conserved RNA were considered. In γ-Proteobacteria, including *E. coli,* the motif is found upstream of the *moaABCDE* operon (Fig. 23a), which encodes proteins responsible for molybdopterin (MPT) biosynthesis (Schwarz, 2005). In some bacteria, representatives also reside upstream of *modABC*, which encodes a high affinity molybdate transporter complex (Grunden and Shanmugam, 1997). There are also three species of γ-Proteobacteria (*Haemophilus somnus*, *Haemophilus ducreyi* and *Actinobacillus pleuropneumoniae* serovar 1) where Moco motifs are located upstream of both the molybdopterin biosynthetic operon and a gene predicted to encode molybdopterin oxidoreductase. The Moco motif is also found upstream of different arrangements of genes for Moco biosynthesis enzymes, for high affinity molybdate transporters, and for enzymes that use Moco to catalyze reactions. In general, these genomic contexts point to Moco or a biosynthetic intermediate of this cofactor as the potential ligand for this riboswitch candidate. Several of the known riboswitch classes sense and respond to other common coenzymes, and therefore Moco was considered as the most likely ligand for this riboswitch candidate.

Molybdenum cofactor biosynthesis is a complex and ancient pathway that is conserved from eubacteria to humans. The only organisms that do not require molybdenum or Moco utilize tungsten and the analogous coenzyme Tuco in its place (Schwarz, 2005). In *E. coli* there are five known operons involved in Moco metabolism: *moa*, *mob*, *mod*, *moe* and *mog* (Shanmugam *et al.*, 1999), which encode a total of 15 proteins. The first two steps of Moco biosynthesis are catalyzed by genes encoded by the *moa* operon (Fig. 23b). Specifically, guanosine-5'-triphosphate (GTP) is converted to cyclic pyranopterin monophosphate (cPMP) in a reaction catalyzed by the *S-*adenosylmethionine-dependent enzyme, MoaA, and the uncharacterized homohexameric MoaC (Wuebbens and Rajagopalan, 1995). With a half life of less than one hour, cPMP is the most stable Moco biosynthetic intermediate (Santamaria-Araugo *et al.,* 2004). The heterotetrameric MPT synthase complex formed by MoaD and MoaE then catalyzes the transfer of two sulfur atoms to cPMP, resulting in a MPT dithiolate (Pitterle *et al.,* 1993).

Moco is subsequently synthesized from MPT by the products of *mogA* and *moeA*, through a series of independent steps involving the adenylation of MPT and insertion of Mo to form molybdenum cofactor (Nichols and Rajagopalan, 2002). In *E. coli,* Moco is further modified to bis-molybdopterin guanine dinucleotide cofactor (bis-MGD) by MobA (Lake et al., 2000) The bis-MGD is then inserted into molybdenum-containing enzymes. In other eubacteria, Moco can be modified to yield other derivatives that also find use as coenzymes (Schwarz, 2005). All of the Moco biosynthetic genes noted above, as well as several genes coding for enzymes that use Moco or its derivatives as coenzymes, are associated with Moco RNA motifs in at least some bacteria (Fig. 23b).

### iii. Biochemical analysis of the structural characteristics of a Moco RNA

Given the chemical instability of Moco and its biosynthetic intermediates, it was not practical to test these compounds for direct binding interactions *in vitro.* Therefore, a series of biochemical and genetic experiments to determine if the Moco RNA from *E. coli* has characteristics that are consistent with riboswitch function were conducted. To assess the proposed structural model for this Moco RNA representative, a 138-nucleotide RNA construct (termed 138 *moaA,* Fig. 24a) containing the Moco motif extending from 9 nucleotides upstream of the S' beginning of the base-pairing P1 element to 10 nucleotides beyond the 3' end of P1. 138 *moaA* includes a P3 stem (which is absent in some representatives), the predicted ribosome binding site, and AUG translation start codon of the *moaA* ORF. The RNA also carries two additional G residues at the 5' terminus to facilitate production by *in vitro* transcription. A trace amount of 5' ³²P-labeled 138 *moaA* RNA was subjected to in-line probing analysis (Soukup and Breaker, 1999), which exploits the differences in the frequency of spontaneous cleavage of various internucleotide linkages caused by differences in RNA structure to identify regions of structural rigidity and flexibility.

When separated by polyacrylamide gel electrophoresis (PAGE), the resulting pattern of RNA cleavage products (Fig. 24b) generated during in-line probing is consistent with most of the major secondary structure features that were predicted to form on the basis of comparative sequence analysis (Fig. 22). Specifically, most sites exhibiting a high level of spontaneous phosphoester transfer are localized to the loops of stems P3 and P5, and are also clustered in the junctions that bridge between the predicted base-paired stems. However, the pattern of spontaneous cleavage is not consistent with the secondary structure proposed for much of the P2 stem. Although this stem can have two internal bulges that could possibly exhibit structural flexibility, some RNA fragments observed are the result of cleavage at nucleotides that can be base paired.

The in-line probing results suggest that the P2 stem and the junctions carrying several of the highly conserved nucleotides might undergo a conformational change to form a ligand binding pocket. This is consistent with the behavior of other known riboswitch aptamers, some of which are observed by in-line probing assays to undergo substantial changes in structure on addition of the target metabolite (Nahvi *et al.*, 2002; Winkler *et al.*, 2002; Mandal *et al.*, 2003).

### iv. Moco RNA functions as a gene control element

In *E. coli,* there are two promoter sites and accompanying protein factor binding sites that are known to regulate transcription of the *moa* operon (Fig. 23a). The S2 promoter site is dependent on the anaerobic protein transcription factor, Fnr (Anderson *et al.*, 2000; Spiro and Guest, 1990). This transcriptional upregulation of the operon in response to anaerobic conditions permits increased biosynthesis of Moco, which certain proteins involved in anaerobic respiration require as a coenzyme. The S 1 promoter site of the *moa* operon is activated by molybdate-bound ModE (Anderson *et al.*, 2000), which is a transcription factor that binds molybdate in the cell and upregulates transcription of the *modABCD* operon (Gruden *et al.,* 1999). This ensures that there is sufficient molybdate available in the cell to synthesize Moco. In addition, there is evidence for the control of some genes in the *moa* operon by the copper-responsive factor CueR (Yamamoto and Ishihama, 2005), although the predicted binding site for CueR occurs downstream of the Moco element.

In addition to this multi-layer transcription control network, it was known that a 131-nt stretch between the S 1 promoter and the *moaA* start codon contributed a third regulatory system (Anderson *et al.,* 2000). Specifically, the presence of this region permits repression of the *moa* operon when Moco is present in cells (Anderson *et al.,* 2000; Baker and Boxer, 1991). The effects of the Fnr and ModE transcription factors on *moa* operon expression in *E. coli* can only be observed when this portion of the 5' untranslated region (UTR) is deleted. Intriguingly, this 131-nt region precisely encompasses the Moco RNA motif. Furthermore, no protein regulatory factor has been identified that acts on this portion of the DNA or the corresponding RNA transcript, leaving open the possibility that this portion of the mRNA might serve as a direct sensor for Moco.

The architectural features of Moco RNAs, coupled with the known gene control characteristics of the *E. coli moa* operon, show that Moco RNA representatives are metabolite-sensing riboswitches. To confirm that a Moco RNA functions as a genetic control element, PCR was used to amplify a portion of the *E. coli* genome corresponding to the wild-type *moaA* 5' UTR. This genome segment encodes a 149-nucleotide RNA (termed 149 *moaA)* that begins 9-nt upstream of the 5' beginning of P1 and extends 21-nt beyond the 3' end of P 1 into the *moaA* coding region. Also, two variant DNAs were generated that express the 149 *moaA* RNA with mutations that disrupt (M1) and subsequently restore (M2) base pairing in the P3 stem (Fig. 25a).

These sequences were cloned into a translational fusion plasmid upstream of, and in-frame with a β-galactosidase reporter gene. The fusion was placed under the control of a constitutively active *lacUV5* promoter to eliminate the effects of upstream anaerobic- and molybdate-dependent promoter regulation that naturally restricts expression of the *moaA* operon. This series of plasmids was placed in a strain of *E. coli* wherein the *moaA* gene was deleted. The resulting transformants were transformed with a plasmid containing the *moaABCDE* operon under the control of an arabinose-inducible and glucose-repressible promoter (Guzman *et al.,* 1995). These doubly transformed strains enabled us to determine the role of the 149 *moaA* RNA on reporter gene expression when Moco concentrations are either high or low.

Cells carrying the wild-type 149 *moaA* construct (WT) exhibited low β-galactosidase gene expression when grown in the presence of arabinose (elevated Moco concentrations expected) (Fig. 25b). In contrast, cells with the WT construct exhibit more than a 3-fold increase in reporter gene expression when grown in the presence of glucose (low Moco concentrations expected). This result shows that the Moco RNA element represses gene expression, most likely by responding to a small molecule product in the Moco biosynthetic pathway. The function of this Moco RNA as a genetic 'OFF' switch is consistent with the mechanism predicted by bioinformatics.

Disruption of the conserved secondary structure of Moco RNAs by the introduction of mutations in the P3 stem (M1) results in derepression of gene expression. In contrast, altering P3 sequences using additional mutations that restore base pairing (M2) also restores gene repression on arabinose-induction of Moco biosynthesis. The disruption of gene control and the return to wild-type reporter gene expression levels exhibited by these variants indicates that the Moco RNA is a gene control element that requires its conserved secondary structure for activity.

### v. Molybdenum transport affects Moco RNA gene control function

Molybdate is transported into *E*. *coli* cells via a high-affinity protein-dependent ABC transporter that is encoded by the *mod* operon (Grunden and Shanmugam, 1997; Maupin-Furlow *et al.,* 1995). In media without molybdate supplementation, wild-type *E. coli* has an estimated intracellular molybdate concentration of 1.0 µM, whereas the intracellular molybdate concentration in a molybdate-transporter deficient strain is estimated at 0.2 µM (Scott and Amy, 1989). However, this molybdate deficiency and the adverse effects it causes can be overcome by the addition of sodium molybdate to the growth medium. Under high extracellular sodium molybdate concentrations, the sulfate transport system is able to import molybdate, bringing the intracellular molybdate concentration of the wild-type and molybdate-transporter deficient strains to approximately 5 µM (Scott and Amy, 1989; Rosentel *et al.*, 1995).

To determine whether molybdate is critical for gene regulation by 149 *moaA* RNA, a *modC* knockout strain of *E. coli* was conducted to eliminate high affinity molybdate transporter function. This strain was then transformed with the *moaABCDE* inducible expression plasmid and the wild-type and mutant 149 *moaA* reporter fusion plasmids. When grown in Luria-Bertani (LB) medium (Sambrook and Russell, 2001), which contains trace amounts of molybdate, the *modC* deletion causes the WT 149 *moaA* reporter fusion to exhibit a high level of gene expression (Fig. 25c). In contrast, when the same strain is grown in LB supplemented with 10 mM sodium molybdate, expression is repressed to a level similar to those of the transformant carrying the functional ModC. This shows that the molecule needed to trigger gene repression via the Moco RNA is not present at a sufficient concentration unless an adequate amount of molybdate is available in cells.

Interestingly, when cells are grown in LB supplemented with 10 mM sodium tungstate, the wild-type 149 *moaA* reporter fusion construct in the transformant carrying the *modC* deletion becomes derepressed. As noted above, tungsten can enter cells and replace molybdenum as the metal ion component of the cofactor. However, if the 149 *moaA* RNA functions as a metabolite-sensing riboswitch (*e.g.* Moco), it has the ability to discriminate between a compound coordinated to molybdenum and the analogous compound coordinated with tungsten. In all cases, the reporter fusion constructs carrying the disruptive (M1) and compensatory (M2) mutations yield the expected gene expression profiles.

### vi. Mutations in biosynthesis genes suggest Moco is the regulatory ligand

Given the results above, it was speculated that Moco is the regulatory factor that can be directly sensed by Moco RNAs. To provide further evidence that a specific compound in the Moco biosynthetic pathway is responsible for gene expression control, knockout mutations were made to various genes along the pathway. In addition to the *moaA* knockout examined earlier (Fig. 25b), strains with knockouts (Δ) of *mogA*, *modC* or *moeA* were made and transformed with the *moaABCDE* inducible expression plasmid and the wild-type 149 *moaA* reporter fusion plasmid. The Δ*mogA*, Δ*modC* and Δ*moeA* knockout strains exhibit derepression regardless of the induction of the *moaABCDE* expression plasmid (Fig. 26). The results indicate that these three proteins are essential for the production of the small molecule signal, and that the induction of the *moa* operon cannot compensate for the loss of these proteins.

The gene expression characteristics of the Δ*modC* strain are similar to that observed previously (Fig. 25c), which demonstrates that molybdate transport is required to form the signaling compound. However, the characteristics of the Δ*moeA* strain reveal that molybdate alone is not likely to be the signaling compound. MoeA catalyzes the insertion of molybdenum into molybdopterin (the final step in the Moco biosynthetic pathway), and this activity is required for regulation of gene expression by the 149 *moaA* RNA.

For numerous riboswitch classes, the ligand sensed by the riboswitch aptamer is the final coenzyme or metabolic product in the biosynthetic pathway being regulated (Winkler and Breaker, 2005). Moco is a compound that is widespread in all domains of life, while some bacteria generate derivatives of Moco that are inserted into the active sites of Mo-dependent enzymes. Moco is the most logical ligand for this riboswitch class.

In *E. coli,* before Moco can be inserted into a protein it must be further modified by MobA (Iobbi-Nivol *et al.,* 1995; Johnson *et al.*, 1991). The resulting bis-MGD form of Moco (Fig. 23b) can then be inserted into molybdoproteins. To determine whether a derivative of Moco might be a riboswitch ligand, a Δ*mobA* strain was constructed and its effects assessed on reporter gene expression as described above. When grown in LB with glucose, the same derepression as in all other transformants is seen (Fig. 26). However, repression of the reporter fusion construct is restored when the cells are grown in LB supplemented with arabinose. Even without MobA, the cell is able to produce the small molecule required to repress gene expression controlled by the *moaA* 149 RNA. These results are consistent with Moco being the small molecule responsible for Moco RNA-dependent gene regulation.

### vii. Evidence that Moco RNAs can distinguish between Moco and Tuco

Riboswitches are known for their abilities to differentiate among closely related intermediates within a biosynthetic pathway. Species of Clostridia and several thermophilic archaean species incorporate tungsten rather than molybdenum into molybdopterin, resulting in the formation of the closely-related compound Tuco (Johnson *et al.,* 1993). The atomic and ionic radii of Mo and W are virtually identical, as are their electron affinities (Kletzin and Adams, 1996). Importantly for the current study, it has been shown (Buc *et al.*, 1999) that *E. coli* is capable of incorporating W into a final W-bis-MGD cofactor and then inserting the cofactor into a functional trimethylamine *N-*oxide reductase.

In a previous study (Anderson *et al.,* 2000), the apparent repression of the *moaABCDE* operon by the 131-nt upstream region occurs in the presence of Moco, but it is derepressed in the presence of Tuco. This previous observation is consistent with the data (Fig. 25c), which demonstrates that cells carrying Moco RNA-reporter fusion constructs are not affected by the addition of tungstate to the medium. Thus, Moco RNAs can to distinguish between these two nearly identical cofactors.

On inspection of the phylogenetic distribution and sequence alignment of Moco RNA representatives, it became apparent that there were two major classes of the motif. Of the 176 examples of the Moco RNA motif, 58 are lacking a P3 stem. However, the groupings do not break down strictly along phylogenetic divisions, which is consistent with the propagation of structural variants of a riboswitch aptamer that recognize the same ligand.

Interestingly, the two structural types of Moco RNAs exhibit near perfect correspondence with the utilization of Moco and Tuco amongst bacteria (Table 3), showing that the two RNA types allow the selective recognition of either Moco or Tuco. Organisms that use Moco or Tuco can be tentatively identified by the presence of genes encoding proteins that are predicted to use or transport compounds related to these cofactors. Moco RNAs that include the P3 stem are exclusively associated with genes that encode proteins that synthesize molybdopterin or Moco, and are also associated only with genes encoding enzymes that are known or predicted to use Moco as a coenzyme. In contrast, 17 of the 19 organisms that carry Moco RNA representatives lacking the P3 stem also carry genes that are indicative of Tuco metabolism. Furthermore, the P3-lacking RNAs are almost exclusively associated with genes encoding enzymes associated with molybdopterin biosynthesis (molybdopterin is a precursor to both Tuco and Moco biosynthesis) or with genes encoding enzymes that are known or predicted to use Tuco as a coenzyme.

An apparent exception to the segregation of Moco RNA types with Moco and Tuco metabolism is evident for *Pelobacter carbinolicus*, which has a P3-lacking RNA associated with a predicted molybdate transporter (Table 3, annotation *a*). However, a member of this transporter class is not able to discriminate against tungstate, and therefore the transporter in this organism can actually be important for Tuco biosynthesis. Even in organisms that carry both structural variations of Moco RNAs, the segregation of the P3-inclusive and P3-lacking RNAs to Moco and Tuco metabolic processes, respectively, is largely is maintained.

The striking segregation of the two types of Moco RNAs can be explained by the existence of two Moco RNA types, differing by the presence or absence of the P3 stem, that selectively sense Moco or Tuco, respectively. However, the P3 stem has been deleted from the *E. coli* Moco RNA, but this change completely disrupted gene regulation under all growth conditions tested. Presumably, this deletion alone is insufficient to switch specificity of Moco RNAs from Moco to Tuco.

As noted above, a triple arrangement of RNA elements is present in an apparent seven-gene operon in *S. wolfei.* The genes in this operon are predicted to encode proteins involved in molybdopterin synthesis, molybdate or tungstate transport, and other genes of unknown function. All three RNAs in this series lack P3 stems. In addition, putative intrinsic transcription terminators were observed following the first two RNA motifs, and a long gap between the third motif and the first ORF in the operon. If each riboswitch indeed functions via a separate expression platform, and if the absence of a P3 stem is indicative of Tuco binding, then the triple arrangement can allow cells to be exceptionally responsive to small changes in Tuco concentration, with greater 'digital' gene control character compared to tandem riboswitches describe previously (Welz and Breaker, 2007).

### 2. Conclusions

In *E. coli*, transcription of the *moaABCDE* operon is upregulated by both ModE and FNR. However, translation of this operon is prevented when the Moco RNA and Moco are present. ModE and FNR regulation ensure that the *moaABCDE* transcript is only created when conditions needing (anaerobic growth conditions sensed by FNR) and enabling (sufficient levels of molybdate sensed by ModE) Moco production are present in the cell. If the Moco RNA indeed is a riboswitch, it adds an additional layer of regulation. A Moco-sensing riboswitch would ensure that if there are sufficient levels of Moco already present, as indicated by free Moco binding to the *moaABCDE* transcript, then the Moco biosynthetic proteins are not made. This system allows for a rapid yet efficient response to the changing demand for Moco within the cell.

The experimental and bioinformatics data presented herein shows that RNAs conforming to the Moco RNA motif can serve as Moco- or Tuco-sensing riboswitch aptamers. The RNAs have genomic distributions that are consistent with riboswitch function, and the 149 *moaA* RNA exhibits gene control functions that are dependent on its secondary structure. However, the instability of Moco and its metabolic intermediates precludes us from demonstrating direct binding of these coenzymes to the RNA with commonly used techniques such as in-line probing, equilibrium dialysis or fluorescence-based assays. These latter experiments are typically used to prove that ligand binding occurs in the complete absence of protein factors.

Several lines of evidence show that a protein factor is not involved in metabolite sensing and that the RNA is serving as a direct sensor. Previous studies have identified protein factors responding to oxygen and molybdate that regulate Moco biosynthetic genes in *E. coli* (Anderson *et al.,* 2000; Spiro and Guest, 1990; Gruden *et al.*, 1999). However, no factor has been identified for the regulatory region located immediately upstream of the *moaA* gene in this organism. Moreover, the Moco RNA representatives can be sorted into two distinct architectures (with and without P3), and the distributions of these motif types correlate with an organism's use of Moco, Tuco, or both (Table 3). Such assortment of these structurally distinct RNAs is expected if it is the RNAs that serve as the direct sensors of Moco and Tuco.

Moco RNAs also share several important characteristics with other riboswitch representatives. For example, there are four other classes of riboswitch aptamers known to exist in *E. coli* that respond to coenzyme B₁₂ (Nahvi *et al.,* 2004), thiamine pyrophosphate (Winkler *et al.*, 2002a), flavin mononucleotide (Winkler *et al.*, 2002b) and lysine (Sudarsan *et al.,* 2003). As with Moco RNAs (Weinberg *et al.,* 2007), these riboswitches are all large compared to non-riboswitch gene control elements composed of RNA (Fig. 27), and they all have high levels of nucleotide sequence and structural conservation (Barrick and Breaker, 2007). Moco RNAs and the known riboswitch aptamers from *E. coli* are larger than 100 nucleotides, and carry four to six stems that exhibit evidence of nucleotide covariation that maintains base-pairing even in representatives from distantly related organisms.

The known metabolic regulatory proteins from E. *coli* that bind RNA, such as CspA (Phadtare and Inouye, 1999), BglG (Houman *et al.*, 1990) and others (Liu *et al.*, 1997; Torres-Larios *et al.,* 2002; Vecerek *et al.,* 2005; Richardson and Richardson, 1996; Tang and Guest, 1999), recognize far smaller stretches of RNA. If there is any secondary structure present in these protein-binding sequences, then it generally consists of a single hairpin. One exception to this trend is the ThrRS RNA element (Torres-Larios *et al.,* 2002), which has evolved a structure to favor binding by an aminoacyl-tRNA synthetase that normally binds a large structured tRNA. In contrast to most protein-binding RNA motifs, the Moco RNA from the *E. coli moaA* RNA spans 138 nucleotides and forms at least five conserved stem-loop elements. Thus, the large and extensively conserved structures of Moco RNAs are more characteristic of the known riboswitch RNAs from *E. coli* than of most RNA gene control elements that are bound by protein factors. These characteristics show that Moco RNAs are members of a new-found class of metabolite-sensing riboswitches.

### 3. Experimental procedures

### i. Oligonucleotides and chemicals

Synthetic DNAs were synthesized by the Howard Hughes Medical Institute Keck Foundation Biotechnology Resource Center at Yale University. The following chemicals were purchased from Sigma-Aldrich: carbenicillin, chloramphenicol, kanamycin, L-arabinose, D-glucose, glycerol, sodium molybdate and sodium tungstate.

### ii. Bacterial strains and media

The *E. coli* strain DJ480 (MG1655 Δ*lacX74*) (Cabrera and Jin, 2001) was obtained from D.J. Jin (National Institutes of Health) and NEB5α cells were obtained from New England Biolabs. Plasmids corresponding to the Wanner gene disruption set (pKD46, pKD13 and pCP20) (Datsenko and Wanner, 2000) were obtained from the *E. coli* Genetic Stock Center, Yale University. Media supplements were added as noted for each experiment: carbenicillin, 50 µg mL⁻¹; chloramphenicol, 5 µg mL⁻¹; kanamycin 50 µg mL⁻¹; L-arabinose, 0.2% (w/v); D-glucose, 0.2% (w/v). Glycerol, 0.2% (w/v), is added to the medium when arabinose supplementation is applied (Guzman *et al.*, 1995).

### iii. Preparation of Moco RNA

The 138 *moaA* RNA construct used for in-line probing was prepared by *in vitro* transcription using a template generated by successive PCR amplification reactions. The intergenic region upstream of *moaA* was amplified by PCR from *E. coli* K12 genomic DNA using the primers 5'- GAATTCCCTGGAGTCAGATTATCCGC (SEQ ID NO:4) and 5'- CTGGATCCAGTTGTGAAGCCATGTACAC (SEQ ID NO:5). Following digestion by EcoRI and BamHI, the PCR product was cloned into pRS414 and the integrity of the resulting plasmid was confirmed by DNA sequencing. This plasmid was used as the template for PCR amplification of the DNA construct encoding the 138 *moaA* construct (including the GG addition corresponding to the 5' terminus of the RNA transcript) where the primer for the non-template strand included the promoter sequence for T7 RNA polymerase. RNA molecules were prepared by transcription *in vitro* using T7 RNA polymerase and purified using denaturing PAGE as described previously (Roth *et al.*, 2006).

### iv. In-line probing analysis of 138 moaA RNA

40 pmoles of RNA prepared by *in vitro* transcription was dephosphorylated with alkaline phosphatase (Roche Diagnostics) and 20 pmoles was radiolabeled with γ-³²P [ATP] using T4 polynucleotide kinase (New England Biolabs) following protocols supplied by the manufacturers. In-line probing reactions using radiolabeled RNAs purified by PAGE were assembled as previously described (Mandal *et al.*, 2003) and were incubated for 40 hr at 25°C in a 10 µL volume containing 50 mM Tris-HCl (pH 8.3 at 23°C), 100 mM KCl, 20 mM MgCl₂, and ~5 nM precursor RNA. The resulting RNA fragments were separated by using 10% denaturing PAGE, and were imaged using a Molecular Dynamics PhosphorImager and quantified using ImageQuaNT software.

### v. Generation of moaA, mogA, modC, moeA and mobA knockouts

*E. coli* DJ480 strains with specific gene deletions were generated using the λ-red recombination method and appropriate PCR products as described elsewhere (Datsenko and Wanner, 2000). In brief, mutants were made by PCR amplification using plasmid pKD 13 and the following primers:
*moaA,* 5'- AGGAAGAAATGACTTCGCCTCCCGTATCTGGAAAGGTG TACATGGCTATTCCGGGGATCCGTCGACC (SEQ ID NO:6) and
5'-ACCTGACTCATCTGATCTCTCCTTTTGACGTTTTA GCCGCCAATGTACGATGTAGGCTGGAGCTGCTTCG; *mogA (SEQ ID NO: 7),*
5'-TGTATCATTCTGTTTAACGAGACTGTTTAAACGGAAA AATCTTGATGAATATTCCGGGGATCCGTCGACC (SEQ ID NO:8) and
5'- TTGAGATCCCCCCGCTCGGGGGGATTTTTTTATTCGC TAACGTCGCGTCTTGTAGGCTGGAGCTGCTTCG; *moeA (SEQ ID NO:9)*,
5'-GACATAATAGGCAAATTCGATTTTGCCTCCGCAGGA GTGTTTTCATGGAAATTCCGGGGATCCGTCGACC *(SEQ ID NO: 10)*and
5'-CAGCATCTCCTGATCGCTGAGTTCCGCCATTACAGG CCTCCGAACAACGCTGTAGGCTGGAGCTGCTTCG *(SEQ ID NO: 11)*;
*modC,* 5'-GAATGGCTGGCCAGAATCAGCCGTGAACGGGCGGG GCGCTAATCATGCTGATTCCGGGGATCCGTCGACC *(SEQ ID NO: 12)* and
5'-TTGCCCAGTTCATTTATAGCCACCTGATTAATCAG GCGGTTATCGACACTGTAGGCTGGAGCTGCTTCG *(SEQ ID NO: 13)*;
*mobA*, 5'-GACACGTTAGCAGGGTCAATCCCACAATAAAAGAG GCGATATCGGTGAATATTCCGGGGATCCGTCGACC *(SEQ ID NO: 14)* and
5'- ACTCCACGCGGCAAAGGCGAGTAACGGTATCATC GTTTTTCCTGCCATCGTGTAGGCTGGAGCTGCTTCG *(SEQ ID NO:15).*

The resulting ~1.4 kb long PCR products, containing a kanamycin resistance cassette flanked by FLP recognition target sites and identical 50-nucleotide sequence identities to adjacent chromosomal sequences, were purified and subjected to DpnI digestion. DJ480 cells were transformed with the Red helper plasmid, pKD46, encoding the Red recombinase from phage λ that promotes homologous recombination. Transformants were selected for ampicillin resistance. DJ480/pKD46 cells grown with L-arabinose were transformed with the gene-specific/pKD13 PCR product. Recombination of the PCR products into the chromosome of DJ480 was determined by selection for kanamycin-resistant transformants. All resulting deletion mutants were verified by PCR with both internal primers specific for the kanamycin cassette
(k1 5'-CAGTCATAGCCGAATAGCCT, SEQ ID NO:16 and k2 5'-CGGTGCCCTGAATGAACTGC, SEQ ID NO:17) and flanking gene-specific primers *(moaA* 5'-CGCTAGTATCGGCATAACCAC, SEQ ID NO:18 and 5'-GAATCGCGCAGATAGTGACCG, SEQ ID NO:19, *mogA* 5'-GCTACCTCTTCTGAAGCCTGTCTGT, SEQ ID NO:20 and 5'-ATGGGTGAAGTACTGAACGAGCAGT, SEQ ID NO:21, *moeA* 5'-GATGGATATGGCATGTAAAGGCAGG, SEQ ID NO: 22 and 5'-AGCACGCGAGAATCTTTCAGCGCCT, SEQ ID NO: 23, *modC* 5'-GAGCGGCGAGACTGTGCATTA SEQ ID NO:24 and 5'-GCAACGCCGATGACGCGGTA SEQ ID NO: 25, and *mobA* 5'-CTTCATTCAGACGTTTACATTTCATAG SEQ ID NO: 26 and 5'-CATCCATATCATGGTGCGTATGCTTA, SEQ ID NO: 27).

The kanamycin cassette was then eliminated by site-specific recombination by using the FLP plasmid pCP20. The resulting kanamycin sensitive transformants were verified by PCR with the same primers used for the pre-FLP verification.

### vi. Construction of β-galactosidase translational fusion and mutants

Preparation of a Moco RNA-reporter fusion construct driven by the constitutively active *lac*UV5 promoter lacking the operator binding site was achieved as follows. The nucleotide sequence from 1 to 145 relative to the S 1 transcription start site for the *E. coli moaABCDE* operon (Fig. 23a) was amplified by PCR from *E. coli* strain K12 using the primer 5'-GAATTCCTCATTAGGCACCCCAGGCTTTACACTTTATGCT TCCGGCTCGTATAATGTGTGGAACCACTAAACACTCTAGCCTC (SEQ ID NO: 28), containing the 56 nt *lac*UV5 promoter sequence (underlined) (Dickson *et al.,* 1975), and the primer 5'-CTGGATCCAGTTGTGAAGCCATGTACAC (SEQ ID NO: 29). Following digestion with *Eco*RI and *Bam*HI, the amplification product was cloned into pRS414 plasmid (Simons *et al.*, 1987) which contains a promoterless copy of *lacZ*, resulting in the in-frame fusion between the ninth codon of *lacZ* and the fifth codon of *moaA.* NEB-5α cells were transformed with the resulting plasmid. Transformants were selected for ampicillin resistance and the integrity of the *lac*UV5-Moco motif-*lacZ* translational fusion was confirmed by DNA sequencing. All site-directed mutations were introduced into the Moco riboswitch using the QuikChange site-directed mutagenesis kit (Stratagene) and the appropriate mutagenic DNA primers. All mutations were confirmed by DNA sequencing.

### vii. Construction of MoaABCDE pBAD33 over-expression plasmid

The *moaABCDE* operon was cloned into the pBAD33 plasmid (Guzman *et al.,* 1995) to enable arabinose-inducible control of Moco levels within the cell. A 2.7 kb KpnI-HindIII *moaABCDE* fragment was amplified by PCR from *E. coli* K12 using primers 5'-CTAGGTACCTCTGGAAAGGTGTACATGGCTTCACAAC (SEQ ID NO: 30) and 5'-TAGAAGCTTAACTACCAGCGTTTTGCCGCCTGCTG (SEQ ID NO: 31). The PCR product was purified by agarose gel electrophoresis and subjected to KpnI and HindIII digestion, as was pBAD33. The digested fragment was then ligated into pBAD33 downstream of the arabinose-inducible promoter and transformed into NEB-5α cells. Transformants were selected for chloramphenicol resistance and the fusion was confirmed by DNA sequencing.

### viii. Generation of mutant strains

The *moaA*, *mogA*, *modC*, *moeA* and *mobA* knockout DJ480 strains were transformed simultaneously with both the *lac*UV5-Moco motif-*lacZ* fusion pRS414 plasmid containing wild-type or mutant 149 moaA-reporter fusions and the *moaABCDE* pBAD33 over-expression plasmid. Transformants were selected for both ampicillin and chloramphenicol resistance.

### ix. In vivo analysis of gene control by the 149 moaA RNA from E. coli

To measure gene expression from *lacZ* in the wild-type and mutant reporter strains of *E. coli*, the cells were grown in a 14 mL culture vial with shaking overnight at 37°C in 3 mL of LB with supplementation as indicated. The cells were then diluted to an OD₆₀₀ of 0.05 in 150 µL of LB in 96-well plates and grown to an A₆₀₀ between 0.5 and 0.7 with shaking at 37°C, and then harvested for gene expression experiments. β-galactosidase activity was assayed using a 96-well microplate protocol described elsewhere (Blount *et al.*, 2007).

### x. Bioinformatics analysis of Moco and Tuco metabolism

The assignment of Moco and Tuco metabolism to various organisms and the assignment of Moco RNA motif types to genes involved in three processes (molybdopterin biosynthesis, Moco biosynthesis or use, and Tuco biosynthesis or use; Table 3) were made by sequence homology analysis. To identify organisms that use Tuco, but that have not been experimentally demonstrated to do so, BLAST was used to search for genes homologous to *tupA* and *tupB.* These genes have been shown in *Eubacterium acidaminophilum* to encode transporters specific for tungstate (Makdessi *et al.*, 2001). Tuco metabolism in *Pelobacter carbinolicus* and *Desulfotalea psychrophilia* were assigned based on the presence of *wtpA* homologs, which have been shown to be distinct but selective transporters of tungstate (Bevers *et al.*, 2006). Assignments of Moco RNA motifs to the three processes listed above were made by examining the annotated functions of protein products of adjoining genes, or by assignment of functions using sequence homology if annotation was not available.

Table 3 Correlation between the presence or absence of P3 and the presence of Moco- or Tuco-related genes. Organisms carrying Moco related genes were identified by searching the genomes of each organism for genes homologous to known Moco-dependent proteins and proteins involved in molybdate transport. Organisms carrying Tuco-related genes were identified as described for Moco, and as published elsewhere *(Bevers et al.,* 2006; Iyer *et al.*, 2006). Since genes important for the biosynthesis of molybdopterin (moly) could be involved in either Moco or Tuco production, the presence of these genes was indicated in a separate column. The absence of an asterisk does not mean that the organism is lacking genes related to the compound designated, but rather indicates that we did not find evidence for the presence of these genes in either or literature or genomic database searches. Genomic searches for genes relied on annotated genomes from the NCBI Entrez Genome Project database (http://www.ncbi.nlm.nih.gov/genomes/lproks.cgi).

Notes: The shaded boxes *a* through c identify the only instances where the correlation between RNA types and Moco or Tuco gene control appears to be violated. However, *a* designates a case where a P3-lacking RNA is associated with *modA*, whose protein product is a transporter that is unable to distinguish between molybdate and tungstate (Chan *et al.,* 1995). For *b*, a P3-lacking RNA is associated with a two-gene operon carrying the Tuco-associated gene COG2414 (encoding a tungsten-dependent aldehyde ferredoxin reductase) (Harborne *et al.,* 1992) and *nirB* (encoding a nitrate reductase and that usually requires Moco) (Clarke *et al.*, 2000). In *c*, P3-lacking RNAs are associated with two genes that encode putative Moco-utilizing sulfite oxidase enzymes. Thus, *a* and *b* do not violate the correlation, while other possibilities would need to be invoked to explain the observations in c (see text). Also in *S. wolfei*, one P3-inclusive RNA is associated with *troA*, which is predicted to encode a component of a metal transport complex. A similar arrangement is present in *Desulfitobacterium hafniense* DCB-2. However, the structural organization of TroA and a nitrogenase MoFe protein (which is a Moco-containing protein) are similar (Chan *et al.*, 1995). Therefore, it appears that the P3-inclusive RNA is associated with a gene for MoFe nitrogenase.

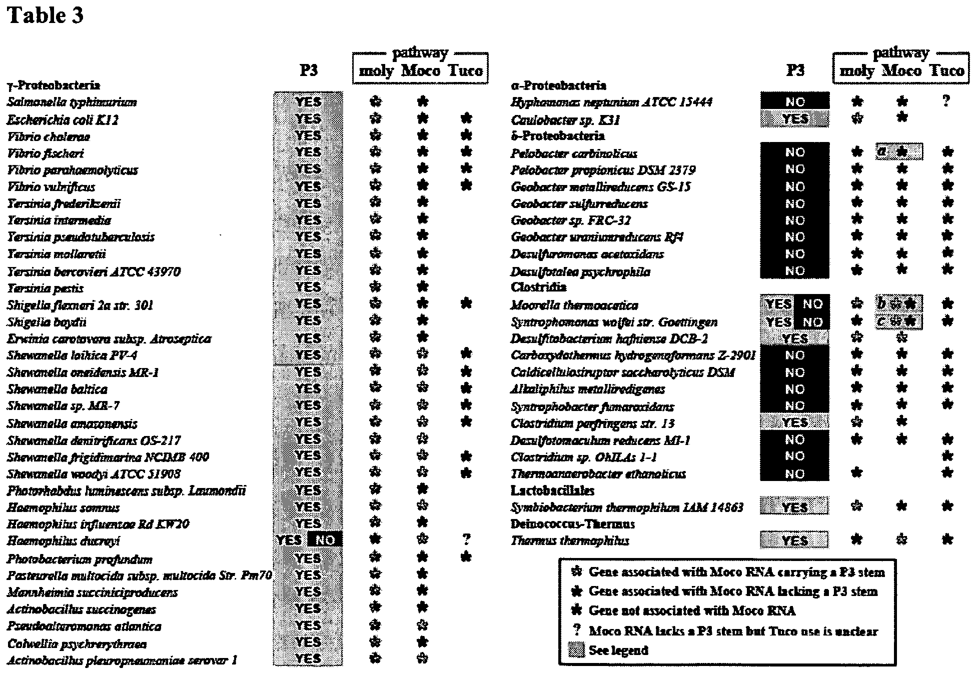

### F. Example 6: The aptamer core of SAM-IV riboswitches mimics the ligand-binding site of SAM-I riboswitches

A novel family of riboswitches, called "SAM-IV", is the fourth distinct set of mRNA elements to be reported that regulate gene expression via direct sensing of S-adenosylmethionine (SAM or AdoMet). SAM-IV riboswitches share conserved nucleotide positions with the previously described SAM-I riboswitches, despite rearranged structures and nucleotide positions with family-specific nucleotide identities. Sequence analysis and molecular recognition experiments suggest that SAM-I and SAM-IV riboswitches share similar ligand binding sites, but have different scaffolds. These findings show that RNA has considerable structural versatility, and reveal that riboswitches exploit this potential to expand the scope of RNA in genetic regulation.

Riboswitches are mRNA elements that specifically bind a particular metabolite, and regulate gene expression in response to changing metabolite concentrations (Mandal and Breaker 2004; Winkler and Breaker 2005; Batey 2006; Coppins et al. 2007). S-adenosylmethionine, a cofactor for many methylase enzymes, is recognized by three previously published riboswitch classes: the SAM-I (Winkler et al. 2003; McDaniel et al. 2003; Epshtein et al. 2003), SAM-II (Corbino et al. 2005) and SAM-III (or S_{MK}) (Fuchs et al. 2006) riboswitches. While all three riboswitch families specifically recognize SAM, they have no apparent similarity in sequence or structure.

The discovery of multiple distinct SAM-binding riboswitches, like for example the discovery of multiple self-cleaving ribozymes, supports the view that RNA has sufficient structural sophistication to solve the same biochemical challenges in diverse ways. Furthermore, structural studies and sequence analyses of two classes of large ribozymes, group I introns and RNase P RNAs, show that distinct scaffolds in these RNAs support the same positioning of nucleotides in the catalytic core (Michel and Westhof 1990; Krasilnikov et al. 2004; Torres-Larios et al. 2006; Vicens and Cech 2006).

While searching for new riboswitches in bacteria using bioinformatics (Yao et al. 2007; Weinberg et al. 2007), a conserved, structured RNA motif that was classified as a novel set of SAM-binding riboswitches was identified. These "SAM-IV" riboswitches are predominantly found in Actinomycetales, e.g., *Mycobacterium tuberculosis* (the causative agent of tuberculosis). SAM-IV riboswitches have similarities to the ligand-binding core of SAM-I riboswitches, but have numerous differences elsewhere in architecture and nucleotide identities. Thus, SAM-I and SAM-IV riboswitches can have distinct scaffolds but highly similar ligand-binding cores. The findings with SAM-IV riboswitches show that structural mimicry is not restricted to large ribozymes, in turn suggesting that it is widespread among diverse kinds of structured RNAs.

### 1. RESULTS AND DISCUSSION

### i. Identification of the SAM-IV motif

The SAM-IV motif was found using the CMfinder comparative genomics pipeline (Weinberg et al. 2007; Yao et al. 2007). Most SAM-IV instances are positioned upstream of genes involved in sulfur metabolism, and presumably in their 5' UTRs, which shows that SAM-IV corresponds to a SAM-binding riboswitch. Almost all known occurrences of SAM-IV RNAs are in the order Actinomycetales.

### ii. The SAM-IV motif core resembles the core of SAM-I

Highly conserved regions of SAM-IV with those of SAM-I, SAM-II and SAM-III riboswitches were compared. SAM-IV shares key sequence and structural features of the SAM-I binding core, but significant differences in nucleotide identities are found elsewhere, and there are considerable deviations in overall architecture (Fig. 28). The core as all nucleotides in the SAM-I 3-D structure (Montange and Batey 2006) having an atom within 5Å of a ligand atom (Fig. 28, bold letters) was identified, and mapped SAM-I positions onto analogous SAM-IV positions based on apparent similarities in conserved structural features. Nucleotide identities were classified as common to both motifs (Fig. 28, shading) when surrounding conserved nucleotide identities or other features show that the common identity is unlikely to arise by chance.

Both motifs have a multi-stem junction, and extensive similarity in nucleotide identity in the P3 stem (Fig. 28). The junctions between P 1 and P2 also share similarity, and both motifs appear to form a pseudoknot between the terminal loop of P2 and the junction 3' of P3. To accommodate this pseudoknot, P2 in SAM-I has a canonical kink-turn (Lescoute et al. 2005; Montange and Batey 2006). Interestingly, the internal loop in P2 in SAM-IV can function analogously to the kink turn, allowing these RNAs to adopt a pseudoknot structure.

However, the two motifs have distinct architectural elements and distinct patterns of nucleotide conservation in many places (Fig. 28, light shading). The P4 hairpin in the SAM-I core is absent in SAM-IV, but a different P4 hairpin is found outside of P1 in SAM-IV. These P4 hairpins have dissimilar conserved nucleotide identities. In addition to the added P4 outside the core, SAM-IV can form an additional pseudoknot that SAM-I lacks. Also, SAM-IV has a considerably different P2 from SAM-I, in that its loops and stem lengths are different from that of SAM-I, and there appears to be little similarity in sequence. Indeed, the kink-turn in P2 is highly conserved in SAM-I riboswitches (Barrick and Breaker 2007), but no SAM-IV riboswitch sequences have this structural motif. Other differences in conserved nucleotide identities between SAM-I and SAM-IV are in the tip of P3, base of P1 and junction 3' to P3.

Because of these differences, SAM-IV riboswitches have escaped detection by homology searches based on SAM-I in at least three previous studies. In the first study, Actinobacterial genomes were searched for regulatory RNAs including SAM-I aptamers using the PAT program (Seliverstov et al. 2005). Second, Gram-positive bacteria were searched for SAM-I aptamers and other gene control elements in sulfur metabolism using the RNA-PATTERN program (Rodionov et al. 2004). Third, searches for homologs of known riboswitches in all bacteria have used SequenceSniffer, and RAVENNA (Weinberg and Ruzzo 2006), which exploits statistical profile techniques to model probabilistically a conserved sequence and secondary structure. All efforts failed to detect SAM-IV riboswitches, although many actinomycetes carry both SAM-I and SAM-IV riboswitches at distinct genome coordinates.

SAM-I and SAM-IV similarities are primarily restricted to their cores, and therefore SAM-I and SAM-IV riboswitches can share similar binding sites and molecular recognition characteristics. This is supported by the SAM-I atomic-resolution model (Montange and Batey 2006), in which six nucleotides were proposed to directly interact with the ligand (Fig. 28). The nucleotide identity at these six positions is strictly conserved in all SAM-I representatives but one. Of the six positions in SAM-IV proposed to correspond to these ligand-contacting positions, five are conserved in all known SAM-IV representatives with the same nucleotide identity as in SAM-I riboswitches. The sixth, U88, is typically a cytosine in SAM-IV, but all four nucleobases are observed at this position.

### iii. Molecular recognition characteristics of SAM-IV RNAs

To assess molecular recognition of SAM-IV RNAs, in-line probing assays (Soukup and Breaker 1999) were applied to a 132-nucleotide RNA (termed 132 *Sc* RNA) comprising the SAM-IV found in *Streptomyces coelicolor.* In these assays, 132 *Sc* RNA binds SAM with an apparent dissociation constant (*K*_{D}) of ~150 nM, versus 20 µM for S-adenosylhomocysteine (SAH). Adenosine and methionine have *K*_{D} values poorer than 1 mM (Fig. 29). These values demonstrate that the 132 Sc RNA aptamer binds SAM with similar affinity and specificity as other classes of SAM riboswitch aptamers.

SAM-IV riboswitches do not conserve the nucleotide corresponding to U88 in SAM-I (Fig. 28). Because the 02 carbonyl oxygen at this position in SAM-I riboswitches is expected to sense the positive charge at the sulfur in the SAM ligand (Montange and Batey 2006), the affinities of SAM-I and SAM-IV riboswitches for compounds that differ at the sulfur position were compared: SAM, SAH, SAH sulfone (Borchardt and Wu 1974) and two aza derivatives, AzaAdoMet and MeAzaAdoMet (Thompson et al. 1999). 132 Sc RNA has a cytosine in place of uracil as the nucleobase in the position analogous to U88. Although cytosine preserves the 02 carbonyl, the additional hydrogen bond formed by a Watson-Crick G-C pair might disturb the interaction with the positive charge of the ligand, which could explain the perfect conservation of U88 in SAM-I riboswitches.

Molecular recognition of 132 Sc RNA was compared to that of a previously studied SAM-I riboswitch called 124 *yitJ* (Winkler et al. 2003). To measure discrimination by a riboswitch against a modification in the ligand, the ratio of the K_{D} for the modified ligand divided by the *K*_{D} for SAM. SAM-I and SAM-IV riboswitches bind SAM and SAM derivatives to yield similar *K*_{D} ratios was measured (Table 4). These data show that SAM-IV molecular recognition is similar to that of SAM-I, despite the poor conservation of U88 in SAM-IV.

### iv. SAM-IV is a genetic regulatory element

To determine if SAM-IV regulates gene expression *in vivo*, the SAM-IV-containing intergenic region upstream of *S. coelicolor* locus SCO2146 was cloned as a translation fusion with the *xylE* reporter gene. Because a promoter could not be easily identified in the SAM-IV intergenic region, the region was placed under the control of a glycerol-inducible promoter (Kieser et al. 2000).

Most known SAM riboswitches lower gene expression upon binding SAM, thereby reducing expression of genes whose protein products are not needed when SAM levels are high. To test if SAM-IV is a gene regulation element, mutations expected to disrupt or restore function of the SAM-IV riboswitch were introduced. The first mutation (M1) modified positions expected to directly contact SAM, by assuming that these nucleotides are analogous to those in SAM-I aptamers. The second mutation (M2) disrupts base pairing in the conserved secondary structure. A third mutation (M3) restores base pairing lost in M2 via compensatory mutations, which can restore wild-type activity.

XylE activity was measured in *S. coelicolor* cells carrying each of the four riboswitch-reporter fusions after growth in complex media, in which cellular concentrations of SAM are expected to be ample. As anticipated, strains with M1 and M2 riboswitches showed higher reporter gene expression than those with wild-type or M3 riboswitches (Fig. 30). These results indicate that the SAM-IV RNA from *S. coelicolor* is a genetic control element, which molecular recognition experiments indicate should respond to SAM. Reporter gene expression was measured under conditions in which cellular SAM concentrations are depleted, but these results were inconclusive due to exceedingly low levels of reporter gene expression with the constructs used.

Gene regulation of sulfur metabolism is of particular interest in *Streptomyces*, as SAM levels affect antibiotic production and sporulation in these species (Kim et al. 2003; Okamoto et al. 2003). Some genes apparently regulated by SAM-IV (e.g. SCO2146) are predicted as selenocysteine lyases. They can have a role in sulfur metabolism, for example.

### v. Evolutionary history of SAM-IV

A model that can account for divergent evolution is herein given. In this model, a SAM-I-like ancestor loses its P4 stem, and any negative effect later caused by the loss of P4 was compensated by the gain of a different P4 stem 3' to the P1 stem, leading eventually to a SAM-IV-like RNA. Three facts are consistent with this model. First, SAM-IV is almost restricted to Actinomycetales, while SAM-I is found in this taxon and many others, suggesting that, if they share an ancestor, it would probably resemble SAM-I. Second, P4 was found to be only involved in scaffolding (Montange and Batey 2006), so it can be more easily replaced. Third, a few SAM-I riboswitches entirely lack the P4 stem and loop, so P4 loss can be tolerated.

### vi. Classification and significance of SAM-IV riboswitches

Proteins are classified into families and superfamilies by varying criteria (Orengo and Thornton 2005). The original criteria were based solely on sequence homology (Dayhoff et al. 1976). Other groups have continued this precedent (e.g. Barker et al. 1996), but some also consider structural or functional similarity (Murzin et al. 1995). Generally, proteins classified into families have clear homology, while proteins grouped into superfamilies appear to be more distantly related and homology is considered only "probable" (Murzin et al. 1995; Dayhoff et al. 1976). Given the incompatible patterns of conservation and rearranged structures between SAM-I and SAM-IV riboswitches, and since their similarity eludes state-of-the-art methods of sequence analysis, we propose that they comprise distinct families. However, the similarity of their cores is suggestive enough to classify them as a single superfamily. 3-D structural studies on SAM-IV riboswitches might help to refine their classification. Riboswitches have been formed into "classes" and "types". A class defines a set of riboswitches sharing structural and functional properties, while type is used to group RNAs that belong to the same class, yet carry distinct sub-structures. To relate this terminology with that used to group homologous proteins, "class" corresponds to "family", and "type" corresponds to "subfamily".

Additional riboswitch families show structural diversity, although possibly of a different nature from the relationship between SAM-I and SAM-IV riboswitches. For example, preQ₁-I riboswitches can be partitioned into sub-families based on loop sequences (Roth et al. 2007). Also an adenosylcobalamin-binding riboswitch reported previously (Nahvi et al. 2004) lacks a normally conserved domain, while its 3' flanking sequence is essential for ligand recognition. This variant exhibited better affinity for purinylcobalamin than does a typical adenosylcobalamin riboswitch, so the binding core of the variant can be altered.

The results demonstrate that riboswitch RNAs can use diverse architectures to position key nucleotides into the same binding site. In view of similar findings for group I introns and RNase P RNAs, it is hereby concluded that this phenomenon is exhibited by a wide range of RNAs. This robust natural use of the structural repertoire of RNA supports the conclusion that RNA is a powerful and versatile polymer for molecular sensing and genetic control.

### 2. MATERIALS AND METHODS

### i. Bioinformatics

The SAM-IV motif was identified using the CMfinder comparative genomics pipeline, and its alignment inferred using previously established strategies (Weinberg et al. 2007; Yao et al. 2007), but version 21 of RefSeq (Pruitt et al. 2005) was used. SAM-I alignment data were published elsewhere (Barrick and Breaker 2007). Conservation statistics presented in Figure 28 were calculated as previously reported (Weinberg et al. 2007).

### ii. Microbial genetics and plasmids

To test riboswitch control of gene expression, we cloned the SAM-IV-containing intergenic region in *S. coelicolor* upstream of a *xylE* reporter gene (Kieser et al. 2000) encoding catechol 2,3-dioxygenase. This enzyme converts catechol to 2-hydroxymuconate semialdehyde, whose absorbance at 375 nm permits a colorimetric assay. We used pMT3226 (Kieser et al. 2000), an integrative *Streptoymces* plasmid that replicates in *Escherichia coli* and includes an apramycin resistance gene and a *xylE* reporter gene downstream of a glycerol-inducible promoter. To construct a translation fusion with the *xylE* gene, a second *Bam*HI site was added to pMT3226 by QuikChange (Stratagene) with primer 5'- GAAGAGGTGACGTCATGGATCCGAACAAAGGTGTA ATGC (SEQ ID NO: 32) and its reverse complement. The *S. coelicolor* intergenic region containing SAM-IV was amplified from genomic DNA by PCR with primers 5'-CTAGGATCCGCCACGCGTAGCGGCCCTGGTGTGT (SEQ ID NO: 33) and 5'-GTAGGATCCACAGCGGTGGGTACGGACATGGCG (*Bam*HI sites underlined, SEQ ID NO: 34). To aid amplification of the high-G+C DNA product, PCR reactions contained 1.3 M betaine (Sigma-Aldrich) and 5% DMSO (Frackman et al. 1998). To assemble "pEZ35", pMT3226 and the PCR product were cut with *Bam*HI, the small fragment from pMT3226 was removed by agarose gel purification, and the DNA molecules were ligated, with verification of correct sequence and orientation by sequencing. Vectors containing mutant riboswitches (M1, M2, M3) were made by QuikChange. Plasmids were introduced into *S. coelicolor* M145 by conjugation (Kieser et al. 2000). Spore stocks of exconjugants were harvested from cells grown on MS agar (Kieser et al. 2000) at 28°C, and stored at -20°C in 15% glycerol. Apramycin sulfate (Sigma-Aldrich) was used at 100 µg/mL for *E. coli* or 50 µg/mL for *S. coelicolor.*

To start genetics experiments, spore stocks were thawed on ice, washed in 1 mL of triton X-100 buffer (Hodgson 1982) and resuspended in 1 mL dH₂O. This mixture was inoculated into a 25 mL culture to A₄₅₀ ~ 0.03, measured relative to H₂O (Hodgson 1982). Media was 24 g/L Tryptic Soy Broth (TSB) without dextrose (Becton Dickinson), 4% glycerol, 20 µg/mL apramycin sulfate (except for cells lacking the vector) and 12.5 µg/mL sodium nalidixate (Sigma-Aldrich), to reduce the risk of contamination. Cultures were grown for 50.5 hours in 250 mL flasks with shaking at 28°C. Then, XylE activity was measured in cell-free extracts (Kieser et al. 2000). Since cell debris remaining in the crude extract add noise to XylE enzyme curves, the supernatant was extracted after centrifugation twice, and was then filtered through a 0.2 µm-pore membrane (Whatman), followed by a 20-minute centrifugation. Reaction included 1 mL catechol-free assay buffer, 100 µL cell-free extract, 10 µL 20 mM catechol, and absorbance at 375 nm was measured every 6 seconds for 20 minutes on a Varian 50 Bio spectrophotometer. Despite filtering, debris sometimes remained that absorbed at all visible wavelengths, including 375 nm. When the change at 480 nm (a wavelength not noticeably absorbed by the XylE reaction product) was more than 0.0004 over 20 minutes and comparable in magnitude to that of 375 nm, we discarded the result, and prepared a new reaction. XylE activity was estimated using the change in A₃₇₅ over the full 20 minutes, which can be more reproducible than the use of just the linear range, as reported previously (Kieser et al. 2000).

### iii. In-line probing assays

DNA corresponding to the aptamer in the pEZ35 plasmid (above) was amplified by PCR with primers 5'-TAATACGACTCACTATAGGTTTTTCGACAGGTCATGAGTGACAGTC (T7 RNA polymerase promoter sequence underlined, SEQ ID NO: 35) and 5'-AGGGGTCCGCGCTTGCCGTGGACCTTGCTG (SEQ ID NO: 36). 5' ³²P-labeled RNA molecules were prepared from PCR products by *in vitro* transcription, dephosphorylation and radiolabeling with [γ-³²P]ATP using protocols described previously (Roth et al. 2007). In-line probing reactions were prepared and 5' ³²P-labeled fragments were resolved by denaturing PAGE and imaged as described previously (Roth et al. 2007).

**Table 4.** Molecular recognition by SAM-I and SAM-IV. *K*_{D} values are listed for SAM derivatives, as is the ratio of the *K*_{D} for a given compound to that for SAM. SAM-I K_{D} values were calculated previously (Lim et al. 2006). Compound structures were depicted previously (Lim et al. 2006) using the following numbering scheme: SAM (compound **1**), SAH (**2**), SAH sulfone (**4**), AzaAdoMet (**5**), MeAzaAdoMet (**6**).

**Table 4**

| Compound | *K*_{D [compound]} (nM) | | *K*_{D [compound]} / *K*_{D [SAM]} | |
|---|---|---|---|---|
| | SAM-I | SAM-IV | SAM-I | SAM-IV |
| SAM | 5 | 150 | 1 | 1 |
| MeAzaAdoMet | 4 | 300 | 0.8 | 2 |
| SAH sulfone | 30 | 4,000 | 6 | 27 |
| AzaAdoMet | 200 | 8,000 | 40 | 53 |
| SAH | 400 | 20,000 | 80 | 133 |

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "an", and "the" include plural reference unless the context clearly dictates otherwise. Thus, for example, reference to "a riboswitch" includes a plurality of such riboswitches, reference to "the riboswitch" is a reference to one or more riboswitches and equivalents thereof known to those skilled in the art, and so forth.

"Optional" or "optionally" means that the subsequently described event, circumstance, or material may or may not occur or be present, and that the description includes instances where the event, circumstance, or material occurs or is present and instances where it does not occur or is not present.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described. Publications cited herein and the material for which they are cited are hereby specifically incorporated by reference. Nothing herein is to be construed as an admission that the present invention is not entitled to antedate such disclosure by virtue of prior invention. No admission is made that any reference constitutes prior art. The discussion of references states what their authors assert, and applicants reserve the right to challenge the accuracy and pertinency of the cited documents. It will be clearly understood that, although a number of publications are referred to herein, such reference does not constitute an admission that any of these documents forms part of the common general knowledge in the art.

Throughout the description and claims of this specification, the word "comprise" and variations of the word, such as "comprising" and "comprises," means "including but not limited to," and is not intended to exclude, for example, other additives, components, integers or steps.

Those skilled in the art will recognize, or be able to ascertain using no more than routine experimentation, many equivalents to the specific embodiments of the method and compositions described herein. Such equivalents are intended to be encompassed by the following claims.

### References

Abreu-Goodger, C., and Merino, E. 2005. RibEx: a web server for locating riboswitches and other conserved bacterial regulatory elements. Nucleic Acids Res. 33: W690-692.
Akakura, R., and Winans, S. C. (2002). Mutations in the occQ operator that decrease OccR-induced DNA bending do not cause constitutive promoter activity. Journal Of Biological Chemistry 277, 15773-15780.
Akimoto, H. et al. Queuine analogues. Their synthesis and inhibition of growth of mouse L5178Y cells in vitro. J. Med. Chem. 29, 1749-1753 (1986).
Akimoto, H., Imamiya, E., Hitaka, T., Nomura, H. & Nishimura, S. Synthesis of queuine, the base of naturally occurring hypermodified nucleoside (queuosine), and its analogues. J. Chem. Soc. Perkin Trans. 1, 1637-1644 (1988).
Altschul, S.F., Madden, T.L., Schaffer, A.A., Zhang, J., Zhang, Z., Miller, W. and Lipman, D.J. (1997) Gapped BLAST and PSI-BLAST: a new generation of protein database search programs. Nucleic Acids Research, 25, 3389-3402.
Anderson, L.A., McNairn, E., Lubke, T., Pau, R.N. & Boxer, D.H. ModE-dependent molybdate regulation of the molybdenum cofactor operon moa in Escherichia coli. J. Bacteriol. 182, 7035-7043 (2000).
Arsène-Ploetze, F., Nicoloff, H., and Bringel F. 2005. Lactobacillus plantarum ccl gene is non-essential, arginine-repressed and codes for a conserved protein in Firmicutes. Arc. Microbiol. 183: 307-316.
Ausubel, F.M., Brent, R., Kingston, R.E., Moore, D.D., Seidman, J.G., Smith, J.A., and Struhl, K. (1992). Short Protocols in Molecular Biology: A Compendium of Methods from Current Protocols in Molecular Biology (New York: John Wiley & Sons, Inc.).
Axmann, I.M., Kensche, P., Vogel, J., Kohl, S., Herzel, H. and Hess, W.R. (2005) Identification of cyanobacterial non-coding RNAs by comparative genome analysis. Genome Biology, 6, R73.
Bader, G., Gomez-Ortiz, M., Haussmann, C., Bacher, A., Huber, R. & Fischer, M. Structure of the molybdenum-cofactor biosynthesis protein MoaB of Escherichia coli. Acta Cryst. D60, 1068-1075 (2004).
Baker, K.P. & Boxer, D.H. Regulation of the chlA locus of Escherichia coli K12: involvement of molybdenum cofactor. Mol Microbiol. 5, 901-907 (1991).
Barker, W.C., Pfeiffer, F., and George, D.G. 1996. Superfamily classification in PIR-International Protein Sequence Database. Methods Enzymol. 266: 59-71.
Barrick, J. E., Corbino, K. A., Winkler, W. C., Nahvi, A., Mandal, M., Collins, J., Lee, M., Roth, A., Sudarsan, N., et al. 2004. New RNA motifs suggest an expanded scope for riboswitches in bacterial gene control. Proc. Natl. Acad. Sci. USA 101: 6421-6426.
Barrick, J.E. & Breaker, R.R. The distributions, mechanisms, and structures of metabolite-binding riboswitches. Genome Biol. 8, R239 (2007).
Bassler, B.L. and Losick, R. (2006) Bacterially Speaking. Cell, 125, 237-246.
Batey, R. T., Gilbert, S. D., and Montagne, R. K. 2004. Structure of a natural guanine-responsive riboswitch complexed with the metabolite hypoxanthine. Nature 432: 411-415.
Batey, R.T. (2006) Structures of regulatory elements in mRNAs. Curr Opin Struct Biol, 16, 299-306.
Baugh, P.E., Collison, D., Garner, C.D. & Joule, J.A. Molybdenum metalloenzymes. In: Comprehensive Biological Catalysis, M. Sinnott, Ed., Academic Press, Vol. III, pp. 377-400 (1998).
Bevers, L.E., Hagedoorn, P.L., Krijger, G.C. & Hagen, W.R. Tungsten transport protein A (WtpA) in Pyrococcus furiosus: the first member of a new class of tungstate and molybdate transporters. J. Bacteriol. 188, 6498-6505 (2006).
Beyhan, F. H. Yildiz, Mol. Microbiol. 63, 995 (2007).
Bienz, M. and Kubli, E. 2001. Wild-type tRNA TyrG reads the TMV RNA stop codon, but Q base-modified tRNA TyrQ does not. Nature 294: 188-190.
Blaise, M. et al. A minimalist glutamyl-tRNA synthetase dedicated to aminoacylation of the tRNAAsp QUC anticodon. Nucleic Acids Res. 32, 2768-2775 (2004).
Blount, K.F., Wang, J.X., Lim, J., Sudarsan, N. & Breaker, R.R. Antibacterial lysine analogs that target lysine riboswitches. Nat. Chem. Biol. 3, 44-49 (2007).
Bocobza, S., Adato, A., Mandel, T., Shapira, M., Nudler, E., and Aharoni, A. (2007). Riboswitch-dependent gene regulation and its evolution in the plant kingdom. Genes Dev. 21, 2874-2879.
Borchardt, R.T. and Wu, Y.S. 1974. Potential inhibitors of S-adenosylmethionine-dependent methyltransferases. 1. Modification of the amino acid portion of S-adenosylhomocysteine. J. Med. Chem. 17: 862-868.
Breaker, R.R. (2004). Natural and engineered nucleic acids as tools to explore biology. Nature 432, 838-845.
Breaker, R.R. (2006). Riboswitches and the RNA World. In: The RNA World. Gesteland, R.F., Cech, T.R., Atkins, J.F. eds. Cold Spring Harbor Laboratory Press, Cold Spring Harbor Laboratory, NY., pp. 89-107.
Breaker, Science 319, 1795 (2008).
Buc, J. et al. Enzymatic and physiological properties of the tungsten-substituted molybdenum TMAO reductase from Escherichia coli. Mol. Microbiol 32, 159-168 (1999).
Bunka, D.H., and Stockley, P.G. (2006). Aptamers come of age - at last. Nat. Rev. Microbiol. 4, 588-596.
Cabrera, J.E. & Jin, D.J. Growth phase and growth rate regulation of the rapA gene, encoding the RNA polymerase-associated protein RapA in Escherichia coli. J. Bacteriol. 183, 6126-6134 (2001).
Cameron, M. Volar, L. A. Bannister, R. J. Redfield, Nucleic Acids Res. 36, 10 (2008).
Chan et al., Proc. Natl. Acad. Sci. USA 101, 17084 (2004).
Chan, M.K., Mukund, S., Kletzin, A., Adams, M.W. & Rees, D.C. Structure of a hyperthermophilic tungstopterin enzyme, aldehyde ferredoxin oxidoreductase. Science 267, 1463-1469 (1995).
Cheah, M.T., Wachter, A., Sudarsan, N., and Breaker, R.R. (2007). Control of alternative RNA splicing and gene expression by eukaryotic riboswitches. Nature 447, 497-U497.
Clarke, T.E., Ku, S.-K., Dougan, D.R., Vogel, H.J. & Tari, L.W. The structure of the ferric siderophore binding protein FhuD complexed with gallichrome. Nat. Struct. Biol. 7, 287-291 (2000).
Claverie, J.M. (2005) Fewer genes, more noncoding RNA. Science, 309, 1529-1530.
Cochrane, J.C., Lipchock, S.V., and Strobel, S.A. (2007). Structural investigation of the GlmS ribozyme bound to its catalytic cofactor. Chem. Biol. 14, 97-105.
Coppins, R.L., Hall, K.B., and Groisman, E.A. 2007. The intricate world of riboswitches. Curr. Opin. Microbiol. 10: 176-181.
Corbino; K. A., Barrick, J. E., Lim, J. L., Welz, R., Tucker, B., Puskarz, I., Mandal, M., Rudnick, N. D., and Breaker, R. R. 2005. Evidence for a second class of S-adenosylmethiothine riboswitches and other regulatory RNA motifs in alpha-proteobacteria. Genome Biol. 6: R70.
Costa, F. Michel, EMBO J. 14, 1276 (1995).
Costa, M. & Michel, F. Frequent use of the same tertiary motif by self-folding RNAs. EMBO J. 14, 1276-1285 (1995).
Costa, M. and Michel, F. (1997) Rules for RNA recognition of GNRA tetraloops deduced by in vitro selection: comparison with in vivo evolution. EMBO J, 16, 3289-3302.
Coventry, A., Kleitman, D.J. and Berger, B. (2004) MSARI: Multiple sequence alignments for statistical detection of RNA secondary structure. Proceedings of the National Academy of Sciences of the Unites States of America, 101, 12102-12107.
Curtis, S.E. (1988) Structure, organization and expression of cyanobacterial ATP synthase genes. Photosynthesis Research, 18, 223-244.
Das, R., Laederach, A., Pearlman, S. M., Herschlag, D., and Altman, R. B. (2005). SAFA: Semi-automated footprinting analysis software for high-throughput quantification of nucleic acid footprinting experiments. RNA-a Publication of the RNA Society 11, 344-354.
Datsenko, K.A. & Wanner, B.L. One-step inactivation of chromosomal genes in Escherichia coli K-12 using PCR products. Proc. Natl. Acad Sci. USA 97, 6640-6645 (2000).
Dayhoff, M.O., Barker, W.C., and Hunt, L.T. 1976. Protein superfamilies. In Atlas of Protein Sequence and Structure, vol. 5, suppl. 2 (ed. M.O. Dayhoff). National Biomedical Research Foundation, Washington, DC.
Della Ragione, F., Porcelli, M., Carteni-Farina, M., Zappia, V., and Pegg, A.E. (1985). Escherichia coli S-adenosylhomocysteine/5'-methylthioadenosine nucleosidase. Purification, substrate specificity and mechanism of action. Biochem. J. 232, 335-341.
Dewey, V. & Kidder, G. W. Partial purification and properties of a nucleoside hydrolase from Crithidia. Arch. Biochem. Biophys. 157, 380-387 (1973).
Dickson, R.C., Abelson, J., Barnes, W.M. & Reznikoff, W.S. Genetic regulation: the Lac control region. Science 187, 27-35 (1975).
Dillon, P. J. and Rosen, C. A. 1990. A rapid method for the construction of synthetic genes using the polymerase chain-reaction. Biotechniques 9: 298-300.
Durand, J. M. et al. vacC, a virulence-associated chromosomal locus of Shigella flexneri, is homologous to tgt, a gene encoding tRNA-guanine transglycosylase (Tgt) of Escherichia coli K-12. J. Bacteriol. 176, 4627-4634 (1994).
Eddy, S. R. & Durbin, R. RNA sequence analysis using covariance models. Nucleic Acids Res. 22, 2079-2088 (1994).
Eddy, S. R. INFERNAL. Version 0.55. Distributed by the author. Dept. of Genetics, Washington University School of Medicine. St. Louis, Missouri. (2003).
Eddy, S.R. (2005) Infernal User's Guide.
Edwards, T.E., and Ferré-D'Amaré, A.R. (2006). Crystal structures of the thi-box riboswitch bound to thiamine pyrophosphate analogs reveal adaptive RNA-small molecule recognition. Structure 14, 1459-1468.
Egli et al., Proc. Natl. Acad. Sci. USA 87, 3235 (1990).
Epshtein, V., Mironov, A. S., and Nudler, E. 2003. The riboswitch-mediated control of sulfur metabolism in bacteria. Proc. Natl. Acad. Sci. USA 100: 5052-5056.
Famulok, M. RNAs turn on in tandem. Science 306, 233-234 (2004).
Finn, R. D., Mistry, J., Schuster-Böckler, B., Griffiths-Jones, S., Hollich, V., Lassmann, T., Moxon, S., Marshall, M., Khanna, A., Durbin, R., Eddy, S. et al. 2006. Pfam: clans, webtools and services. Nucleic Acids Res. 24: D247-251.
Frackman, S., Kobs, G., Simpson, D., and Storts, D. 1998. Betaine and DMSO: enhancing agents for PCR. Promega Notes 65: 27.
Frey, B., Janel, G., Michelsen, U. & Kersten, H. Mutations in the Escherichia coli fnr and tgt genes: control of molybdate reductase activity and the cytochrome d complex by fnr. J. Bacteriol. 171, 1524-1530 (1989).
Frey, B., McCloskey, J., Kersten, W. & Kersten, H. New function of vitamin B12: cobamide-dependent reduction of epoxyqueuosine to queuosine in tRNAs of Escherichia coli and Salmonella typhimurium. J. Bacteriol. 170, 2078-2082 (1988).
Fuchs, R. T., Grundy, F. J., and Henkin, T. M. 2006. The SMK box is a new SAM-binding RNA for translational regulation of SAM synthesis. Nat. Struct. Mol. Biol. 13: 226-233.
Galperin, A. N. Nikolskaya, E. V. Koonin, FEMS Microbiol. Lett. 203, 11 (2001).
Garcia Martin, N. Ivanova, V. Kunin, F. Warnecke, K. W. Barry, A. C. McHardy, C. Yeates, S. He, A. A. Salamov, E. Szeto, et al., Nat. Biotechnol. 24, 1263 (2006).
Gaur, R. and Varshney, U. 2005. Genetic analysis identifies a function for the queC (ybaX) gene product at an initial step in the queuosine biosynthetic pathway in Escherichia coli. J. Bacteriol. 187: 6893-6901.
Gebhardt, K., Shokraei, A., Babaie, E., and Lindqvist, B. H. (2000). RNA aptamers to S-adenosylhomocysteine: kinetic properties, divalent cation dependency, and comparison with anti-S-adenosylhomocysteine antibody. Biochemistry 39, 7255-7265.
Gerstein, M., Sonnhammer, E.L.L. and Chothia, C. (1994) Volume changes in protein evolution. Journal of Molecular Biology, 236, 1067-1078.
Gilbert, S. D., Stoddard, C. D., Wise, S. J. & Batey, R. T. Thermodynamic and kinetic characterization of ligand binding to the purine riboswitch aptamer domain. J. Mol. Biol. 359, 754-768 (2006).
Gilbert, S.D., Stoddard, C.D., Wise, S.J., and Batey, R.T. (2006) Thermodynamic and kinetic characterization of ligand binding to the purine riboswitch aptamer domain. J. Mol. Biol. 359, 754-768.
Gill, M. Pop, , R. T. Deboy, P. B. Eckburg, P. J. Turnbaugh, B. S. Samuel, J. I. Gordon, D. A. Relman, C. M. Fraser-Liggett, K. E. Nelson, Science 312, 1355 (2006).
Gold L., Polisky, B., Uhlenbeck, O., and Yarus, M. (1995) Diversity of oligonucleotide functions. Annu. Rev. Biochem. 64, 763-797.
Gottfried, P., Kolot, M. and Yagil, E. (2001) The effect of mutations in the Xis-binding sites on site-specific recombination in coliphage HK022. Mol Genet Genomics, 266, 584-590.
Govind, J. A. Fralick, R. D. Rolfe, J. Bacteriol. 188, 2568 (2006).
Great Britain patent number 981458 (1965).
Griffiths-Jones, S. RALEE-RNA ALignment Editor in Emacs. Bioinformatics 21, 257-259 (2005).
Griffiths-Jones, S., Moxon, S., Marshall, M., Khanna, A., Eddy, S.R. and Bateman, A. (2005) Rfam: annotating non-coding RNAs in complete genomes. Nucleic Acids Research, 33, 121-124.
Gruden, A.M., Self, W.T., Villain, M., Blalock, J.E. & Shanmugan, K.T. An analysis of the binding of repressor protein ModE to modABCD (molybdate transport) operator/promoter DNA of Escherichia coli. J. Biol. Chem. 274, 24308-24315 (1999).
Grunden, A.M. & Shanmugam, K.T. Molybdate transport and regulation in bacteria. Arch. Microbiol. 168, 345-354 (1997).
Guérout-Fleury, A. M., Frandsen, N. & Stragier, P. Plasmids for ectopic integration in Bacillus subtilis. Gene 180, 57-61 (1996).
Gündüz, U. & Katze, J. R. Queuine salvage in mammalian cells. Evidence that queuine is generated from queuosine 5'-phosphate. J. Biol. Chem. 259, 1110-1113 (1984).
Gündüz, U. & Katze, J. R. Salvage of the nucleic acid base queuine from queuine-containing tRNA by animal cells. Biochem. Biophys. Res. Commun. 109, 159-167 (1982).
Gusarov, I. & Nudler, E. The mechanism of intrinsic transcription termination. Mol. Cell 3, 495-504 (1999).
Guzman, L.M., Belin, D., Carson, M.J. & Beckwith, J. Tight regulation, modulation, and high-level expression by vectors containing the arabinose PBAD promoter. J. Bacteriol. 177, 4121-4130 (1995).
Harada F. and Nishimura, S. 1972. Possible anticodon sequences of tRNAHis, tRNAAsn, and tRNAAsp from Escherichia coli B. Universal presence of nucleoside Q in the first position of the anticodons of these transfer ribonucleic acids. Biochemistry 11: 301-308.
Harborne, N.R., Griffiths, L., Busby, S.J. & Cole, J.A. Transcriptional control, translation and function of the products of the five open reading frames of the Escherichia coli nir operon. Mol. Microbiol. 6, 2805-2813 (1992).
He, L. and Hannon, G.J. (2004) microRNAs: small RNAs with a big role in gene regulation. Nature Reviews. Genetics, 5, 522-531.
Hendrix, D.K., Brenner, S.E. and Holbrook, S.R. (2005) RNA structural motifs: building blocks of a modular biomolecule. Q Rev Biophys, 38, 221-243.
Henkin, T.M. and Yanofsky, C. (2002) Regulation by transcription attenuation in bacteria: how RNA provides instructions for transcription termination/antitermination decisions. Bioessays, 24, 700-707.
Hermes, M., Osswald, H., Mattar, J., and Kloor, D. (2004). Influence of an altered methylation potential on mRNA methylation and gene expression in HepG2 cells. Experimental Cell Research 294, 325-334.
Heus, H.A. & Pardi, A. Structural features that give rise to the unusual stability of RNA hairpins containing GNRA loops. Science 253, 191-194 (1991).
Hodgson, D.A. 1982. Glucose repression of carbon source uptake in Streptomyces coelicolor A3(2) and its perturbation in mutants resistant to 2-deoxyglucose. J. Gen. Microbiol. 128: 2417-2430.
Houman, F., Diaz-Torres, M.R. & Wright, A. Transcriptional antitermination in the bgl operon of E. coli is modulated by a specific RNA binding protein. Cell 62, 1153-1163 (1990).
Hurt, J. K., Olgen, S., and Garcia, G. A. 2007. Site-specific modification of Shigella flexneri virF mRNA by tRNA-guanine transglycosylase in vitro. Nucleic Acids Res. 35: 4905-4913.
Iobbi-Nivol, C., Palmer, T., Whitty, P.W., McNairn, E. & Boxer, D.H. The mob locus of Escherichia coli K12 required for molybdenum cofactor biosynthesis is expressed at very low levels. Microbiology 141, 1663-1671 (1995).
Itoh, Y., Watson, J.M., Haas, D., and Leisinger, T. (1984) Genetic and molecular characterization of the Pseudomonas plasmid pVS1. Plasmid 11, 206-220.
Iwata-Reuyl, D. Biosynthesis of the 7-deazaguanosine hypermodified nucleosides of transfer RNA. Bioorg. Chem. 31, 24-43 (2003).
Iyer, L.M., Burroughs, A.M. & Aravind, L. The prokaryotic antecedents of the ubiquitin-signaling system and the early evolution of ubiquitin-like β-grasp domains. Genome Biol. 7, R60 (2006).
Jenal, J. Malone, Annu. Rev. Genet. 40, 385 (2006).
Johnson, J.L., Indermaur, L.W. & Rajagopalan, K.V. Molybdenum cofactor biosynthesis in Escherichia coli. Requirement of the chlB gene product for the formation of molybdopterin guanine dinucleotide. J. Biol. Chem. 266, 12140-12145 (1991).
Johnson, J.L., Rajagopalan, K.V., Mukund, S. & Adams, M.W. Identification of molybdopterin as the organic component of the tungsten cofactor in four enzymes from hyperthermophilic Archaea. J. Biol. Chem. 268, 4848-4852 (1993).
Joseph, J. R. Fantino, M. L. Herbaud, F. Denizot, FEMS Microbiol. Lett. 205, 91 (2001).
Kanehisa, M., Goto, S., Hattori, M., Aoki-Kinoshita, K.F., Itoh, M., Kawashima, S., Katayama, T., Araki, M. and Hirakawa, M. (2006) From genomics to chemical genomics: new developments in KEGG. Nucleic Acids Res, 34, D354-357.
Kieser, T., Bibb, M.J., Buttner, M.J., Chater, K.F., and Hopwood, D.A. 2000. Practical Streptomyces Genetics. The John Innes Foundation, Norwich, UK.
Kim, B.-C., Qian, X., Leang, C., Coppi, M.V. and Lovley, D.R. (2006) Two Putative c-Type Multiheme Cytochromes Required for the Expression of OmcB, an Outer Membrane Protein Essential for Optimal Fe(III) Reduction in Geobacter sulfurreducens. J Bact, 188, 3138-3142.
Kim, D.J., Huh, J.H., Yang, Y.Y., Kang, C.M., Lee, I.H., Hyun, C.G., Hong, S.K., and Suh, J.W. 2003. Accumulation of S-adenosyl-L-methionine enhances production of actinorhodin but inhibits sporulation in Streptomyces lividans TK23. J. Bact. 185: 592-600.
Kim, J. N., Roth, A., and Breaker, R. R. 2007. Guanine riboswitch varients from Mesoplasma florum selectively recognize 2'-deoxyguanosine. Proc. Natl. Acad. Sci. USA 104: 16092-16097.
Kima, V.N. and Nam, J.-W. (2006) Genomics of microRNA. Trends Genet, 22, 165-173.
King, E.O., Ward, M.K., and Raney, D.E. (1954) Two simple media for the demonstration of pyocyanin and fluorescin. J. Lab. Clin. Med. 44, 301-307
Kirn, T.J., Jude, B.A. and Taylor, R.K. (2005) A colonization factor links Vibrio cholerae environmental survival and human infection. Nature, 438, 863-866.
Klein, R.J. and Eddy, S.R. (2003) RSEARCH: finding homologs of single structured RNA sequences. BMC Bioinformatics, 4, 44.
Kletzin, A. & Adams, M.W. Tungsten in biological systems. FEMS Microbiol. Rev. 18, 5-63 (1996).
Krasilnikov, A.S., Xiao, Y., Pan, T., and Mondragon, A. 2004. Basis for structural diversity in homologous RNAs. Science 306: 104-107.
Kubodera, T., Watanabe, M., Yoshiuchi, K., Yamashita, N., Nishimura, A., Nakai, S., Gomi, K., and Hanamoto, H. (2003) Thiamine-regulated gene expression of Aspergillus oryzae thiA requires splicing of the intron containing a riboswitch-like domain in the 5'-UTR. FEBS Lett. 555, 516-520.
Kuchino, Y., Kasai, H., Nihei, K., and Nishimura, S. 1976. Biosynthesis of the modified nucleoside Q in transfer RNA. Nucleic Acid Res. 3: 393-398.
Lake, M.W., Temple, C.A., Rajagopalan, K.V., & Schindelin, H. The crystal structure of the Escherichia coli MobA protein provides insight into molybdopterin guanine dinucleotide biosynthesis. J. Biol. Chem. 275, 40211-40217 (2000).
Lee, J. M. Matewish, J. L. Kessler, M. Hyodo, Y. Jayakawa, S. Lory, Mol. Microbiol. 65, 1474 (2007).
Lemay, J. F., Penedo, J. C., Tremblay, R., Lilley, D. M. & Lafontaine, D. A. Folding of the adenine riboswitch. Chem. Biol. 13, 857-868 (2006).
Lescoute, A., Leontis, N.B., Massire, C., and Westhof, E. 2005. Recurrent structural RNA motifs, isostericity matrices and sequence alignments. Nucleic Acids Res. 33: 2395-2409.
Li, Y. and Breaker, R. R. 1999. Kinetics of RNA degradation by specific base catalysis of transesterification involving the 2'-hydroxyl group. J. Am. Chem. Soc. 121: 5364-5372.
Lim, J., Grove, B.C., Roth, A., and Breaker, R.R. (2006a). Characteristics of ligand recognition by a glmS self-cleaving ribozyme. Angew. Chem. Int. Ed. Engl. 45, 6689-6693.
Lim, J., Winkler, W.C., Nakamura, S., Scott, V., and Breaker, R.R. (2006b). Molecular-recognition characteristics of SAM-binding riboswitches. Angewandte Chemie-International Edition 45, 964-968.
Lim, S. Beyhan, J. Meir, F. H. Yildiz, Mol. Microbiol. 60, 331 (2006c).
Liu, M.Y. et al. The RNA molecule CsrB binds to the global regulatory protein CsrA and antagonizes its activity in Escherichia coli. J. Biol. Chem. 272, 17502-17510 (1997).
Luisi, B., Orozco, M., Sponer, J., Luque, F. J., and Shakked, Z. 1998. On the potential role of the amino nitrogen atom as a hydrogen bond acceptor in macromolecules. J. Mol. Biol. 279: 1123-1136.
Makdessi, K., Andreesen, J. R. and Pich, A. (2001) Tungstate uptake by a highly specific ABC transporter in Eubacterium acidaminophilum. J. Biol. Chem. 276, 24557-24564.
Mandal, M. and Breaker, R. R. 2004a. Gene regulation by riboswitches. Nature Rev. Mol. Cell. Biol. 5: 451-463.
Mandal, M. and Breaker, R. R. 2004b. Adenine riboswitches and gene activation by disruption of a transcription terminator. Nat. Struct. Mol. Biol. 11: 29-35.
Mandal, M., Boese, B., Barrick, J.E., Winkler, W.C. & Breaker, R.R. Riboswitches control fundamental biochemical pathways in Bacillus subtilis and other bacteria. Cell 113, 577-586 (2003).
Mandal, M., Lee, M., Barrick, J.E., Weinberg, Z., Emilsson, G.M., Ruzzo, W.L. & Breaker, R.R. A glycine-dependent riboswitch that uses cooperative binding to control gene expression. Science 306, 275-279 (2004c).
Marchler-Bauer, A., Anderson, J.B., Cherukuri, P.F., DeWeese-Scott, C., Geer, L.Y., Gwadz, M., He, S., Hurwitz, D.I., Jackson, J.D., Ke, Z. et al. (2005) CDD: a Conserved Domain Database for protein classification. Nucleic Acids Research, 33, 192-196.
Maupin-Furlow, J.A. et al. Genetic analysis of the modABCD (molybdate transport) operon of Escherichia coli. J. Bacteriol. 177, 4851-4856 (1995).
McCutcheon, J.P. and Eddy, S.R. (2003) Computational identification of non-coding RNAs in Saccharomyces cerevisiae by comparative genomics. Nucleic Acids Research, 31, 4119-4128.
McDaniel, B. A., Grundy, F. J., Artsimovitch, I., and Henkin, T. M. 2003. Transcription termination control of the S box system: direct measurement of S-adenosylmethionine by the leader RNA. Proc. Natl. Acad. Sci. USA 100: 3083-3088.
Mehta, T., Coppi, M.V., Childers, S.E. and Lovley, D.R. (2005) Outer membrane c-type cytochromes required for Fe(III) and Mn(IV) oxide reduction in Geobacter sulfurreducens. Appl Environ Microbiol, 71, 8634-8641.
Meibom, K.L., Blokesch, M., Dolganov, N.A., Wu, C.-Y. and Schoolnik, G.K. (2005) Chitin Induces Natural Competence in Vibrio cholerae. Science, 310, 1824-1827.
Meibom, K.L., Li, X.B., Nielsen, A.T., Wu, C.-Y., Roseman, S. and Schoolnik, G.K. (2004) The Vibrio cholerae chitin utilization program. PNAS, 101, 2524-2529.
Meier, F., Suter, B., Grosjean, H., Keith, G., and Kubli, E. 1985. Queuosine modification of the wobble base in tRNA HIS influences 'in vivo' decoding properties. EMBO J. 4: 823-827.
Methé, B.A., Nelson, K.E., Eisen, J.A., Paulsen, I.T., Nelson, W., Heidelberg, J.F., Wu, D., Wu, M., Ward, N., Beanan, M.J. et al. (2003) Genome of Geobacter sulfurreducens: Metal Reduction in Subsurface Environments. Science, 302, 1967-1969.
Michel, F. and Westhof, E. 1990. Modelling of the three-dimensional architecture of group I catalytic introns based on comparative sequence analysis. J. Mol. Biol. 216: 585-610.
Migawa, M. T., Hinkley, J. M., Hoops, G. C. & Townsend, L. B. A two step synthesis of the nucleoside Q precursor 2-amino-5-cyanopyrrolo[2,3-d]pyrimidine-4-one (preQ0) Synth. Commun. 26, 3317-3322 (1996).
Miller, J. H. (1992). A Short Course in Bacterial Genetics 72 (New York: Cold Spring Harbor Laboratory Press).
Miller, R. L., Sabourin, C. L., Krenitsky, T. A., Berens, R. L. & Marr, J. J. Nucleoside hydrolases from Trypanosoma cruzi. J. Biol. Chem. 259, 5073-5077 (1984).
Mironov, A.S., Gusarov, I., Rafikov, R., Lopez, L.E., Shatalin, K., Kreneva, R.A., Perumov, D.A., and Nudler, E. (2002) Sensing small molecules by nascent RNA: a mechanism to control transcription in bacteria. Cell 111, 747-756.
Montange, R.K. and Batey, R.T. 2006. Structure of the S-adenosylmethionine riboswitch regulatory mRNA element. Nature 441: 1172-1175.
Murzin, A.G., Brenner, S.E., Hubbard, T., and Chothia, C. 1995. SCOP: a structural classification of proteins database for the investigation of sequences and structures. J. Mol. Biol. 247: 536-540.
Nahvi, A., Barrick, J.E. & Breaker, R.R. Coenzyme B12 riboswitches are widespread genetic control elements in prokaryotes. Nucleic Acids Res. 32, 143-150 (2004).
Nahvi, A., Sudarsan, N., Ebert, M.S., Zou, X., Brown, K.L. & Breaker, R.R. Genetic control by a metabolite bidning mRNA. Chem. Biol. 9, 1043-1049 (2002).
Nahvi, N. Sudarsan, M. S. Ebert, X. Zou, K. L. Brown, R. R. Breaker, Chem. Biol. 9, 1043 (2002).
Neph, S. and Tompa, M. (2006) MicroFootPrinter: a Tool for Phylogenetic Footprinting in Prokaryotic Genomes. Nucleic Acids Research, 34.
Nichols, J. & Rajagopalan, K.V. Escherichia coli MoeA and MogA. Function in metal incorporation step of molybdenum cofactor biosynthesis. J. Biol. Chem. 277, 24995-52000 (2002).
Nissen, P., Ippolito, J. A., Ban, N., Moore, P. B. & Steitz, T. A. RNA tertiary interactions in the large ribosomal subunit: the A-minor motif. Proc. Natl. Acad. Sci. USA 98, 4899-4903 (2001).
Noeske, J. et al. An intermolecular base triple as the basis of ligand specificity and affinity in the guanine- and adenine-sensing riboswitch RNAs. Proc. Natl. Acad. Sci. USA 102, 1372-1377 (2005).
Noeske, J., Richter, C., Grundle, M. A., Nasiri, H. R., Schwalbe, H., and Wohnert, J. 2005. An intermolecular base triple as the basis of ligand specificity and affinity in the guanine- and adenine-sensing riboswitch RNAs. Proc. Natl. Acad. Sci. USA 104: 1372-1377.
Noguchi, S., Nishimura, Y., Hirota, Y., and Nishumura, S. 1982. Isolation and characterization of an Escherichia coli mutant lacking tRNA-guanine transglycoslyase. J. Biol. Chem. 257: 6544-6550.
Nygård, O., Vollset, S.E., Refsum, H., Brattström, L., and Ueland, P.M. (1999) Total homocysteine and cardiovascular disease. J. Intern. Med. 246, 425-454.
Okada, N., Noguchi, S., Kasai, H., Shindo-Okada, N., Ohgi, T., Goto, T., and Nishimura, S. 1979. Novel mechanism of post-transcriptional modification of tRNA. J. Biol. Chem. 254: 3067-3073.
Okada, N., Noguchi, S., Nishimura, S., Ohgi, T., Goto, T., Crain, P. F., and McCloskey, J. A. 1978. Structure of a nucleoside Q precursor isolated from E. coli tRNA: 7-(aminomethyl)-7-deazaguanine. Nucleic Acids Res. 5: 2289-2296.
Okamoto, S., Lezhava, A., Hosaka, T., Okamoto-Hosoya, Y., and Ochi, K. 2003. Enhanced expression of S-adenosylmethionine synthetase causes overproduction of actinorhodin in Streptomyces coelicolor A3(2). J. Bact. 185: 601-609.
Old, I.G., Hunter, M.G., Wilson, D.T.R., Knight, S.M., Weatherston, C.A., and Glass, R. E. (1988). Cloning and characterization of the genes for the two homocysteine transmethylases of Escherichia coli. Mol. Gen. Genet. 211, 78-87.
Orengo, C.A. and Thornton, J.M. 2005. Protein families and their evolution-a structural perspective. Annu. Rev. Biochem. 74: 867-900.
Osborne, S.E., and Ellington, A.D. (1997). Nucleic acid selection and the challenge of combinatorial chemistry. Chem. Rev. 97, 349-370.
Pedersen, J.S., Bejerano, G., Siepel, A., Rosenbloom, K., Lindblad-Toh, K., Lander, E.S., Kent, J., Miller, W. and Haussler, D. (2006), PLoS Computational Biology.
Phadtare, S. & Inouye, M. Sequence-selective interactions with RNA by CspB, CspC and CspE, members of the CspA family of Escherichia coli. Mol. Microbiol. 33, 1004-1014 (1999).
Pitterle, D.M., Johnson, J.L. & Rajagopalan, K.V. In vitro synthesis of molybdopterin from precursor Z using purified converting factor. Role of protein-bound sulfur in formation of the dithiolene. J. Biol. Chem. 268, 13506-13509 (1993).
Posey, J.E., Pytlos, M.J., Sinden, R.R. and Roth, D.B. (2006) Target DNA Structure Plays a Critical Role in RAG Transposition. PLoS Biology, 4, 350.
Potamana, V.N., Shlyakhtenkob, L.S., Oussatchevaa, E.A., Lyubchenkob, Y.L. and Soldatenkov, V.A. (2005) Specific Binding of Poly(ADP-ribose) Polymerase-1 to Cruciform Hairpins. J Mol Biol, 348, 609-615.
Pruitt, K. D., Tatusova, T., and Maglott, D. R. 2007. NCBI Reference Sequence (RefSeq): a curated non-redundant sequence database of genomes, transcripts and proteins. Nucleic Acids Res. 35: D61-65.
Pruitt, K., Tatusova, T. and Maglott, D. (2005) NCBI Reference Sequence (RelSeq): a curated non-redundant sequence database of genomes, transcripts and proteins. Nucleic Acids Research, 33, 501-504.
Ptashne, M. & Gann, A. Genes and Signals (Cold Spring Harbor Laboratory Press, Cold Spring Harbor, New York, 2002).
Reader, J. S., Metzgar, D., Schimmel P., and de Crécy-Lagard, V. 2004. Identification of four genes necessary for biosynthesis of the modified nucleoside queuosine. J. Biol. Chem. 279: 6280-6285.
Reguera, G., McCarthy, K.D., Mehta, T., Nicoll, J.S., Tuominen, M.T. and Lovley, D.R. (2005) Extracellular electron transfer via microbial nanowires. Nature, 435, 1098-1101.
Regulski et al., Mol. Microbiol. 68, 918 (2008).
Rentmeister, A., Mayer, G., Kuhn, N., and Famulok, M. (2007) Conformational changes in the expression domain of the Escherichia coli thiM riboswitch. Nucleic Acids Res. (in press).
Reuter, K., Slany, R., Ullrich, F. & Kersten, H. Structure and organization of Escherichia coli genes involved in biosynthesis of the deazaguanine derivative queuine, a nutrient factor for eukaryotes. J. Bacteriol. 173, 2256-2264 (1991).
Rey, D.A., Nentwich, S.S., Koch, D.J., Ruckert, C., Puhler, A., Tauch, A., and Kalinowski, J. (2005) The McbR repressor modulated by the effector substance S-adenosylhomocysteine controls directly the transcription of a regulon involved in sulphur metabolism of Corynebacterium glutamicum ATCC 13032. Mol. Microbiol. 56, 871-887
Richardson, L.V. & Richardson, J.P. Rho-dependent termination of transcription is governed primarily by the upstream Rho utilization (rut) sequences of a terminator. J. Biol. Chem. 271, 21597-21603 (1996).
Rodionov, D. A., Vitreschak, A. G., Mironov, A. A., and Gelfand, M. S. 2002 Comparative genomics of thiamin biosynthesis in prokaryotes. J. Biol. Chem. 277: 48949-48959.
Rodionov, D. A., Vitreschak, A. G., Mironov, A. A., and Gelfand, M. S. 2003a. Comparative genomics of the vitamin B12 metabolism and regulation in prokaryotes. J. Biol. Chem. 278: 41148-41159.
Rodionov, D.A., Vitreschak, A.G., Mironov, A.A., and Gelfand, M.S. (2003b). Regulation of lysine biosynthesis and transport genes in bacteria: yet another riboswitch? Nucleic Acids Res. 31, 6748-6757.
Rodionov, D.A., Vitreschak, A.G., Mironov, A.A., and Gelfand, M.S. (2004). Comparative genomics of the methionine metabolism in Gram-positive bacteria: a variety of regulatory systems. Nucleic Acids Res. 32, 3340-3353.
Römling, M. Gomelsky, M. Y. Galperin, Mol. Microbiol. 57, 629 (2005).
Rosentel, J.K., Healy, F., Maupin-Furlow, J.A., Lee, J.H. & Shanmugam, K.T. Molybdate and regulation of mod (molybdate transport), fdhF, and hyc (formate hydrogenlyase) operons in Escherichia coli. J. Bacteriol. 177, 4857-4864 (1995).
Ross et al., FEBS Lett. 186, 191 (1985).
Ross et al., Nature 325, 279 (1987).
Roth, A., Nahvi, A., Lee, M., Jona, I. & Breaker, R.R. Characteristics of the glmS ribozyme suggest only structural roles for divalent metal ions. RNA 12, 607-619 (2006).
Roth, A., Winkler, W. C., Regulski, E. E., Lee B. W. K., Lim, J., Jona, I., Barrick, J. E., Ritwik, A., Kim, J. N., Welz, R. et al. 2007. A riboswitch selective for the queuosine precursor preQ1 contains an unusually small aptamer domain Nature Struct. Mol. Biol. 14: 308-317.
Rusch, A. L. Halpern, G. Sutton, K. B. Heidelberg, S. Williamson, S. Yooseph, D. Wu, J. A. Eisen, J. M. Hoffman, K. Remington, et al., PLoS Biol. 5, e77 (2007).
Ryan, Y. Fouhy, J. F. Lucey, J. Maxwell Dow, J. Bacteriol. 188, 8327 (2006).
Ryjenkov, R. Simm, U. Römling, M. Gomelsky, J. Biol. Chem. 281, 30310 (2006).
Salazar, J. C., Ambrogelly, A., Crain, P. F., McCloskey, J. A. & Soll, D. A truncated aminoacyl-tRNA synthetase modifies RNA. Proc. Natl. Acad. Sci. USA 101, 7536-7541 (2004).
Sambrook, J. & Russell, D.W. In: Molecular Cloning: A Laboratory Manual. 3rd ed., Cold Spring Harbor Laboratory Press, Cold Spring Harbor, NY (2001).
Sanishvili, R., Beasley, S., Skarina, T., Glesne, D., Joachimiak, A., Edwards, A. & Savchenko, A. The crystal structure of Escherichia coli MoaB suggests a probable role in molybdenum cofactor synthesis. J. Biol. Chem. 279, 42139-42146 (2004).
Santamaria-Araujo, J.A. et al. The tetrahydropyranopterin structure of the sulfur-free and metal-free molybdenum cofactor precursor. J. Biol. Chem. 279, 15994-159949
(2004).
Saran, D., Frank, J., and Burke, D.H. (2003) The tyranny of adenosine recognition among RNA aptamers to coenzyme A. BCM Evol. Biol. 3, 26.
Schaffer, A.A., Aravind, L., Madden, T.L., Shavirin, S., Spouge, J.L., Wolf, Y.I., Koonin, E.V. and Altschul, S.F. (2001) Improving the accuracy of PSI-BLAST protein database searches with composition-based statistics and other refinements. Nucleic Acids Res, 29, 2994-3005.
Schauder, S., Shokat, K., Surette, M.G., and Bassler, B.L. (2001). The LuxS family of bacterial autoinducers: biosynthesis of a novel quorum-sensing signal molecule. Molec. Microbiol. 41, 463-476.
Schwarz, G. Molybdenum cofactor biosynthesis and deficiency. Cell Mol. Life Sci. 62, 2792-2810 (2005).
Schyns, G., Potot, S., Geng, Y., Barbosa, T.M., Henriques, A., and Perkins, J. B. 2005. Isolation and characterization of new thiamine-deregulated mutants of Bacillus subtilis. J. Bacteriol 187: 8127-8136.
Scott, D. & Amy, N.K. Molybdenum accumulation in chlD mutants of Escherichia coli. J. Bacteriol. 171, 1284-1287 (1989).
Seetharaman, S., Zivarts, M., Sudarsan, N., and Breaker, R. R. 2001. Immobilized RNA switches for the analysis of complex chemical and biological mixtures. Nat. Biotechnol. 19: 336-341.
Seliverstov, A.V., Putzer, H., Gelfand, M.S. and Lyubetsky, V.A. (2005) Comparative analysis of RNA regulatory elements of amino acid metabolism genes in Actinobacteria. BMC Microbiol, 5, 54.
Serganov, A., Polonskaia, A., Phan, A.T., Breaker, R.R., and Patel, D.J. (2006). Structural basis for gene regulation by a thiamine pyrophosphate-sensing riboswitch. Nature 441, 1167-1171.
Serganov, A., Yuan, Y.R., Pikovskaya, O., Polonskaia, A., Malinina, L., Phan, A. T., Hobartner, C., Micura, R., Breaker, R. R., and Patel, D. J. 2004. Structural basis for discriminative regulation of gene expression by adenine- and guanine-sensing mRNAs. Chem. Biol. 11: 1729-1741.
Shanmugam, K.T. et al. Proposed nomenclature for the genes involved in molybdenum metabolism in Escherichia coli and Salmonella typhimurium. Mol. Microbiol. 6, 3452-3454 (1992).
Sheppard, C.A., Trimmer, E.E., and Matthews, R.G. (1999). Purification and properties of NADH-dependent 5,10-methylenetetrahydrofolate reductase (MetF) from Escherichia coli. J. Bact. 181, 718-725.
Shimizu, S., Shiozaki, S., Ohshiro, T., and Yamada, H. (1984). Occurrence of S-adenosylhomocysteine hydrolase in prokaryote cells - characterization of the enzyme from Alcaligenes faecalis and role of the enzyme in the activated methyl cycle. Eur. J. Biochem. 141, 385-392.
Simm, M. Morr, A. Kader, M. Nimtz, U. Römling, Mol. Microbiol. 53, 1123 (2004).
Simons, R.W., Houman, F. & Kleckner, N. Improved single and multicopy lac-based cloning vectors for protein and operon fusions. Gene 53, 85-96 (1987).
Slany, R. K., Bosl, M. & Kersten, H. Transfer and isomerization of the ribose moiety of AdoMet during the biosynthesis of queuosine tRNAs, a new unique reaction catalyzed by the QueA protein from Escherichia coli. Biochimie 76, 389-393 (1994).
Slany, R., Bosl, M., Crain, R. F., and Kersten, H. 1993. A new function of S-adenosylmethionine: The ribosyl moiety of AdoMet is the precursor of the cyclopentenediol moiety of the tRNA wobble base queuine. Biochemistry 32: 7811-7817.
Soukup, G. A. and Breaker, R. R. 1999. Relationship between internucleotide linkage geometry and the stability of RNA. RNA 5: 1308-1325.
Soukup, J. K. and Soukup, G. A. 2004. Riboswitches exert genetic control through metabolite-induced conformational change. Curr. Opin. Struct. Biol. 14: 344-349.
Spiro, S. & Guest, J.R. FNR and its role in oxygen-related gene expression in Escherichia coli. FEMS Microbiol. Rev. 6, 399-428 (1990).
Stoddard, C.D. & Batey, R.T. Mix-and-match riboswitches. ACS Chem. Biol. 1, 751-754 (2006).
Storz, G., Altuvia, S. and Wassarman, K.M. (2005) An abundance of RNA regulators. Annu Rev Biochem, 74, 199-217.
Sudarsan, J. K. Wickiser, S. Nakamura, M. S. Ebert, R. R. Breaker, Genes Dev. 17, 2688 (2003).
Sudarsan, N., Barrick, J.E., and Breaker, R.R. (2003a). Metabolite-binding RNA domains are present in the genes of eukaryotes. RNA 9, 644-647.
Sudarsan, N., Hammond, M.C., Block, K.F., Welz, R., Barrick, J.E., Roth, A. & Breaker, R.R. Tandem riboswitch architectures exhibit complex gene control functions. Science 314, 300-304 (2006).
Sudarsan, N., Wickiser, J.K., Nakamura, S., Ebert, M.S., and Breaker, R.R. (2003b). An mRNA structure in bacteria that controls gene expression by binding lysine. Genes Dev. 17, 2688-2697.
Tabor, C.W., and Tabor, H. (1984). Methionine adenosyltransferase (S-adenosylmethionine synthetase) and S-adenosylmethionine decarboxylase. Advances Enzymol. Related Areas Mol. Biol. 56, 251-282.
Takusagawa, F., Fujioka, M., Spies, A., and Schowen, R.L. (1998). S-adenosylmethionine (AdoMet)-dependent methyltransferases. In: Comprehensive Biological Catalysis, M. Sinnott, ed., Academic Press, San Diego, CA, pp1-30.
Tamayo, A. D. Tischler, A. Camilli, J. Biol. Chem. 280, 33324 (2005).
Tamayo, J. T. Pratt, A. Camilli, Annu. Rev. Microbiol. 61, 131 (2007).
Tang, Y. & Guest, J.R. Direct evidence for mRNA binding and post-transcriptional regulation by Escherichia coli aconitases. Microbiology 145, 3069-3079 (1999).
Tatusov, R. L. et al. The COG database: an updated version includes eukaryotes. BMC Bioinformatics 4, 41 (2003).
Tatusov, R. L. et al. The COG database: new developments in phylogenetic classification of proteins from complete genomes. Nucleic Acids Res. 29, 22-28 (2001).
Thompson, M.J., Mekhalfia, A., D.P., H., and Blackburn, G.M. 1999. Synthesis of two stable nitrogen analogues of S-adenosyl-L-methionine. J. Org. Chem. 64: 7467-7473.
Thore, S., Leibundgut, M., and Ban, N.N. (2006). Structure of the eukaryotic thiamine pyrophosphate riboswitch with its regulatory ligand. Science 312, 1208-1211.
Tischler, A. Camilli, Infect. Immun. 73, 5873 (2005).
Torarinsson, E., Sawera, M., Havgaard, J.H., Fredholm, M. and Gorodkin, J. (2006) Thousands of corresponding human and mouse genomic regions unalignable in primary sequence contain common RNA structure. Genome Res, 16, 885-889.
Torres-Larios, A. et al. Structural basis of translational control by Escherichia coli threonyl tRNA synthetase. Nat. Struct. Biol. 9, 343-347 (2002).
Torres-Larios, A., Swinger, K.K., Pan, T., and Mondragon, A. 2006. Structure of ribonuclease P-a universal ribozyme. Curr. Opin. Struct. Biol. 16: 327-335.
Tringe, C. von Mering, A. Kobayashi, A. A. Salamov, K. Chen, H. W. Chang, M. Podar, J. M. Short, E. J. Mathur, J. C. Detter, et al., Science 308, 554 (2005).
Tucker, B. J. and Breaker, R. R. 2005. Riboswitches as versatile gene control elements. Curr. Opin. Struct. Biol. 15: 342-348.
Turnbaugh, R. E.Ley, M. A.Mahowald, V. Magrini, E. R. Mardis, J. I. Gordon, Nature 444, 1027 (2006).
Tyson, G.W., Chapman, J., Hugenholtz, P., Allen, E.E., Ram, R.J., Richardson, P.M., Solovyev, V.V., Rubin, E.M., Rokhsar, D.S. and Banfield, J.F. (2004) Community structure and metabolism through reconstruction of microbial genomes from the environment. Nature, 428, 37-43.
Ueland, P.M. (1982). Pharmacological and biochemical aspects of S-adenosylhomocysteine and S-adenosylhomocysteine hydrolase. Pharmacological Reviews 34, 223-285.
Ulrich, E. V. Koonin, I. B. Zhulin, Trends Microbiol. 13, 52 (2005).
Urbonavi 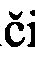 ius, J., Qian, Q., Durand J. M. B., Hagervall, T. G., and Bjork, G. R. 2001. Improvement of reading frame maintenance is a common function for several rRNA modification. *EMBO J.* **20:** 4863-4873.
Urbonavi 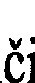 ius, J., Qian, Q., Durand, J. M., Hagervall, T. G. & Björk, G. R. Improvement of reading frame maintenance is a common function for several tRNA modifications. EMBO J. 20, 4863-4873 (2001).
Van Lanen, S. G., Reader, J. S., Swairjo, M. A., de Crécy-Lagard, V., Lee, B., and Iwata-Reuyl, D. 2005. From cyclohydrolase to oxidoreductase: Discovery of nitrile reductase activity in a common fold. Proc. Natl. Acad. Sci. USA 102: 4264-4269.
Vecerek, B., Moll, I. & Blasi, U. Translational autocontrol of the Escherichia coli hfq RNA chaperone gene. RNA 11, 976-984 (2005).
Venter, J.C., Remington, K., Heidelberg, J.F., Halpern, A.L., Rusch, D., Eisen, J.A., Wu, D., Paulsen, I., Nelson, K.E., Nelson, W. et al. (2004) Environmental Genome Shotgun Sequencing of the Sargasso Sea. Science, 304, 66-74.
Vicens, Q. and Cech, T.R. 2006. Atomic level architecture of group I introns revealed. Trends Biochem. Sci. 31: 41-51.
Wachter, A., Tunc-Ozdemir, M., Grove, B.C., Green, P.J., Shintani, D.K., and Breaker, R.R. (2007) Riboswitch control of gene expression in plants by alternative 3' end processing of mRNAs. The Plant Cell (in press).
Wan, X.-F. and Xu, D. (2004) Intrinsic Terminator Prediction and Its Application in Synechococcus sp. WH8102. J Comp Sci & Tech, 20, 465-482.
Wang, J. X., Lee, E. R., Rivera, D., Lim, J., and Breaker, R. R. Riboswitches that sense S-adenosylhomocysteine and activate genes involved in coenzyme recycling. 2008. Mol. Cell (in press).
Washietl, S., Hofacker, I.L., Lukasser, M., Huttenhofer, A. and Stadler, P.F. (2005) Mapping of conserved RNA secondary structures predicts thousands of functional noncoding RNAs in the human genome. Nature Biotechnology, 23, 1383-1390.
Wassman et al., Structure 15, 915 (2007).
Waters, W. Lu, J. D. Rabinowitz, B. L. Bassler, J. Bacteriol. 190, 2527 (2008).
Weinberg, J. E. Barrick, Z. Yao, A. Roth, J. N. Kim, J. Gore, J. X. Wang, E. R. Lee, K. F. Block, N. Sudarsan, et al., Nucleic Acids Res. 35, 4809 (2007).
Weinberg, Z. and Ruzzo, W.L. (2004) Exploiting conserved structure for faster annotation of non-coding RNAs without loss of accuracy. Bioinformatics, 20, i334-i341.
Weinberg, Z. and Ruzzo, W.L. (2004), RECOMB04: Proceedings of the Eighth Annual International Conference on Computational Molecular Biology, San Diego, CA, pp. 243-251.
Weinberg, Z. and Ruzzo, W.L. (2006) Sequence-based heuristics for faster annotation of non-coding RNA families. Bioinformatics, 22, 35-39.
Weinberg, Z., Barrick, J. E., Yao, Z., Roth, A., Kim, J. N., Gore, J., Wang, J. X., Lee, E. R., Block, K. F., Sudarsan N., et al. 2007. Identification of 22 candidate structured RNAs in bacteria using the CMfinder comparative genomics pipeline. Nucleic Acids Res. 35: 4809-4819.
Weinberg, Z., Barrick, J. E., Yao, Z., Roth, A., Kim, J.N., Gore, J., Wang, J.X., Lee, E.R., Block, K.F., Sudarsan, N., et al. (2007). Identification of 22 candidate structured RNAs in bacteria using the CMfinder comparative genomics pipeline. Nucleic Acids Res. (doi:10.1093).
Welz, R. & Breaker, R.R. Ligand binding and gene control characteristics of tandem riboswitches in Bacillus anthracis. RNA 13, 573-582 (2007).
West, R. A. 4-Hydroxypyrrolo[2,3-d]pyrimidine: Mannich reaction. J. Org. Chem. 26, 4959-4961 (1961).
Wickiser, J. K., Cheah, M. T., Breaker, R. R. & Crothers, D. M. The kinetics of ligand binding by an adenine-sensing riboswitch. Biochemistry 44, 13404-13414 (2005).
Wickiser, J. K., Winkler, W. C., Breaker, R. R. & Crothers, D. M. The speed of RNA transcription and metabolite binding kinetics operate an FMN riboswitch. Mol. Cell 18, 49-60 (2005).
Wickiser, J.K., Cheah, M.T., Breaker, R.R., and Crothers, D.M. (2005b) The kinetics of ligand binding by an adenine-sensing riboswitch. Biochemistry 44, 13404-13414.
Wickiser, J.K., Winkler, W.C., Breaker, R.R., and Crothers, D.M. (2005a) The speed of RNA transcription and metabolite binding kinetics operate an FMN riboswitch. Mol. Cell 18, 49-60.
Winkler, W. C. and Breaker, R. R. 2005. Regulation of bacterial gene expression by riboswitches. Annu. Rev. Microbiol. 59: 487-517.
Winkler, W. C. Nahvi, A., and Breaker, R. R. 2002a. Thiamine derivatives bind messenger RNAs directly to regulate bacterial gene expression. Nature 419: 952-956.
Winkler, W. C. Riboswitches and the role of noncoding RNAs in bacterial metabolic control. Curr. Opin. Chem. Biol. 9, 594-602 (2005).
Winkler, W. C., Nahvi, A., Sudarsan, N., Barrick, J. E., and Breaker, R. R. 2003. An mRNA structure that controls gene expression by binding S-adenosylmethionine. Nat. Struct. Biol. 10: 701-707.
Winkler, W.C., Cohen-Chalamish, S. & Breaker, R.R. An mRNA structure that controls gene expression by binding FMN. Proc. Natl. Acad. Sci. U S A 99, 15908-15913 (2002b).
Winkler, W.C., Nahvi, A., Roth, A., Collins, J.A. & Breaker, R.R. Control of gene expression by a natural metabolite-responsive ribozyme. Nature 428, 281-286 (2004).
Wolfe, K. L. Visick, J. Bacteriol. 190, 463 (2008).
Woyke, H. Teeling, N. N. Ivanova, M. Huntemann, M. Richter, F. O. Gloeckner, D. Boffelli, I. J. Anderson, K. W. Barry, H. J. Shapiro, et al., Nature 443, 950 (2006).
Wuebbens, M.M. & Rajagopalan, K.V. Investigation of the early steps of molybdopterin biosynthesis in Escherichia coli through the use of in vivo labeling studies. J. Biol. Chem. 270, 1082-1087 (1995).
Yamamoto, K. & Ishihama, A. Transcriptional response of Escherichia coli to external copper. Mol. Microbiol. 56, 215-227 (2005).
Yao, Z., Barrick, J.E., Weinberg, Z., Neph, S., Breaker, R.R., Tompa, M., and Ruzzo, W.L. (2007). A computational pipeline for high throughput discovery of cis-regulatory noncoding RNA in prokaryotes. PLoS Comput. Biol. 3, e126.
Yao, Z., Weinberg, Z. and Ruzzo, W.L. (2006) CMfinder-a covariance model based RNA motif finding algorithm. Bioinformatics, 22, 445-452.
Yarnell, W.S. & Roberts, J.W. Mechanism of intrinsic transcription termination and antitermination. Science 284, 611-615 (1999).
Zengel, J.M. and Lindahl, L. (1994) Diverse mechanisms for regulating ribosomal protein synthesis in Escherichia coli. Prog Nucleic Acid Res Mol Biol, 47, 331-370.
Zhang, S. Kim, B. L. Gaffney, R. A. Jones, J. Am. Chem. Soc. 128, 7015 (2006).
Zuker, M. (2003) Mfold web server for nucleic acid folding and hybridization prediction. Nucleic Acids Res, 31, 3406-3415.

## Claims

1. A regulatable gene expression construct comprising a nucleic acid molecule encoding an RNA comprising a riboswitch operably linked to a coding region, wherein the riboswitch regulates expression of the RNA, wherein the riboswitch and coding region are heterologous, wherein the riboswitch is an SAM-responsive riboswitch, wherein the riboswitch comprises a SAM-IV motif, wherein the riboswitch preferably comprises an aptamer domain and an expression platform domain, wherein the aptamer domain and the expression platform domain are heterologous, wherein the riboswitch further preferably comprises two or more aptamer domains and an expression platform domain, wherein at least one of the aptamer domains and the expression platform domain are heterologous.

2. The construct of claim 1, wherein at least two of the aptamer domains exhibit cooperative binding, and/or wherein the riboswitch has the SAM-IV consensus structure of Figure 28.

3. The construct of claim 1, wherein the riboswitch comprises an aptamer domain and an expression platform domain wherein the aptamer domain is derived from a naturally occurring SAM-responsive riboswitch, wherein the aptamer domain is preferably the aptamer domain of a naturally-occurring SAM-responsive riboswitch, wherein the aptamer domain has more preferably the consensus structure of an aptamer domain of the naturally-occurring riboswitch, wherein the aptamer domain most preferably consists of only base pair conservative changes of the naturally-occurring riboswitch.

4. A riboswitch, wherein the riboswitch is a non-natural derivative of a naturally occurring riboswitch, wherein the riboswitch is an SAM-responsive riboswitch, wherein the riboswitch comprises a SAM-IV motif, wherein the riboswitch preferably comprises an aptamer domain and an expression platform domain, wherein the aptamer domain and the expression platform domain are heterologous, wherein the riboswitch is most preferably activated by a trigger molecule, wherein the riboswitch produces a signal when activated by the trigger molecule.

5. A method of detecting a compound of interest, the method comprising bringing into contact a sample and a riboswitch, wherein the riboswitch is activated by the compound of interest, wherein the riboswitch produces a signal when activated by the compound of interest, wherein the riboswitch produces a signal when the sample contains the compound of interest, wherein the riboswitch is a SAM-responsive riboswitch or a derivative of a SAM-responsive riboswitch, wherein the SAM-responsive riboswitch comprises a SAM-IV motif, wherein the riboswitch preferably changes conformation when activated by the compound of interest, wherein the change in conformation produces a signal via a conformation dependent label, and/or wherein the riboswitch preferably changes conformation when activated by the compound of interest, wherein the change in conformation causes a change in expression of an RNA linked to the riboswitch, wherein the change in expression produces a signal, wherein the signal is more preferably produced by a reporter protein expressed from the RNA linked to the riboswitch.

6. A method comprising
(a) testing a compound for altering gene expression of a gene encoding an RNA comprising a riboswitch, wherein the alteration is via the riboswitch, wherein the riboswitch is a SAM-responsive riboswitch or a derivative of a SAM-responsive riboswitch, wherein the SAM-responsive riboswitch comprises a SAM-IV motif,
(b) altering gene expression by bringing into contact a cell and a compound that altered gene expression in step (a), wherein the cell comprises a gene encoding an RNA comprising a riboswitch,
wherein the compound inhibits expression of the gene by binding to the riboswitch.

7. A method of identifying riboswitches, the method comprising assessing in-line spontaneous cleavage of an RNA molecule in the presence and absence of a compound, wherein the RNA molecule is encoded by a gene regulated by the compound, wherein a change in the pattern of in-line spontaneous cleavage of the RNA molecule indicates a riboswitch, wherein the RNA comprises a SAM-responsive riboswitch or a derivative of a SAM-responsive riboswitch, wherein the SAM-responsive riboswitch comprises a SAM-IV motif.

8. An *in vitro* method of altering gene expression, the method comprising bringing into contact a compound and a cell, wherein the cell comprises a gene encoding an RNA comprising a SAM-responsive riboswitch, wherein the riboswitch comprises a SAM-IV motif, wherein the compound alters expression of the gene by binding to the SAM-responsive riboswitch.

9. The method of any of claim 8, wherein the cell has been identified as being in need of altered gene expression.

10. The method of claim 8, wherein the cell is a bacterial cell.

11. The method of claim 10, wherein the compound kills or inhibits the growth of the bacterial cell.

12. The method of claim 8, wherein the compound inhibits bacterial growth in a biofilm.

13. Use of a regulatable gene expression construct of any of claims 1-3 for detection of a compound of interest.

14. Use of a riboswitch of claim 4 for the detection of a compound of interest.

15. Use of a riboswitch of claim 4 for altering gene expression *in vitro.*
